# FASCICULE DE BREVET EUROPEEN

(11) **EP 0 729 933 B1**
(45) Date de publication et mention de la délivrance du brevet: **26.01.2000**
(21) Numéro de dépôt: 96400417.0
(22) Date de dépôt: 28.02.1996
(51) Int. Cl.: C07C 49/755, C07C 309/73, C07D 209/48, C07C 323/40

(54) **Dérivés tricycliques, leur préparation et leur application à la préparation de la colchicine et de la thiocolchicine optiquement actives ou racémiques et d'analogues ou dérivés et intermédiaires**
Trizyklische Derivate, ihre Herstellung, ihre Verwendung zur Herstellung von optisch aktivem oder racemischem Colchizin, Thiocolchizin, dessen Analoge oder Derivate, und Intermediate
Tricyclic derivatives, their preparation, their use in the preparation of optically active or racemic colchicine, thiocolchicine and analogues or derivatives, and intermediates

(30) Priorité: 03.03.1995 FR 9502480
(43) Date de publication de la demande: 04.09.1996
(73) Titulaire: HOECHST MARION ROUSSEL, 92800 Puteaux (FR)
(72) Inventeur: Brion, Francis, F-93220 Gagny (FR); Chappert, Bernadette, F-75020 Paris (FR); Diolez, Christian, F-91120 Palaiseau (FR); Marie, Christian, F-93130 Noisy le Sec (FR); Mazurie, Alain, F-93410 Vaujours (FR); Middendorp, Michel, F-77120 Faremoutiers (FR); Pronine, Didier, F-93110 Rosny sous Bois (FR); Toromanoff, Edmond, F-75020 Paris (FR)
(74) Mandataire: Vieillefosse, Jean-Claude

(56) Documents cités:
- Aucun document pertinent relevé

## Description

L'invention a pour objet de nouveaux dérivés tricycliques, leur préparation, leur application à la préparation de la colchicine et de la thiocolchicine optiquement actives ou racémiques et d'analogues ou dérivés et des intermédiaires.

L'invention a ainsi pour objet les composés de formule (I) : 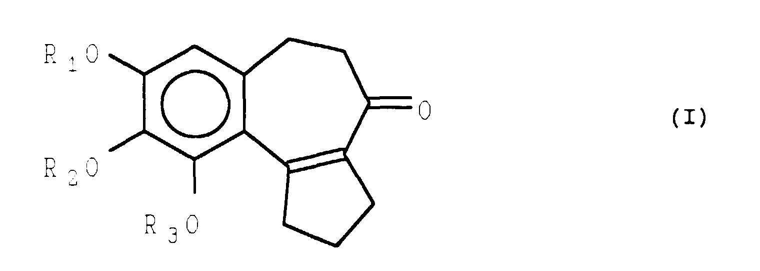 dans laquelle :
soit (a) R₁ et R₂ représentent tous les deux un groupement alkyle et R₃ représente un atome d'hydrogène ou le groupement A-SO₂-,
soit (b) R₂ et R₃ représentent tous les deux un atome d'hydrogène ou tous les deux un groupement alkyle et R₁ représente un groupement A-SO₂-,
soit (c) R₁, R₂ et R₃ représentent tous les trois un atome d'hydrogène ou tous les trois un groupement alkyle,
soit (d) R₁ représente le groupement A-SO₂- ou un atome d'hydrogène et R₂ et R₃ forment ensemble avec les atomes d'oxygène auxquels ils sont liés un groupement de formule -O-X-O- dans laquelle X représente un groupement de formule -B(OR₄)-, où R₄ représente un atome d'hydrogène, un groupement alkyle, un groupement -C(O)-, ou un groupement de formule -CR₅R₆-, où R₅ et R₆ représentent un atome d'hydrogène, un groupement alkyle ou phényle éventuellement substitué par 1 à 3 groupements choisis parmi les radicaux alkyle, hydroxy et alkyloxy, ou R₅ et R₆, ensemble avec l'atome de carbone auquel ils sont liés, représentent un groupement carbocyclique à 5 ou 6 chaînons,
soit (e) R₃ représente le groupement A-SO₂- et R₁ et R₂ forment ensemble avec les atomes d'oxygène auxquels ils sont liés, le groupement -O-X-O-,
soit (f) R₁ représente un atome d'hydrogène et R₂ et R₃ représentent tous les deux, un groupement alkyle ou forment ensemble avec les atomes d'oxygène auxquels il sont liés un groupement de formule -O-X-O- tel que défini ci-dessus,
"A" représente un groupement alkyle, un groupement phényle non substitué ou substitué par 1 à 3 groupements alkyles ou un groupement naphtyle non substitué ou substitué par 1 à 5 groupements alkyles, les groupements alkyle ou alkyloxy étant ramifiés ou non ramifiés, et renfermant de 1 à 6 atomes de carbone.

Dans la formule (I) et dans ce qui suit, par alkyle on entend un radical linéaire ou ramifié, renfermant de 1 à 6 atomes de carbone, c'est-à-dire un radical méthyle, éthyle, propyle, butyle, pentyle ou hexyle, linéaire ou ramifié.

Lorsque R₁ et/ou R₂ et/ou R₃ représentent un radical alkyle, ceux-ci peuvent être identiques ou différents. Il en est de même pour R₅ et R₆.

Par alkyloxy, on entend un radical linéaire ou ramifié, renfermant de 1 à 6 atomes de carbone, c'est-à-dire l'un des radicaux découlant des radicaux alkyle mentionnés plus haut.

L'invention a notamment pour objet :
- les composés de formule (I) telle que définie plus haut, caractérisés en ce que R₁, R₂ et R₃ représentent tous les trois un atome d'hydrogène, ou tous les trois un groupement alkyle et plus particulièrement un composé caractérisé en ce que R₁, R₂ et R₃ représentent tous les trois un radical méthyle, ou tous les trois un atome d'hydrogène ;
- les composés de formule (I) telle que définie plus haut, caractérisés en ce que R₁ représente un groupement A-SO₂-, tel que défini plus haut, et R₂ et R₃ représentent tous les deux un groupement alkyle ou tous les deux un atome d'hydrogène et plus particulièrement un composé caractérisé en ce que R₁ représente un groupement tosyle et R₂ et R₃ représentent tous les deux un radical méthyle ou tous les deux un atome d'hydrogène ;
- les composés de formule (I) telle que définie plus haut, caractérisés en ce que R₃ représente un groupement A-SO₂- tel que défini plus haut et R₁ et R₂ représentent tous les deux un radical alkyle et plus particulièrement, un composé caractérisé en ce que R₃ représente un groupe tosyle et R₁ et R₂ représentent tous les deux un radical méthyle ;
- les composés de formule (I) telle que définie plus haut, caractérisés en ce que R₁ et R₂ représentent tous les deux un groupement alkyle et R₃ représente un atome d'hydrogène et plus particulièrement un composé caractérisé en ce que R₁ et R₂ représentent un radical méthyle et R₃ représente un atome d'hydrogène ;
- les composés de formule (I) telle que définie plus haut, caractérisés en ce que R₁ représente un atome d'hydrogène et R₂ et R₃ représentent tous les deux un radical méthyle.

L'invention a encore pour objet un procédé de préparation des composés de formule (I) telle que définie plus haut, caractérisé en ce que
(i) l'on soumet un composé de formule (a) : 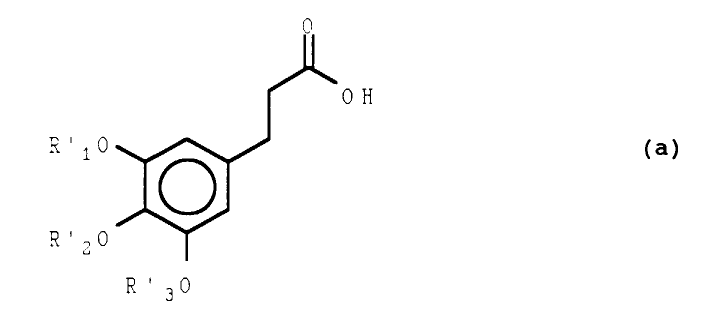 dans laquelle
   R'₁, R'₂ et R'₃ ont respectivement la même signification que les groupements R₁, R₂ et R₃ tels que définis à la revendication 1, à l'exception de la valeur hydrogène et pour R'₃ de la valeur A-SO₂-,
   à l'action d'un agent d'halogénation, pour obtenir l'halogénure d'acyle correspondant,
(ii) que l'on soumet à l'action d'un réactif de formule (b) : 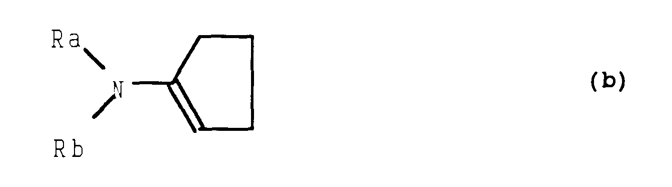 dans laquelle Ra et Rb identiques ou différents, représentent un groupement alkyle, ou Ra et Rb ensemble avec l'atome d'azote auquel ils sont liés, représentent un hétérocycle à 5 ou 6 chaînons comprenant éventuellement un autre hétéroatome choisi parmi O et N ;
   pour obtenir un composé de formule (c) 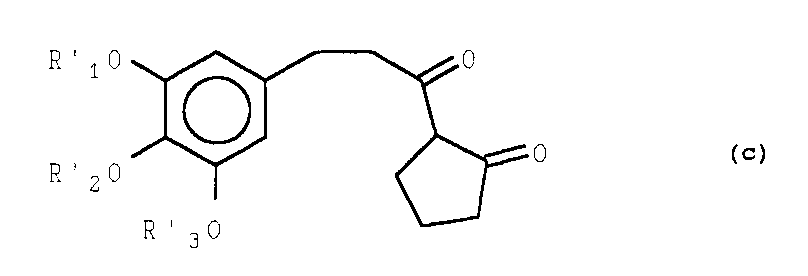
(iii) que l'on soumet à l'action d'un agent d'halogénation pour obtenir un composé de formule (d): 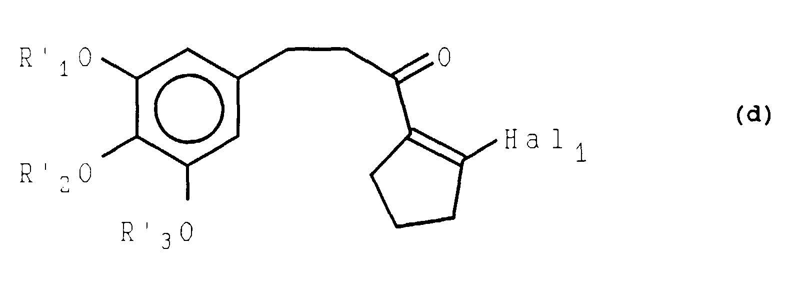 dans laquelle Hal₁ représente un atome d'halogène ;
(iv) que l'on soumet à l'action d'un acide de Lewis, pour obtenir un composé de formule (e) : 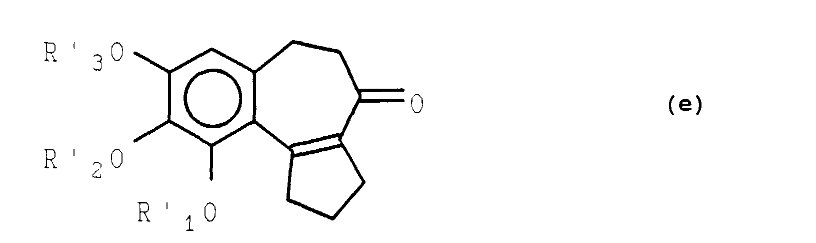 correspondant aux composés de formule (I) (a) telle que définie plus haut, dans laquelle R₃ représente un groupement A-SO₂-, aux composés de formule (I) (c) telle que définie plus haut, dans laquelle R₁, R₂ et R₃ représentent tous les trois un radical alkyle, et aux composés de formule (I) (e), telle que définie plus haut et composé de formule (e), que, le cas échéant,
(v) -soit si R'₁ représente un groupement A-SO₂- et R'₂ et R'₃ ne représentent pas ensemble le groupement -X-, l'on soumet à l'action d'un agent d'hydrolyse pour obtenir un composé de formule (f) : 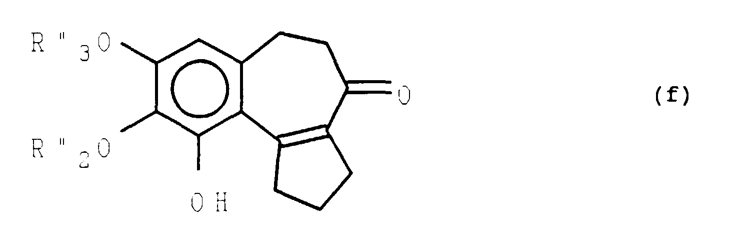 dans laquelle R"₂ et R"₃ représentent tous les deux un radical alkyle ; correspondant aux composés de formule (I) (a) telle que définie plus haut dans laquelle R₃ représente un atome d'hydrogène ;
   que, le cas échéant,
(vi) l'on soumet à l'action d'un agent d'alkylation, pour obtenir un composé de la formule (g) : 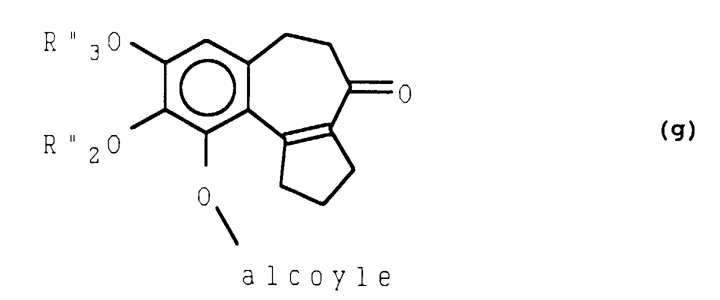 correspondant aux composés de formule (I) (c) telle que définie plus haut, dans laquelle R₁, R₂ et R₃ représentent un radical alkyle, que le cas échéant,
(vii) l'on soumet à une hydrolyse de l'ensemble des groupements alkyloxy pour obtenir le composé de formule (h) : 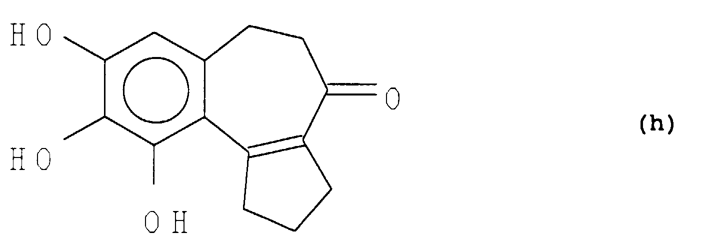 correspondant au composé de formule (I) (c) telle que définie plus haut, dans laquelle R₁, R₂ et R₃ représentent un atome d'hydrogène,
(viii) soit soumet un composé de la formule (e) dans laquelle R'₁, R'₂ et R'₃ représentent tous les trois un radical alkyle, à une hydrolyse de l'ensemble des groupements alkyloxy, pour obtenir le composé de formule (h) définie ci-dessus,
(ix) que, le cas échéant, l'on traite par un réactif de protection des diols choisi parmi les composés de formules :

   B(OR₄)₃, C(O)R₅R₆, C(O)Y₂ et CR₅R₆(Y)₂,

   dans lesquelles R₄, R₅ et R₆ sont tels que définis plus haut et Y représente un atome d'halogène ou un groupement de formule Ar-O-, dans laquelle Ar représente un groupement phényle éventuellement substitué par 1 à 3 substituants choisi parmi les radicaux alkyle, alkyloxy, hydroxy, amino, alkylamino, dialkylamino et nitro ;
   ou bien par un réactif de formule CH₂(Y)₂, Y étant défini comme plus haut, en présence d'un réactif de formule P(Hal)₅, dans laquelle Hal représente un atome d'halogène, suivi d'une hydrolyse, pour obtenir un composé de formule (i) : 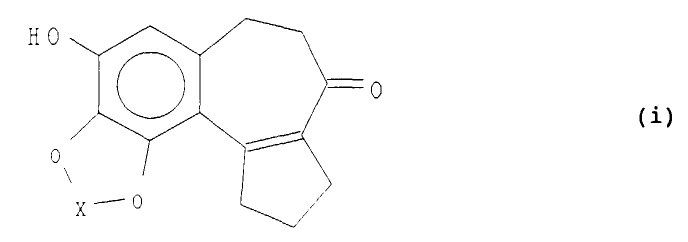 correspondant aux composés de formule (I) (d) telle que définie plus haut, dans laquelle R₁ représente un atome d'hydrogène,
   que l'on traite par un agent de formule A-SO₂Y, dans laquellle A et Y sont définis comme plus haut, pour obtenir un composé de formule (j) : 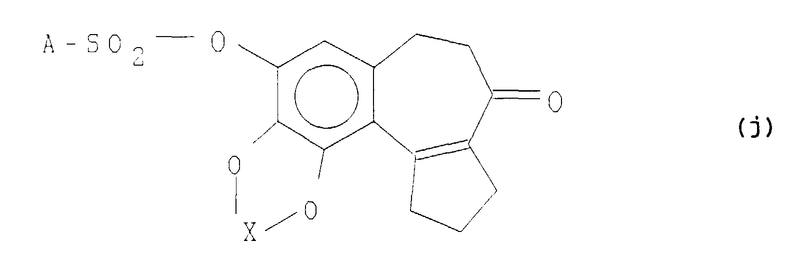 correspondant aux composés de formule (I) (d) telle que définie plus haut, dans laquelle R₁ représente le groupement A-SO₂-, que, le cas échéant,
(x) l'on soumet à l'action d'un réactif de déprotection du diol, pour obtenir un composé de formule (k) : 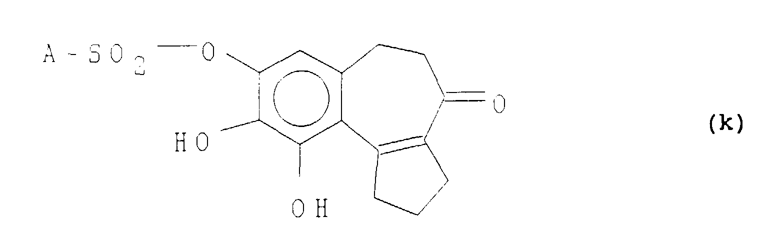 correspondant aux composés de formule (I) (b) telle que définie plus haut, dans laquelle R₂ et R₃ représentent un atome d'hydrogène, que, le cas échéant,
(xi) l'on soumet à l'action d'un agent d'alkylation pour obtenir un composé de formule (l) : 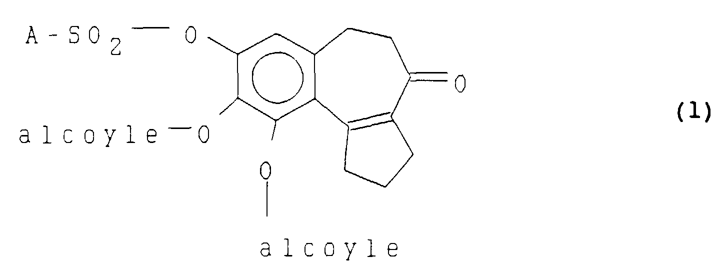 correspondant aux composés de formule (I) (b) telle que définie plus haut, dans laquelle R₂ et R₃ représentent un radical alkyle,
(xii) ou bien soumet le cas échéant un composé de formule (j) ou (l) à l'action d'un agent d'hydrolyse du radical A-SO₂-O- pour obtenir respectivement un composé de formule (i) 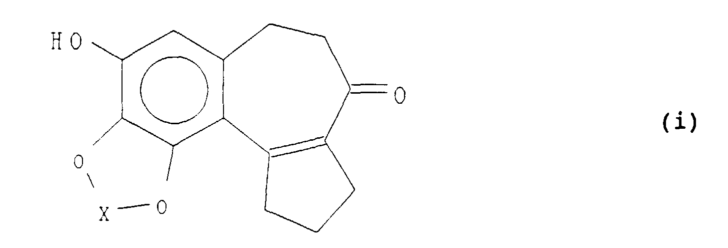 ou (1') 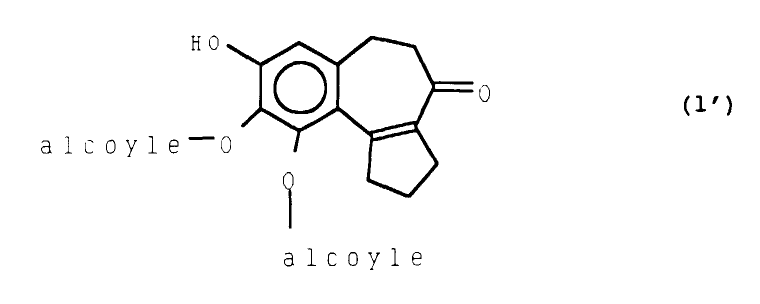 correspondant aux composés de formule (I) (f) telle que définie plus haut.

L'agent d'halogénation que l'on fait agir sur le composé de formule (a) est par exemple le chlorure de thionyle, le chlorure d'oxalyle ou tout autre agent connu de l'homme du métier pour préparer un halogénure d'acide.

Le réactif de formule (b) est préparé au départ de la cyclopentanone et d'une amine secondaire, par exemple la diéthylamine, la pipéridine, la pipérazine ou, de préférence, la morpholine. On opère en présence d'un catalyseur acide fort, par exemple l'acide paratoluène sulfonique.

L'action de l'énamine de formule (b) sur l'halogénure d'acide est réalisée de préférence en présence d'une amine tertiaire telle que la triéthylamine ou la pyridine.

L'agent d'halogénation que l'on fait agir sur le composé de formule (c) peut être, par exemple, le chlorure de thionyle, le phosgène, l'oxychlorure de phosphore ou, de préférence, le chlorure d'oxalyle.

L'acide de Lewis utilisé pour cycliser le composé de formule (d) est par exemple le chlorure d'aluminium, le tétrachlorure de titane, ou de préférence le chlorure ferrique, ou le tétrachlorure d'étain. La réaction, comme celles qui précèdent, peut être conduite, par exemple, dans un solvant halogéné tel que le chlorure de méthylène, le chloroforme ou le dichloroéthane.

L'hydrolyse conduisant au composé de formule (f), (i) ou (l') peut être effectuée par l'action de la soude, la potasse ou encore du carbonate de sodium ou de potassium, en présence d'un alcanol aqueux.

L'hydrolyse conduisant au composé de formule (h) peut être effectuée par l'action d'un agent désalkylant tel que le tribromure de bore, le chlorure d'aluminium, l'acide chlorhydrique, ou encore un mercaptan tel que le thiophénol.

L'agent d'alkylation que l'on fait agir sur les composés de formules (f) et (k) peut être tout agent classique connu de l'homme du métier pour alkyler les phénols. On peut citer par exemple un halogénure d'alkyle tel que l'iodure de méthyle ou d'éthyle, un sulfate d'alkyle tel que le sulfate de méthyle ou d'éthyle, ou encore le diazométhane. On peut le cas échéant opérer en présence d'une base telle qu'un hydroxyde ou un carbonate alcalin, au sein d'un solvant organique, par exemple un solvant halogéné, et le cas échéant en présence d'un agent de transfert de phase, par exemple un ammonium quaternaire tel que le bromure de tétra-n-butyl ammonium.

Le réactif de protection des diols que l'on fait agir sur le composé de formule (h) peut être un dérivé du bore tel que l'acide borique, un borate de trialkyle, par exemple de triméthyle ou triéthyle, ou encore le borax,

Il peut également être le formol ou une cétone, par exemple l'acétone ou la méthyléthylcétone. On opère alors en milieu acide fort et anhydre. Il peut encore être le phosgène, utilisé en milieu alcalin anhydre, par exemple en présence de triéthylamine ou de pyridine. Il peut encore être du type 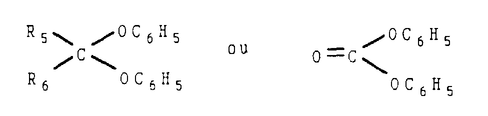 et l'on opère alors en milieu acide fort anhydre, par exemple en présence d'un acide sulfonique. Il peut encore être du type 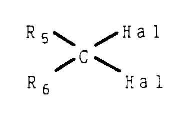 et l'on opère alors en milieu basique anhydre, par exemple en présence d'une amine tertiaire.

Le réactif de formule A-SO₂Y que l'on fait agir sur le composé de formule (i) est notamment un halogénure d'acide sulfonique ASO₃H, dans lequel A représente par exemple un radical alkyle tel que méthyle ou éthyle, phényle éventuellement substitué par un à trois alkyles tel que tolyle, xylyle ou 2,4,6-triisopropylphényl, ou encore naphtyle.

La déprotection du diol est réalisée soit par un agent d'hydrolyse acide, par exemple un acide fort tel que l'acide chlorhydrique, l'acide sulfurique ou l'APTS, ou par un oxydant, par exemple l'eau oxygénée dans le cas d'une protection par un dérivé du bore. Dans le cas d'une protection sous forme d'acétonide ou de carbonate, la déprotection est réalisée en milieu acide fort aqueux, par exemple par l'acide sulfurique, chlorhydrique ou encore l'APTS.

L'invention a encore pour objet l'application des composés de formule (I), telle que définie plus haut, à la préparation des composés de formule (II) : 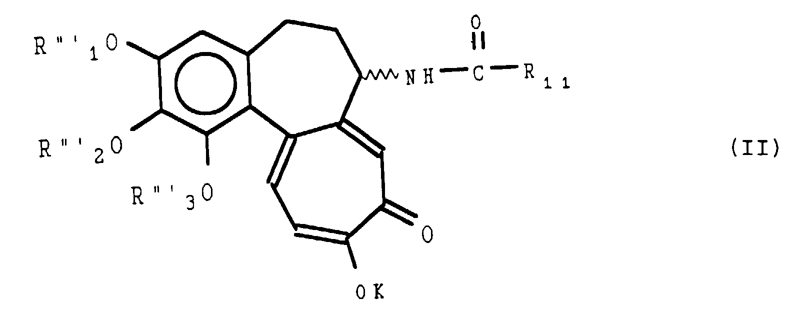 dans laquelle R"'₁, R"'₂ et R"'₃ ont les mêmes significations que les groupements R₁, R₂ et R₃ tels que définis plus haut, à l'exception de la valeur hydrogène, et, pour R₂-R₃, de la valeur X autre que -CR₅R₆- et, pour R₁-R₂, de la valeur X autre que -CR₅R₆-, R₁₁ représente un radical alkyle, le trait ondulé indique que le groupement NH-C(O)R₁₁ se trouve en position α ou β, ou que le composé est sous la forme d'un mélange d'isomères α + β, et K représente un radical alkyle, un radical benzyle éventuellement substitué sur le phényle par 1 à 3 radicaux alkyles, ou un radical -SO₂-A', dans lequel A' est choisi indépendamment parmi les valeurs de A mentionnées plus haut, caractérisée en ce que :
(i) soit l'on soumet un composé de formule (I), telle que définie plus haut, dans laquelle les groupements R_{1,} R₂ ou R₃ ne représentent pas un atome d'hydrogène, à l'action d'un agent de formule (m) : 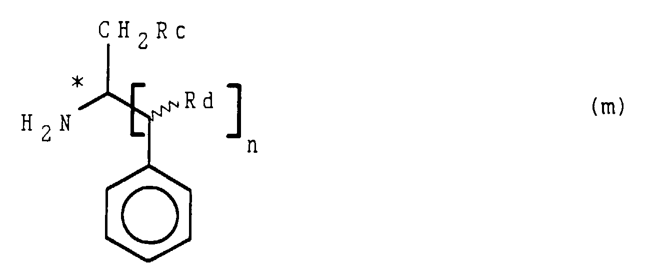 sous forme racémique ou optiquement active, dans laquelle Rc et Rd, identiques ou différents, représentent un atome d'hydrogène ou un radical hydroxy et n représente le chiffre 0 ou 1 ;
   puis traite l'intermédiaire imino ainsi obtenu par un agent réducteur pour obtenir un composé de formule (n) : 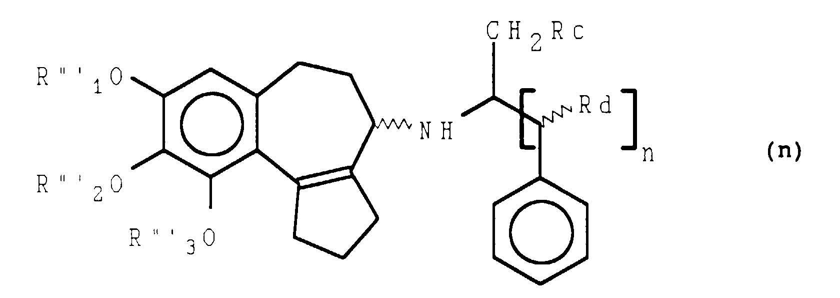 dans laquelle Rc et Rd ont la définition indiquée ci-dessus, et le trait ondulé reliant NH au cycle symbolise, selon que l'on a utilisé un agent de formule (m) racémique ou optiquement actif, respectivement un mélange d'isomères ou l'un ou l'autre isomère,
   composé (n) que ou bien l'on soumet à l'hydrogénolyse du groupement alkylamino, pour obtenir un composé de formule (o): 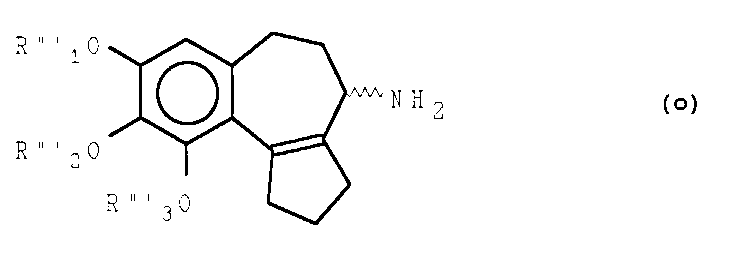 dans lequel le trait ondulé est défini comme ci-dessus, dont on protège le groupement amino avec un groupement protecteur bivalent pour obtenir un composé de formule (p) : 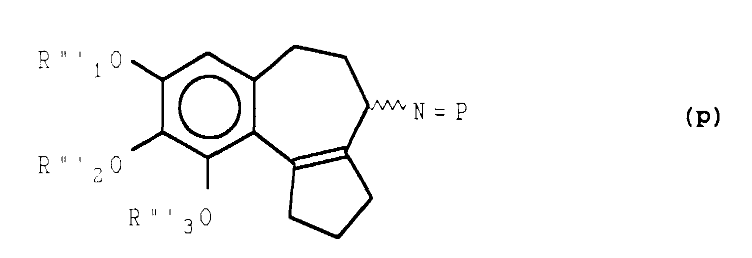 dans laquelle P représente un groupement protecteur bivalent, et le trait ondulé a la signification indiquée ci-dessus, ou bien composé de formule (n) telle que définie ci-dessus, que l'on soumet à l'action d'un agent d'hydrolyse du groupement R‴₁O pour obtenir un composé de formule (n') : 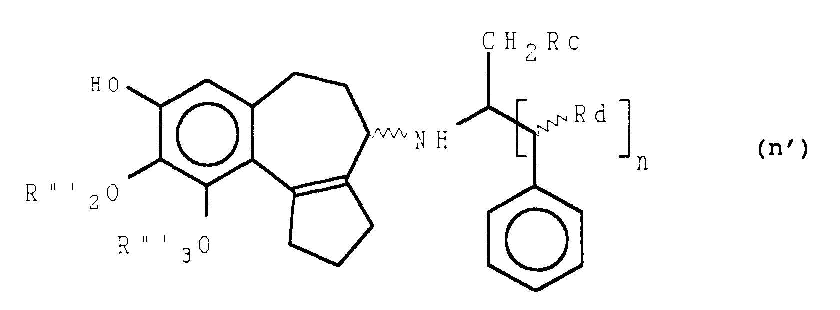 dans laquelle R"'₂, R"'₃, Rc, Rd, n et les traits ondulés ont la signification indiquée précédemment, que l'on soumet à l'hydrogénolyse du groupement alkylamino, pour obtenir un composé de formule (o') : 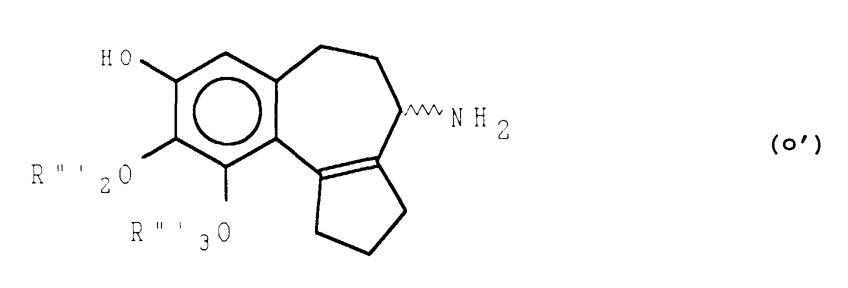 dans lequel le trait ondulé est défini comme ci-dessus, dont on protège le groupement amino avec un groupement protecteur bivalent pour obtenir un composé de formule (p') : 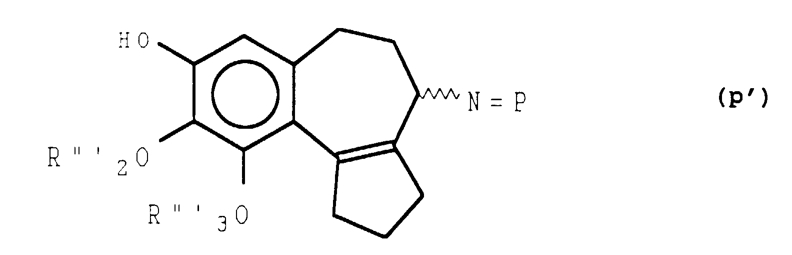 dans laquelle P et le trait ondulé ont la signification indiquée ci-dessus, que l'on soumet à l'action d'un agent protecteur du radical hydroxy pour obtenir un composé de formule (p) telle que définie ci-dessus ;
(ii) soit l'on soumet un composé de formule (I) (f) telle que définie plus haut, à l'action d'un agent de formule (m) définie ci-dessus, traite l'intermédiaire imino obtenu par un agent réducteur pour obtenir un composé de formule (n') telle que définie ci-dessus, puis poursuit la synthèse comme indiqué ci-dessus ;
(iii) soit l'on soumet un composé de la formule (I) telle que définie plus haut, à l'action d'un agent de formule R₈O-NH₂, ou un sel de ce composé, dans laquelle R₈ représente un atome d'hydrogène ou un radical alkyle, pour obtenir un composé de formule (q) : 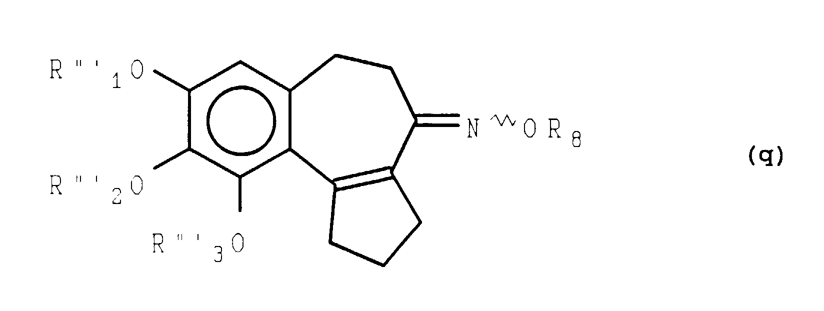 dans laquelle le trait ondulé indique que le composé consiste en un mélange d'isomères syn et anti,
(iv) soit l'on soumet un composé de formule (I) telle que définie plus haut, à l'action ou bien d'un agent réducteur chiral, ou bien d'un agent réducteur non chiral, pour obtenir l'alcool correspondant, de formule (r) : 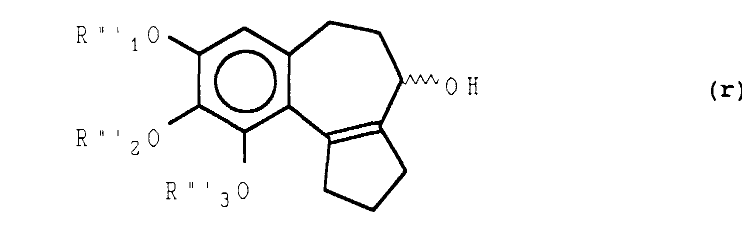 dans laquelle le trait ondulé entre l'atome d'azote et le cycle à 7 chaînons indique que ce produit consiste selon le réducteur utilisé, ou bien en un isomère α ou β, ou bien en un mélange d'isomères α + β, mélange que le cas échéant l'on dédouble, puis soit soumet l'alcool à l'action d'un agent de formule HN=P, dans laquelle P a la signification donnée précédemment, pour obtenir un composé de formule (p) : 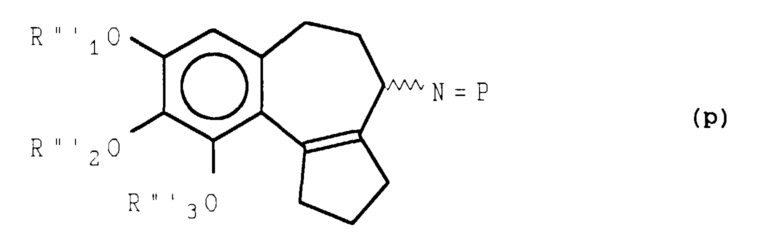 dans laquelle le trait ondulé entre l'atome d'azote et le cycle à 7 chaînons indique que ce produit consiste en un isomère α ou β, ou à un mélange racémique d'isomères, soit soumet l'alcool à l'action d'un réatif de formule ASO₂Hal, dans laquelle A est défini comme plus haut et Hal représente un atome d'halogène pour obtenir le composé de formule (r₁) : 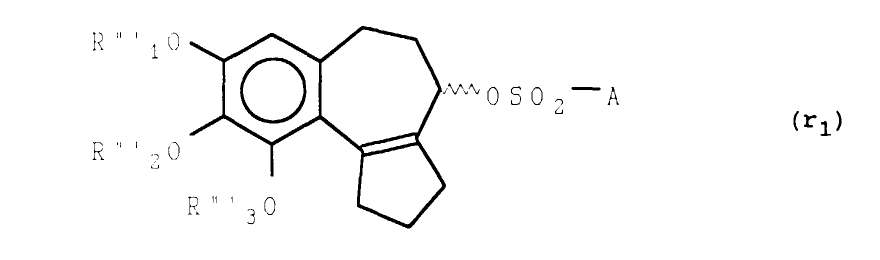 dans laquelle le trait ondulé et A ont les significations ci-dessus, que l'on fait agir avec l'ammoniac, pour obtenir le composé de formule (o) ci-dessus, dont on protège le groupement amino avec un groupement protecteur bivalent, pour obtenir le composé de formule (p) ci-dessus, puis,
(v) l'on soumet un composé de formule (p) ou (q) à l'action d'un agent de bromation en présence d'un composé de formule R₇OH, dans laquelle R₇ représente un atome d'hydrogène ou un radical alkyle, pour obtenir un composé de formule (s) : 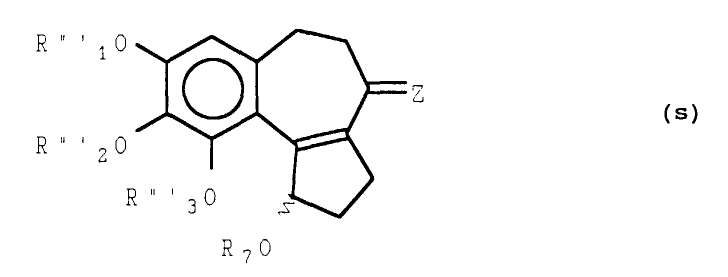 dans laquelle le trait ondulé indique que la liaison peut se trouver en position alpha ou béta ou que ce produit peut consister en un mélange d'isomères et dans laquelle le groupement =Z représente un groupement de formule = N 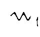 OR₈ ou 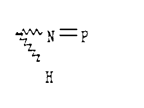 telles que définies dans les formules p et q, ci-dessus,
(vi) que l'on soumet à une déshydratation ou une désalcoxylation en milieu acide anhydre pour obtenir un composé de formule (t) : 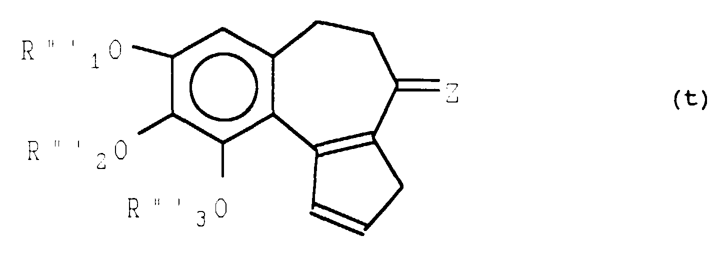
(vii) que l'on soumet en présence d'une base ou d'un métal réducteur, à l'action d'un agent de formule 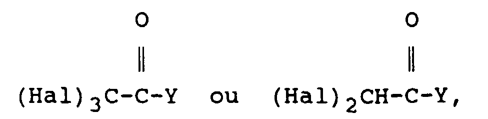 dans laquelle Hal représente un atome d'halogène et Y est défini comme plus haut, pour obtenir un composé de formule (u) : 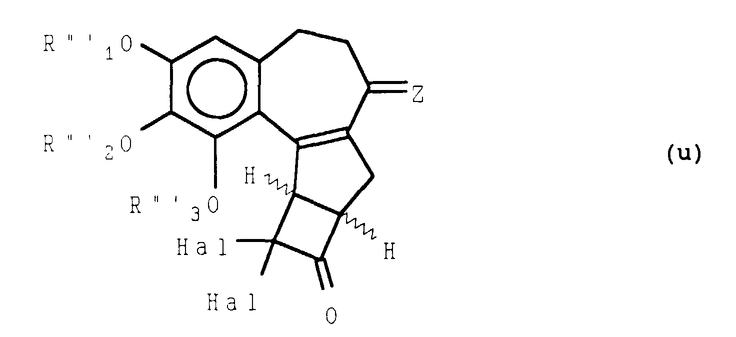 dans laquelle les traits ondulés indiquent que les atomes d'hydrogène se trouvent tous les deux en position alpha ou tous les deux en position béta ;
(viii) que l'on soumet à l'action d'un acide carboxylique en présence d'une base et en milieu aqueux, pour obtenir un composé de formule (v), lequel existe en équilibre avec son tautomère (v') : 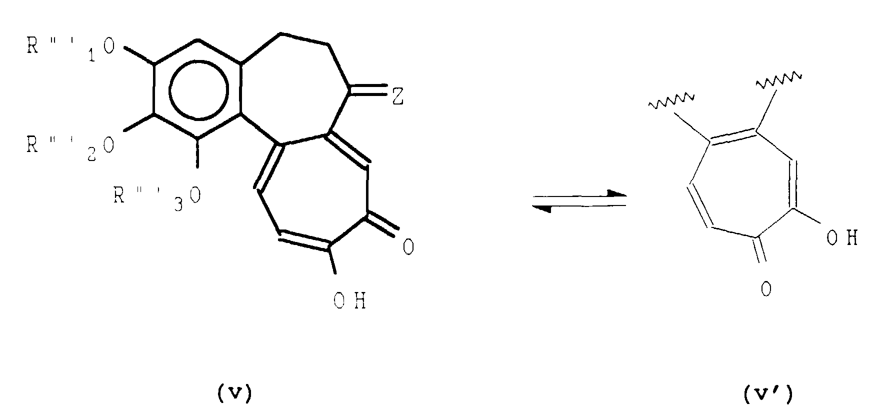 lequel composé, ou bien,
(ix) si =Z représente un groupement de formule 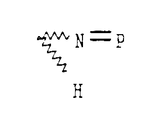 tel que défini plus haut, soit l'on soumet à une réaction de déprotection du groupement amino pour obtenir soit l'amine libre de formule (w), lequel existe en équilibre avec son tautomère (w') : 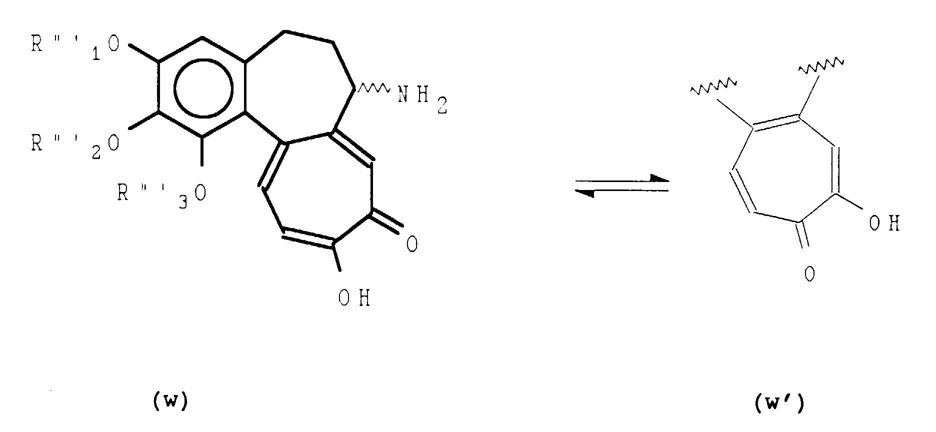 dans laquelle le trait ondulé indique que le groupement NH₂ se trouve en position alpha ou béta, ou que le produit consiste en un mélange d'isomères,
   que l'on soumet à l'action d'un réactif de formule R₁₁-C(O)Y ou de formule (R₁₁CO)₂O, dans laquelle le groupement R₁₁ représente un groupement alkyle et Y est défini comme plus haut, pour obtenir un composé de formule (x) : 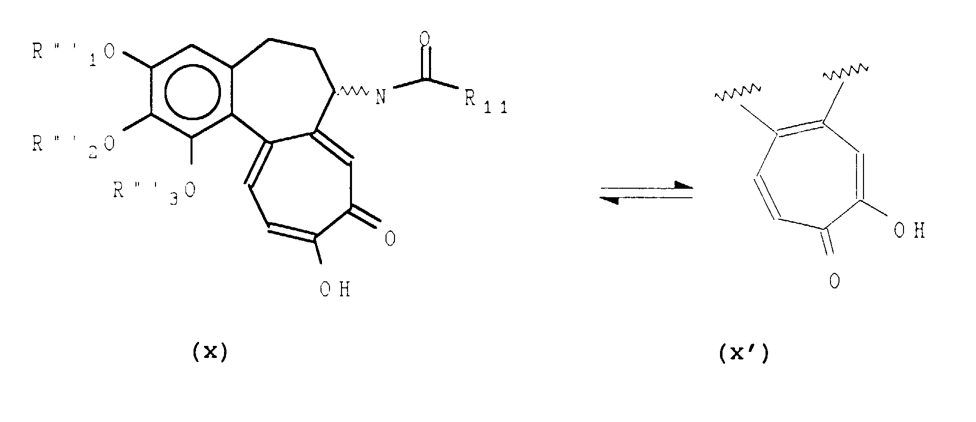 dans laquelle le trait ondulé a la signification précédente, soit l'on soumet à une réaction de déprotection du groupement amino et à un agent d'hydrolyse du groupement R"'₁ dans le cas où ce groupement représente la valeur A-SO₂- telle que définie plus haut, pour obtenir un composé de formule (w₁) : 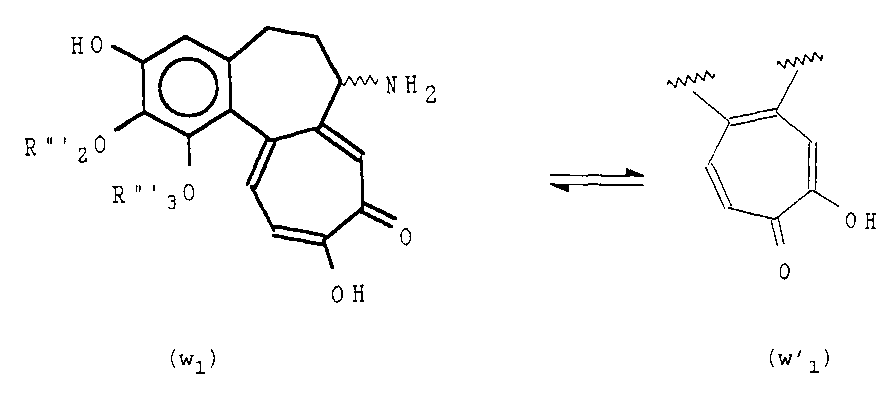 que l'on soumet à l'action d'un composé de formule R₁₁C(O)Y ou (R₁₁CO)₂O telle que définie plus haut, pour obtenir un composé de formule (x₁) : 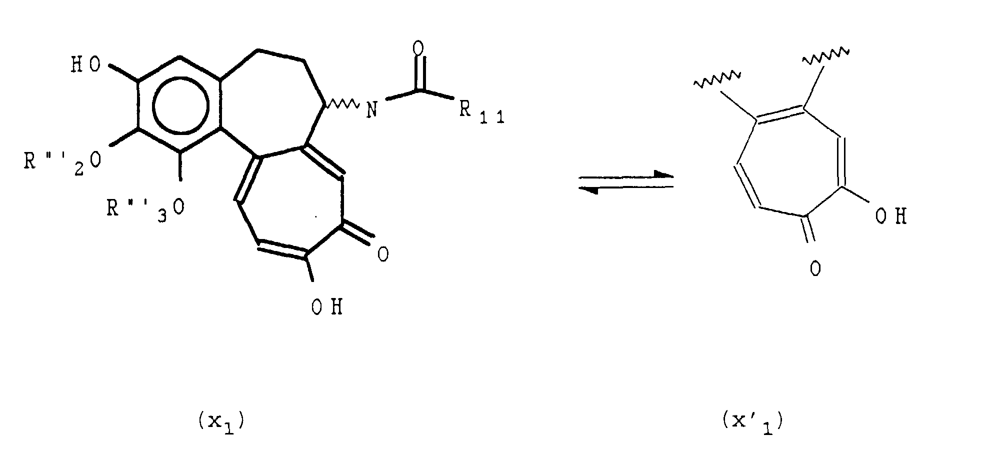 dont on protège la fonction OH par action d'un réactif de formule ASO₂Y tel que défini plus haut, pour obtenir un composé correspondant de formule (x)/(x') telle que définie plus haut, ou bien,
(x) si =Z représente un groupement de formule =N∼OR₈, l'on soumet à l'action d'un agent protecteur de formule B(Hal)₃ ou B(OR₄)₃ dans lesquelles R₄ et Hal sont définis comme précédemment, puis à l'action d'un agent réducteur, pour obtenir un composé de formule (y) : 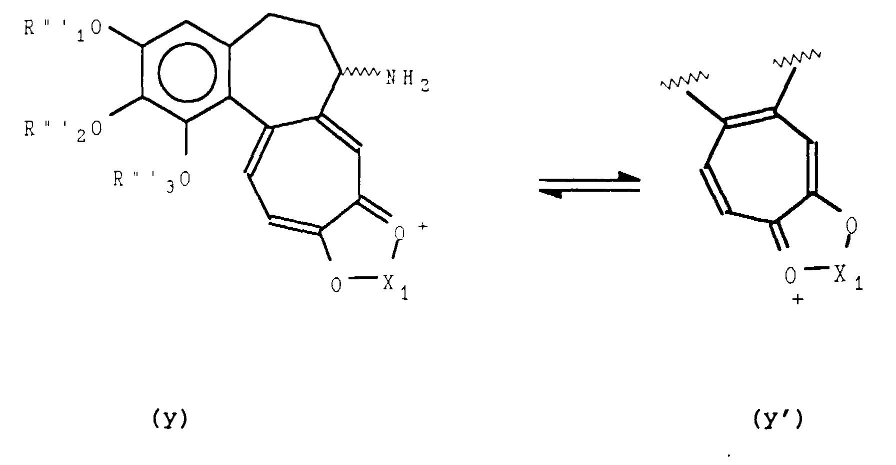 dans laquelle le trait ondulé indique que ce produit consiste en un mélange d'isomères, et X₁ représente un groupement de formule -B(OR₄)₂- ou -B(Hal)₂ que l'on soumet à l'action d'un composé de formule R₁₁C(O)Y ou de formule (R₁₁CO)₂O, telle que définie plus haut puis à l'action d'un réactif de déprotection du groupement alpha-hydroxy cétone, pour obtenir un composé de formule (x")/(x"₁) correspondant au produit de formule (x)/(x') telle que définie ci-dessus, dans laquelle le trait ondulé indique que le produit consiste en un mélange d'isomères ;
(xi) lequel composé de formule (x) ou (x') l'on soumet à l'action d'un agent susceptible d'introduire le groupement K défini plus haut, pour obtenir un composé de formule (II) : 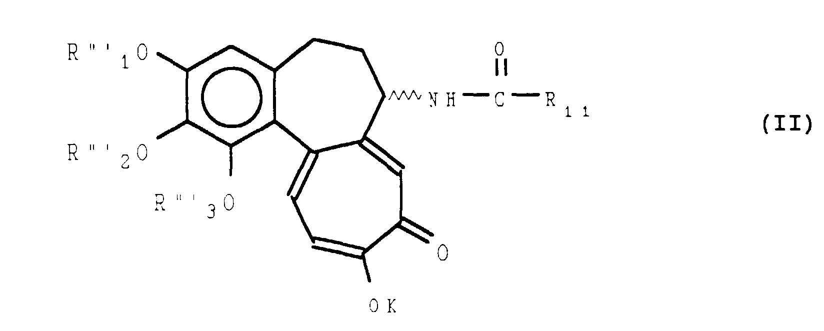 lequel existe en mélange avec son régioisomère de formule : 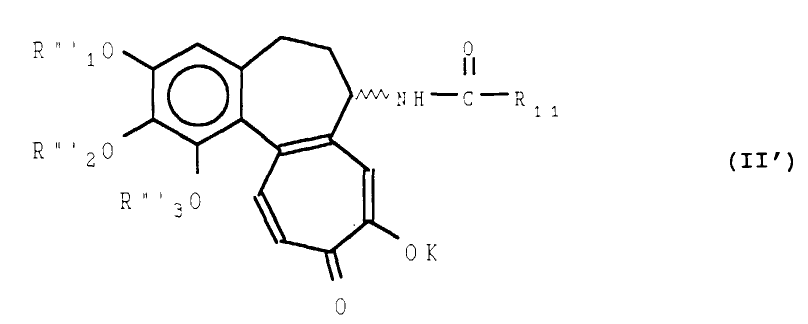 formules (II) et (II') dans lesquelles le trait ondulé indique que le groupement se trouve en position alpha ou béta, ou que ce produit consiste en un mélange d'isomères et les termes R"'₁, R"'₂, R"'₃, R₁₁, K et alkyle ayant les significations données précédemment, mélange dont on sépare les constituants.

La formation de l'imine par action de l'agent (m) sur le composé (I) est effectuée notamment en présence de tétrachlorure de titane et d'une base, notamment une amine tertiaire, ou en présence d'un catalyseur acide, notamment de la silice ou un acide sulfonique tel que l'APTS.

L'agent (m) préféré est l'α-méthylbenzylamine, mais on peut encore utiliser la noréphédrine ou la norpseudoéphédrine.

L'agent réducteur que l'on fait agir sur l'imine intermédiaire est par exemple un hydrure tel que le borohydrure de sodium ou l'hydrure d'aluminium-lithium ou encore l'hydrure de diisobutylaluminium. On peut encore utiliser l'hydrogène en présence d'un catalyseur tel que le palladium ou le platine.

L'hydrogénolyse du groupement alkylamino est effectuée par l'hydrogène en présence d'un catalyseur tel que le palladium ou le platine.

Le groupement protecteur bivalent de l'amine est notamment un groupement phtalimido, obtenu par l'action de l'anhydride phtalique ou d'un dérivé d'acide orthophtalique de type 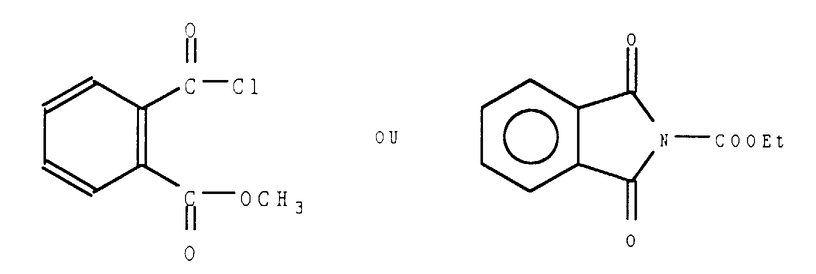 sur le composé (o) ou (o'), au sein d'un solvant tel que le toluène, en présence d'une base, par exemple une base azotée telle que la triéthylamine.

Le groupement protecteur peut encore être du type maléimide, notamment un groupement diphénylmaléimide.

L'hydrolyse du groupement R"'₁O peut être effectuée par l'action de la soude, de la potasse ou encore du carbonate de sodium ou de potassium, en présence d'un alcanol aqueux, lorsque R"'₁ représente un groupe A-SO₂- ou par l'action d'un agent désalkylant tel que le tribromure de bore, le chlorure d'aluminium, l'acide chlorhydrique ou encore un mercaptan tel que le thiophénol lorsque R"'₁ représente un radical alkyle.

Le réactif de protection que l'on fait agir sur le composé de formule (p') peut être un agent d'alkylation classique connu pour alkyler les phénols choisis parmi ceux indiqués ci-dessus ou bien un agent de formule A-SO₂Y tel que défini ci-dessus.

L'agent de formule R₈O-NH₂ est l'hydroxylamine ou un dérivé alkylé, par exemple la méthylhydroxylamine, et est de préférence utilisé sous forme de sel, notamment le chlorhydrate.

L'agent réducteur permettant de préparer l'alcool (r) est notamment un hydrure tel que l'un de ceux mentionnés plus haut. L'alcool est alors obtenu sous forme d'un mélange d'isomères. Une réduction chirale peut être réalisée par action d'une oxazaborolidine chirale, selon la méthode dite de Corey.

Le dédoublement de l'alcool peut être effectué par les méthodes classiques connues de l'homme du métier. On peut par exemple opérer par voie enzymatique, à l'aide d'une lipase en présence d'acétate de vinyle, au sein d'un solvant organique, par exemple un éther tel que le terbutyl méthyl éther.

Le réactif de formule HN=P est notamment le phtalimide ou un maléimide tel que le diphénylmaléimide. On opère en présence de triphénylphosphine et d'un azodicarboxylate d'alkyle de formule R₈O₂C-N=N-CO₂-R₁₀, où R₈ et R₁₀ représentent des alkyles identiques ou différents.

Le réactif de formule ASO₂Hal que l'on fait réagir avec l'alcool de formule (r) est de préférence le chlorure de méthane ou de p-toluène sulfonyle.

Les composés de formule (o) et (p) sont obtenus dans une configuration inverse de celle de l'alcool de formule (r) de départ.

L'agent de bromation est notamment le perbromure de pyridinium, le brome, le N-bromo succinimide ou la dibromo diméthylhydantoïne.

Le composé de formule R₇-OH est l'eau ou de préférence un alcanol inférieur, par exemple le méthanol ou l'éthanol.

Le composé de formule (t) est obtenu par action d'un acide fort tel que l'acide chlorhydrique, l'acide sulfurique, l'acide paratoluène sulfonique ou encore une résine sulfonique dans des conditions anhydres, sur le composé de formule (s).

Le réactif de formule Hal₃C-C(O)-Y ou Hal₂CH-C(O)-Y est de préférence le chlorure ou le bromure de tri ou dichloro ou dibromo acétyle. On opère dans le cas de Hal₂CH-C(O)-Y en présence d'une base, notamment une amine tertiaire, telle que la triéthylamine ou la pyridine, dans des conditions anhydres.

Dans le cas de Hal₃C-C(O)-Y, on opère en présence d'un métal réducteur tel que le zinc. On obtient un mélange de diastéréoisomères.

Le réarrangement en tropolone est effectué par action d'un acide carboxylique en présence d'une base qui peut être une base aminée, par exemple la triéthylamine, ou un hydroxyde alcalin, en présence d'eau et d'un solvant miscible à l'eau, tel que l'acétone, le dioxanne ou le tétrahydrofuranne. On obtient un produit se présentant sous la forme d'un équilibre entre les deux tautomères.

La déprotection de l'amine est effectuée par action de l'hydrazine utilisée de préférence sous forme d'hydrate.

Le réactif de formule R₁₁-C(O)Y ou (R₁₁CO)₂O est de préférence un dérivé de l'acide acétique, par exemple le chlorure ou bromure d'acétyle ou l'anhydride acétique.

L'hydrolyse sélective du groupement R"'₁ est effectuée de préférence par action d'un thioalcoolate alcalin tel que le thiométhylate de sodium, dans un solvant tel que le diméthylformamide ou le diméthylsulfoxyde. On peut encore utiliser la soude ou la potasse, ou encore le carbonate de sodium ou potassium, dans un alcool, tel que le méthanol ou l'éthanol.

Le réactif de formule ASO₂Y est de préférence un halogénure tel que le chlorure ou bromure de mésyle ou de tosyle ou de 2,4,6-triisopropylphényl sulfonyle ou encore un dérivé benzylé tel que le mésylate ou tosylate de benzyle.

Le réactif formant le groupement protecteur des fonctions hydroxy et cétone du composé (x₁)/(x'₁) est le trifluorure, le trichlorure ou le tribromure de bore, ou encore le borate de triméthyle ou triéthyle.

L'agent réducteur que l'on fait agir sur l'oxime intermédiaire est un métal tel que le zinc, en présence d'un acide sulfonique ou carboxylique, tel que l'acide méthane sulfonique ou l'acide acétique.

La déprotection des fonctions hydroxy et cétone est effectuée par hydrolyse acide ou basique ou encore par un sel tel que l'acétate de sodium ou de potassium, ou le carbonate ou bicarbonate de sodium ou de potassium dans un alcool, tel que le méthanol ou l'éthanol et en présence d'eau.

L'agent susceptible d'introduire le groupement K est par exemple un halogènure d'alkyle tel que l'iodure de méthyle ou éthyle, un sulfate d'alkyle, tel que le sulfate de méthyle ou d'éthyle, le diazométhane, un halogénure de benzyle tel que le chlorure ou le bromure de benzyle, le mésylate ou le tosylate de benzyle, ou l'un des réactifs de formule ASO₂Y cités précédemment.

La séparation des constituants du mélange de composés (II) et (II') est effectuée par les méthodes connues de l'homme de métier, notamment par chromatographie.

L'invention a encore pour objet une application telle que définie précédemment, caractérisée en ce que l'on soumet un composé de formule (v)/(v') telle que définie précédemment, dans laquelle Z représente un groupement 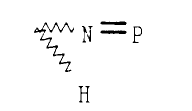 à l'action d'un agent susceptible d'introduire le groupement K, tel que défini précédemment, pour obtenir un composé de formule (III) : 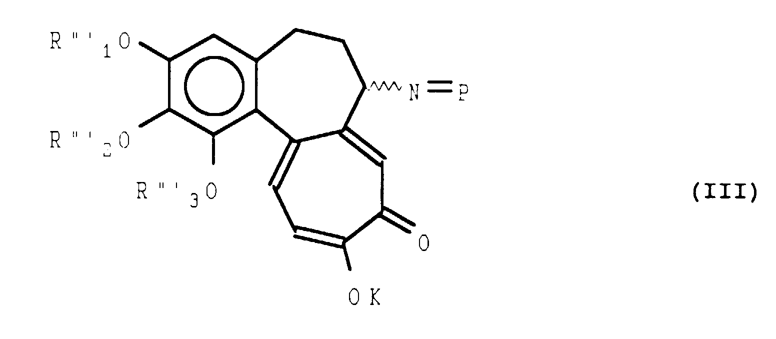 lequel existe en mélange avec son régioisomère de formule : 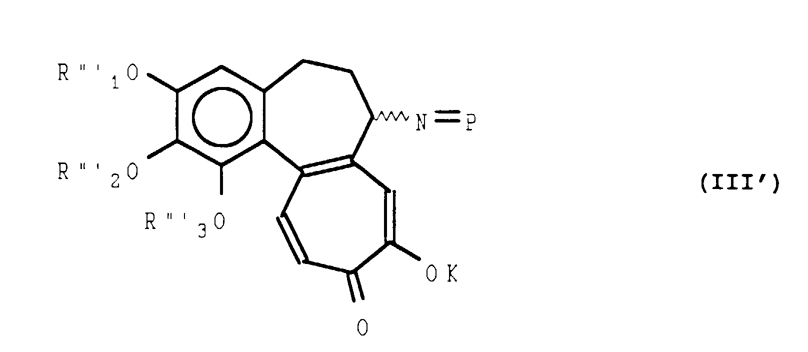 formules (III) et (III') dans lesquelles le trait ondulé indique que le groupement se trouve en position alpha ou béta, ou que ce produit consiste en un mélange d'isomères et les termes R‴₁, R‴₂, R‴₃, R₁₁, K et alkyle ayant les significations données précédemment, mélange dont le cas échéant, on sépare les constituants, puis soumet le mélange ou les constituants séparés, à une réaction de déprotection de la fonction amino, pour obtenir soit le composé de formule (IV) : 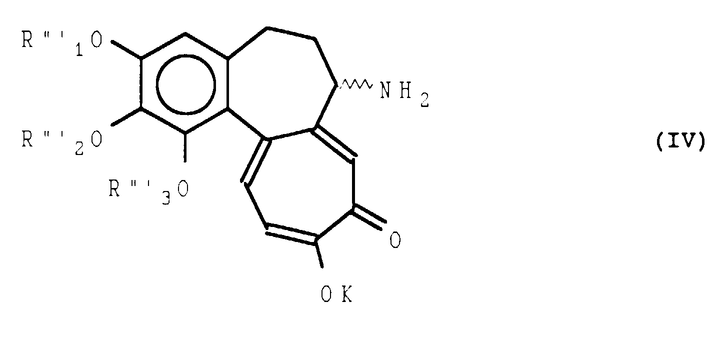 sous forme de mélange avec son régioisomère de formule 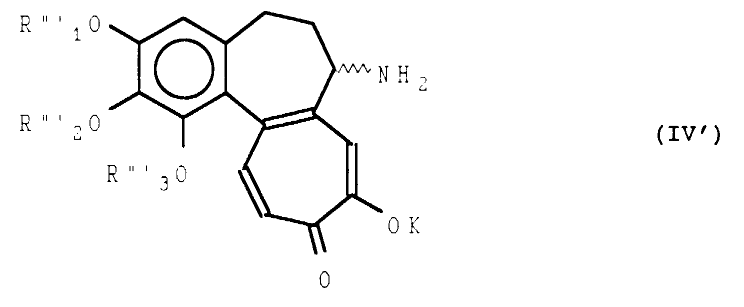 mélange que, le cas échéant, l'on sépare,
soit l'un ou l'autre de ces régioisomères, puis soumet le mélange ou les constituants séparés, à l'action d'un composé de formule R₁₁C(O)Y ou (R₁₁CO)₂O, telle que définie plus haut, pour obtenir soit le composé de formule (II) telle que définie plus haut, en mélange avec son régioisomère de formule (II'), mélange que, le cas échéant, l'on sépare en ses constituants, soit l'un ou l'autre de ces régioisomères.

Dans l'application ci-dessus, l'agent susceptible d'introduire le groupement K, le réactif de déprotection de la fonction amino et les composés de formule R₁₁C(O)Y et (R₁₁CO)₂O sont les mêmes que ceux qui ont été définis plus haut. La séparation des divers régioisomères peut être effectuée par les méthodes connues de l'homme du métier, notamment la chromatographie.

L'invention a encore pour objet une application telle que définie précédemment, caractérisée en ce que l'on soumet un composé de formule (v)/(v') telle que définie précédemment, dans laquelle (z) représente un groupement =N-OR₈, à l'action d'un agent susceptible d'introduire le groupement K, pour obtenir un composé de formule (V) : 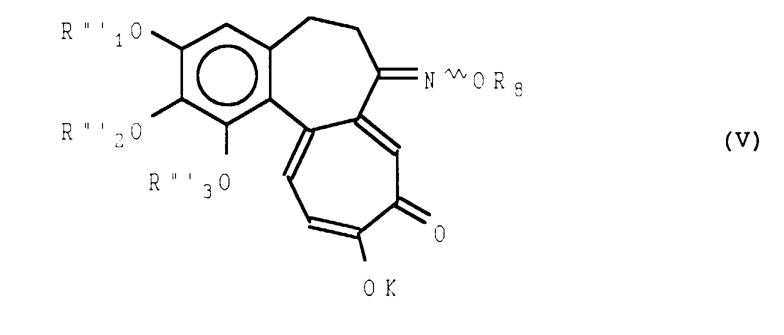 lequel existe en mélange avec son régioisomère de formule 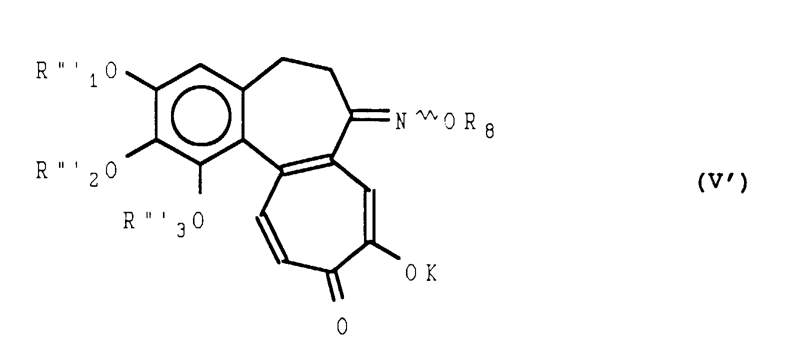 formules (V) et (V') dans lesquelles le trait ondulé, R₈ et les termes R"'₁, R"'₂, R"'₃, K et alkyle ont les significations données précédemment, mélange dont le cas échéant, on sépare les constituants, puis soumet le mélange ou les constituants séparés, à l'action d'un agent réducteur, pour obtenir soit le composé de formule (IV₁) : 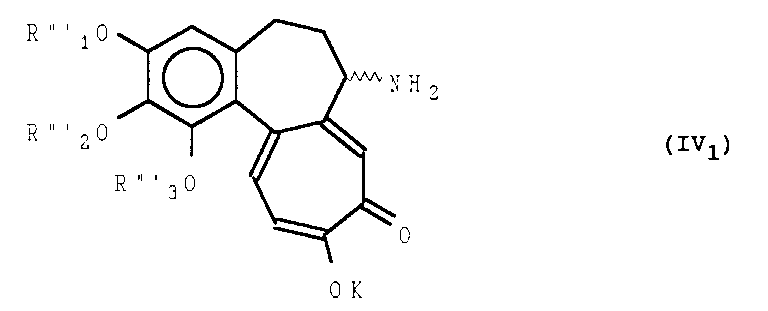 sous forme de mélange avec son régioisomère de formule 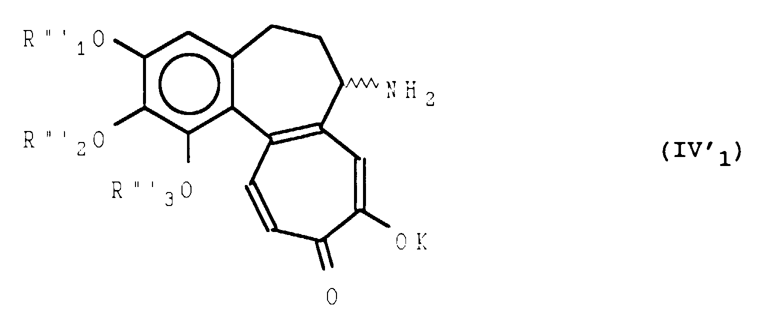 le trait ondulé symbolisant un mélange d'isomères, mélange de régioisomères que, le cas échéant, l'on sépare,
soit l'un ou l'autre de ces régioisomères, puis poursuit la synthèse comme décrit précédemment pour les composés de formule (IV)/(IV').

Dans l'application ci-dessus, l'agent susceptible d'introduire le groupement K et l'agent réducteur de l'oxime sont les mêmes que ceux qui ont été définis plus haut. La séparation des régioisomères peut être effectuée par les méthodes connues de l'homme du métier, notamment la chromatographie.

Le composé de formule (II) dans laquelle R"'₁, R"'₂ et R"'₃ représentent un radical méthyle, K représente un radical méthyle, R₁₁ représente un radical méthyle et le trait ondulé symbolise l'un ou l'autre isomère ou un mélange d'isomères, correspond respectivement à la colchicine optiquement active ou à la colchicine racémique.

L'invention a encore pour objet une application telle que définie précédemment, caractérisée en ce que en outre l'on soumet un produit de formule (II₁) : 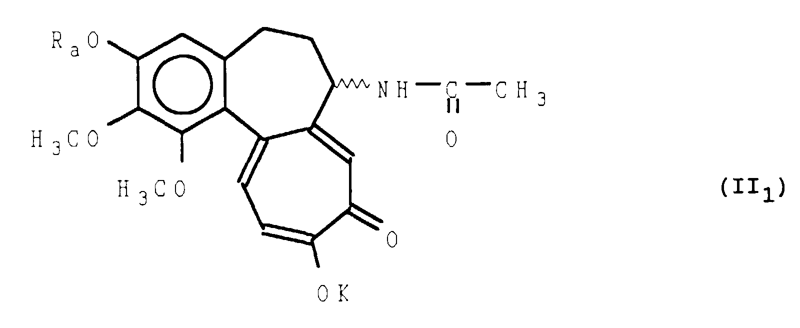 dans laquelle K est défini comme précédemment, Rₐ représente un radical ASO₂- tel que défini précédemment et le trait ondulé symbolise l'un ou l'autre isomère ou un mélange d'isomères, à l'action d'un réactif de formule CH₃S⁻Na⁺ pour obtenir un composé de formule (VI) : 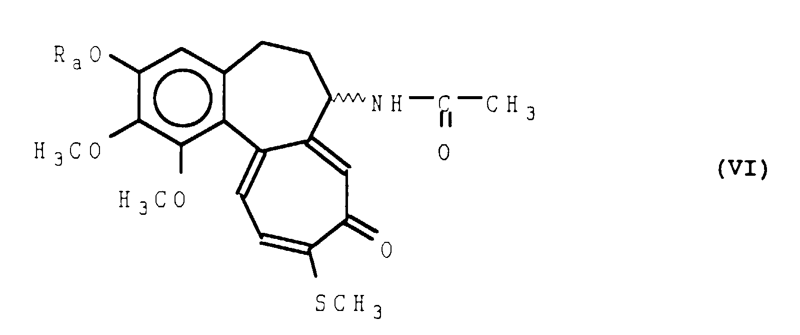 dans laquelle Rₐ et le trait ondulé ont la signification précédente que l'on soumet à un réactif de déprotection du radical hydroxy pour obtenir le composé de formule (VII) : 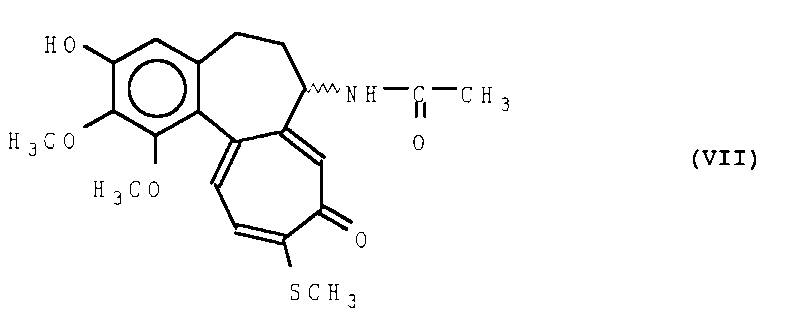 optiquement actif ou racémique.

La réaction avec le sel de sodium du méthylmercaptan est de préférence effectuée dans le diméthylformamide ou le diméthylsulfoxyde.

Le réactif de déprotection du radical hydroxy est de préférence également le sel de sodium du méthylmercaptan les conditions utilisées correspondent alors à l'utilisation d'un excès de réactif.

Le composé de formule (VII) est un composé connu sous le nom de "thio substance C" ou encore "desméthylthiocolchicine".

L'invention a encore pour objet une application telle que définie précédemment, caractérisée en ce que en outre l'on soumet un composé de formule (II₂) : 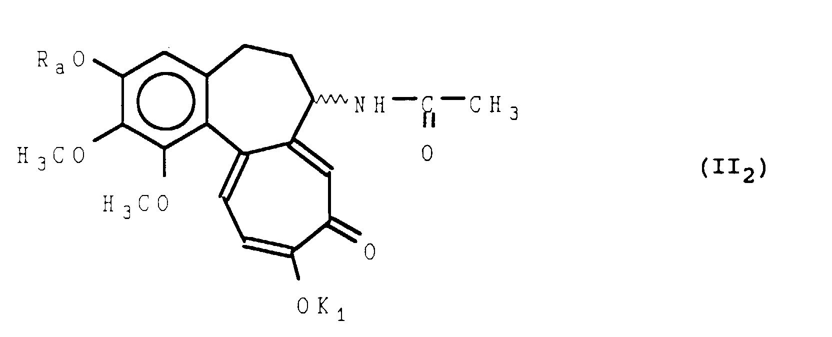 dans laquelle Rₐ est défini comme précédemment, K₁ représente un radical méthyle et le trait ondulé symbolise l'un ou l'autre isomère ou un mélange d'isomères, à l'action d'un agent de déprotection du radical hydroxy, pour obtenir le composé de formule (VIII) : 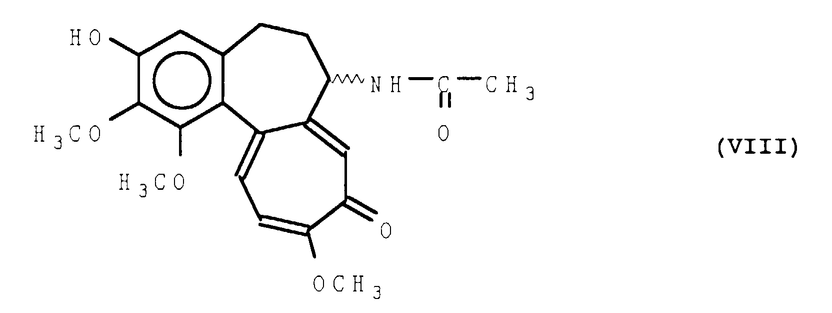 optiquement actif ou racémique.

Le réactif de déprotection du radical hydroxy est l'un de ceux qui ont été cités plus haut dans le cadre de l'obtention du composé de formule (f).

Le composé de formule (VIII) est un composé connu sous le nom de "substance C" ou encore "desméthylcolchicine".

L'invention a encore pour objet une application telle que définie précédemment, caractérisée en ce que en outre l'on soumet un composé de formule (II₃) : 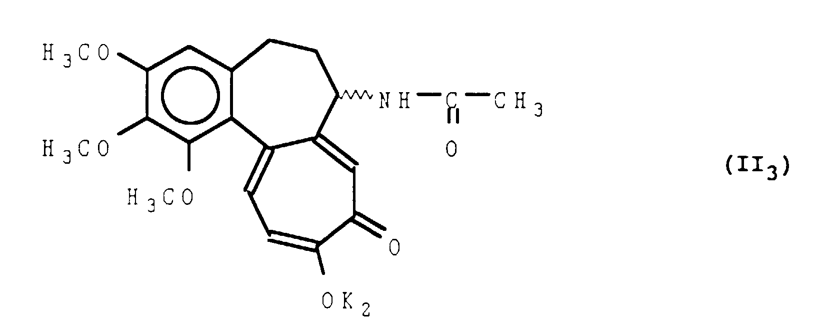 dans laquelle K₂ représente un radical benzyle éventuellement substitué sur le phényle par 1 à 3 radicaux alkyle, ou un radical -SO₂A' tel que défini précédemment et le trait ondulé symbolise l'un ou l'autre isomère, ou un mélange d'isomères, à l'action d'un réactif de formule CH₃S⁻Na⁺, pour obtenir le composé de formule (IX) : 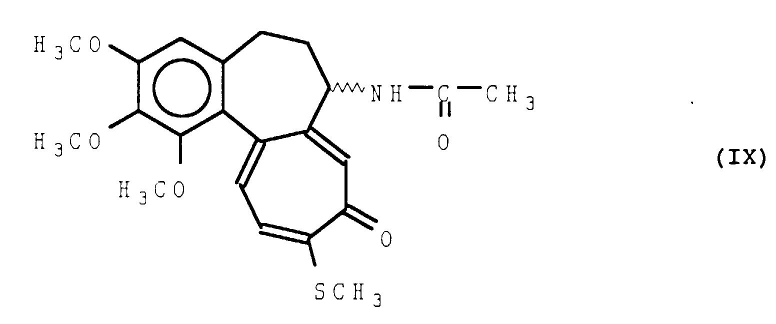 optiquement actif ou racémique.

Les conditions de la réaction sont identiques à celles qui ont été mentionnées ci-dessus.

Le composé de formule (IX) est un composé connu sous le nom de thiocolchicine (voir par exemple le brevet français 1099138).

L'invention a encore pour objet une application telle que définie précédemment, caractérisée en ce que en outre l'on soumet un composé de formule (II₄) : 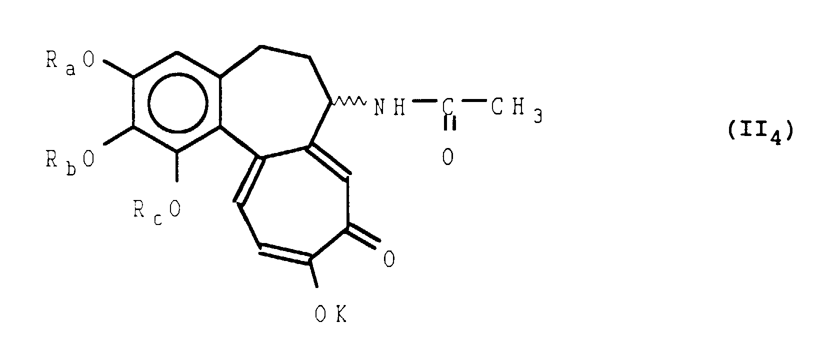 dans laquelle Rₐ et K sont définis comme précédemment, R_{b} et R_{c} forment ensemble un groupement CR₅R₆ tel que défini précédemment et le trait ondulé symbolise l'un ou l'autre isomère ou un mélange d'isomères, à l'action d'un réactif de déprotection du diol, pour obtenir un composé de formule (X) : 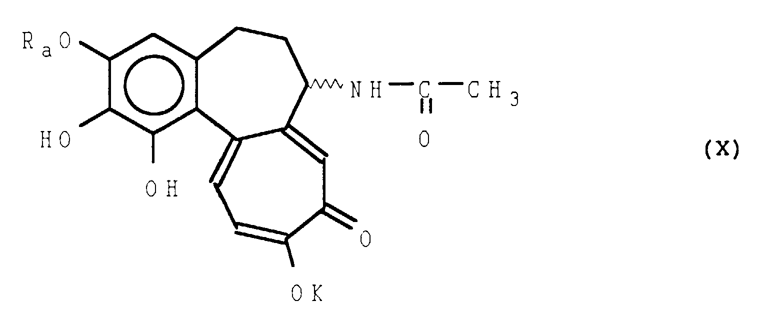 dans laquelle Rₐ, K et le trait ondulé ont la signification précédente que l'on traite par un agent de méthylation pour obtenir un composé de formule (II₁) telle que définie précédemment, puis poursuit la synthèse comme décrit précédemment au départ dudit composé de formule (II₁).

Le réactif de déprotection du diol peut être l'un de ceux qui ont été cités plus haut dans le cadre de l'obtention du composé de formule (k).

L'invention a encore pour objet une application telle que définie précédemment, caractérisée en ce que en outre l'on soumet un composé de formule (II₅) : 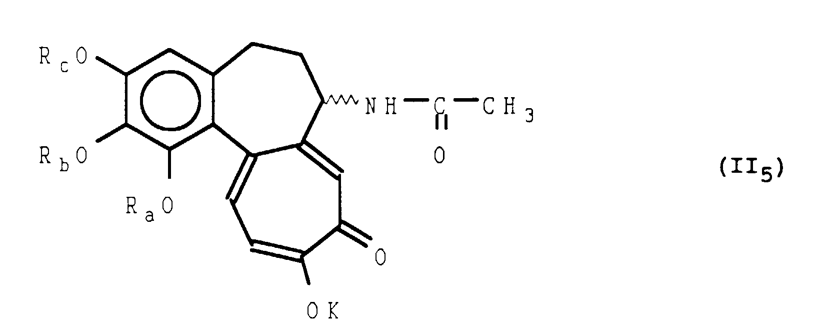 dans laquelle Rₐ, R_{b}, R_{c}, K et le trait ondulé sont définis comme dans la formule (II₄), à l'action d'un agent de déprotection du diol, pour obtenir un composé de formule (XI) : 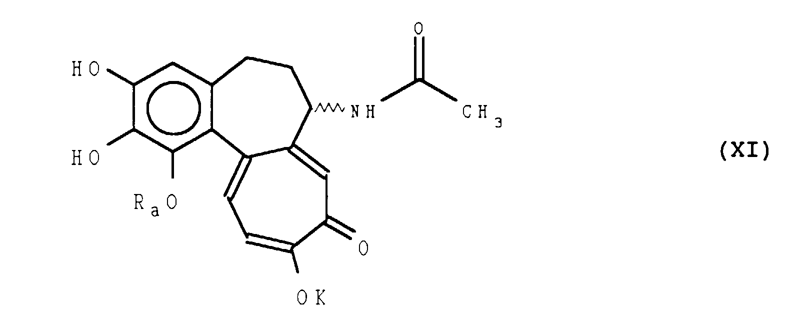 dans laquelle Rₐ, K et le trait ondulé ont la signification précédente, que l'on soumet à l'action d'un agent d'hydrolyse pour obtenir le composé de formule (XII) : 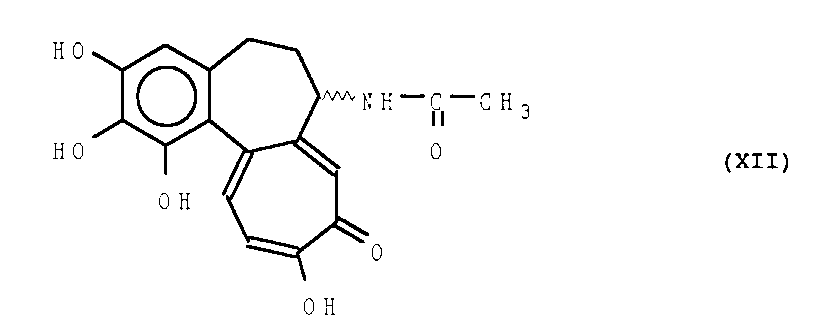 que l'on soumet à un agent de méthylation, pour obtenir la colchicine optiquement active ou racémique.

L'invention a encore pour objet des composés intermédiaires obtenus lors de la mise en oeuvre des procédés et applications ci-dessus. Ces composés sont les suivants :
1) Les composés de formule (F₁) : 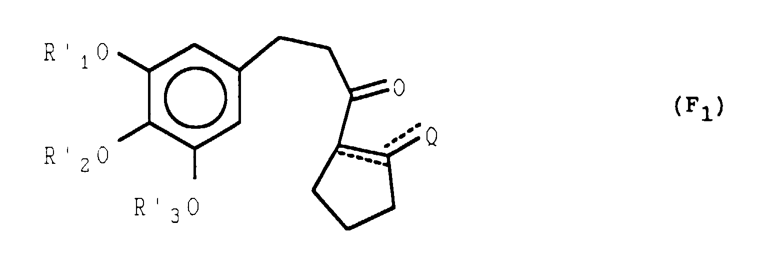 dans laquelle soit Q représente un radical oxo, soit le pointillé dans le cycle représente une seconde liaison et Q représente un atome d'halogène, R'₁, R'₂ et R'₃ ont la signification indiquée plus haut.
2) Les composés de formule (F₂) : 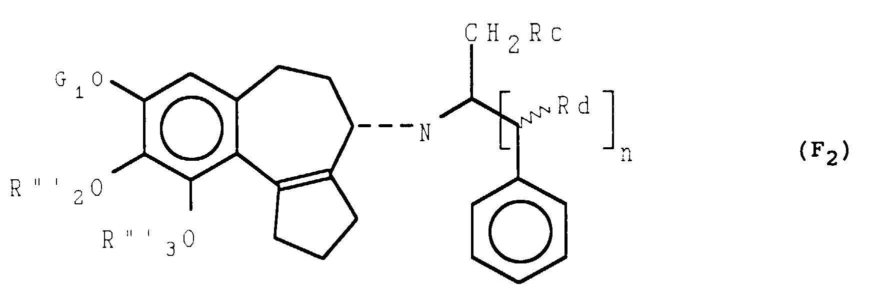 dans laquelle G₁ représente un atome d'hydrogène ou le radical R"'₁ tel que défini ci-dessus et Rd ainsi que R"'₂ et R"'₃ ont la signification indiquée plus haut, le trait pointillé symbolise soit une double liaison, soit une simple liaison et, dans ce cas, l'azote porte un atome d'hydrogène et le composé se présente sous la forme d'un mélange d'isomères (R,S) ou d'isomère (R) ou (S).
3) Les composés de formule (F₃) : 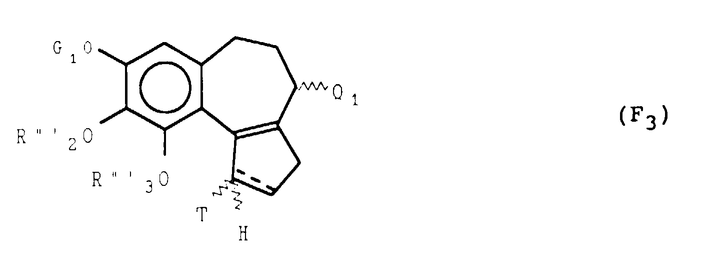 dans laquelle R"'₂ et R"'₃ ont la signification indiquée plus haut, et ou bien G₁ représente le radical R"'₁ tel que défini précédemment et :
   - soit Q₁ représente un radical NH₂ de configuration (R), (S) ou (R,S) et T représente un atome d'hydrogène ;
   - soit Q₁ représente un groupe N=P de configuration (R), (S) ou (R,S), P étant défini comme plus haut, et ou bien T représente un atome d'hydrogène ou un radical hydroxy ou alcoxy renfermant de 1 à 6 atomes de carbone, de configuration (R), (S) ou (R,S), ou bien le trait pointillé représente une seconde liaison ;
   - soit Q₁ représente un groupe = N 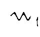 OR₈ dans lequel R₈ a la signification indiquée plus haut et le trait ondulé indique que la liaison peut être syn ou anti ou que le produit peut consister en un mélange d'isomères syn et anti et ou bien T représente un atome d'hydrogène ou un radical hydroxy ou alcoxy renfermant de 1 à 6 atomes de carbone, de configuration (R), (S) ou (R,S), ou bien le trait pointillé représente une seconde liaison ;
   - soit Q₁ représente un radical OH ou OSO₂A, A étant défini comme plus haut, de configuration (R), (S) ou (R,S) et T représente un atome d'hydrogène,
   ou bien G₁ représente un atome d'hydrogène et
   Q₁ représente soit un radical NH₂ de configuration (R), (S) ou (R,S) soit un groupe N=P de configuration (R), (S) ou (R,S), P étant défini comme ci-dessus et T représente un atome d'hydrogène, le pointillé ne représentant pas de double liaison.
4) Les composés de formule (u) : 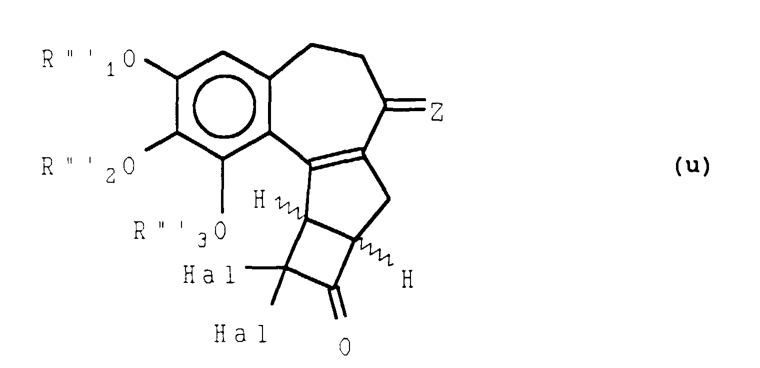 dans laquelle R"'₁, R"'₂, R"'₃, Hal et Z sont tels que définis plus haut.
5) Les composés de formule (F₄) : 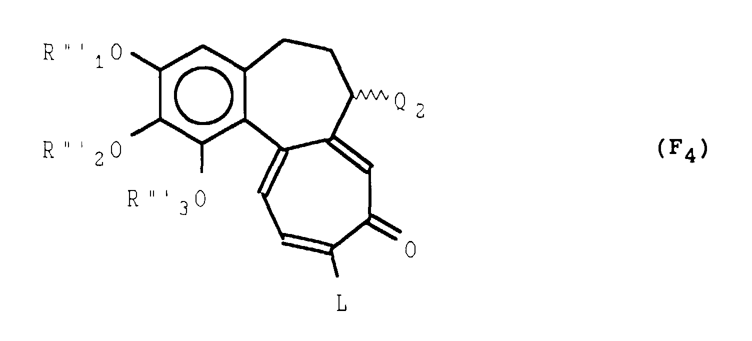 ainsi que leurs tautomères ou régioisomères de formule : 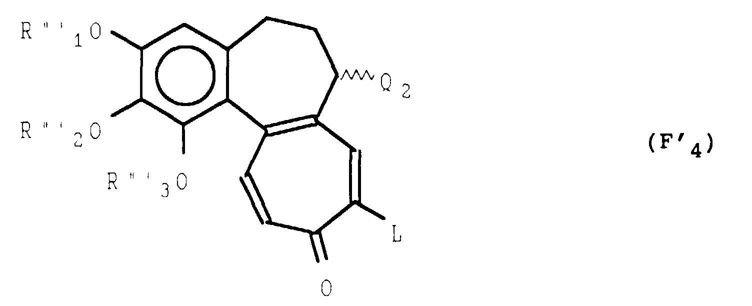 dans laquelle R"'₁, R"'₂ et R"'₃ ont la signification indiquée plus haut et 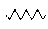 Q₂ représente :
   - soit un groupe = N 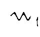 OR₈ tel que défini plus haut et L représente un radical hydroxy,
   - soit un groupe 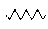 N = P dans lequel P a la signification indiquée plus haut, le trait ondulé indique que ce groupe se trouve dans la configuration (R), (S) ou (R,S) et L représente un radical hydroxy ou -OK tel que défini plus haut, à l'exception des composés dans lesquels R"'₁, R"'₂ et R"'₃ représentent un radical alkyle, L représente un radical hydroxy ou alkoxy et P représente un groupement =C-phényle ou CH₃-C-OC₂H₅ ;
   - soit un groupe 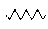 NH₂ de configuration (R), (S) ou (R,S) et L représente un radical hydroxy, à l'exception des composés dans lesquels R"'₁, R"'₂ et R"'₃ représentent un radical alkyle ;
   - soit un groupe 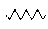 NH₂ configuration (R,S) et L forme avec l'oxygène de la cétone en position 9, un groupe 0-X₁-O⁺ tel que défini plus haut,
   - soit un groupe 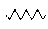 NH₂ de configuration (R), (S) ou (R,S) et L représente un radical OK tel que défini plus haut, à l'exception des composés dans lesquels R"'₁, R"'₂ et R"'₃ représentent un radical alkyle et K représente un radical alkyle ;
   - soit un groupe = N 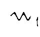 OR₈ tel que défini plus haut et L représente un radical OK tel que défini plus haut.
6) Les composés de formule (F₅) : 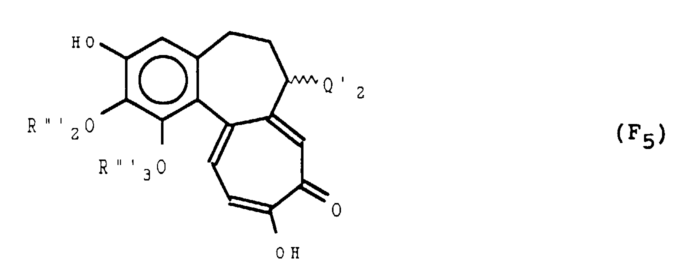 ainsi que leurs tautomères de formule : 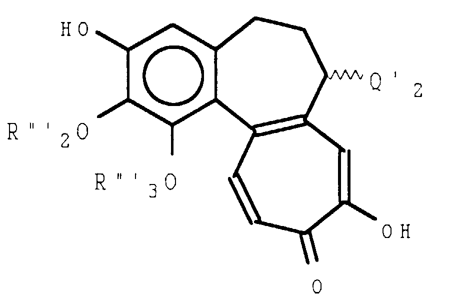 dans laquelle R"'₂ et R"'₃ ont la signification indiquée plus haut, à l'exception de la valeur alkyle et 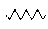 Q'₂ représente :
   - soit un groupe 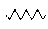 NH-CO-R₁₁ dans lequel R₁₁ a la signification indiquée plus haut,
   - soit un groupe 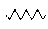 NH₂ de configuration (R), (S) ou (R,S).
7) Les composés de formule (F₆) : 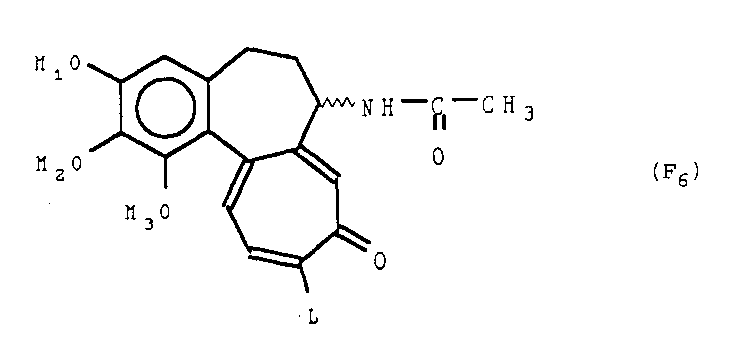 dans laquelle le trait ondulé indique que le substituant se trouve dans la configuration (R), (S) ou (R,S) et
   - soit M₁ et M₂ représentent un atome d'hydrogène, M₃ représente un radical Ra tel que défini plus haut et L représente un radical OK, K étant tel que défini plus haut,
   - soit M₂ et M₃ représentent un atome d'hydrogène, M₁ représente un radical Ra tel que défini ci-dessus et L représente un radical OK tel que défini ci-dessus,
   - soit M₁ représente un radical Ra tel que défini ci-dessus, M₂ et M₃ représentent un radical méthyle et L représente un radical thiométhyle.

Les exemples suivants illustrent l'invention sans toutefois la limiter.

### EXEMPLE 1 : 2,3,5,6-tétrahydro-8,9,10-trihydroxybenz[e]azulèn-4(1H)-one

### Stade A : Acide-3,4,5-triméthoxy-benzènepropanoïque

On ajoute 6,8 g de carbonate de potassium à une solution de 21,44 g d'acide 3,4,5-triméthoxyphénylepropénoïque et 45 ml d'eau puis on hydrogène pendant une heure sous une pression de 1200-1300 mbar en présence de 1,8 g de charbon actif à 10 % de palladium, on absorbe ainsi 2,1 l d'hydrogène. On filtre, lave à l'eau et acidifie avec 50 ml d'acide chlorhydrique (2N). On essore, lave à l'eau et sèche sous pression réduite à température ambiante. On obtient ainsi 19,8 g du produit attendu (P.F. = 102-103°C).
Spectre I.R. (CHCI₃) 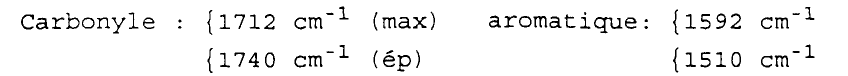 Spectre R.M.N. (CDCl₃)

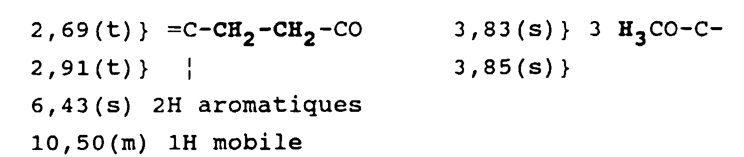

### Stade B : Chlorure de 3,4,5-triméthoxy-benzenepropanoyle

On sèche avec 1,5 g de sulfate de magnésium une solution de 6 g du produit obtenu au stade A dans 21 ml de chlorure de méthylène, après filtration on refroidit à 5°C et ajoute 2,2 ml de chlorure de thionyle puis agite la solution 20 heures à température ambiante. On évapore à sec sous pression réduite en procédant à deux entraînements avec du cyclohexane on recueille ainsi 6,46 g du produit recherché. (P.F. = 60°C)

### Stade C : 2-[3-(3,4,5-triméthoxyphényl)-1-oxopropyl]-cyclopentanone

A une solution refroidie à 5°C de 2,4 ml de 1-(N-morpholinyl)cyclopentène obtenu comme décrit ci-après, 2,31 ml de triéthylamine et 15 ml de chlorure de méthylène on ajoute en 1 heure 30 à +5°C une solution de 4,27 g du produit obtenu au stade B dans 15 ml de chlorure de méthylène. On agite 1 heure à +5°C puis en laissant la température remonter on ajoute 10 ml d'acide chlorhydrique 2 N, agite 1 heure à température ambiante, décante, lave à l'eau puis avec une solution saturée de bicarbonate de sodium, sèche, filtre et évapore à sec sous pression réduite. On obtient 5 g du produit attendu. On purifie le produit brut par dissolution dans 10 volumes d'acétate d'éthyle, extrait avec une solution de soude N, lave la phase alcaline avec de l'acétate d'éthyle, on acidifie à pH 1 avec de l'acide chlorhydrique concentré, extrait avec du chlorure de méthylène, sèche et évapore à sec sous pression réduite. On recueille 2,75 g de produit purifié.
Spectre I.R. (CHCl₃) : 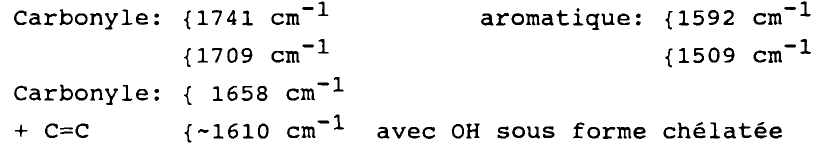 Spectre R.M.N. (CDCl₃)

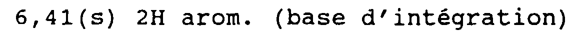

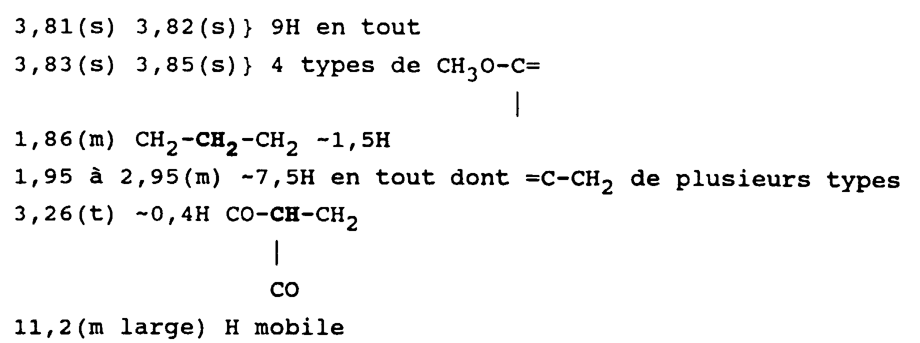

### Préparation de 1-(N-morpholinyl)-cyclopentène utilisé au stade C :

On agite pendant 4 heures 30 au reflux, en éliminant l'eau formée, une solution de 100 ml de cyclohexane, 20 ml de cyclopentanone, 50 ml de morpholine et 100 mg d'acide paratoluène sulfonique. Après évaporation du solvant sous pression réduite, on distille sous 12 - 13 mbar de pression et recueille 27,44 g de produit recherché (Eb. = 83°C).

### Stade D : 1-(2-chloro-1-cyclopenten-1-yl)-3-(3,4,5-triméthoxyphényl)-propan-1-one

A une solution de 23 g de produit obtenu au stade C et 230 ml de chloroforme, on ajoute à température ambiante 13 ml de chlorure d'oxalyle. On agite trois heures à température ambiante, on concentre à pression réduite en procédant à deux entraînements au cyclohexane. On obtient 28 g de produit brut que l'on recristallise dans un mélange de 50 ml de cyclohexane et 50 ml d'éther diisopropylique après concentration partielle. On essore, lave avec de l'éther diisopropylique et sèche sous pression réduite. On obtient 16,24 g du produit attendu. (P.F. = 93°C)
Spectre I.R. (CHCl₃) :
1659 cm⁻¹ : Carbonyle
1599 cm⁻¹
1586 cm⁻¹ : C=C + aromatique
1508 cm⁻¹
Spectre R.M.N. CDCl₃
1,93(m) : CH₂ central
2,69(m)-2,81(m) : C-**CH**_{**2**}**-**C= du cyclopentène
2,85(t,j=7,5) - 3,08(t,j=7,5) : les autres =C-**CH**_{**2**}**-**C
2,44 : **CH**_{**3**}-C=
3,68 - 3,81 : les OCH₃
6,59-6,68 (d,j=2) : les CH= aromatiques couplés méta
7,31-7,80(d,j=8) : les aromatiques.

### Stade E : 2,3,5,6-tétrahydro-8,9,10-triméthoxy-benz[e]azulèn-4(1H)-one

On agite 20 heures à température ambiante 900 mg du produit obtenu au stade D, 9 ml de 1,2-dichloroéthane et 0,9 ml de chlorure stannique. On ajoute ensuite 9 ml d'eau et glace et décante, lave à l'eau, réextrait une fois avec du chlorure de méthylène, sèche sur sulfate de magnésium, filtre et évapore à sec sous pression réduite, pour obtenir 1 g du produit attendu (brut) que l'on purifie par chromatographie sur silice en éluant avec du cyclohexane à 10 % d'acétate d'éthyle, puis à 25 % d'acétate d'éthyle. Après concentration on recueille 700 mg de produit que l'on cristallise dans 5 ml de n-hexane, puis refroidit à 0°C, essore, lave avec le minimum de n-hexane, sèche sous pression réduite à température ambiante pour obtenir 630 mg de produit attendu.
(P. F. = 101-102°C).
Spectre RMN (CDCl₃)

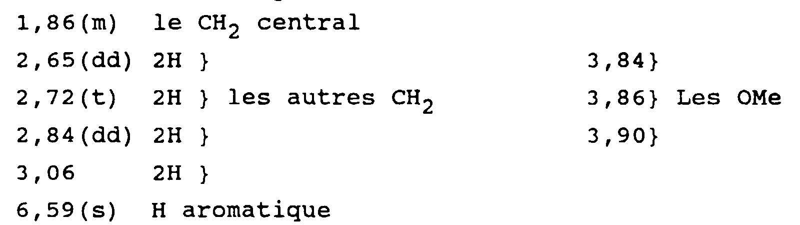

### Stade F : 2,3,5,6-tétrahydro-8,9,10-trihydroxy-benz[e]azulèn-4(1H)-one

En opérant comme au stade F (c) de l'exemple 2 on obtient le produit déméthylé attendu.

### EXEMPLE 2, Synthèse alternative de : 2,3,5,6-tétrahydro-8,9,10-trihydroxy-benz[e]azulèn-4(1H)-one

### Stade A : Acide 3,4-diméthoxy 5-[[(4-méthylphényl)-sulfonyl]oxy]-benzenepropanoïque

On opère comme au stade A de l'exemple 1 en utilisant 29,76 g de l'acide 3,4-diméthoxy-5-[[[(4-méthylphényl)-sulfonyl]oxy]phényl]-cinnamique dont la préparation est donnée ci-après, 43,5 g de carbonate de potassium, 60 ml de méthanol et 1,48 g de charbon actif palladié à 10 %. On obtient ainsi 28,23 g du produit recherché sous forme de cristaux incolores (P. F. = 148-149°C).
Spectre U.V. (EtOH)
Pour M = 380,4
max 226nm ε= 22100
infl 263nm ε= 2000
infl 269nm ε= 2400
max 274nm ε= 2800
infl 279nm ε= 2500
infl 307nm ε= 450
Spectre R.M.N. (CDCl₃)

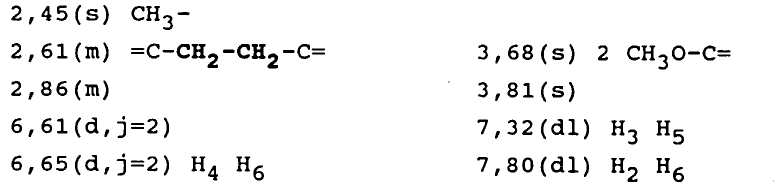

### Stade B : Chlorure de 3,4-diméthoxy 5-[[(4-méthylphényl)-sulfonyl]oxy]-benzenepropanoyle

On opère comme au stade B de l'exemple 1 en utilisant 1,9 g du produit obtenu au stade A, 9,5 ml de chlorure de méthylène et 0,7 ml de chlorure de thionyle. On obtient 2,24 g du produit recherché utilisé tel quel pour le stade suivant.

### Stade C : 2-[3-[3,4-diméthoxy-5-[[(4-méthylphényl)-sulfonyl]oxy]-phényl]-1-oxopropyl]-cyclopentanone

On opère comme au stade C de l'exemple 1 à partir de 2,24 g du chlorure d'acide obtenu au stade B et en utilisant 770 mg de 1-(N-morpholinyl)-cyclopentène (préparé au stade C de l'exemple 1), 6 ml de chlorure de méthylène et 0,77 ml de triéthylamine. Après recristallisation dans l'éther diisopropylique on obtient 1,27 g du produit recherché (P. F. = 84°C).
Spectre I.R. (CHCl₃) 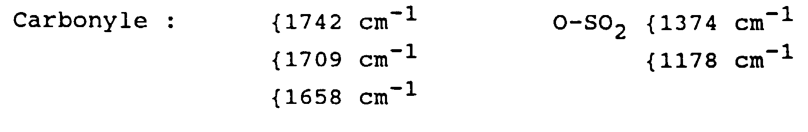 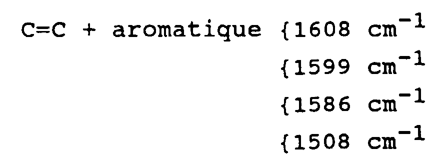 Spectre R.M.N (CDCl₃) 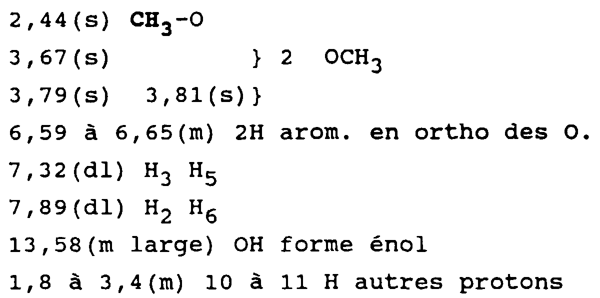 Spectre U.V.
**1** - EtOH (+ dioxane) pour M = 446,52
max 225 nm ε= 23000
max 282nm ε= 7900
infl 270, 277, 290, 300 ,313 nm
**2** - EtOH (NaOH 0,1N)
max 310 nm ε= 21600
infl 268, 272, 276 nm

### Stade D : 1-(2-chloro-1-cyclopenten-1-yl)-3-[3,4-diméthoxy-5-[[(4-méthylphényl)sulfonyl]oxy]-phényl]-propan-1-one

On opère comme au stade D de l'exemple 1 en utilisant 8,7 g du produit obtenu au stade C, 70 ml de chloroforme et 3,5 ml de chlorure d'oxalyle. Après cristallisation dans l'éther diisopropylique on obtient 7,75 g du produit recherché (P. F. = 73°C). Ce produit est utilisé tel quel pour le stade suivant.

Un échantillon analytique a été obtenu par recristallisation dans 2,5 volumes de chlorure de méthylène et 5 volumes d'éther diisopropylique suivie par concentration à 3 volumes, essorage, lavage à l'éther diisopropylique et séchage sous pression réduite à température ambiante (P.F. = 77 - 78°C).
Spectre I.R. (CHCl₃) 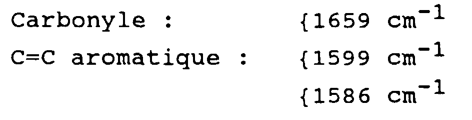 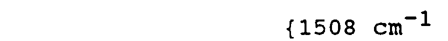 Spectre U.V. (EtOH)
max 227nm ε= 26100
infl 248nm ε= 12800
infl 272nm ε= 5300
infl 280nm ε= 3200
infl 320nm
Spectre R.M.N. (CDCl₃)

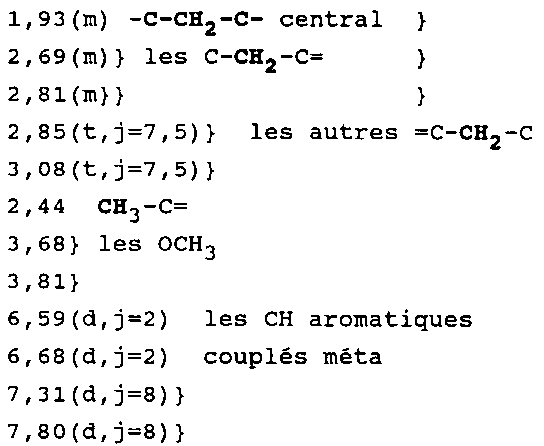

### Stade E : 8,9-diméthoxy-10-[[(4-méthylphényl)sulfonyl]oxy]-2,3,5,6-tétrahydro-benz[e]azulèn-4(1H)-one

A une solution de 2,32 g du produit obtenu au stade C dans 50 ml de 1,2-dichloroéthane on ajoute à température ambiante 1,65 g de chlorure ferrique à 98 %. On agite 48 heures à température ambiante puis coule sur un mélange d'eau et glace, agite énergiquement pendant 15 mn et extrait avec du chlorure de méthylène, lave à l'eau, puis avec une solution aqueuse saturée de chlorure de sodium. Après sèchage et évaporation à sec sous pression réduite on obtient 2,15 g du produit brut que l'on chromatographie en éluant avec du cyclohexane à 50 % d'acétate d'éthyle, on recueille 1,8 g de produit que l'on chromatographie à nouveau et recristallise dans le mélange chloroforme/éther diisopropylique pour obtenir 720 mg du produit recherché (P.F. = 138°C).
Spectre I.R. (CHCl₃) 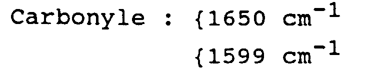 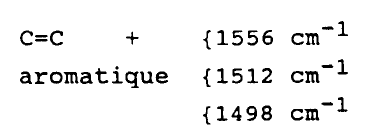 Spectre U.V. (EtOH)
max 230nm ε= 25300
infl 254nm ε= 9400
max 323nm ε= 10300
Spectre R.M.N. (CDCl₃)

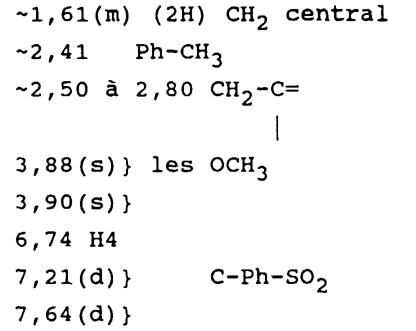

### Stade F (a) : 8,9-diméthoxy-10-hydroxy-2,3,5,6-tétrahydrobenz[e]azulèn-4(1H)-one

On chauffe au reflux pendant 2 heures un mélange de 350 g du produit obtenu au stade E ci-dessus, 1750 ml de méthanol, 350 ml d'eau déminéralisée et 350 ml de lessive de soude pure (concentrée). On refroidit le milieu réactionnel vers 2°C ± 2°C et introduit en 45 minutes 467 ml d'acide chlorhydrique concentré en maintenant la température à 2°C ± 2°C. On ajoute alors 1645 ml d'eau déminéralisée en 10 mn et en maintenant la température à 2°C ± 2°C, puis le milieu réactionnel est agité 30 minutes toujours à 2°C ± 2°C. On essore les cristaux formés, lave par clairçages à 5 reprises avec à chaque fois 700 ml d'eau déminéralisée à 20°C puis sèche à 40°C sous pression réduite pour obtenir 199,1 g du produit recherché.

### Stade F (b) : 2,3,5,6-tétrahydro-8,9,10-triméthoxybenz[e]azulèn-4(1H)-one

On agite pendant 2 heures 30 à 20°C, 60 g du produit obtenu en (a), 600 ml de 1,2-dichloroéthane, 342 ml de soude 2N, 1,2 g de bromure de tétrabutylammonium et 33 ml du sulfate diméthylique. On introduit alors, 39 ml de triéthylamine afin de détruire l'excès de sulfate diméthylique et agite une heure à 20°C ± 2°C. On ajoute 342 ml d'eau déminéralisée, agite 15 minutes à 20°C ± 2°C, décante, réextrait la phase aqueuse deux fois avec à chaque fois 120 ml de 1,2-dichloroéthane. Les phases 1,2-dichloroéthane sont réunies et lavées par 4 x 240 ml d'eau déminéralisée, puis par 1 x 300 ml d'acide chlorhydrique N, puis par 3 x 240 ml d'eau déminéralisée (jusqu'à la neutralité). Les phases organiques réunies sont séchées sur sulfate de sodium, filtrées et concentrées à pression ordinaire à 83°C jusqu'à un volume résiduel de 480 ml.

### Stade F (c) : 2,3,5,6-tétrahydro-8,9,10-trihydroxybenz[e]azulèn-4(1H)-one

On chauffe au reflux, pendant une heure, 480 ml de la solution obtenue en (b) avec 102,3 g de chlorure d'aluminium anhydre. On refroidit le milieu à 0°C ± 2°C puis ajoute en deux heures un mélange de 600 ml d'eau déminéralisée et 192 ml d'acide sulfurique pur (concentré) préalablement refroidi vers 0°C en maintenant la température du milieu réactionnel inférieure à 20°C. On introduit, en 5 minutes à 20°C ± 2°C, 300 ml d'eau déminéralisée et agite 16 heures à 20°C ± 2°C, essore, lave deux fois avec à chaque fois 60 ml de 1,2-dichloroéthane, puis à l'eau déminéralisée, sèche sous pression réduite et obtient 52,2 g du produit recherché.

### Préparation de l'Acide 3,4-diméthoxy 5-[[(4-méthylphényl)-sulfonyl]oxy]-cinnamique utilisé au départ de l'exemple 2.

### Stade A : 3,4-diméthoxy-5-[[(4-méthylphényl)-sulfonyl]oxy]-benzoate de méthyle.

On ajoute en 10 minutes à température ambiante 303 ml de triéthylamine à un mélange agité de 200 g de gallate de méthyle et 2 litres de chlorure de méthylène. Après dissolution on refroidit à 0-5°C puis ajoute en 1 heure à cette température 130 ml de dichlorodiméthylsilane, agite encore 30 minutes à cette température. En maintenant la température à 0-5°C on ajoute en 25 minutes 303,2 ml de triéthylamine puis en 15 minutes 227,6 g de chlorure de tosyle. On agite encore une heure à 0-5°C, et ajoute en 10 minutes sous agitation et en laissant évoluer la température jusqu'à 20-22°C, 200 ml d'acide acétique, puis 500 ml d'eau déminéralisée, on agite encore 15 minutes à 20°C. On distille le chlorure de méthylène à volume constant (3,3 l) sous pression réduite en remplaçant par de l'eau déminéralisée, on agite 2 heures à 20°C, essore, lave avec de l'eau déminéralisée pour obtenir 523 g (poids humide) de 3,4-dihydroxy-5-[[(4-méthylphényl)-sulfonyl]oxy]-benzoate de méthyle (3-tosylgallate de méthyle). Le produit humide obtenu est repris par 2,17 l de soude (2N) et 2,17 l de chlorure de méthylène. On agite à 20°C jusqu'à dissolution puis ajoute à 20°C, 18 g de bromure de tétrabutylammonium puis, en 15 minutes à 20°C, 237 ml de sulfate diméthylique. Le milieu réactionnel est agité 1,5 heure à 20-22°C. On ajoute, à 20-22°C, 78 ml de triéthylamine et agite une nuit à 20-22°C, puis décante et lave avec 400 ml d'eau déminéralisée et ajoute 20 ml d'acide acétique pur à la phase organique agite 15 minutes, ajoute 400 ml d'eau déminéralisée, puis décante. On concentre à sec les phases organiques réunies, d'abord à pression atmosphérique puis sous pression réduite à 40 mm Hg et 60°C extérieur. On entraîne avec 400 ml de méthanol puis, reprend l'extrait sec obtenu avec 600 ml de méthanol chauffe au reflux jusqu'à dissolution totale du produit, puis refroidit à 0-5°C agite une heure à cette température. On essore et lave par deux fois avec 200 ml de méthanol à -10°C et sèche à 40°C sous pression réduite on recueille ainsi 330,4 g de : 3,4-diméthoxy-5-[[(4-méthylphényl)-sulfonyl]oxy]-benzoate de méthyle. Le produit brut est purifié par recristallisation dans 330 ml de toluène. Après 2 heures d'agitation à -10°C on essore, lave par deux fois 82 ml de toluène refroidi à -15°C et sèche sous pression réduite à 40°C pour obtenir 230,3 g du produit purifié recherché.

### Stade B : acide 3,4-diméthoxy 5-[[(4-méthylphényl)-sulfonyl]oxy]-cinnamique

**a)** On refroidit à 0°C, 600 ml de toluène et ajoute 202 ml d'une solution de Vitride® à 70 % dans le toluène à 0°C et ajoute en une heure 67,6 ml de morpholine à 0-2°C, on laisse remonter la température jusqu'à 18°C. On utilise la solution ainsi obtenue immédiatement pour l'étape suivante.
**b)** On agite 10 minutes à 20-22°C, 200 g de 3,4-diméthoxy-5-[[(4-méthylphényl)-sulfonyl]oxy]-benzoate de méthyle obtenu au stade A et 1400 ml de toluène jusqu'à dissolution totale. On ajoute en une heure à 10°C la solution du réactif obtenue ci-dessus. On agite encore une heure en laissant la température remonter à 18°C.
   On introduit en une heure, à 10°C, une solution refroidie à 10°C de 200 ml d'acide sulfurique concentré et 1000 ml d'eau déminéralisée. On agite 16 heures à 20°C puis décante la phase organique, lave par 5 x 200 ml d'eau déminéralisée, sèche, filtre et lave par 3 x 100 ml de chlorure de méthylène. La solution d'aldéhyde intermédiaire ainsi obtenue est utilisée telle quelle à l'étape suivante.
**c)** On chauffe 16 heures à 70°C ± 2°C (en éliminant, à pression ordinaire, le chlorure de méthylène) la solution d'aldéhyde intermédiaire obtenue ci-dessus, 200 ml de 2-picoline, 120 g d'acide malonique et 20 ml de pipéridine.
   On refroidit à 20-22°C, et en maintenant cette température on ajoute en 15 minutes une solution de 200 ml d'acide chlorhydrique concentré et 400 ml d'eau déminéralisée. On agite 2 heures à 20-22°C, puis refroidit à 0°C, essore les cristaux formés, lave à l'eau déminéralisée, sèche sous pression réduite à 40°C pour obtenir 171,7 g de l'acide 3,4-diméthoxy 5-[[(4-méthylphényl)-sulfonyl]oxy}phényl]-cinnamique attendu.

### EXEMPLE 3 : 9,10-diméthoxy-8-[[(4-méthylphényl)sulfonyl]oxy]-2,3,5,6-tétrahydro-benz[e]azulèn-4-(1H)-one (a) : 9,10-dihydroxy-8-[[(4-méthylphényl)sulfonyl]oxy]-2,3,5,6-tétrahydro-benz[e]azulèn-4(1H)-one

On agite pendant 1 heure 30 à 20°C ± 2°C, 30 g de 2,3,5,6-tétrahydro-8,9,10-trihydroxy-benz[e]azulèn-4(1H)-one obtenu selon l'exemple 1 ou 2, 300 ml de tétrahydrofuranne, 60 ml de triéthylamine et 12,9 ml de triméthylborate. On ajoute 30 g de chlorure de tosyle et agite 16 heures à 20°C ± 2°C puis en 10 minutes à 20°C ± 2°C verse le milieu réactionnel sur un mélange agité de 900 ml d'eau déminéralisée et 150 ml d'acide chlorhydrique concentré, puis on ajoute 90 ml de tétrahydrofuranne et 60 ml de chlorure de méthylène. On agite la solution obtenue une heure à 20°C, puis introduit 150 ml de chlorure de méthylène et agite encore 15 minutes, décante et réextrait par 2 x 75 ml de chlorure de méthylène. Les phases organiques réunies sont lavées avec 4 x 150 ml d'eau déminéralisée et réextraites par 75 ml de chlorure de méthylène, après concentration sous pression réduite de 20 mbars jusqu'à refus de distillation à 50°C pour obtenir 47,6 g du produit recherché.

### (b) : 9,10-diméthoxy-8-[[(4-méthylphényl)sulfonyl]oxy]-2,3,5,6-tétrahydro-benz[e]azulèn-4(1H)-one

On agite 16 heures à 20°C, 47,6 g du produit obtenu ci-dessus en (a), 300 ml de chlorure de méthylène, 300 ml de soude (2N), 0,6 g du bromure de tétrabutylammonium et 30 ml de sulfate diméthylique. On introduit alors 30 ml de triéthylamine afin de détruire l'excès de sulfate diméthylique, le milieu réactionnel est agité encore une heure à 20°C ± 2°C, puis ajoute 150 ml d'eau déminéralisée agite encore 15 minutes puis décante. La phase aqueuse était réextraite avec 2 x 75 ml de chlorure de méthylène et les phases organiques réunies sont lavées par 3 x 120 ml d'eau déminéralisée puis 120 ml d'acide chlorhydrique N et 3 x 120 ml d'eau déminéralisée, les phases organiques sont réunies et séchées sur sulfate de sodium, puis on ajoute en 1 heure 120 g de gel de silice (60 Mesh) à 20°C ± 2°C sous agitation et agite encore une heure à 20°C, filtre, lave avec du chlorure de méthylène et concentre à sec sous pression réduite à 50°C pour obtenir 47,4 g du produit recherché.
On purifie le produit brut par recristallisation dans 390 ml d'éthanol après distillation de 90 ml d'éthanol, on agite 3 heures à 0°C ± 2°C. On essore, lave avec 30 ml d'éthanol à 0°C, puis sèche sous pression réduite à 40°C pour obtenir 41,1 g du produit recherché (P.F. = 129°C).

### Exemples 4-7 : Synthèse Asymétrique de "Thiosubstance C"

### EXEMPLE 4 : (S) 2-[9,10-diméthoxy-8-[[(4-méthylphényl)sulfonyl]oxy]-1,2,3,4,5,6-hexahydro-benz[e]azulèn-4-yl]-1H-isoindole-1,3(2H)-dione

### Stade A : 4-méthylbenzenesulfonate de S (R*,R*) 9,10-diméthoxy-1,2,3,4,5,6-hexahydro-4-[(1-phényléthyl)-amino]benz[e]azulèn-8-ol, éthanedïoate

A une solution de 6,8 ml de S(-)-alpha méthyle benzylamine dans 45 ml de tétrahydrofuranne, on ajoute en 30 minutes à 5-9°C, 26,25 ml de chlorure de titane en solution 1M dans le 1,2-dichloroéthane agite 5 minutes puis introduit en une heure 9 g du produit de l'exemple 3 en solution dans 45 ml de 1,2-dichloroéthane et 14,7 ml de triéthylamine on agite 30 minutes vers 10°C puis 1 heure 30 en laissant la température évoluer. On refroidit vers 10°C et ajoute en 3 minutes 9 ml de méthanol puis 2,52 g d'hydroborure de sodium en 40 minutes, on agite une heure à 10°C puis 16 heures à température ambiante. 840 mg d'hydroborure de sodium sont ajoutés et le milieu réactionnel agité encore 45 minutes, refroidi vers 3°C et hydrolysé par addition de 84 ml de soude N, après 10 minutes à 10°C on filtre, lave par 90 ml de chlorure de méthylène, décante, lave avec 40 ml d'eau et réextrait par 40 ml de chlorure de méthylène, sèche sur sulfate de sodium, filtre et évapore à sec sous pression réduite pour obtenir 18,3 g de produit brut.

### Première Purification

On dissout le produit brut dans 33 ml de méthanol et 110 ml d'éther diisopropylique, puis 225 ml d'acide phosphorique N, agite 5 minutes et décante, réextrait par un mélange de 84 ml d'acide phosphorique N et 16 ml de méthanol puis 30 ml d'eau, lave avec 90 ml d'éther diisopropylique. Les phases aqueuses acides sont réunies et alcalinisées par 22,5 g de bicarbonate de sodium en présence de 100 ml d'acétate d'éthyle, on lave à l'eau, sèche sur sulfate de sodium, filtre et évapore à sec sous pression réduite pour obtenir 9,24 g du produit sous forme de résine.
[α]_{D}= -124° à 0,63 % dans Méthanol). Le rapport des diastéréoisomères S:R en position 4 (S étant l'isomère recherché) est estimé à 85/15 d'après le rapport de surface des pics à 6,63 et 6,72 ppm du spectre R.M.N. (correspondant aux protons H₄).

### Deuxième Purification

On ajoute en 20 minutes sous agitation, 7,15 g du produit obtenu ci-dessus dans une solution de 1,69 g d'acide oxalique (2H₂O) 6,75 ml de méthanol. On agite encore 30 minutes, essore, lave à l'éther à 5 % de méthanol, sèche sous pression réduite à température ambiante pour obtenir 6,12 g de l'oxalate du produit attendu.
(P.F. = 172°C, [α]_{D}= -170° à 0,44 % dans le méthanol)

### Stade B : 4-méthylbenzènesulfonate de (S) 4-amino-9,10-diméthoxy-1,2,3,4,5,6-hexahydro-benz[e]azulèn-8-ol

On agite pendant 5 heures à température ambiante sous atmosphère d'hydrogène (400 mbar), 800 mg du produit obtenu au stade A, 32 ml d'un mélange méthanol-acide acétique 2-1 et 240 mg d'hydroxyde de palladium à 20 % sur charbon actif humide (50 % d'eau). On filtre et lave avec du méthanol, puis concentre à sec sous pression réduite à 40°C. L'huile obtenue est reprise avec 15 ml d'acétate d'éthyle et 15 ml d'une solution aqueuse saturée de bicarbonate de sodium, décante, lave, sèche sur sulfate de sodium filtre et évapore à sec sous pression réduite pour obtenir 590 mg de produit que l'on chromatographie sur silice en éluant avec CH₂Cl₂ à 10 % de méthanol. On receuille ainsi 330 mg du produit recherché. [α]_{D}= -133° (à 0,45 % dans le méthanol).
Spectre U.V. Dans l'EtOH
max 223nm ε= 30500
max 263nm ε= 12400
Spectre R.M.N. (CDCl₃)

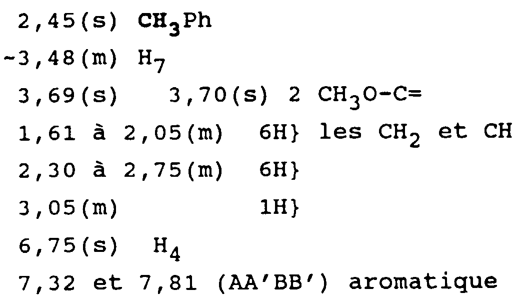

### Stade C : (S) 2-[9,10-diméthoxy-8-[[(4-méthylphényl)sulfonyl]oxy]-1,2,3,4,5,6-hexahydro-benz[e]azulèn-4-yl]-1H-isoindole-1,3(2H)-dione

A une solution de 4,3 g de produit obtenu comme au stade B dans 43 ml de toluène, on ajoute 2,63 g d'anhydride phtalique et 1,4 ml de triéthylamine puis on chauffe pendant huit heures à reflux en éliminant l'eau formée. On refroidit à 10°C et ajoute 25 ml d'eau et 7 ml d'acide chlorhydrique (2N), décante et réextrait une fois avec de l'acétate d'éthyle, lave avec 10 ml de soude (N) puis avec une solution aqueuse saturée de chlorure de sodium, sèche sur sulfate de magnésium, filtre et évapore à sec sous pression réduite pour obtenir 6,4 g du produit brut, que l'on dissout dans 6,5 ml de chlorure de méthylène puis on introduit en 15 minutes 6,4 g d'oxyde d'aluminium après 5 minutes d'agitation on filtre, lave au chlorure de méthylène et concentre à sec sous pression réduite, entraîne au méthanol, évapore à sec. Le résidu est recristallisé dans 25 ml de méthanol, on essore, lave avec 2 x 4 ml de méthanol et sèche sous pression réduite à 40°C pour obtenir 4,42 g du produit recherché.
(P.F. = ~139°C)
[α]_{D}= -66° (c = 0,6 % dans CHCl₃ ; Rendement = 79 %).

### Exemple 5 : Mélange tautomérique de : (S) 2-[1,2-diméthoxy-10-hydroxy-3-[[(4-méthylphényl)sulfonyl]oxy]-9-oxo-5,6,7,9 -tétrahydro-benzo[a]heptalèn-7-yl]-1H-isoindole-1,3(2H)-dione (Forme "Normale") et

### (S) 2-[1,2-diméthoxy-9-hydroxy-3-[[(4-méthylphényl)sulfonyl] oxy]-10-oxo-5,6,7,10-tétrahydro-benzo[a]heptalèn-7-yl]-1H-isoindole-1,3(2H)-dione (forme "iso")

### Stade A : (4S) 2-[1,2,3,4,5,6-hexahydro-8-[[(4-méthylphényl)sulfonyl]oxy]-1,9,10-triméthoxy-benz[e]azulèn-4-yl]-1H-isoindole-1,3(2H)-dione: Isomères (1R) et (1S)

A une solution refroidie à 5°C, de 7,7 g du produit obtenu à l'exemple 4 dans 77 ml de chlorure de méthylène et 77 ml de méthanol on ajoute 4,45 ml de pyridine, puis en une fois 5,28 g de bromhydrate de perbromure de pyridinium, on agite le milieu réactionnel trois heures à 5°C. On verse dans l'eau glacée et acidifie par 55 ml d'acide chlorhydrique (N), extrait au chlorure de méthylène, lave à l'eau contenant un peu de thiosulfate de sodium (pour éliminer l'excès d'oxydant), puis à l'eau, sèche sur sulfate de sodium, filtre et concentre à sec sous pression réduite pour obtenir 8,6 g du produit brut que l'on chromatographie sur silice en éluant avec du chlorure de méthylène à 5 % d'acétate d'éthyle on recueille ainsi 4,38 g du diastéréoisomère A ([α]D = -76° à 0,5 % dans CHCl₃) et 666 mg du diastéréoisomère B (P.F. 168°C et [α]_{D} = -37° à 0,7 % dans CHCl₃) après cristallisation chlorure de méthylène/éther diisopropylique.

### Diastéréoisomère A (produit majoritaire)

Spectre R.M.N. (CDCl₃)

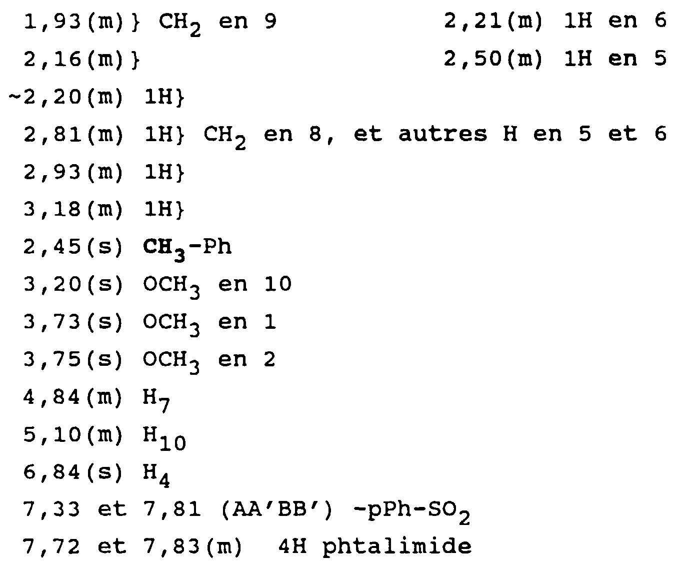

### Diastéréoisomere B (produit minoritaire)

Spectre R.M.N. (CDCl₃) 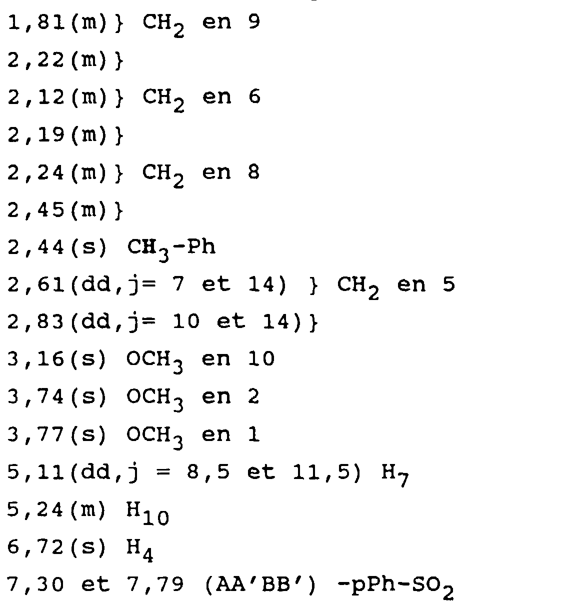 7,69 et 7,78(m) 4H phtalimide

### Stade B : (S) 2-[9,10-diméthoxy-8-[[(4-méthylphényl)sulfonyl]oxy]-3,4,5,6-tétrahydro-benz[e]azulèn-4yl]-1H-isoindole-1,3(2H)-dione

On agite trois heures à température ambiante, 4,1 g produit (diastéréoisomère A) obtenu au stade A ci-dessus, 82 ml de chlorure de méthylène et 205 mg d'acide paratoluène sulfonique. On verse dans l'eau glacée, extrait avec du chlorure de méthylène, lave à l'eau, sèche sur sulfate de sodium, filtre et concentre à sec sous pression réduite pour obtenir 4 g de produit que l'on chromatographie sur silice éluant cyclohexane/acétate d'éthyle 2-1. On obtient 3,34 g du composé attendu.
Spectre I.R. (CHCl₃) 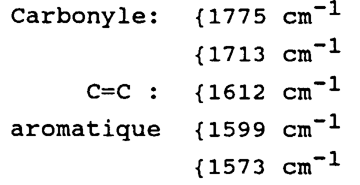 Spectre U.V. (EtOH)
max 221nm ε= 71000
max 288nm ε= 9900
infl 233, 240, 270, 277nm
Spectre R.M.N.(CDCl₃)

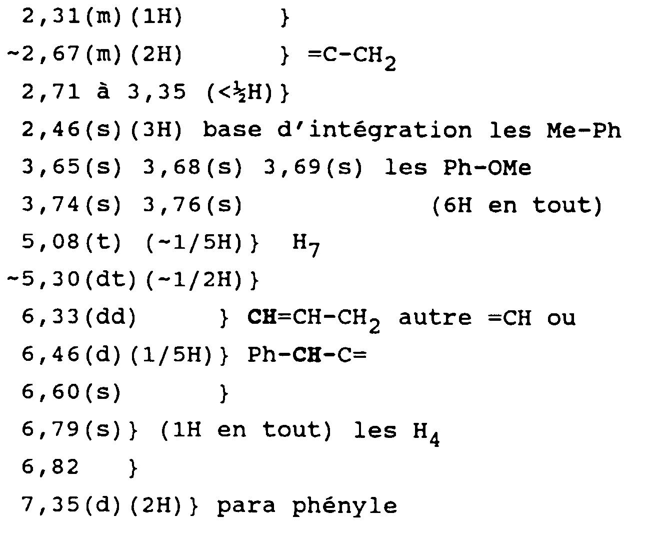

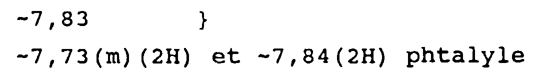

### Stade C : Mélange de : (7S) 2-[10,10-dichloro-1,2-diméthoxy-3-[[(4-méthylphényl)sulfonyl ]oxy]-5,6,7,8,8a,9,10,10a-octahydro-9-oxo-benzo[h]cyclobuta[a]azulèn-7-yl]-1H-isoindole-1,3(2H)-dione et son diastéréoisomère au niveau de la condensation cyclobuta[a]azulène (Diastéréoisomères A et B)

A une solution de 2,27 g du produit obtenu au stade B ci-dessus dans un mélange de 36 ml de chlorure de méthylène et 18 ml de n-pentane, on ajoute rapidement à 5°C, 2,32 ml de chlorure de dichloroacétyle, puis en cinq minutes 3,4 ml de triéthylamine. On agite le milieu réactionnel trois heures à 20°C, puis verse dans l'eau et extrait avec du chlorure de méthylène, lave à l'eau, sèche sur sulfate de sodium, filtre et évapore à sec sous pression réduite pour obtenir 4,7 g de produit que l'on chromatographie sur silice en éluant avec cyclohexane/acétate d'éthyle (2-1). On recueille ainsi 2,3 g d'un mélange de deux diastéréoisomères que l'on chromatographie à nouveau sur silice en éluant avec cyclohexane/acétate d'éthyle (75/25). On obtient ainsi, 500 mg de l'isomère A, 942 mg de l'isomère B et 416 mg du mélange des deux isomères.

### Diastéréoisomère A

[α]_{D}= -75° (0,5 % dans CHCl₃)
Spectre I.R. (CHCl₃)

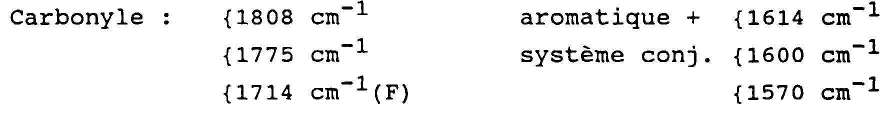

### Diastéréoisomère B

[αl_{D}= -86° (0,5 % dans CHCl₃)
Spectre I.R. (CHCl₃) 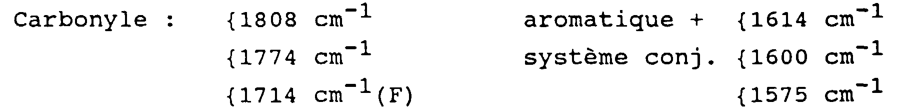 Spectre R.M.N. (CDCl₃)

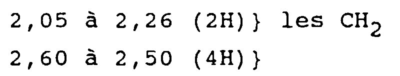

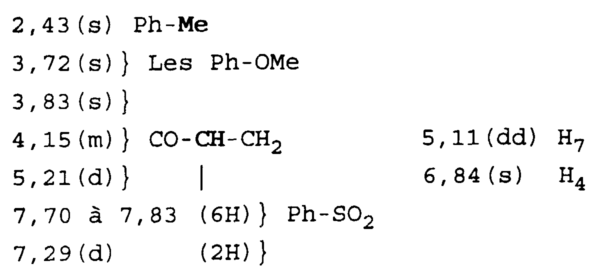

### Stade D : Mélange tautomérique de : (S) 2-[1,2-diméthoxy-10-hydroxy-3-[[(4-méthylphényl)sulfonyl]oxy] -9-oxo-5,6,7,9-tétrahydro-benzo[a]heptalèn-7-yl]-1H-isoindole-1,3(2H)-dione (forme "normal") et

### (S) 2-[1,2-diméthoxy-9-hydroxy-3-[[(4-méthylphényl)sulfonyl] oxy]-10-oxo-5,6,7,10-tétrahydro-benzo[a]heptalèn-7-yl]-1H-isoindole-1,3(2H)-dione (forme "iso")

On agite 72 heures à température ambiante, 260 mg du diastéréoisomère A obtenu au stade C ci-dessus, 13 ml d'acétone à 20 % d'eau, 0,75 ml d'acide acétique glacial et 1,8 ml de triéthylamine. On verse le mélange réactionnel sur un mélange d'eau et glace, extrait avec du chlorure de méthylène, lave à l'eau, sèche sur sulfate de sodium, filtre et concentre à sec sous pression réduite pour obtenir 244 mg du produit recherché.

### EXEMPLE 6 : (S) N-[1,2-diméthoxy-3,10-bis[[(4-méthylphényl) sulfonyl]oxy]-9-oxo-5,6,7,9-tétrahydro-benzo[a]heptalèn-7-yl]-acétamide (forme "normale") et

### (S) N-[1,2-diméthoxy-3,9-bis[[(4-méthylphényl)sulfonyl]oxy]-10-oxo-5,6,7,10-tétrahydro-benzo[a]heptalèn-7-yl]-acétamide (forme "iso").

### Stade A : (S) 7-amino-1,2-diméthoxy-3-[[(4-méthylphényl)sulfonyl]oxy]-10-hydroxy-6,7-dihydro-benzo[a]heptalèn-9(5H)one (forme "normale") + tautomère "iso"

On agite pendant 5 jours au reflux 614 mg du produit obtenu au stade D de l'exemple 5 en solution dans un mélange de 10 ml de chloroforme, 10 ml de méthanol et 55 µl d'hydrate d'hydrazine. On évapore presque à sec, reprend avec du chlorure de méthylène, filtre et concentre le filtrat à sec sous pression réduite. On obtient 600 mg du produit recherché. Stade B : (S) N-[1,2-diméthoxy-10-hydroxy-3-[[(4-méthylphényl)sulfonyl]oxy]-9-oxo-5,6,7,9-tétrahydro-benzo[a]heptalèn-7-yl]-acétamide (Forme "Normale") + Tautomère "Iso"

On dissout 600 mg du produit obtenu au stade A ci-dessus dans un mélange de 2,4 ml d'anhydride acétique et 3,4 ml de pyridine puis le milieu réactionnel est agité 16 heures à 20°C. On ajoute de l'eau et agite 15 minutes, puis verse sur de l'acide chlorhydrique N et extrait avec du chlorure de méthylène, lave à l'eau, sèche sur sulfate de sodium, filtre et évapore à sec sous pression réduite pour obtenir 600 mg du produit recherché.

### Stade C : (S) N-[1,2-diméthoxy-3,10-bis[[(4-méthylphényl) sulfonyl]oxy]-9-oxo-5,6,7,9-tétrahydro-benzo[a]heptalèn-7-yl]-acétamide (forme "normal") et

### (S) N-[1,2-diméthoxy-3,9-bis[[(4-méthylphényl)sulfonyl]oxy]-10-oxo-5,6,7,10-tétrahydro-benzo[a]heptalèn-7-yl]-acétamide (forme "Iso")

On agite pendant 2 heures 30 un mélange de 1,5 g de produit obtenu au stade B ci-dessus, 570 mg de chlorure de tosyle, 455 mg de bicarbonate de sodium, 15 ml de chlorure de méthylène, 15 ml d'eau et 75 mg d'hydrogénosulfate de tétrabutylammonium. On décante lave à l'eau, sèche sur sulfate de sodium, filtre et évapore à sec sous pression réduite pour obtenir 2,01 g de produit tosylé, dont les formes tautomériques (formes "normale" et "iso") sont fixées par cette acylation. Ce produit, qui consiste en un mélange des deux isomères tosylés, est recristallisé par dissolution dans 6 ml d'acétate d'éthyle et cristallisation par ajout de 6 ml d'éther diisopropylique, on essore et lave avec un mélange acétate d'éthyle - éther diisopropylique (1/1) après sèchage sous pression réduite à température ambiante on obtient 1,76 g du produit attendu (mélange d'isomères rapport des formes "normale"/"iso" : 68/32).

### Séparation des isomères par chromatographie :

On chromatographie sur silice 1,75 g du mélange des deux isomères obtenus ci-dessus en éluant avec du chlorure de méthylène à 15 % d'acétone. Les fractions contenant l'isomère "iso" sont concentrées à sec pour obtenir 515 mg de produit que l'on met en suspension dans 6 ml de mélange éther diisopropylique/acétate d'éthyle (2:1), on essore, lave à l'éther diisopropylique, sèche sous pression réduite à température ambiante pour obtenir 499 mg du produit "Iso", sous forme de cristaux incolores
(Déc.~ 205°C).
[α]_{D} = -171° (à 0,28 % dans CHCl₃).

Les fractions contenant l'isomère "Normal" sont concentrées à sec pour obtenir 1,11 g de produit que l'on cristallise dans 12 ml d'éther diisopropylique/acétate d'éthyle (2:1), essore, lave à l'éther diisopropylique et sèche sous pression réduite à température ambiante pour obtenir 1,07 g du produit "normal" (Déc. 197°C).
[α]_{D} = -147° (à 0,39 % dans CHCl₃).

### Isolement de l'isomère " normal" par traitement alcalin :

L'isomère "normal" recherché peut être obtenu par le traitement alcalin du mélange des deux tautomères tosylés "normal"/"iso", qui provoque la dégradation spécifique du produit "Iso" non-recherché.

On agite pendant 1 heure 30, 3 g du mélange des deux isomères tosylés obtenus au stade C ci-dessus, en suspension dans 15 ml de méthanol et 4,4 ml de lithine 2N. On essore, lave par 2 x 7,5 ml puis 1 x 5 ml d'eau, sèche sous pression réduite à température ambiante pour obtenir 1,94 g de l'isomère "normal".
[α]_{D} = -145° (à 0,46 % dans CHCl₃).
Contrôles de l'isomère "normal" obtenu par séparation chromatographique :
Spectre I.R. (CHCl₃)
~3440 cm⁻¹ =C-NH
1684 cm⁻¹ Carbonyle
1626 cm⁻¹} autre Carbonyle
1599 cm⁻¹} C=C aromatique
1585 cm⁻¹} Amide II
1514 cm⁻¹}
1496 cm⁻¹}

### EXEMPLE 7 : (S) N-[1,2-diméthoxy-3-hydroxy-10-méthylthio-9-oxo-5,6,7,9-tétrahydro-benzo[a]heptalèn-7-yl]-acétamide (Thiosubstance C)

### Préparation du mercaptide de sodium

On agite 47 heures à 35-40°C, 8 g de sodium, 360 ml de tétrahydrofuranne et 19,5 ml (216 mmoles) de diméthyle disulfure. On siphone alors la suspension sous agitation modérée pour enlever l'excès de sodium, le solvant est évaporé sous pression réduite à 40°C, on obtient 27,2 g de produit que l'on cristallise dans l'éther diisopropylique, après séchage sous pression réduite on obtient 20,36 g du mercaptide de sodium. A un mélange de 520 mg de mercaptide de sodium et 5 ml de diméthylformamide on ajoute à 20°C en trois minutes, 1 g du produit obtenu au stade C de l'exemple 6 (isomère "normale") et agite une heure à température ambiante. On ajoute alors un mélange de 6 ml d'eau, 4 ml d'acide chlorhydrique 2N à 0°C puis 10 ml d'acétate d'éthyle, décante, sèche sur sulfate de sodium, filtre et évapore à sec sous pression réduite. Après cristallisation dans un mélange d'acétate d'éthyle-éther diisopropylique (2:1), on recueille 400 mg du produit recherché.
[α]_{D} = -334° (à 0,26 % dans EtOH).
Endotherme de Fusion à 309°C
Spectre I.R. (CHCl₃) 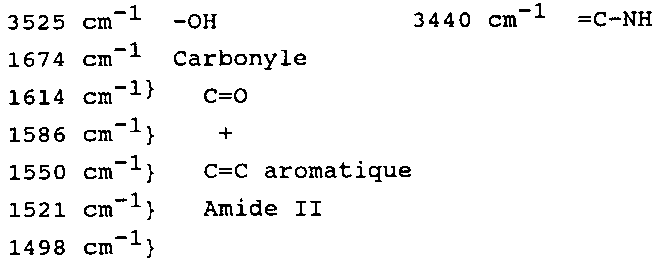 Spectre R.M.N.

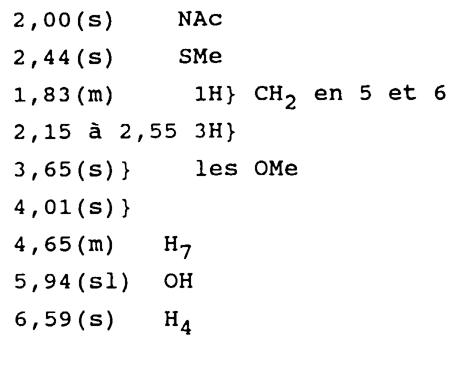

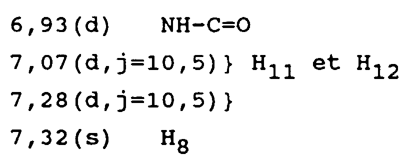

### Exemples 8-12 : Synthèse Racémique

### EXEMPLE 8 : O-méthyloxime de 9,10-diméthoxy-8-[[(4-méthylphényl)sulfonyl]oxy]-2,3,5,6-tétrahydro-benz[e]azulèn-4(1H) -one

A une solution de 3,5 g du produit obtenu au stade B de l'exemple 3 dans 17,5 ml de chlorure de méthylène et 35 ml de méthanol on ajoute à 20°C, 1,05 g de chlorhydrate de méthoxylamine à 98 % et 1,3 g de pyridine et agite le milieu réactionnel 16 heures à 20°C. On verse sur eau, glace et un excès d'acide chlorhydrique 0,1N, extrait au chlorure de méthylène, lave à l'eau, sèche, filtre et évapore à sec sous pression réduite pour obtenir 5 g du produit recherché (mélange d'isomères syn et anti de l'oxime). Le produit brut est chromatographié sur silice en éluant avec du chlorure de méthylène à 5 % d'éther diisopropylique. On concentre à sec et cristallise par concentration dans le mélange éther diisopropylique-chlorure de méthylène on obtient 1,4 g d'isomère majoritaire pur (P.F. ∼110°C) et 1,2 g du mélange d'isomères syn ou anti au niveau du groupement O-méthyloxime.
Spectre I.R. (CHCl₃) de l'isomère majoritaire :
Absence de carbonyle 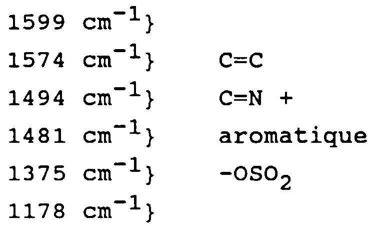 Spectre R.M.N. (CDCl₃) de l'isomère majoritaire :

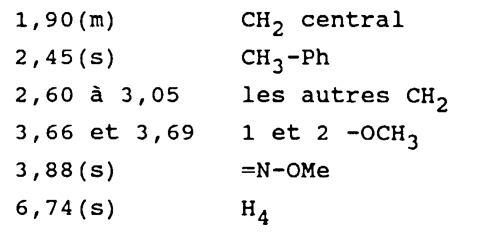

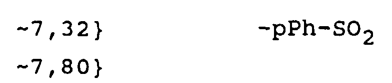

### EXEMPLE 9 : Mélange tautomérique de : 7-O-méthyloxime de 1,2-diméthoxy-10-hydroxy-3-[[(4-méthylphényl)sulfonyl]oxy]-5,6-dihydro-benzo[a]heptalène-7,9-dione (forme "normale") et 7-O-méthyloxime de 1,2-diméthoxy-9-hydroxy-3-[[(4-méthylphényl) sulfonyl]oxy]-5,6-dihydro-benze[a]heptaléne-7,10-dione (forme "iso")

### Stade A : O-méthyloxime de 8-[[(4-méthylphényl)sulfonyl] oxy]-2,3,5,6-tétrahydro-1,9,10-triméthoxy-benz[e]azulèn-4(1H)-one, (racémisé en position 1).

A une solution de 1,14 g d'isomère majoritaire obtenu à l'exemple 8 dans 12,5 ml de chlorure de méthylène et 12,5 ml de méthanol, on ajoute à température ambiante, 0,8 ml de pyridine et 1 g de bromhydrate de perbromure de pyridinium. Après 24 heures d'agitation à 20°C, le milieu réactionnel est versé sur un mélange eau, glace et acide chlorhydrique (N) en excès, extrait avec du chlorure de méthylène, lavé à l'eau avec un peu de thiosulfate de sodium, puis à l'eau pure, sèche sur sulfate de sodium, filtre et évapore à sec sous pression réduite pour obtenir 1,2 g du produit brut que l'on chromatographie sur silice en éluant avec du chlorure de méthylène à 5 % d'éther diisopropylique, les fractions contenant uniquement le mélange racémique des deux isomères (de position 1) du produit recherché sont évaporées à sec. Le produit obtenu est cristallisé dans le mélange éther diisopropylique/n-pentane pour obtenir 377 mg du produit recherché. (P.F. = 117°C)
Spectre I.R. (CHCl₃) 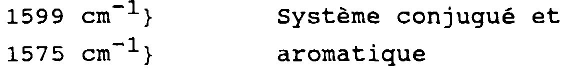 Spectre R.M.N. (CDCl₃)

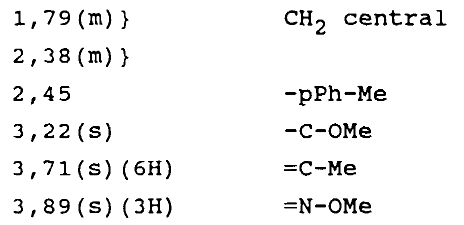

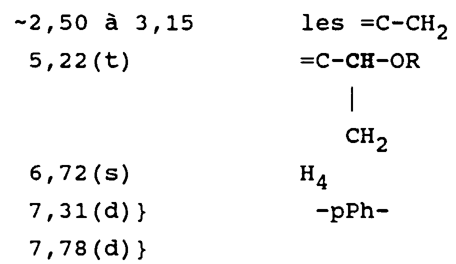

### Stade B : O-méthyloxime de 5,6-dihydro-9,10-diméthoxy-8-[[(4-méthylphényl)sulfonyl]oxy]-benz[e]azulèn-4(1H)-one

On agite deux heures à température ambiante 230 mg du produit obtenu au stade B ci-dessus, 6,25 ml de chlorure de méthylène, 23 mg d'acide paratoluène sulfonique. On verse sur un mélange d'eau, de glace et 2 ml d'une solution aqueuse saturée de bicarbonate de sodium, extrait au chlorure de méthylène, lave à l'eau, sèche sur sulfate de sodium, filtre et évapore à sec sous pression réduite. On chromatographie le produit obtenu sur silice en éluant avec du chlorure de méthylène, on évapore à sec, le produit obtenu est utilisé tel quel pour le stade suivant.

### Stade C : Mélange racémique de : 7-O-méthyloxime de 10,10-dichloro-1,2-diméthoxy-5,6,8,8a,10,10a-hexahydro-3-[[(4-méthylphényl)sulfonyl]oxy]-benzo[h]cyclobuta[a]azulène-7,9-dione et son diastéréoisomère au niveau de la condensation cyclobuta[a]azulène

On dissout le produit obtenu au stade B dans 4 ml de chlorure de méthylène et 1 ml de n-pentane, puis on ajoute, en 5 minutes, une solution de 0,4 ml de chlorure de dichloroacétyle, 0,6 ml de triéthylamine et 4 ml de n-pentane. On agite 2 heures à température ambiante, verse sur eau et glace, extrait avec du chlorure de méthylène, lave à l'eau, sèche sur sulfate de sodium, traite avec du charbon actif, filtre puis évapore à sec sous pression réduite pour obtenir 700 mg de produit brut, que l'on chromatographie sur silice en éluant au chlorure de méthylène. Les fractions ne contenant que le mélange des cycloadduits sont concentrées à sec, on cristallise le produit obtenu dans du méthanol contenant un peu d'éther diisopropylique. On essore les cristaux ainsi obtenus, lave avec le minimum de méthanol et sèche sous pression réduite à température ambiante pour obtenir 89 mg du produit recherché.
(P.F. = 170°C)
Spectre I.R. (CHCl₃) 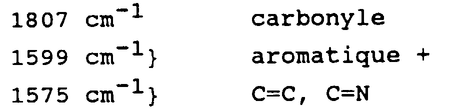 Spectre R.M.N. (CDCl₃)

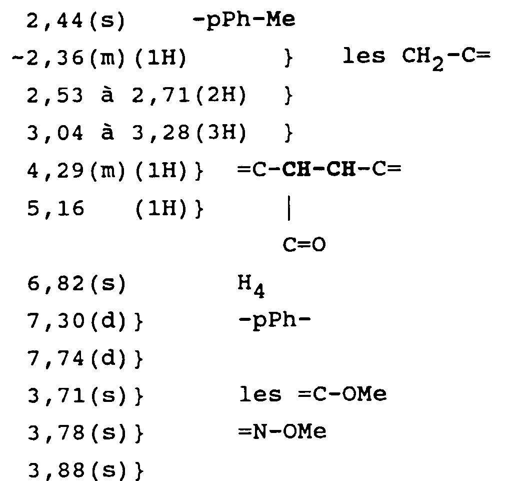

### Stade D : Mélange tautomérique de : 7-O-méthyloxime de 1,2-diméthoxy-10-hydroxy-3-[[(4-méthylphényl)sulfonyl]oxy]-5,6-dihydro-benzo[a]heptalène-7,9-dione (forme "normal") et 7-O-méthyloxime de 1,2-diméthoxy-9-hydroxy-3-[[(4-méthylphényl) sulfonyl]oxy]-5,6-dihydro-benzo[a]heptalène-7,10-dione (forme "iso")

A une solution de 166 mg du produit obtenu au stade C dans 8 ml d'acétone à 20 % d'eau, on ajoute à 20°C, 0,46 ml d'acide acétique et 1,12 ml de triéthylamine. On agite cinq jours à température ambiante puis verse sur de l'eau, extrait au chlorure de méthylène, lave à l'eau, sèche sur sulfate de sodium, traite par charbon actif, filtre et concentre à sec sous pression réduite pour obtenir 170 mg du produit recherché.

### EXEMPLE 10 : Mélange tautomérique de : N-[1,2-diméthoxy-3-[[(4-méthylphényl)sulfonyl]oxy]-9-oxo-5,6,7,9-tétrahydro-10-[[[2,4,6-tris(1-méthyléthyl)phényl]sulfonyl]oxy]benzo[a]heptalèn-7-yl]-acétamide (forme "normal"), racémique au niveau de la position 7 du noyau benzo[a]heptalèn et N-[1,2-diméthoxy-3-[[(4-méthylphényl)sulfonyl]oxy]-10-oxo-5,6,7,9-tétrahydro-9-[[[2,4,6-tris(1-méthyléthyl)phényl] sulfonyl]oxy]-benzo[a]heptalèn-7-yl]-acétamide (forme "iso"), racémique au niveau de la position 7 du noyau benzo[a]heptalène

### Stade A : Mélange tautomérique de : (T-4)-[7-méthoxyimino-1,2-diméthoxy-10-hydroxy-3-[[(4-méthylphényl)sulfonyl]oxy]-9-oxo-5,6,7,9-tétrahydro-benzo[a]heptalènato]difluoro-bore et (T-4)-[7-méthoxyimino-1,2-diméthoxy-9-hydroxy-3-[[(4-méthylphényl)sulfonyl]oxy]-10-oxo-5,6,7,10-tétrahydro-benzo[a]heptalènato]difluoro-bore

A une solution de 1,023 g du produit obtenu à l'exemple 9 dans 2 ml de chlorure de méthylène et 10 ml de méthanol, on ajoute, à 20°C, 0,5 ml de diéthyle étherate de trifluoroborure puis agite trois heures à température ambiante. On verse le mélange réactionnel sur un mélange d'eau et glace, extrait chlorure de méthylène, lave avec une solution aqueuse saturée de bicarbonate de sodium, puis à l'eau, sèche sur sulfate de sodium, traite avec du charbon actif, filtre et évapore à sec sous pression réduite à <30°C pour obtenir 1,4 g du produit brut recherché. On chromatographie ce produit sur silice en éluant avec du chlorure de méthylène à 5 % d'éther diisopropylique et 0,1 % de triéthylamine. On recueille 917 mg du produit recherché.
Spectre I.R. (CHCl₃) 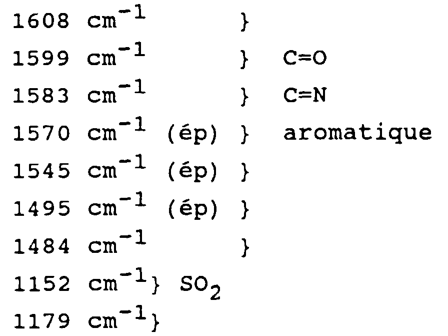 Spectre U.V.
**1** - Dans EtOH : pour M = 559,35
max 227nm ε= 38000
max 339nm ε= 14500
infl 250, 380 nm
**2** - Dans EtOH-Acide chlorhydrique 0,1N :
max 228nm ε= 40700
max 339nm ε= 16900
max 456nm ε= 900
infl 250, 380 nm
**3** - Dans EtOH-NaOH 0,1N :
max 258nm ε= 25100
max 356nm ε= 23100
max 410nm ε= 10600
Spectre R.M.N. (CDCl₃)

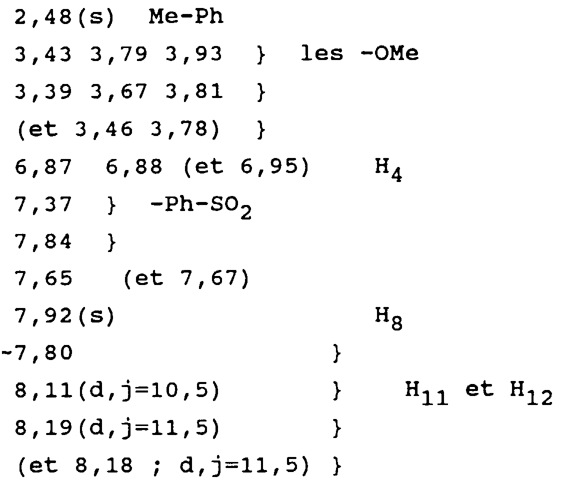

### Stade B : Mélange tautomérique de : (T-4)-[7-amino-1,2-diméthoxy-10-hydroxy-3-[[(4-méthylphényl)sulfonyl]oxy]-9-oxo-5,6,7,9-tétrahydro-benzo[a]heptalènato]difluoro-bore (Forme "Normale") - racémique en position 7 et (T-4)-[7-amino-1,2-diméthoxy-9-hydroxy-3-[[(4-méthylphényl) sulfonyl]oxy]-10-oxo-5,6,7,10-tétrahydro-benzo[a] heptalènato]difluoro-bore (Forme "Iso") - racémique en position 7

A une solution de 2,83 g du produit obtenu au stade A ci-dessus, dans 50 ml de tétrahydrofuranne, on ajoute 920 mg de zinc électrolytique (Vieille Montagne) et 3,4 ml d'acide méthane sulfonique et agite 72 heures à température ambiante. Le mélange réactionnel est versé dans le mélange d'eau, glace, bicarbonate de sodium et chlorure de méthylène, décante et réextrait deux autres fois au chlorure de méthylène, lave à l'eau, sèche sur sulfate de sodium, filtre et concentre à sec sous pression réduite pour obtenir 2,8 g du produit brut recherché. On chromatographie ce produit sur silice en éluant avec cylcohexane-acétate d'éthyle : (1:1) et 1 % triéthylamine. On recueille ainsi 1,67 g du produit recherché.
Spectre R.M.N. (CDCl₃)

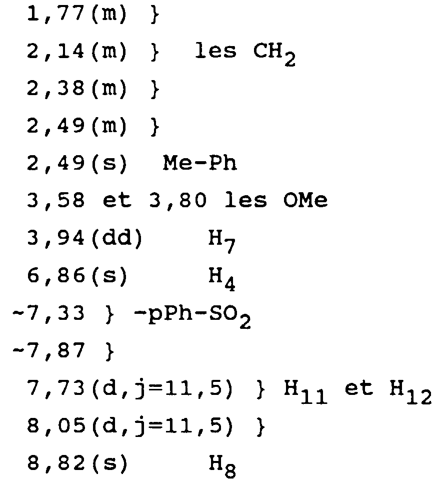

### STADE C : Mélange tautomérique de : (T-4)-[7-acétylamino-1,2-diméthoxy-10-hydroxy-3-[[(4-méthylphényl)sulfonyl]oxy]-9-oxo-5,6,7,9-tétrahydro-benzo[a]heptalènato]difluoro-bore (forme "normale") - racémique en position 7 et (T-4)-[7-acétylamino-1,2-diméthoxy-9-hydroxy-3-[[(4-méthylphényl)sulfonyl]oxy]-10-oxo-5,6,7,10-tétrahydro-benzo[a] heptalènato]difluoro-bore (forme "iso") - racémique en position 7

On agite 48 heures à température ambiante, 475 mg du produit obtenu au stade B ci-dessus, 3 ml de pyridine et 2 ml d'anhydride acétique. On verse le milieu réactionnel sur 20 ml d'eau légèrement acidulée en présence de chlorure de méthylène et agite 20 minutes. On décante et réextrait deux fois au chlorure de méthylène, lave à l'acide chlorhydrique (2N) puis à l'eau, sèche sur sulfate de sodium et évapore sous pression réduite pour obtenir 540 mg d'extrait sec que l'on chromatographie sur silice en éluant avec de l'acétate d'éthyle à 1 % de triéthylamine, pour obtenir 226 mg du produit recherché.
Spectre R.M.N. (CDCl₃)

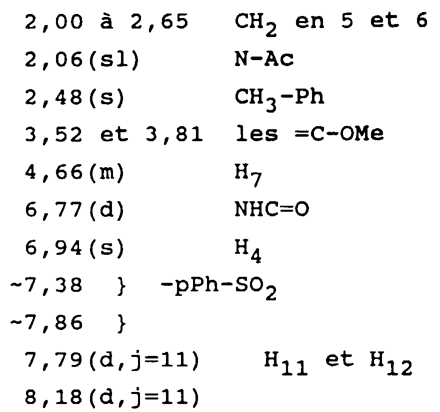

### Stade D : Mélange tautomérique de : N-[1,2-diméthoxy-10-hydroxy-3-[[(4-méthylphényl)sulfonyl]oxy]-9-oxo-5,6,7,9-tétrahydro-benzo[a]heptalèn-7-yl]-acétamide (forme "normale"), racémique au niveau de position 7 du noyau benzo[a]heptalène et [1,2-diméthoxy-9-hydroxy-3-[[(4-méthylphényl)sulfonyl]oxy]-10-oxo-5,6,7,9-tétrahydro-benzo[a]heptalén-7-yl]-acétamide (Forme "iso"), racémique au niveau de position 7 du noyau benzo[a]heptalène.

On agite 16 heures à température ambiante, 200 mg du produit obtenu au stade C dans 4 ml de méthanol et 0,7 ml d'une solution aqueuse (2M) d'acétate de sodium. On verse dans l'eau, essore le précipité, lave à l'eau et sèche sous pression réduite à 20°C pour obtenir 146 mg du produit recherché.

### Stade E : Mélange tautomérique de : N-[1,2-diméthoxy-3-[[(4-méthylphényl)sulfonyl]oxy]-9-oxo-5,6,7,9-tétrahydro-10-[[[2,4,6-tris(1-méthyléthyl)phényl]sulfonyl]oxy]benzo[a]heptalèn-7-yl]-acétamide (forme "normale"), racémique au niveau de position 7 du noyau benzo[a]heptalène et N-[1,2-diméthoxy-3-[[(4-méthylphényl)sulfonyl]oxy]-10-oxo-5,6,7,9-tétrahydro-9-[[[2,4,6-tris(1-méthyléthyl)phényl]sulfonyl]oxy]-benzo[a]heptalèn-7-yl]-acétamide (forme "iso"), racémique au niveau de position 7 du noyau benzo[a]heptalène

A une solution de 79 mg du produit obtenu au stade D dans 1,5 ml de chlorure de méthylène, on ajoute 1,5 ml d'eau, 25 mg de bicarbonate de sodium, 94 mg de chlorure de [2,4,6-tris(1-méthyléthyl)phényl]-sulfonyle et 8 mg d'hydrogénosulfate de tétrabutylammonium et on agite 16 heures à 20°C. Le milieu réactionnel est versé dans l'eau, extrait avec du chlorure de méthylène, lavé à l'eau, séché sur sulfate de magnésium, filtre et concentre à sec sous pression réduite pour obtenir 164 mg d'un mélange de produit recherché (forme "normale") et son regioisomère (forme "iso"). Après chromatographie sur silice (éluant cyclohexane-acétate d'éthyle (1:1)). On obtient 38 mg de l'isomère "iso" et 67 mg du produit recherché isomère forme "normale".
Spectre I.R. (CHCl₃)

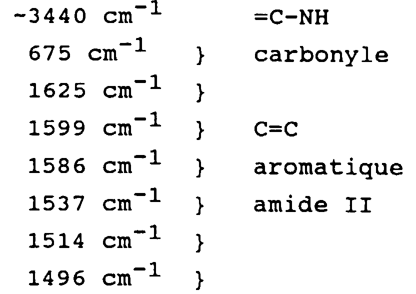

### EXEMPLE 11 : Mélange tautomérique de : 2-[10-hydroxy-9-oxo-5,6,7,9-tétrahydro-1,2,3-triméthoxy-benzo[a]heptalén-7-yl]-1H-isoindole-1,3(2H)-dione, racémique en position 7 du noyau benzo[a]heptalène et 2-[9-hydroxy-10-oxo-5,6,7,10-tétrahydro-1,2,3-triméthoxybenzo[a]heptalèn-7-yl]-1H-isoindole-1,3(2H)-dione, racémique en position 7 du noyau benzo[a]heptalène.

### Stade A : 1,2,3,4,5,6-hexahydro-8,9,10-triméthoxy-benz[e] azulèn-4-ol, mélange racémique au niveau de position 4.

A une solution de 5,766 g du produit obtenu au stade E de l'exemple 1, dans 75 ml de méthanol et 5 ml de chlorure de méthylène, on ajoute en une fois 757 mg d'hydroborure de sodium et agite le milieu réactionnel pendant 16 heures à température ambiante. On verse dans l'eau, extrait deux fois au chlorure de méthylène, lave à l'eau, sèche sur sulfate de sodium, filtre et concentre à sec. On reprend le produit ainsi obtenu avec du chlorure de méthylène et de l'éther diisopropylique et concentre à petit volume, puis essore et lave à l'éther diisopropylique pour obtenir 5,55 g du produit recherché.

### Stade B : 2-[1,2,3,4,5,6-hexahydro-8,9,10-triméthoxy-benz[e]azulèn-4-yl]-1H-isoindole-1,3(2H)dione, mélange racémique en position 4 du noyau benz[e]azulène.

A un mélange, sous agitation, de 5 g de triphénylphosphine, 2,811 g de phtalimide et 80 ml de tétrahydrofuranne, on ajoute 5,55 g du produit obtenu au stade A ci-dessus et 3,327 g de azodicarboxylate de diéthyle et 13,5 ml de tétrahydrofuranne et agite le milieu réactionnel pendant 16 heures à température ambiante. Après ce temps on introduit à nouveau les mêmes quantités de triphénylphosphine, phtalimide et azo dicarboxylate de diéthyle on agite encore 16 heures puis on verse le milieu réactionnel dans l'eau, extrait au chlorure de méthylène, lave deux fois à l'eau, sèche sur sulfate de sodium, filtre et concentre à sec on obtient 30 g du produit que l'on chromatographie sur silice en éluant avec un mélange chlorure de méthylène-acétate d'éthyle (2:1), on recueille ainsi 6,16 g du produit recherché.

### Stade C : 2-[1,2,3,4,5,6-hexahydro-1,8,9,10-tétraméthoxybenz[e]azulèn-4-yl]-1H-isoindole-1,3(2H)dione, mélange racémique, en position 4, du noyau benz[e]azulène (stéréochimie en position 1 du noyau benz[e]azulène pas établie)

On refroidit à 0°C une suspension de 6,16 g du produit obtenu au stade B, 62 ml de méthanol et 4,75 ml de pyridine puis on ajoute 5,64 g de bromhydrate de perbromure de pyridinium en une fois, on laisse la température remonter et agite 2 heures à température ambiante, puis ajoute 25 ml de méthanol et agite encore une heure, on ajoute à nouveau 0,94 g de bromhydrate de perbromure de pyridinium, agite encore 10 heures. On verse alors le milieu réactionnel dans de l'eau glacée en provoquant une précipitation du produit recherché, on ajoute du chlorure de méthylène et 29,4 ml d'acide chlorhydrique (2N), extrait deux fois au chlorure de méthylène, lave à l'eau, sèche sur sulfate de sodium, filtre et concentre à sec pour obtenir 7,2 g du produit brut que l'on chromatographie sur silice en éluant avec du chlorure de méthylène à 5 % d'éther diisopropylique pour obtenir 2,9 g du produit que l'on dissout dans du chlorure de méthylène, puis après ajout d'éther diisopropylique la solution est concentrée à petit volume, glacée et essorée pour obtenir 2,54 g du produit recherché.
P.F. = 200°C.

### Stade D : 2-[10,10-dichloro-5,6,7,8,8a,9,10,10a-octahydro-9-oxo-1,2,3-triméthoxy-benzo[h]cyclobuta[a]azulèn-7-yl]-1H-isoindole-1,3(2H)-dione, racémique en position 7 du noyau benzo[h]cyclobuta[a]azulène + son Diastéréoisomère au Niveau de la Condensation cyclobuta[a]azulène, (racémique en position 7 du noyau benzo[h]cyclobuta[a]azulène) et 2-[3,4,5,6-tétrahydro-8,9,10-triméthoxy-benz[e]azulèn-4-yl]-1H-isoindole-1,3(2H)dione, mélange racémique en position 4 du noyau benz[e]azulène (=diène intermédiaire non isolé)

a) A une solution de 1 g du produit obtenu au stade C, dans 40 ml de chlorure de méthylène, on ajoute, à 5°C, 50 mg d'acide paratoluène sulfonique et agite 30 minutes à température ambiante.
b) On refroidit à 5° et ajoute 10 ml d'eau et 1,62 ml de triéthylamine, puis 1,12 ml de chlorure de dichloroacétyle et agite une heure à température ambiante. On ajoute alors, 0,54 ml de triéthylamine et 0,37 ml de chlorure de dichloroacétyle et agite encore 30 minutes, verse dans l'eau glacée, extrait au chlorure de méthylène, lave à l'eau, sèche sur sulfate de sodium, filtre et concentre à sec pour obtenir 2,58 g de produit brut que l'on chromatographie sur silice en éluant avec du chlorure de méthylène à 5 % d'éther diisopropylique, et les fractions contenant l'ensemble des deux isomères du produit recherché sont concentrées à sec pour obtenir 640 mg du produit recherché. On chromatographie à nouveau ce produit en éluant avec du chlorure de méthylène-acétate d'éthyle (2:1), on recueille ainsi 59 mg de l'isomère minoritaire isomériquement pur, 124 mg de l'isomère majoritaire isomériquement pur et 419 mg d'un mélange des deux isomères.

### Stade E : Mélange tautomérique de 2-[10-hydroxy-9-oxo-5,6,7,9-tétrahydro-1,2,3-triméthoxy-benzo[a]heptalèn-7-yl]-1H-isoindole-1,3(2H)-dione, racémique en position 7 du noyau benzo[a]heptalène et de 2-[9-hydroxy-10-oxo-5,6,7,10-tétrahydro-1,2,3-triméthoxybenzo[a]heptalèn-7-yl]-1H-isoindole-1,3(2H)-dione, racémique en position 7 du noyau benzo[a]heptalène.

On agite 3 heures à température ambiante 419 mg du mélange des deux isomères obtenus au stade D avec 8,5 ml d'acétone à 20 % d'eau, 0,553 ml d'acide acétique et 1,3 ml de triéthylamine, puis on verse le milieu réactionnel dans l'eau glacée, extrait au chlorure de méthylène, lave à l'acide chlorhydrique (N) glacé puis à l'eau, sèche sur sulfate de sodium, filtre et concentre à sec pour obtenir 365 mg du produit recherché.

### EXEMPLE 12 : 2-[9-oxo-5,6,7,9-tétrahydro-1,2,3-triméthoxy-10-[[[2,4,6-tris(1-méthyléthyl)phényl]sulfonyl]oxy]benzo[a]heptalèn-7-yl]-1H-isoindole-1,3(2H)-dione (Forme "Normale"), racémique en position 7 du noyau benzo[a]heptalène

On agite 48 heures à température ambiante, 365 mg du produit obtenu au stade E de l'exemple 11 avec 7 ml de chlorure de méthylène, 7 ml d'eau, 129 mg de bicarbonate de sodium, 36,5 mg de l'hydrogénosulfate de tétrabutylammonium et le chlorure de [2,4,6-tris(1-méthyléthyl)phényl]-sulfonyle. On coule le milieu réactionnel dans l'eau glacée, extrait au chlorure de méthylène, lave à l'eau, sèche sur sulfate de sodium, filtre et concentre à sec pour obtenir 965 mg du produit brut que l'on chromatographie sur silice en éluant avec du chlorure de méthylène à 5 % d'éther diisopropylique on obtient 151 mg du produit recherché que l'on chromatographie une seconde fois en éluant avec du chlorure de méthylène à 5 % d'éther diisopropylique, on recueille 75 mg du produit recherché.

### EXEMPLE 13 : 4-méthylbenzènesulfonate de (S) 4-amino-8,9-diméthoxy-1,2,3,4,5,6-hexahydro-benz[e]azulen-10-ol

### Stade A : (R) 8,9-diméthoxy-10-[[(4-méthylphényl)sulfonyl]oxy]-2,3,5,6-tétrahydro-benz[e]azulen-4(1H)-β-ol

A une solution de 200 mg de 8,9-diméthoxy-10-[[(4-méthylphényl)sulfonyl]oxy]-2,3,5,6-tétrahydro-benz[e]azulen-4(1H)-one (obtenu au stade E de l'exemple 2) dans 2 ml de tétrahydrofuranne, on ajoute 0,046 mmoles de complexe oxaza borolidine (dont la préparation est donnée ci-après) en solution dans 100 µl de tétrahydrofuranne puis on ajoute, en 15 minutes, 0,56 ml de complexe borane - tétrahydrofuranne (0,88 M) en laissant la température s'élever jusqu'à 30°C environ. On refroidit à 0°C et ajoute sans dépasser 10°C, 5 ml d'eau glacée, on agite 30 minutes puis extrait avec du chlorure de méthylène, lave à l'eau, sèche et évapore à sec sous pression réduite. On recueille 224 mg de produit que l'on chromatographie sur silice en éluant avec du chloroforme à 3 % d'acétone. On obtient ainsi 197 mg du produit recherché.
Spectre RMN 330 MHz (CDCl₃)
1,6 à 2,8 : les CH₂
2,45 : CH₃-φ-
3,64 à 3,89 : les O-CH₃
4,27 (large) : CH₂-CH-OH
6,71 : H4 en α de OSO₂-φ
7,31 à 7,79 : les aromatiques

### Préparation du complexe oxazaborolinique utilisé au stade A :

A une solution de 250 mg de (R)-(+)-αα-diphényle-2-pyrrolidine-méthanol dans 20 ml de toluène on ajoute goutte à goutte, à température ambiante, 90 µl de triméthyl boroxine et on poursuit l'agitation 15 minutes. On élimine le toluène et le réactif en excès par distillation sous pression réduite pour obtenir le produit cristallisé que l'on utilise tel quel.

### Stade B : 4-méthylbenzènesulfonate de (S) 4-amino-8,9-diméthoxy-1,2,3,4,5,6-hexahydro-benz[e]azulen-10-ol

On refroidit à -55°C une solution de 40 mg du produit obtenu au stade A, ci-dessus, dans 0,8 ml de chlorure de méthylène, on ajoute goutte à goutte 44 µl de triéthylamine, agite 10 minutes à -55°C puis on introduit lentement 20 µl de chlorure de méthane sulfonyle en solution dans 50 µl de chlorure de méthylène. On agite encore 10 minutes à -55°C et ajoute, sans dépasser -50°C, 0,5 ml d'ammoniac liquéfié. On agite 2 heures à -50°C, puis laisse la température remonter pour éliminer l'ammoniac en excès. On refroidit à 0+5°C, puis on introduit 2 ml d'eau glacée et agite 20 minutes à cette température, on extrait avec de l'acétate d'éthyle, lave à l'eau, filtre et évapore à sec sous pression réduite pour obtenir 38,3 mg de produit brut que l'on chromatographie sur silice en éluant avec le mélange toluène-méthanol-ammoniaque (80-20-1). On recueille ainsi 24,2 mg du produit recherché.
Spectre RMN 300 MHz (CDCl₃)
1,5 à 2,8 : les CH₂
2,44 : CH₃-φ-
3,70 à 3,86 : les O-CH₃
3,74 : CH-NH₂
6,71 : H4 en α de OSO₂-φ
7,35 à 7,83: les aromatiques

### EXEMPLE 14 : 4-méthylbenzènesulfonate de (S) 4-acétylory-8,9-diméthoxy-1,2,3,4,5,6-hexahydro-benz[e]azulen-10-ol

### Stade A : (R,S) 8,9-diméthoxy-10-[[(4-méthylphényl)sulfonyl]oxy]-2,3,5,6-tétrahydro-benz[e]azulen-4(1H)-ol

En opérant comme au stade A de l'exemple 11 à partir de 8,9-diméthoxy-10-[[(4-méthylphényl)sulfonyl]oxy]-2,3,5,6-tétrahydro-benz[e]azulen-4(1H)-one (obtenu au stade E de l'exemple 2), on a obtenu le produit recherché.

### Stade B : 4-méthylbenzènesulfonate de (S) 4-acétyloxy-8,9-diméthoxy-1,2,3,4,5,6-hexahydro-benz[e]azulen-10-ol

A un mélange de 150 mg du produit obtenu au stade A et 7,5 ml de terbutyl méthyl éther, on ajoute 0,81 ml d'acétate vinylique et 150 mg de Lipase PS Amano. On agite la suspension pendant 27 heures à température ambiante. On filtre sur membrane pour éliminer la lipase puis évapore à sec sous pression réduite pour obtenir 138 mg de produit brut que l'on chromatographie sur silice en éluant avec du chloroforme à 5 % d'acétone. On recueille 65,2 mg du produit recherché et 66,4 mg de produit non transformé.
Spectre RMN 300 MHz (CDCl₃)
1,6 à 2,8 : les CH₂
2,45 : CH₃-φ-
3,64 à 3,89 : les O-CH₃
4,27 (large) : CH₂-CH-OH
6,71 : H4 en α de OSO₂-φ
7,30 à 7,79 : les aromatiques.

### EXEMPLE 15 : (S) 2-[9,10-diméthoxy-8-[[(4-méthylphényl)sulfonyl]oxy]-1,2,3,4,5,6-hexahydro-benz[e]azulèn-4-yl]-1H-isoindole-1,3(2H)-dione

### STADE A : (S) 9,10-diméthoxy 8-hydroxy 1,2,3,4,5,6-hexahydro-4-[(1-phényléthyl) amino] benz[e]azulène.

On ajoute 4,5 g de potasse à 10 g de produit obtenu au stade A de l'exemple 4 dans 50 ml de méthanol et chauffe 1 heure au reflux. On refroidit la solution à 20°C, ajoute 20 ml d'acide acétique puis 150 ml d'eau et extrait au dichlorométhane. Après chromatographie sur silice, on obtient 6,4 g de produit attendu.
Spectre RMN (CDCl₃) ppm
6,82 : 2H mobiles
6,47 : H₄
3,64 à 3,84 : les 2 CH₃O
3,3 (t) : H₇.

### STADE B : (S) 4-amino 8-hydroxy 9,10-diméthoxy 1,2,3,4,5,6-hexahydro-benz[e]azulène.

On agite pendant 30 heures à température ambiante sous atmosphère d'hydrogène (400 mbar), 5 g du produit obtenu au stade A, 50 ml de méthanol, 25 ml d'acide acétique et 150 ml d'eau en présence de 2 g d'hydroxyde de palladium à 20% sur charbon actif humide (50% d'eau). On filtre et lave avec du méthanol, puis concentre à sec sous pression réduite à 40°C. Après cristallisation dans l'acétate d'éthyle, on obtient 3 g de produit attendu.
Spectre RMN (CDCl₃) ppm
6,59 (s) : H₄
3,79 et 3,89 (2s) : les 2 CH₃O
3,49 (m) : H₇
1,6 (m large) : H mobiles.

### STADE C : (S) 2-(9,10-diméthoxy 8-hydroxy 1,2,3,4,5,6-hexahydro-benz[e]azulèn-4-yl) 1H-isoindole-1,3(2H)-dione.

A une solution de 3 g de produit obtenu comme au stade B dans 30 ml de toluène, on ajoute 0,8 g d'anhydride phtalique et 2 ml de triéthylamine puis on chauffe pendant 16 heures à reflux. On refroidit à 20°C et lave avec 20 ml d'acide chlorhydrique N. On décante, lave à l'eau, sèche et évapore le solvant. On obtient 4,42 g de produit attendu que l'on utilise tel quel pour le stade suivant.
Spectre RMN (CDCl₃) ppm
6,60 (s) : H₄
5,70 : OH
5,01 (m) : H₇
3,82 et 3,95 (2s) : les 2 CH₃O
7,7 et 7,82 (2m) : les aromatiques.

### STADE D : (S) 2-[9,10-diméthoxy-8-[[(4-méthylphényl)sulfonyl]oxy]-1,2,3,4,5,6-hexahydro-benz[e]azulèn-4-yl]-1H-isoindole-1,3(2H)-dione.

On introduit le produit obtenu au stade C dans 45 ml de dichlorométhane, ajoute 4 ml de triéthylamine et 2,5 g de chlorure de tosyle puis agite 16 heures à 20°C. On lave à l'acide chlorhydrique N, puis à l'eau, sèche et cristallise dans le méthanol. On obtient 5,50 g de produit attendu, identique à celui obtenu à l'exemple 4.

### EXEMPLE 16 : (S) 9,10-diméthoxy 8-hydroxy 1,2,3,4,5,6-hexahydro 4-((1-phényléthyl) amino)-benz[e]azulène.

### STADE A : 9,10-diméthoxy 8-hydroxy 2,3,5,6-tétrahydrobenz[e]-azulèn-4(1H)-one.

On ajoute 4,5 g de potasse puis 10 ml de triéthylamine dans une suspension comprenant 10 g de 9,10-diméthoxy 8-(((4-méthylphényl) sulfonyl) oxy) 2,3,5,6-tétrahydro-benz[e]azulèn-4(1H)-one obtenu comme au stade B de l'exemple 3 et 100 ml de méthanol. On chauffe 1 heure au reflux, acidifie par addition de 20 ml d'acide acétique puis ajoute 20 ml d'eau. On extrait au dichlorométhane, lave à l'eau, évapore le solvant à 40°C sous pression réduite et recueille 5,7 g de produit attendu utilisé tel quel pour le stade suivant.

### STADE B : (S) 9,10-diméthoxy 8-hydroxy 1,2,3,4,5,6-hexahydro ((1-phényléthyl) imino)-benz[e]azulèn-4-yl.

On ajoute 5 g de silice et 6 ml de S (-) méthylbenzylamine à 5 g de produit obtenu au stade A dans 50 ml de toluène. On chauffe 16 heures au reflux en éliminant l'eau par azéotropie. On filtre, évapore le solvant sous pression réduite et obtient 6,9 g de produit attendu utilisé tel quel pour le stade suivant.

### STADE C : (S) 9,10-diméthoxy 8-hydroxy 1,2,3,4,5,6-hexahydro 4-((1-phényléthyl) amino)-benz[e]azulène.

On dissout le produit obtenu à l'exemple 15 dans 700 ml de tétrahydrofuranne, ajoute 2 g de palladium sur charbon actif et hydrogène (1,2 bar) pendant 20 heures. On filtre, évapore le solvant et obtient 6,9 g de produit attendu identique à celui obtenu au stade A de l'exemple 15.

## Revendications

1. Les composés de formule (I): 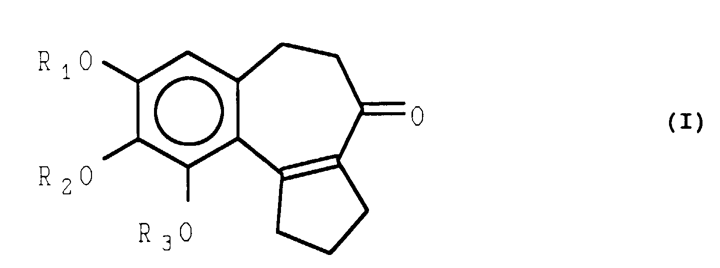 dans laquelle :
soit (a) R₁ et R₂ représentent tous les deux un groupement alkyle et R₃ représente un atome d'hydrogène ou le groupement A-SO₂-,
soit (b) R₂ et R₃ représentent tous les deux un atome d'hydrogène ou tous les deux un groupement alkyle et R₁ représente un groupement A-SO₂-,
soit (c) R₁, R₂ et R₃ représentent tous les trois un atome d'hydrogène ou tous les trois un groupement alkyle,
soit (d) R₁ représente le groupement A-SO₂- ou un atome d'hydrogène et R₂ et R₃ forment ensemble avec les atomes d'oxygène auxquels ils sont liés un groupement de formule -O-X-O- dans laquelle X représente un groupement de formule -B(OR₄)-, où R₄ représente un atome d'hydrogène, un groupement alkyle, un groupement -C(O)-, ou un groupement de formule -CR₅R₆-, où R₅ et R₆ représentent un atome d'hydrogène, un groupement alkyle ou phényle éventuellement substitué par 1 à 3 groupements choisis parmi les radicaux alkyle, hydroxy et alkyloxy, ou R₅ et R₆, ensemble avec l'atome de carbone auquel ils sont liés, représentent un groupement carbocyclique à 5 ou 6 chaînons,
soit (e) R₃ représente le groupement A-SO₂- et R₁ et R₂ forment ensemble avec les atomes d'oxygène auxquels ils sont liés, le groupement -O-X-O-,
soit (f) R₁ représente un atome d'hydrogène et R₂ et R₃ représentent tous les deux un groupement alkyle ou forment ensemble avec les atomes d'oxygène auxquels ils sont liés un groupement de formule -O-X-O- tel que défini ci-dessus,
"A" représente un groupement alkyle, un groupement phényle non substitué ou substitué par 1 à 3 groupements alkyles ou un groupement naphtyle non substitué ou substitué par 1 à 5 groupements alkyles,
les groupements alkyles ou alkyloxy mentionnés ci-dessus, étant ramifiés ou non ramifiés, et renfermant de 1 à 6 atomes de carbone.

2. Les composés de formule (I) telle que définie à la revendication 1, caractérisés en ce que R₁, R₂ et R₃ représentent tous les trois un atome d'hydrogène, ou tous les trois un groupement alkyle tel que défini à la revendication 1.

3. Un composé selon la revendication 2, caractérisé en ce que R₁, R₂ et R₃ représentent tous les trois un radical méthyle, ou tous les trois un atome d'hydrogène.

4. Les composés de formule (I) telle que définie à la revendication 1, caractérisés en ce que R₁ représente un groupement A-SO₂-, tel que défini à la revendication 1, et R₂ et R₃ représentent tous les deux un groupement alkyle tel que défini à la revendication 1, ou tous les deux un atome d'hydrogène.

5. Un composé selon la revendication 4, caractérisé en ce que R₁ représente un groupement tosyle et R₂ et R₃ représentent tous les deux un radical méthyle ou tous les deux un atome d'hydrogène.

6. Les composés de formule (I) telle que définie à la revendication 1, caractérisés en ce que R₃ représente un groupement A-SO₂- tel que défini à la revendication 1 et R₁ et R₂ représentent tous les deux un radical alkyle tel que défini à la revendication 1.

7. Un composé selon la revendication 6, caractérisé en ce que R₃ représente un groupe tosyle et R₁ et R₂ représentent tous les deux un radical méthyle.

8. Les composés de formule (I) telle que définie à la revendication 1, caractérisés en ce que R₁ et R₂ représentent tous les deux un groupement alkyle et R₃ représente un atome d'hydrogène.

9. Un composé selon la revendication 8, caractérisé en ce que R₁ et R₂ représentent tous les deux un radical méthyle.

10. Un composé selon la revendication 1, caractérisé en ce que R₁ représente un atome d'hydrogène et R₂ et R₃ représentent tousles deux un radical méthyle.

11. Procédé de préparation des composés de formule (I) telle que définie à la revendication 1, caractérisé en ce que
(i) l'on soumet un composé de formule (a) : 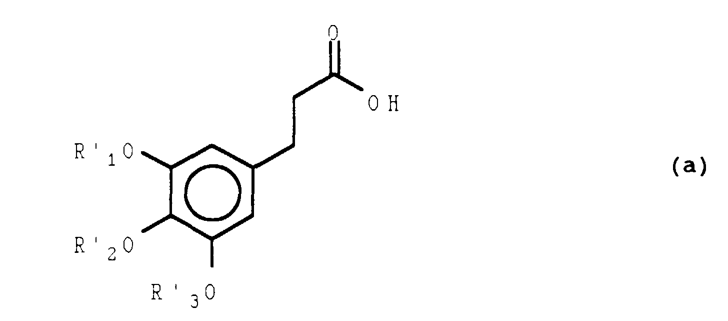 dans laquelle
R₁', R₂' et R₃' ont respectivement la même signification que les groupements R₁, R₂ et R₃ tels que définis à la revendication 1, à l'exception de la valeur hydrogène et pour R₃' de la valeur A-SO₂-,
à l'action d'un agent d'halogénation, pour obtenir l'halogénure d'acyle correspondant,
(ii) que l'on soumet à l'action d'un réactif de formule (b) : 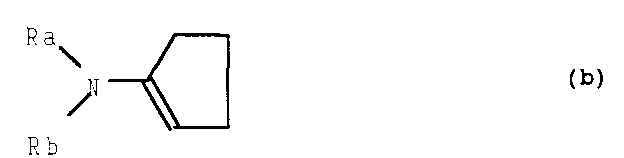 dans laquelle Ra et Rb, identiques ou différents, représentent un groupement alkyle, ou Ra et Rb ensemble avec l'atome d'azote auquel ils sont liés, représentent un hétérocycle à 5 ou 6 chaînons comprenant éventuellement un autre hétéroatome choisi parmi O et N ;
pour obtenir un composé de formule (c) 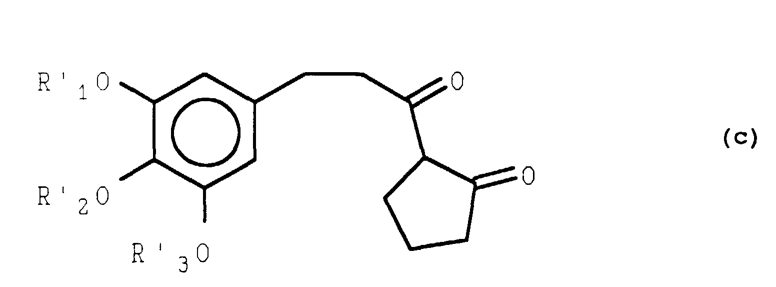
(iii) que l'on soumet à l'action d'un agent d'halogénation pour obtenir un composé de formule (d): 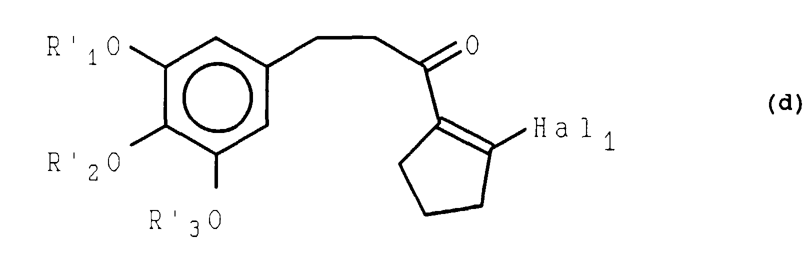 dans laquelle Hal₁ représente un atome d'halogène ;
(iv) que l'on soumet à l'action d'un acide de Lewis, pour obtenir un composé de formule (e) : 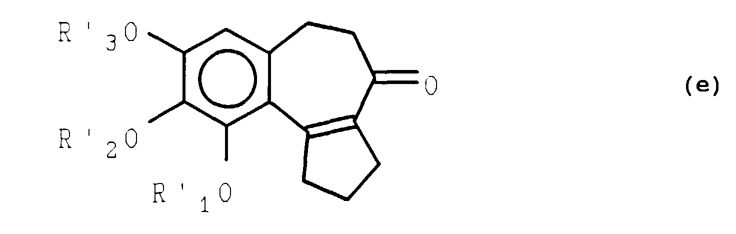 correspondant aux composés de formule (I) (a) telle que définie à la revendication 1, dans laquelle R₃ représente un groupement A-SO₂-, aux composés de formule (I) (c) telle que définie à la revendication 1, dans laquelle R₁, R₂ et R₃ représentent tous les trois un radical alkyle, et aux composés de formule (I) (e), telle que définie à la revendication 1 et composé de formule (e), que, le cas échéant,
(v) -soit si R'₁ représente un groupement A-SO₂- et R'₂ et R'₃ ne représentent pas ensemble le groupement -X-, l'on soumet à l'action d'un agent d'hydrolyse pour obtenir un composé de formule (f) : 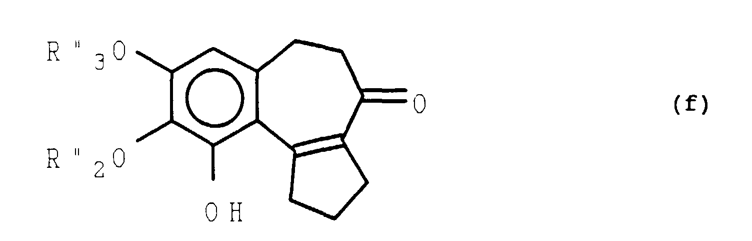 dans laquelle R"₂ et R"₃ représentent tous les deux un radical alkyle ; correspondant aux composés de formule (I)(a) telle que définie à la revendication 1 dans laquelle R₃ représente un atome d'hydrogène ;
que, le cas échéant,
(vi) l'on soumet à l'action d'un agent d'alkylation, pour obtenir un composé de la formule (g) : 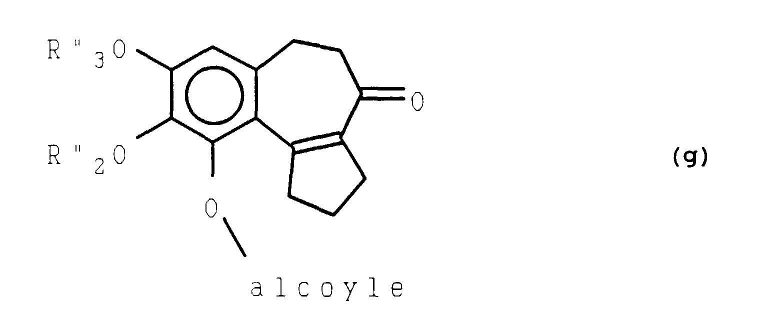 correspondant aux composés de formule (I) (c) telle que définie à la revendication 1, dans laquelle R₁, R₂ et R₃ représentent un radical alkyle, que le cas échéant,
(vii) l'on soumet à une hydrolyse de l'ensemble des groupements alkyloxy pour obtenir le composé de formule (h) : 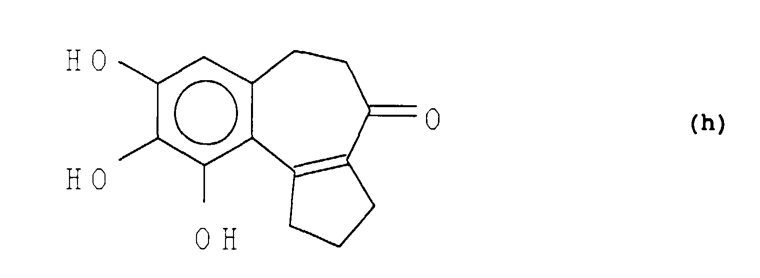 correspondant au composé de formule (I) (c) telle que définie à la revendication 1, dans laquelle R₁, R₂ et R₃ représentent un atome d'hydrogène,
(viii) soit soumet un composé de la formule (e) dans laquelle R'₁, R'₂ et R'₃ représentent tous les trois un radical alkyle, à une hydrolyse de l'ensemble des groupements alkyloxy, pour obtenir le composé de formule (h) définie ci-dessus,
(ix) que, le cas échéant, l'on traite par un réactif de protection des diols choisi parmi les composés de formules :
B(OR₄)₃, C(O)R₅R₆, C(O)Y₂ et CR₅R₆(Y)₂,
dans lesquelles R₄, R₅ et R₆ sont tels que définis à la revendication 1 et Y représente un atome d'halogène ou un groupement de formule Ar-O-, dans laquelle Ar représente un groupement phényle éventuellement substitué par 1 à 3 substituants choisi parmi les radicaux alkyle, alkyloxy, hydroxy, amino, alkylamino, dialkylamino et nitro ;
ou bien par un réactif de formule CH₂(Y)₂, Y étant défini comme plus haut, en présence d'un réactif de formule P(Hal)₅, dans laquelle Hal représente un atome d'halogène, suivi d'une hydrolyse, pour obtenir un composé de formule (i) : 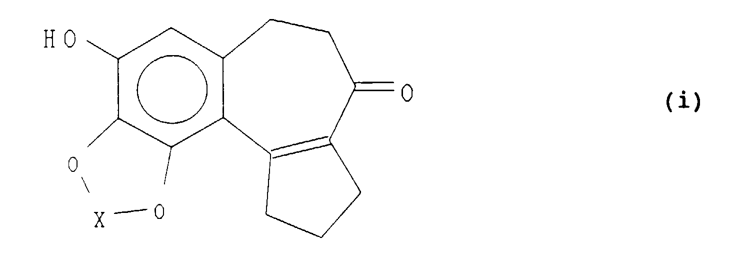 correspondant aux composés de formule (I) (d) telle que définie à la revendication 1, dans laquelle R₁ représente un atome d'hydrogène,
que l'on traite par un agent de formule A-SO₂Y, dans laquelle A est défini comme à la revendication 1 et Y est défini comme plus haut, pour obtenir un composé de formule (j) : 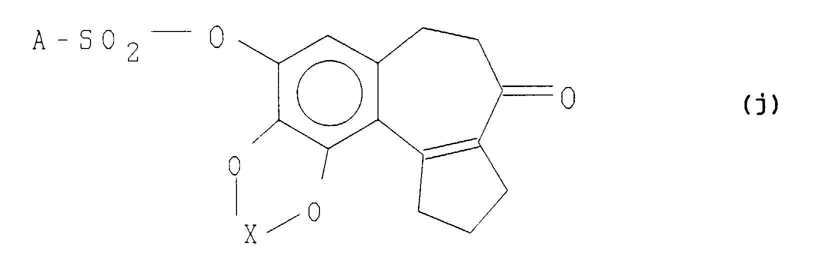 correspondant aux composés de formule (I)(d) telle que définie à la revendication 1, dans laquelle R₁ représente le groupement A-SO₂-, que, le cas échéant,
(x) l'on soumet à l'action d'un réactif de déprotection du diol, pour obtenir un composé de formule (k) : 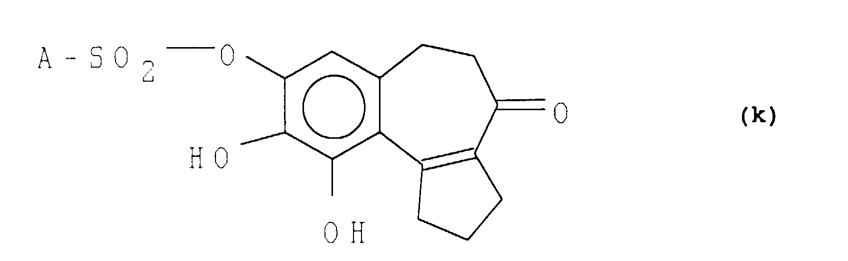 correspondant aux composés de formule (I) (b) telle que définie à la revendication 1, dans laquelle R₂ et R₃ représentent un atome d'hydrogène, que, le cas échéant,
(xi) l'on soumet à l'action d'un agent d'alkylation pour obtenir un composé de formule (l) : 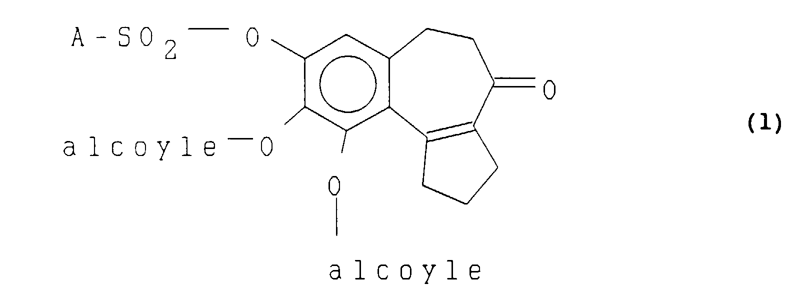 correspondant aux composés de formule (I) (b) telle que définie à la revendication 1, dans laquelle alkyle a la même signification qu'à la revendication 1,
(xii) ou bien soumet le cas échéant un composé de formule (j) ou (l) à l'action d'un agent d'hydrolyse du radical A-SO₂-O- pour obtenir respectivement un composé de formule (i) 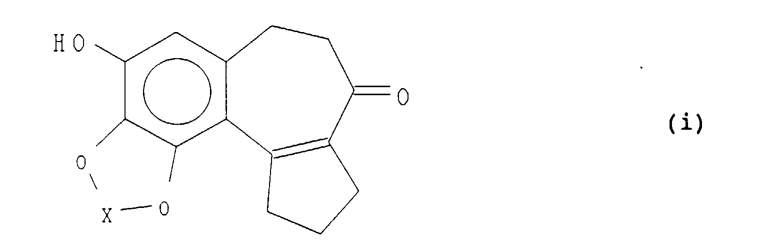 ou (l') 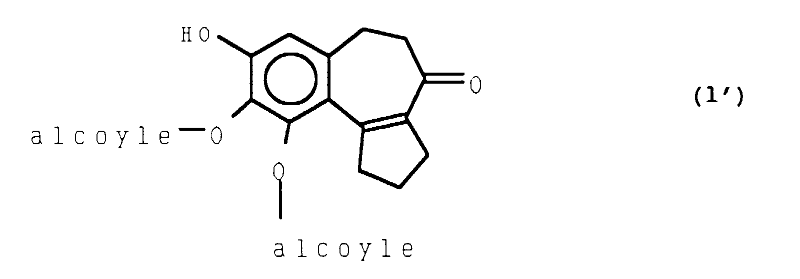 correspondant aux composés de formule (I) (f) telle que définie à la revendication 1.

12. Procédé selon la revendication 11, caractérisé en ce que
- l'agent d'halogénation utilisé au stade (i) est le chlorure de thionyle ;
- le réactif de formule (b) utilisé au stade (ii) est le 1-(N-morpholinyl)-cyclopentène;
- l'agent d'halogénation utilisé au stade (iii) est le chlorure d'oxalyle - (COCl)₂ ;
- l'acide de Lewis utilisé au stade (iv) est le chlorure ferrique ou le tétrachlorure d'étain ;
- le réactif de protection des diols utilisé au stade (ix) est le borate de triméthyle ou de triéthyle ;
- le réactif ASO₂Y que l'on fait agir au stade (ix) et tel que A représente un radical alkyle ou phényle substitué par un à trois radicaux alkyles.

13. Application d'un composé de formule (I) telle que définie à la revendication 1, à la synthèse des composés de formule (II) : 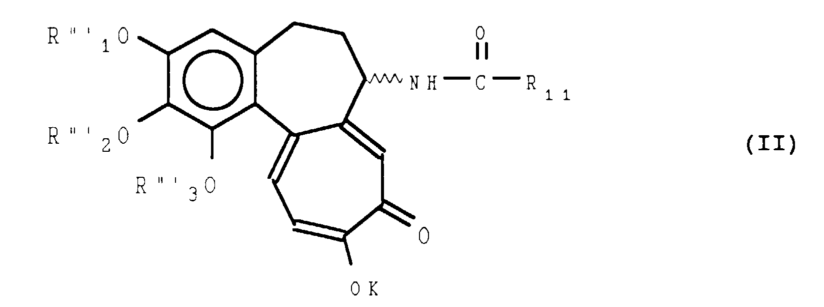 dans laquelle R"'₁, R"'₂ et R"'₃ ont les mêmes significations que les groupements R₁, R₂ et R₃ tels que définis à la revendication 1, à l'exception de la valeur hydrogène, et pour R₂-R₃, de la valeur X autre que -CR₅R₆- et, pour R₁-R₂, de la valeur X autre que -CR₅R₆-, R₁₁ représente un radical alkyle, le trait ondulé indique que le groupement NH-C(O)R₁₁ se trouve en position α ou β, ou que le composé est sous la forme d'un mélange d'isomères α + β, et K représente un radical alkyle, un radical benzyle éventuellement substitué sur le phényle par 1 à 3 radicaux alkyles, ou un radical -SO₂-A', dans lequel A' est choisi indépendamment parmi les valeurs de A mentionnées plus haut, caractérisée en ce que :
(i) soit l'on soumet un composé de formule (I), telle que définie plus haut, dans laquelle les groupements R₁, R₂ ou R₃ ne représentent pas un atome d'hydrogène, à l'action d'un agent de formule (m): 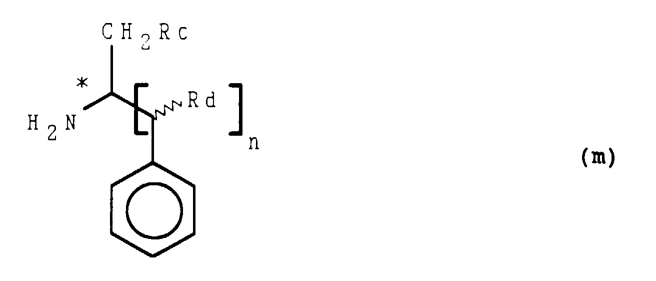 sous forme racémique ou optiquement active, dans laquelle Rc et Rd identiques ou différents représentent un atome d'hydrogène ou un radical hydroxy et n représente le chiffre 0 ou 1 ;
puis traite l'intermédiaire imino ainsi obtenu par un agent réducteur pour obtenir un composé de formule (n) : 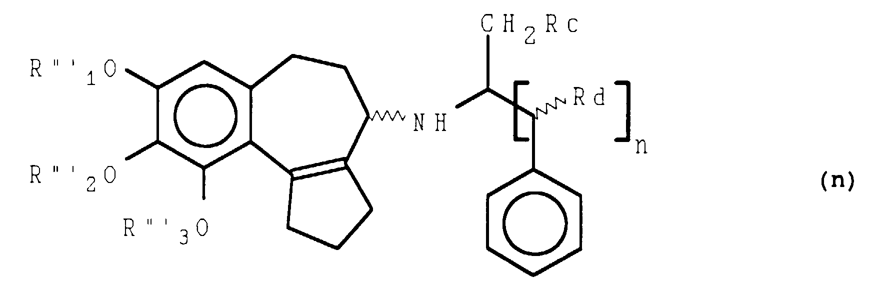 dans laquelle Rc et Rd ont la signification indiquée ci-dessus, et le trait ondulé reliant NH au cycle symbolise, selon que l'on a utilisé un agent de formule (m) racémique ou optiquement actif, respectivement un mélange d'isomères ou l'un ou l'autre isomère,
composé (n) que ou bien l'on soumet à l'hydrogénolyse du groupement alkylamino, pour obtenir un composé de formule (o) : 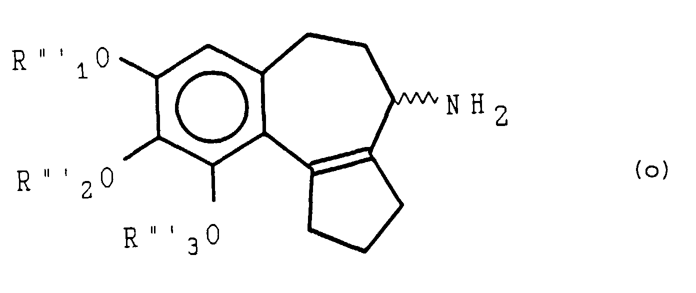 dans lequel le trait ondulé est défini comme ci-dessus, dont on protège le groupement amino avec un groupement protecteur bivalent pour obtenir un composé de formule (p) : 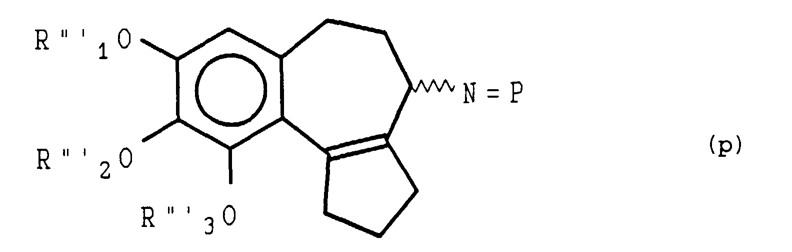 dans laquelle le trait ondulé est défini comme ci-dessus et P représente un groupement protecteur bivalent,
ou bien composé de formule (n) telle que définie ci-dessus, que l'on soumet à l'action d'un agent d'hydrolyse du groupement R"'₁O pour obtenir un composé de formule (n') : 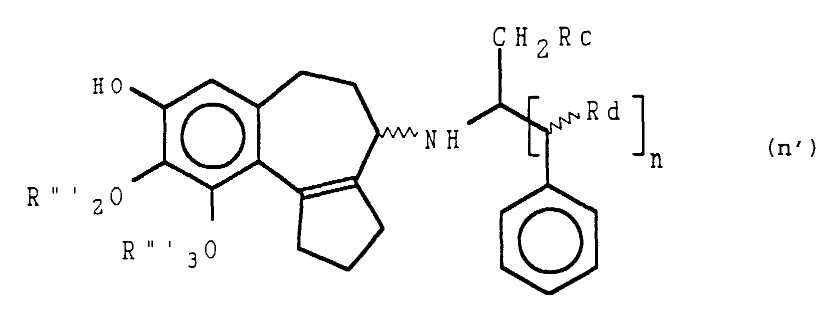 dans laquelle R"'₂, R"'₃, Rc, Rd, n et les traits ondulés ont la signification indiquée précédemment, que l'on soumet à l'hydrogénolyse du groupement alkylamino, pour obtenir un composé de formule (o') : 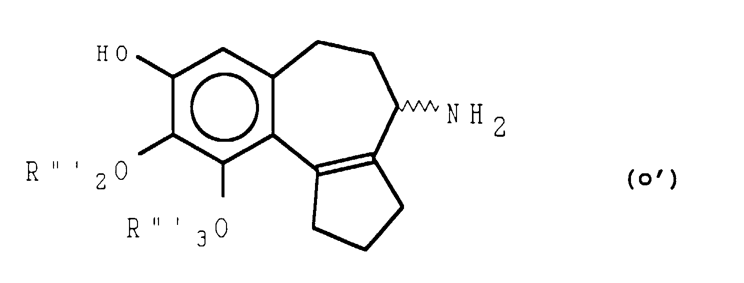 dans lequel le trait ondulé est défini comme ci-dessus, dont on protège le groupement amino avec un groupement protecteur bivalent pour obtenir un composé de formule (p') : 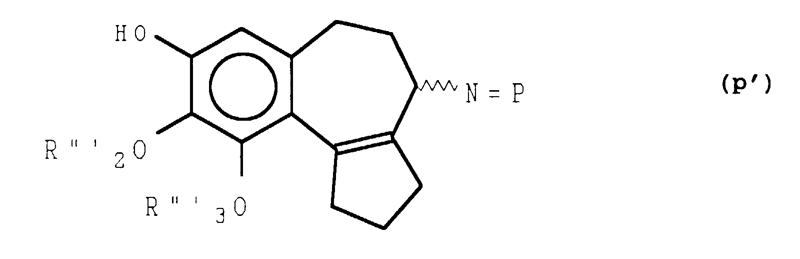 dans laquelle P et le trait ondulé ont la signification indiquée ci-dessus, que l'on soumet à l'action d'un agent protecteur du radical hydroxy pour obtenir un composé de formule (p) telle que définie précédemment ;
(ii) soit l'on soumet un composé de formule (I) (f) telle que définie plus haut, à l'action d'un agent de formule (m) définie ci-dessus, traite l'intermédiaire imino obtenu par un agent réducteur pour obtenir un composé de formule (n') telle que définie ci-dessus, puis poursuit la synthèse comme indiqué ci-dessus ;
(iii) soit l'on soumet un composé de la formule (I) telle que définie plus haut, à l'action d'un agent de formule R₈O-NH₂, ou un sel de ce composé, dans laquelle R₈ représente un atome d'hydrogène ou un radical alkyle, pour obtenir un composé de formule (q) : 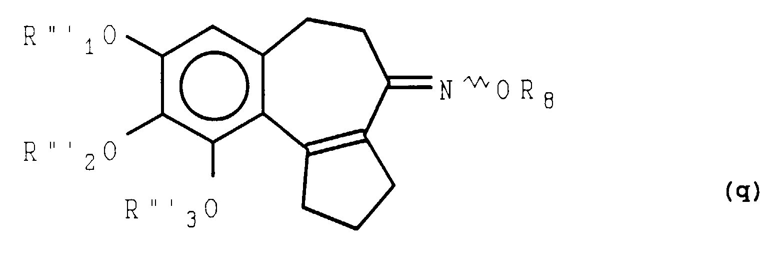 dans laquelle le trait ondulé indique que le composé consiste en un mélange d'isomères syn et anti,
(iv) soit l'on soumet un composé de formule (I) telle que définie plus haut, à l'action ou bien d'un agent réducteur chiral, ou bien d'un agent réducteur non chiral, pour obtenir l'alcool correspondant, de formule (r) : 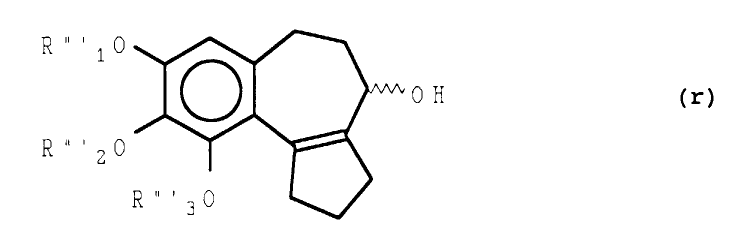 dans laquelle le trait ondulé entre l'atome d'azote et le cycle à 7 chaînons indique que ce produit consiste selon le réducteur utilisé, ou bien en un isomère α ou β, ou bien en un mélange d'isomères α + β, mélange que le cas échéant l'on dédouble, puis soit soumet l'alcool à l'action d'un agent de formule HN=P, dans laquelle P a la signification donnée précédemment, pour obtenir un composé de formule (p) : 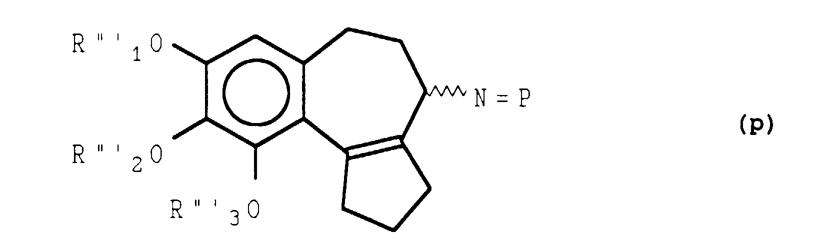 dans laquelle le trait ondulé entre l'atome d'azote et le cycle à 7 chaînons indique que ce produit consiste en un isomère α ou β, ou à un mélange racémique d'isomères, soit soumet l'alcool à l'action d'un réatif de formule ASO₂Hal, dans laquelle A est défini comme plus haut et Hal représente un atome d'halogène pour obtenir le composé de formule (r₁) : 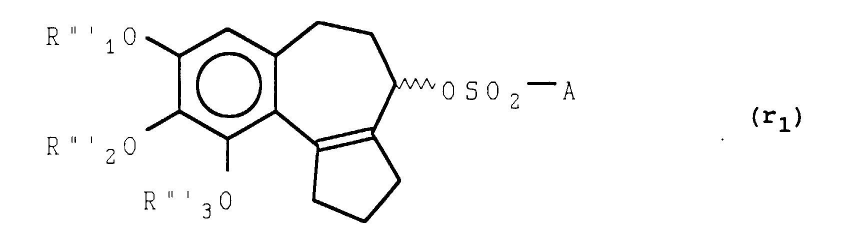 dans laquelle le trait ondulé et A ont les significations ci-dessus, que l'on fait agir avec l'ammoniac, pour obtenir le composé de formule (o) ci-dessus, dont on protège le groupement amino avec un groupement protecteur bivalent, pour obtenir le composé de formule (p) ci-dessus, puis,
(v) l'on soumet un composé de formule (p) ou (q) à l'action d'un agent de bromation en présence d'un composé de formule R₇OH, dans laquelle R₇ représente un atome d'hydrogène ou un radical alkyle, pour obtenir un composé de formule (s) : 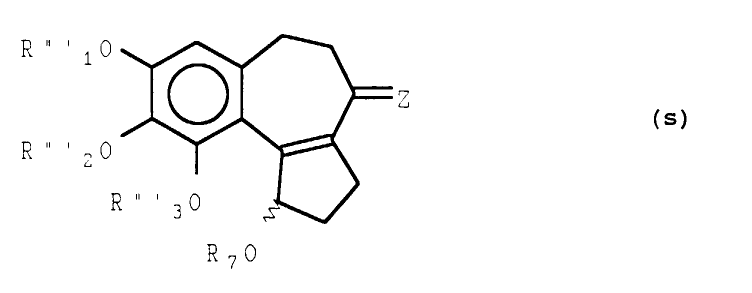 dans laquelle le trait ondulé indique que la liaison peut se trouver en position alpha ou béta ou que ce produit peut consister en un mélange d'isomères et dans laquelle le groupement =Z représente un groupement de formule = N 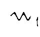 OR₈ ou 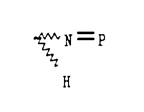 telles que définies dans les formules p et q, ci-dessus,
(vi) que l'on soumet à une déshydratation ou une désalcoxylation en milieu acide anhydre pour obtenir un composé de formule (t) : 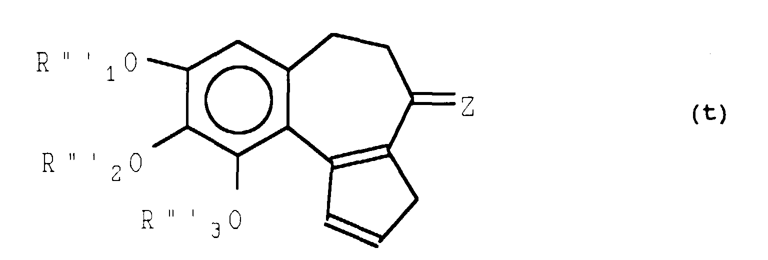
(vii) que l'on soumet en présence d'une base ou d'un métal réducteur, à l'action d'un agent de formule 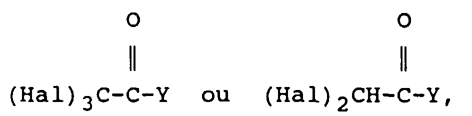 dans laquelle Hal représente un atome d'halogène et Y est défini comme plus haut, pour obtenir un composé de formule (u) : 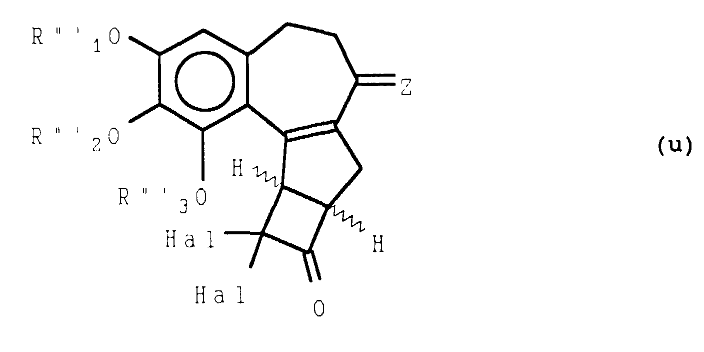 dans laquelle les traits ondulés indiquent que les atomes d'hydrogène se trouvent tous les deux en position alpha ou tous les deux en position béta ;
(viii) que l'on soumet à l'action d'un acide carboxylique en présence d'une base et en milieu aqueux, pour obtenir un composé de formule (v), lequel existe en équilibre avec son tautomère (v') : 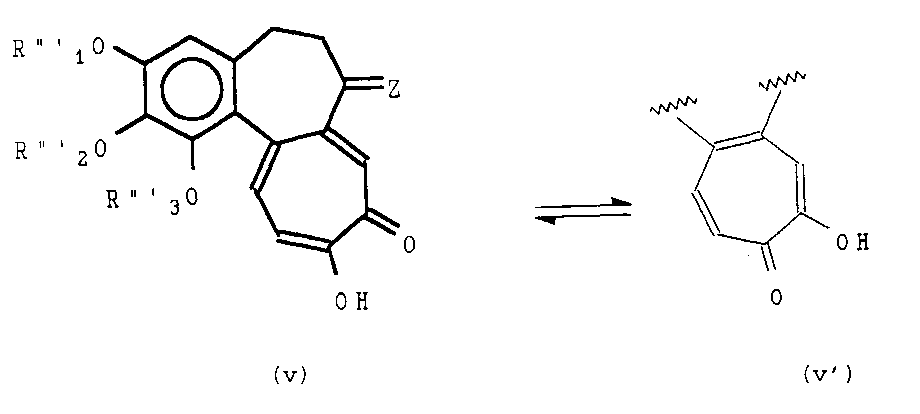 lequel composé, ou bien,
(ix) si =Z représente un groupement de formule 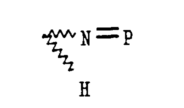 tel que défini plus haut, soit l'on soumet à une réaction de déprotection du groupement amino pour obtenir soit l'amine libre de formule (w), lequel existe en équilibre avec son tautomère (w') : 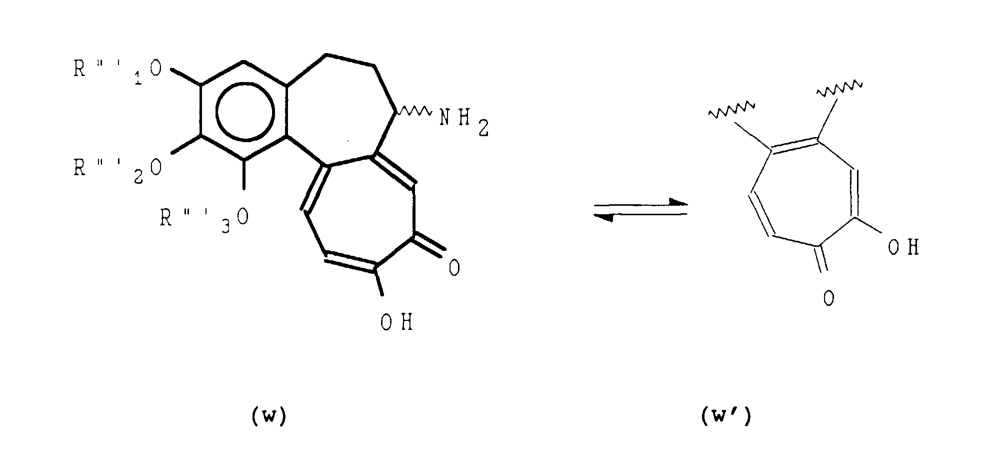 dans laquelle le trait ondulé indique que le groupement NH₂ se trouve en position alpha ou béta, ou que le produit consiste en un mélange d'isomères,
que l'on soumet à l'action d'un réactif de formule R₁₁-C(O)Y ou de formule (R₁₁CO)₂O, dans laquelle le groupement R₁₁ représente un groupement alkyle et Y est défini comme à la revendication 11, pour obtenir un composé de formule (x) : 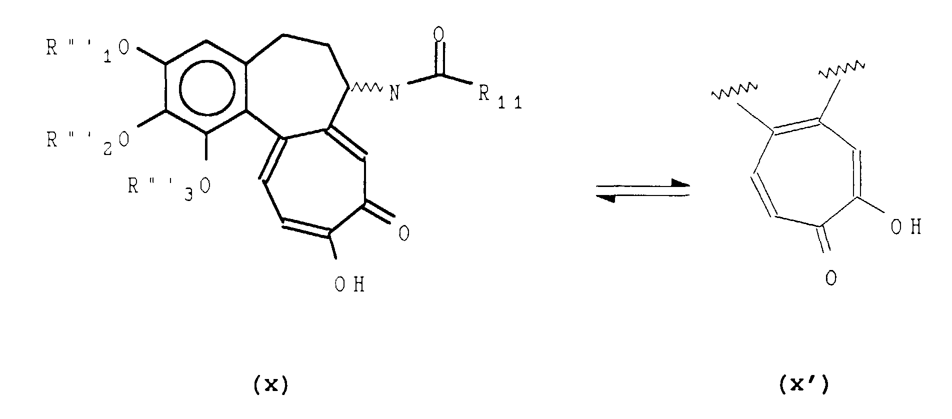 dans laquelle le trait ondulé a la signification précédente, soit l'on soumet à une réaction de déprotection du groupement amino et à un agent d'hydrolyse du groupement R"'₁ dans le cas où ce groupement représente la valeur A-SO₂- telle que définie plus haut, pour obtenir un composé de formule (w₁) : 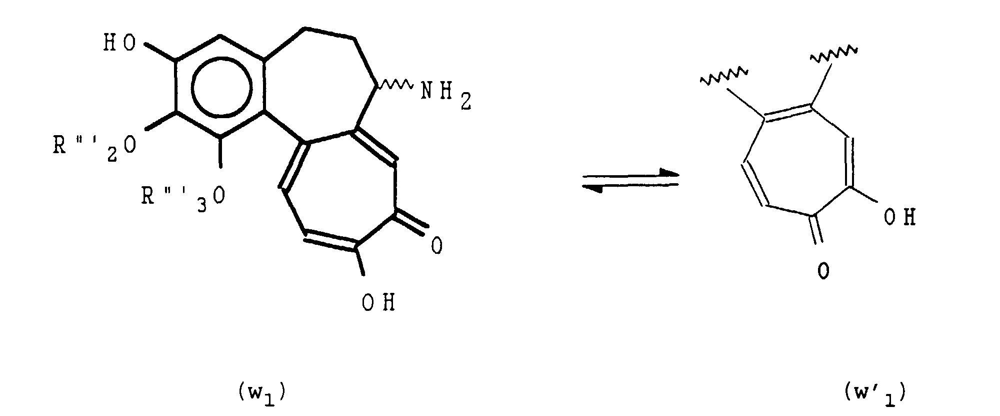 que l'on soumet à l'action d'un composé de formule R₁₁C(O)Y ou (R₁₁CO)₂O telle que définie plus haut, pour obtenir un composé de formule (x₁) : 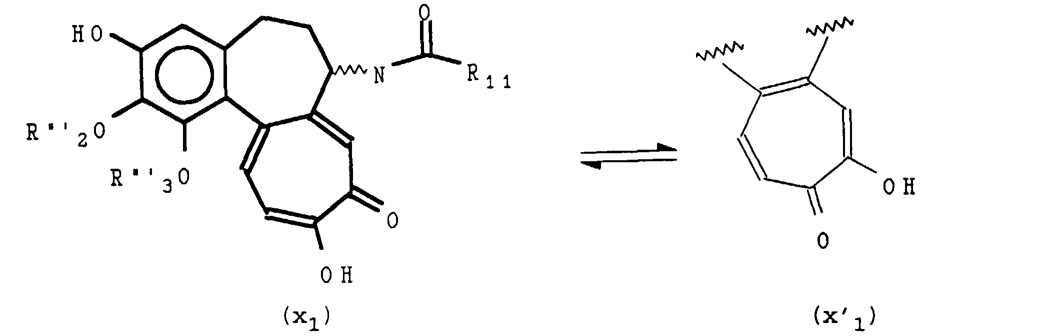 dont on protège la fonction OH par action d'un réactif de formule ASO₂Y tel que défini plus haut, pour obtenir un composé correspondant de formule (x)/(x') telle que définie plus haut, ou bien,
(x) si =Z représente un groupement de formule =N~OR₈, l'on soumet à l'action d'un agent protecteur de formule B(Hal)₃ ou B(OR₄)₃ dans lesquelles R₄ et Hal sont définis comme précédemment, puis à l'action d'un agent réducteur pour obtenir un composé de formule (y) : 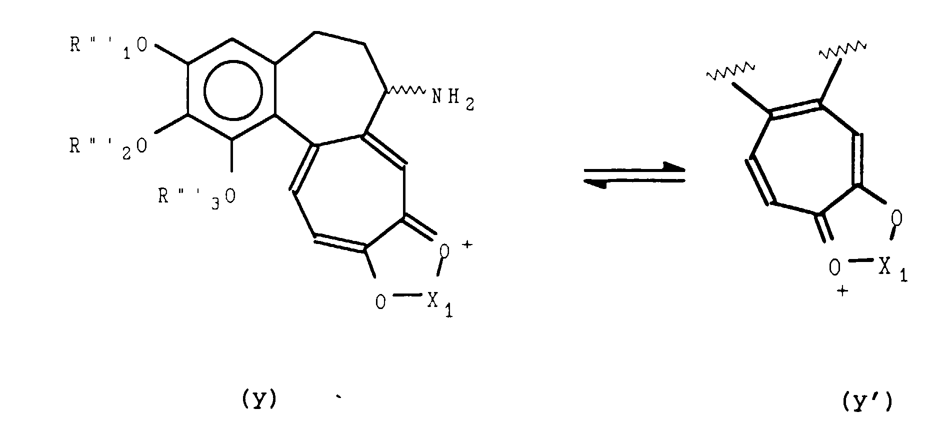 dans laquelle le trait ondulé indique que ce produit consiste en un mélange d'isomères, et X₁ représente un groupement de formule -B(OR₄)₂- ou -B(Hal)₂, que l'on soumet à l'action d'un composé de formule R₁₁C(O)Y ou de formule (R₁₁CO)₂O, telle que définie plus haut puis à l'action d'un réactif de déprotection du groupement alpha-hydroxy cétone, pour obtenir un composé de formule (x")/(x"₁) correspondant au produit de formule (x)/(x') telle que définie ci-dessus, dans laquelle le trait ondulé indique que le produit consiste en un mélange d'isomères ;
(xi) lequel composé de formule (x) ou (x') l'on soumet à l'action d'un agent susceptible d'introduire le groupement K défini plus haut, pour obtenir un composé de formule (II) : 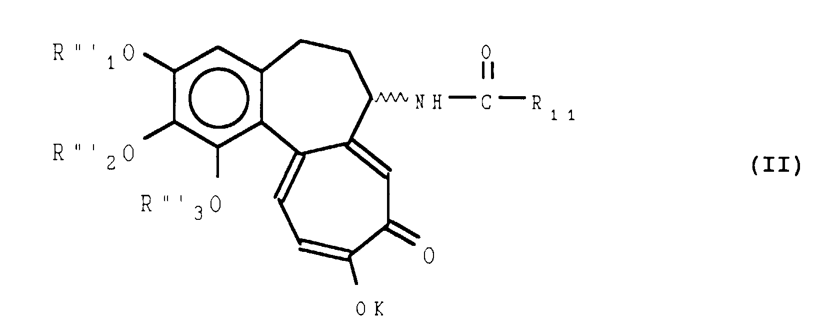 lequel existe en mélange avec son régioisomère de formule : 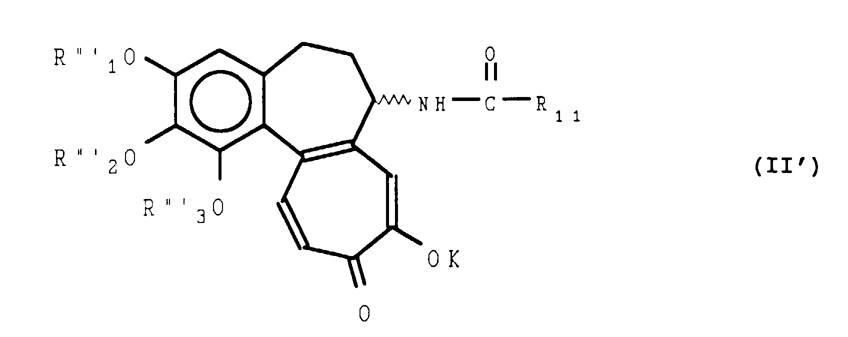 formules (II) et (II') dans lesquelles le trait ondulé indique que le groupement se trouve en position alpha ou béta, ou que ce produit consiste en un mélange d'isomères et les termes R"'₁, R"'₂, R"'₃, R₁₁, K et alkyle ayant les significations données précédemment, mélange dont on sépare les constituants.

14. Application selon la revendication 13, caractérisée en ce que l'amine de formule (m) utilisé au stade (i) ou (ii) est la (S)-α-méthylbenzylamine, la noréphédrine ou la norpseudoéphédrine.

15. Application selon la revendication 13 ou 14, caractérisée en ce que le groupement protecteur du radical amino est un groupement phatlimido ou de type maléimido.

16. Application selon la revendication 13, caractérisée en ce que l'agent de formule R₈O-NH₂ est la méthylhydroxylamine.

17. Application selon l'une quelconque des revendications 13 à 16, caractérisée en ce que le réactif R₇OH est le méthanol.

18. Application selon l'une quelconque des revendications 13 à 17, caractérisée en ce que le dérivé halogéné que l'on fait réagir avec le composé (t) est le chlorure de dichloroacétyle.

19. Application selon l'une quelconque des revendications 13 à 18, caractérisée en ce que R₁₁ représente un radical méthyle.

20. Application selon l'une quelconque des revendications 13 à 19, caractérisée en ce que le réactif ASO₂Y que l'on fait agir sur le composé (x₁)/(x'₁) est le chlorure de mésyle ou tosyle.

21. Application selon l'une quelconque des revendications 13 à 20, caractérisée en ce que l'agent susceptible d'introduire le groupement K est le chlorure de mésyle, de tosyle ou de 2,4,6-triisopropylphényl sulfonyle.

22. Application selon l'une quelconque des revendications 13 à 21, caractérisée en ce que :
- on fait réagir l'agent (m) avec le composé (I) en présence de tétrachlorure de titane et d'une base aminée tertiaire ou en présence d'un catalyseur acide ;
- la protection du groupement amino est effectuée par action de l'anhydride phtalique sur le composé (o) ou (o') ;
- l'agent de formule HN=P est l'anhydride phtalique et la réaction avec le composé (r) est réalisée en présence de triphénylphosphine et d'un azobicarboxylate d'alkyle ;
- le réarrangement en tropolone est effectué par action de l'acide acétique sur le composé (u), en présence d'une base aminée ;
- la déprotection de l'amine est effectuée par l'hydrazine ;
- l'hydrolyse sélective du composé (x)/(x') est effectuée par action d'un thioalcoolate alcalin ;
- le réactif formant le groupement protecteur des fonctions hydroxy et cétone est le trifluorure de bore ;
- l'agent réducteur que l'on fait agir sur l'oxime issue du composé (v)/(v') est le zinc, en présence d'un acide sulfonique ou carboxylique.

23. Application selon la revendication 13, caractérisée en ce que l'on soumet un composé de formule (v)/(v') telle que définie à la revendication 13 dans laquelle Z représente un groupement 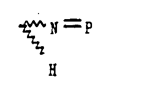 à l'action d'un agent susceptible d'introduire le groupement K tel que défini à la revendication 13, pour obtenir un composé de formule (III) : 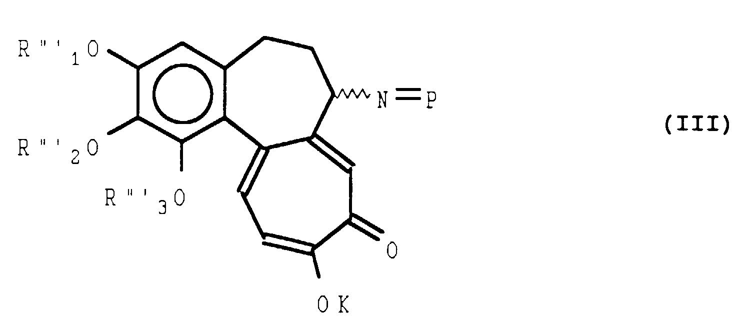 lequel existe en mélange avec son régioisomère de formule : 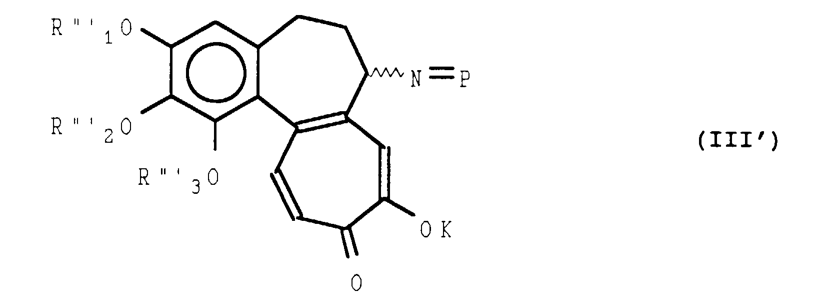 formules (III) et (III') dans lesquelles le trait ondulé indique que le groupement se trouve en position alpha ou béta, ou que ce produit consiste en un mélange d'isomères et les termes R‴₁, R‴₂, R‴₃, R₁₁ et K et alkyle ayant les significations données à la revendication 13, mélange dont le cas échéant, on sépare les constituants, puis soumet le mélange ou les constituants séparés, à une réaction de déprotection de la fonction amino, pour obtenir soit le composé de formule (IV) : 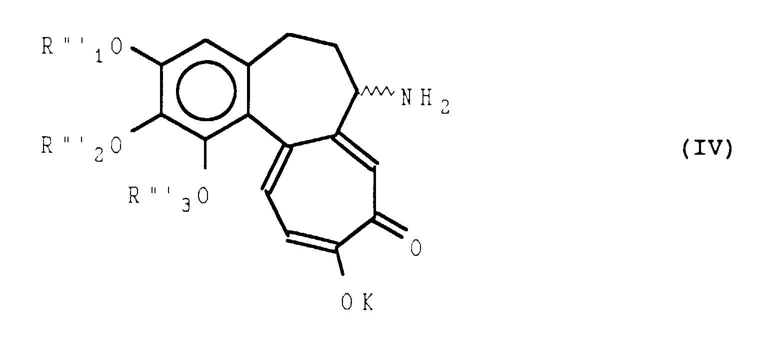 sous forme de mélange avec son régioisomère de formule 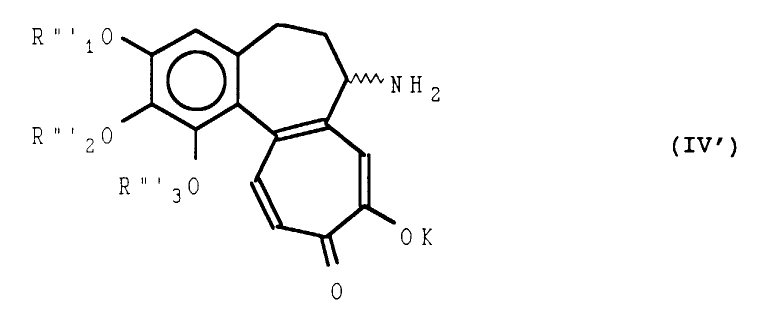 mélange que, le cas échéant, l'on sépare,
soit l'un ou l'autre de ces régioisomères, puis soumet le mélange ou les constituants séparés, à l'action d'un composé de formule R₁₁C(O)Y ou (R₁₁CO)₂O, telle que définie à la revendication 13, pour obtenir soit le composé de formule (II) telle que définie plus haut, en mélange avec son régioisomère de formule (II'), mélange que, le cas échéant, l'on sépare en ses constituants, soit l'un ou l'autre de ces régioisomères.

24. Application selon la revendication 23, caractérisée en ce que l'agent susceptible d'introduire le groupement K, le réactif de déprotection de la fonction amino et R₁₁ sont tels que définis aux revendications 19, 21 et 22.

25. Application selon la revendication 13, caractérisée en ce que l'on soumet un composé de formule (v)/(v') telle que définie précédemment, dans laquelle (z) représente un groupement =N-OR₈, à l'action d'un agent susceptible d'introduire le groupement K, pour obtenir un composé de formule (V) : 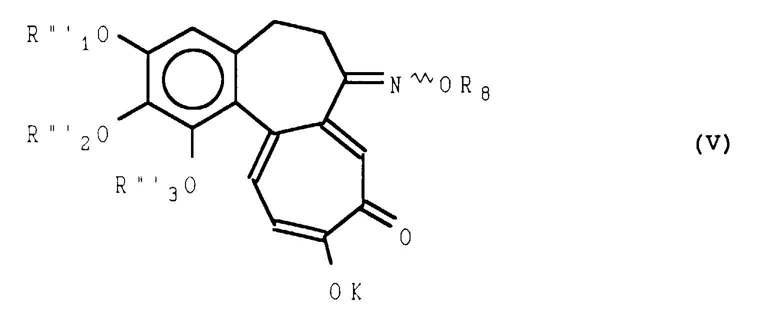 lequel existe en mélange avec son régioisomère de formule 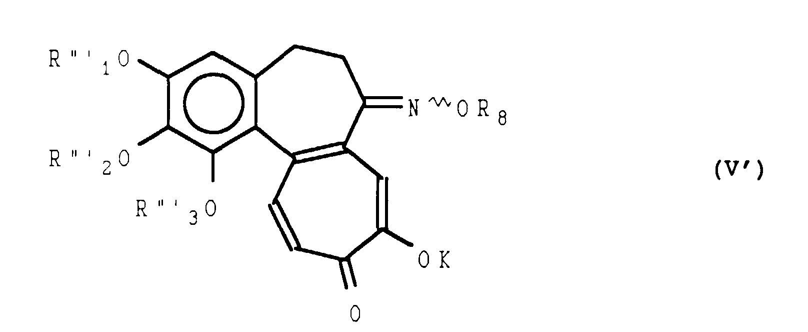 formules (V) et (V') dans lesquelles le trait ondulé, R₈ et les termes R"'₁, R"'₂, R"'₃, K et alkyle ont les significations données à la revendication 13, mélange dont le cas échéant, on sépare les constituants, puis soumet le mélange ou les constituants séparés, à l'action d'un agent réducteur, pour obtenir soit le composé de formule (IV₁) : 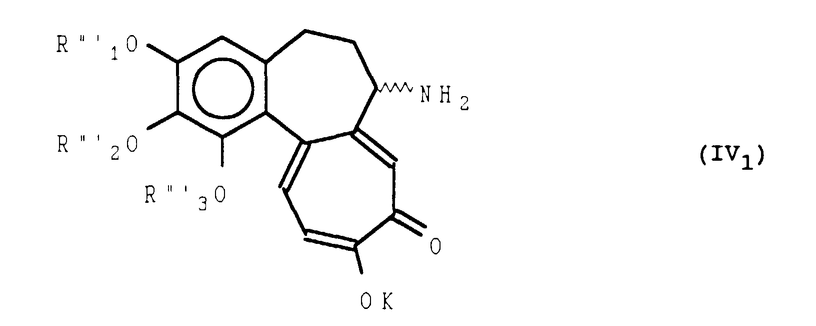 sous forme de mélange avec son régioisomère de formule 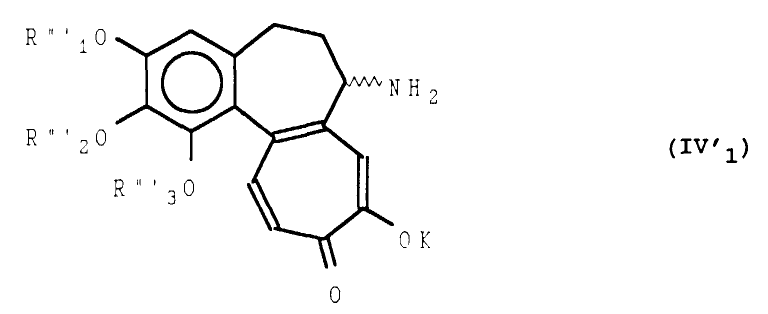 le trait ondulé symbolisant un mélange d'isomères, mélange de régioisomères que, le cas échéant, l'on sépare,
soit l'un ou l'autre de ces régioisomères, puis poursuit la synthèse comme décrit à la revendication 13 pour les composés de formule (IV)/(IV').

26. Application selon la revendication 25, caractérisée en ce que l'agent susceptible d'introduire le groupement K et l'agent réducteur de l'oxime sont tels que définis aux revendications 21 et 22.

27. Application selon la revendication 13, caractérisée en ce que en outre l'on soumet un produit de formule (II₁) : 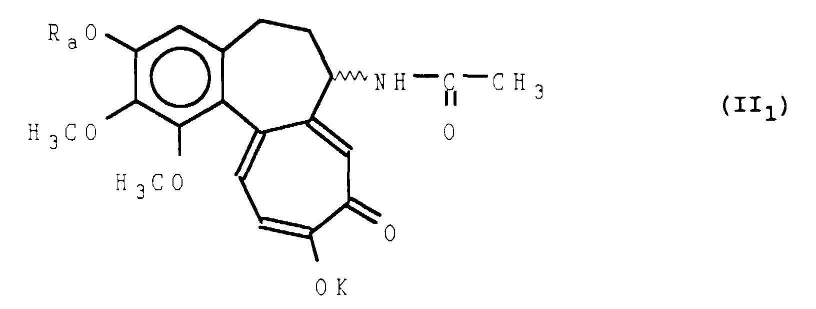 dans laquelle K est défini comme à la revendication 13, Rₐ représente un radical ASO₂- tel que défini à la revendication 13 et le trait ondulé symbolise l'un ou l'autre isomère ou un mélange d'isomères,
à l'action d'un réactif de formule CH₃S⁻Na⁺ pour obtenir un composé de formule (VI) : 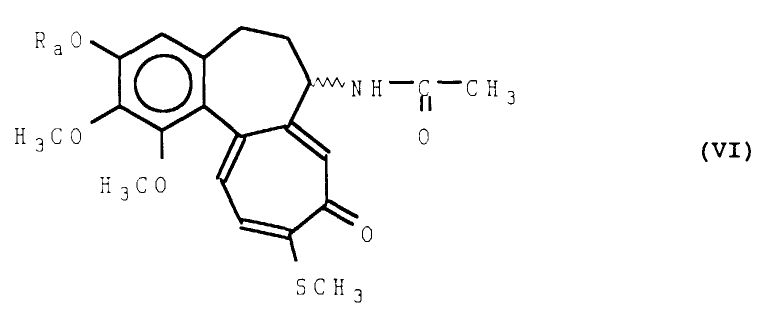 dans laquelle Rₐ et le trait ondulé ont la signification précédente que l'on soumet à un réactif de déprotection du radical hydroxy pour obtenir le composé de formule (VII) : 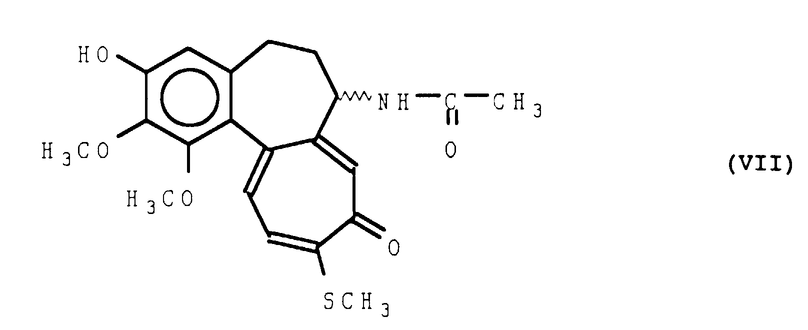 optiquement actif ou racémique.

28. Application selon la revendication 27, caractérisée en ce que l'on substitue le groupement OK et déprotège le radical hydroxy avec le même réactif CH₃SNa.

29. Application selon la revendication 13, caractérisée en ce que en outre l'on soumet un composé de formule (II₂) : 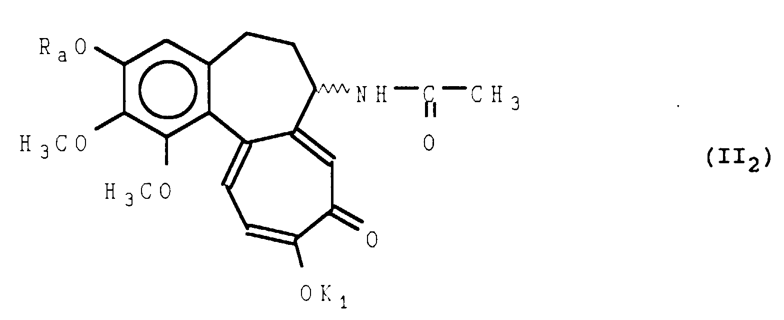 dans laquelle Rₐ est défini comme à la revendication 13, K₁ représente un radical méthyle et le trait ondulé symbolise l'un ou l'autre isomère ou un mélange d'isomères, à l'action d'un agent de déprotection du radical hydroxy, pour obtenir le composé de formule (VIII) : 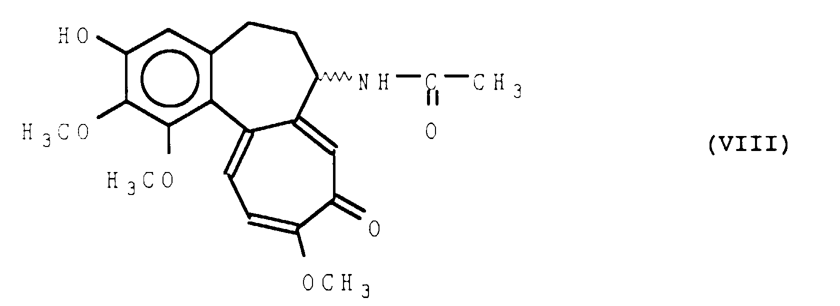 optiquement actif ou racémique.

30. Application selon la revendication 13, caractérisée en ce que en outre l'on soumet un composé de formule (II₃) : 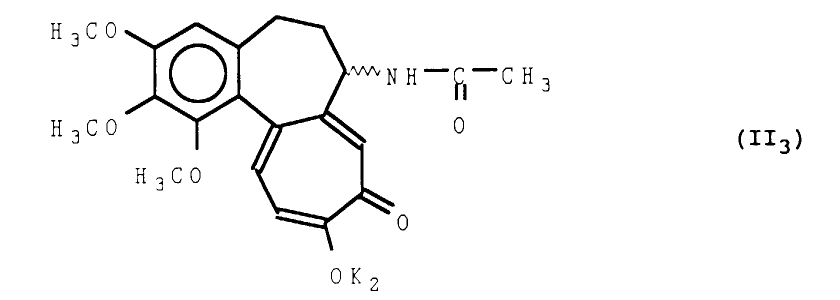 dans laquelle K₂ représente un radical benzyle éventuellement substitué sur le phényle par 1 à 3 radicaux alkyles, ou un radical -SO₂A' tel que défini précédemment et le trait ondulé symbolise l'un ou l'autre isomère, ou un mélange d'isomères, à l'action d'un réactif de formule CH₃S⁻Na⁺, pour obtenir le composé de formule (IX) : 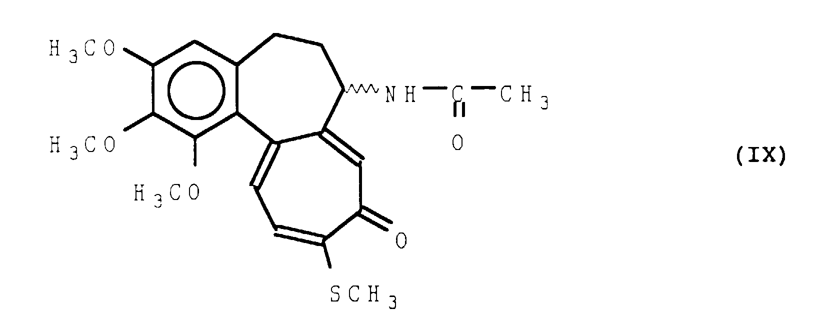 optiquement actif ou racémique.

31. Application selon la revendication 13, caractérisée en ce que en outre l'on soumet un composé de formule (II₄) : 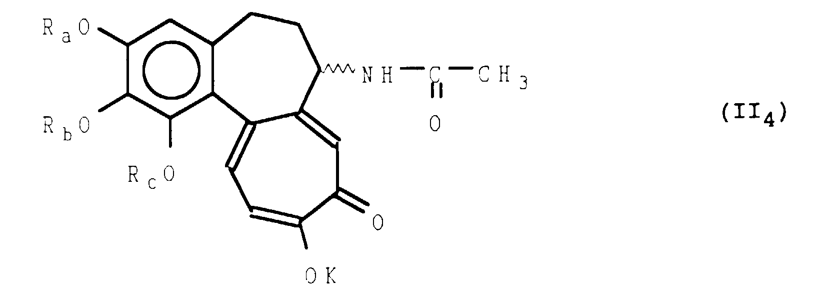 dans laquelle Rₐ et K sont définis comme à la revendication 13, R_{b} et R_{c} forment ensemble un groupement CR₅R₆ tel que défini à la revendication 1 et le trait ondulé symbolise l'un ou l'autre isomère ou un mélange d'isomères, à l'action d'un réactif de déprotection du diol, pour obtenir un composé de formule (X) : 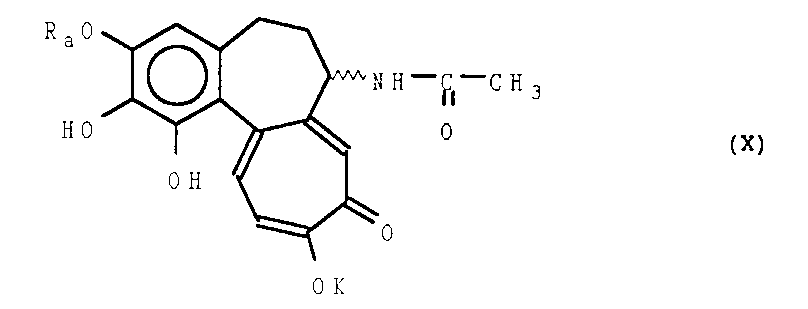 dans laquelle Rₐ, K et le trait ondulé ont la signification précédente que l'on traite par un agent de méthylation pour obtenir un composé de formule (II₁) telle que définie à la revendication 27, puis poursuit la synthèse comme décrit à la revendication 27 au départ dudit composé de formule (II₁).

32. Application selon la revendication 13, caractérisée en ce que en outre l'on soumet un composé de formule (II₅) : 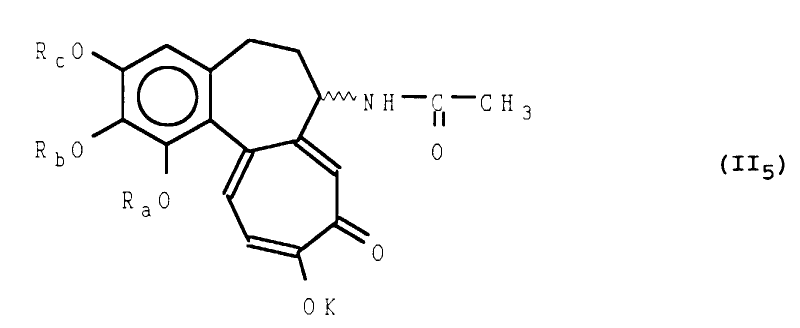 dans laquelle Rₐ, R_{b}, R_{c}, K et le trait ondulé sont définis comme dans la formule (II₄) à la revendication 31, à l'action d'un agent de déprotection du diol, pour obtenir un composé de formule (XI) : 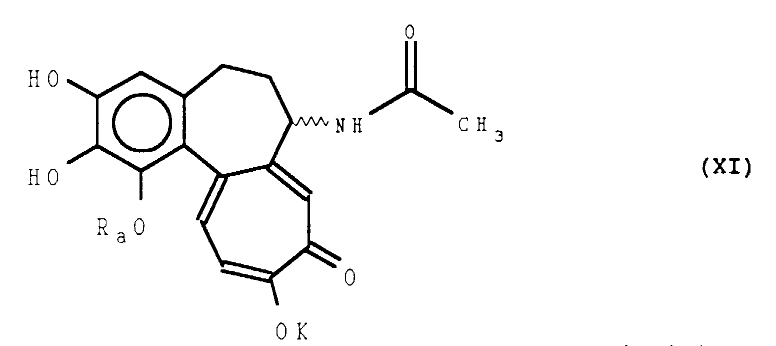 dans laquelle Rₐ, K et le trait ondulé ont la signification précédente, que l'on soumet à l'action d'un agent d'hydrolyse pour obtenir le composé de formule (XII) : 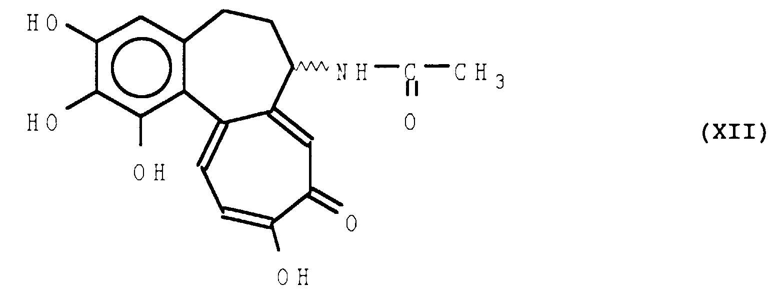 que l'on soumet à un agent de méthylation, pour obtenir la colchicine optiquement active ou racémique.

33. A titre de composés industriels nouveaux, les composés de formule (F₁) : 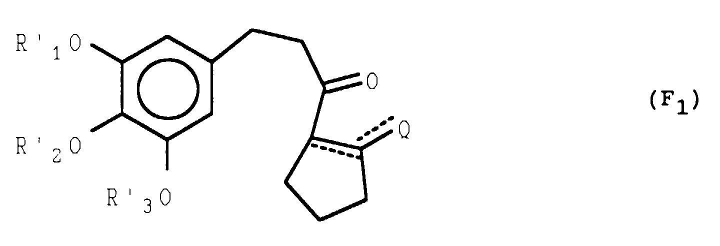 dans laquelle soit Q représente un radical oxo, soit le pointillé dans le cycle représente une seconde liaison et Q représente un atome d'halogène, R'₁, R'₂ et R'₃ ont la signification indiquée à la revendication 11.

34. A titre de composés industriels nouveaux, les composés de formule (F₂) : 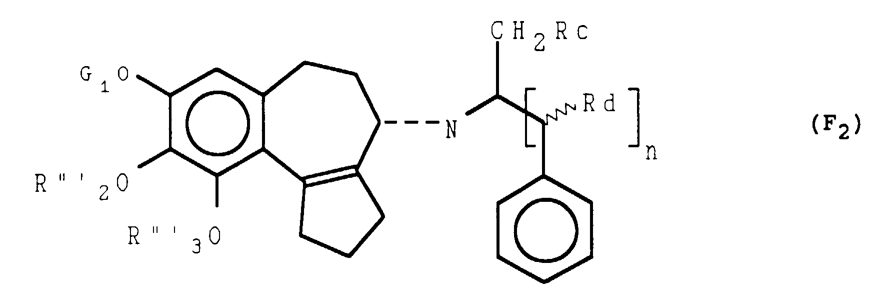 dans laquelle G₁ représente un atome d'hydrogène ou le radical R"'₁ tel que défini ci-dessus et Rc et Rd ainsi que R"'₂ et R"'₃ ont la signification indiquée à la revendication 13, le trait pointillé symbolise soit une double liaison, soit une simple liaison et, dans ce cas, l'azote porte un atome d'hydrogène et le composé se présente sous la forme d'un mélange d'isomères (R,S) ou d'isomère (R) ou (S).

35. A titre de composés industriels nouveaux, les composés de formule (F₃) : 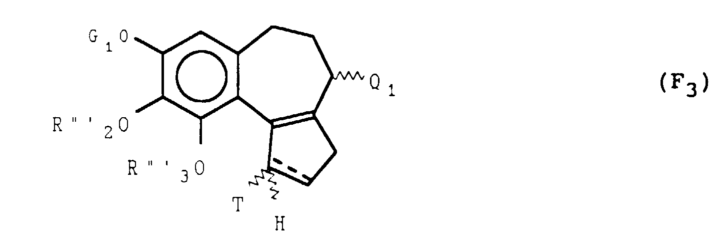 dans laquelle R"'₂ et R"'₃ ont la signification indiquée à la revendication 13, et ou bien G₁ représente le radical R"'₁ tel que défini précédemment et :
- soit Q₁ représente un radical NH₂ de configuration (R), (S) ou (R,S) et T représente un atome d'hydrogène ;
- soit Q₁ représente un groupe N=P de configuration (R), (S) ou (R,S), P étant défini comme à la revendication 13, et ou bien T représente un atome d'hydrogène ou un radical hydroxy ou alcoxy renfermant de 1 à 6 atomes de carbone, de configuration (R), (S) ou (R,S), ou bien le trait pointillé représente une seconde liaison ;
- soit Q₁ représente un groupe = N 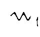 OR₈ dans lequel R₈ a la signification indiquée à la revendication 13, et le trait ondulé indique que la liaison peut être syn ou anti ou que le produit peut consister en un mélange d'isomères syn et anti et ou bien T représente un atome d'hydrogène ou un radical hydroxy ou alcoxy renfermant de 1 à 6 atomes de carbone, de configuration (R), (S) ou (R,S), ou bien le trait pointillé représente une seconde liaison ;
- soit Q₁ représente un radical OH ou OSO₂A, A étant défini comme plus haut, de configuration (R), (S) ou (R,S) et T représente un atome d'hydrogène,
ou bien G₁ représente un atome d'hydrogène et
Q₁ représente soit un radical NH₂ de configuration (R), (S) ou (R,S) soit un groupe N=P de configuration (R), (S) ou (R,S), P étant défini comme à la revendication 13 et T représente un atome d'hydrogène, le pointillé ne représentant pas de double liaison.

36. A titre de composés industriels nouveaux, les composés de formule (u) : 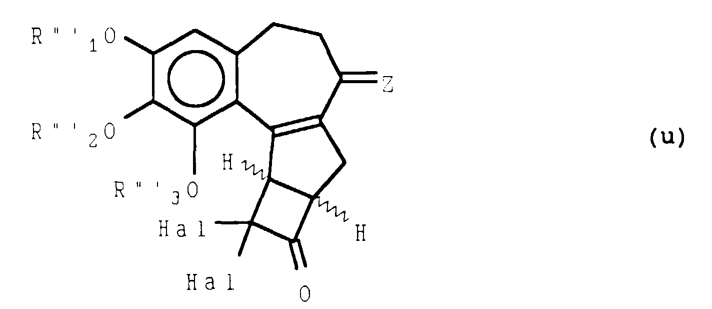 dans laquelle R"'₁, R"'₂, R"'₃, Hal et Z sont tels que définis à la revendication 13.

37. A titre de composés industriels nouveaux, les composés de formule (F₄) : 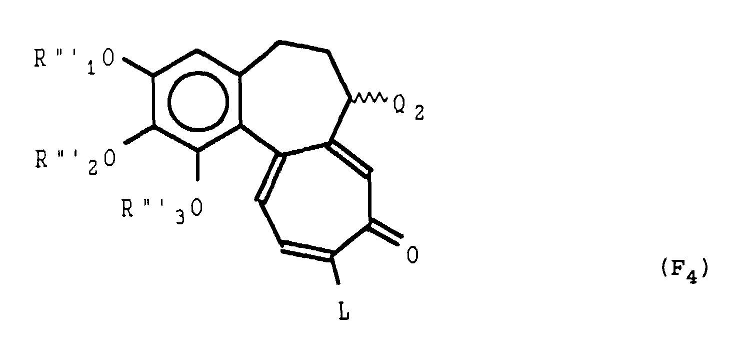 ainsi que leurs tautomères ou régioisomères de formule : 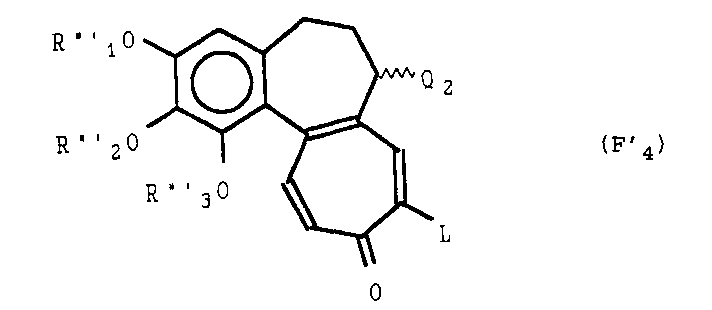 dans laquelle R"'₁, R"'₂ et R"'₃ ont la signification indiquée à la revendication 13 et 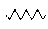 Q₂ représente :
- soit un groupe = N 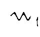 OR₈ tel que défini plus haut et L représente un radical hydroxy,
- soit un groupe 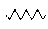 N = P dans lequel P a la signification indiquée à la revendication 13, le trait ondulé indique que ce groupe se trouve dans la configuration (R), (S) ou (R,S) et L représente un radical hydroxy ou -OK tel que défini à la revendication 13, à l'exception des composés dans lesquels R"'₁, R"'₂ et R"'₃ représentent un radical alkyle, L représente un radical hydroxy ou alkoxy et P représente un groupement =C-phényle ou CH₃-C-OC₂H₅ ;
- soit un groupe 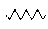 NH₂ de configuration (R), (S) ou (R,S) et L représente un radical hydroxy, à l'exception des composés dans lesquels R"'₁, R"'₂ et R"'₃ représentent un radical alkyle ;
- soit un groupe 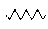 NH₂ configuration (R,S) et L forme avec l'oxygène de la cétone en position 9, un groupe O-X₁-O⁺ tel que défini à la revendication 13 ;
- soit un groupe 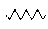 NH₂ de configuration (R), (S) ou (R,S) et L représente un radical OK tel que défini à la revendication 13, à l'exception des composés dans lesquels R"'₁, R"'₂ et R"'₃ représentent un radical alkyle et K représente un radical alkyle ;
- soit un groupe = N 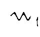 OR₈ tel que défini à la revendication 13 et L représente un radical OK tel que défini à la revendication 13.

38. A titre de composés industriels nouveaux, les composés de formule (F₅) : 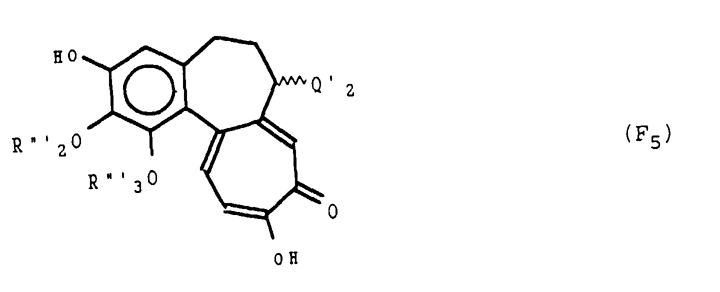 ainsi que leurs tautomères de formule : 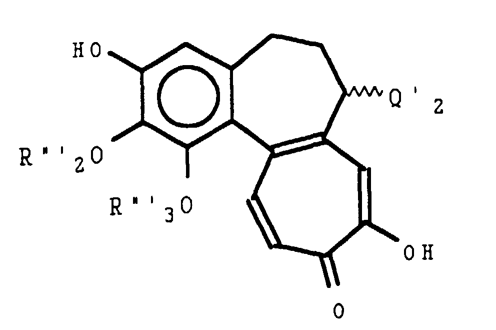 dans laquelle R"'₂ et R"'₃ ont la signification indiquée à la revendication 13, à l'exception de la valeur alkyle et 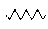 Q'₂ représente :
- soit un groupe 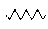 NH-CO-R₁₁ dans lequel R₁₁ a la signification indiquée à la revendication 13,
- soit un groupe 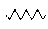 NH₂ de configuration (R), (S) ou (R,S).

39. A titre de composés industriels nouveaux, les composés de formule (F₆) : 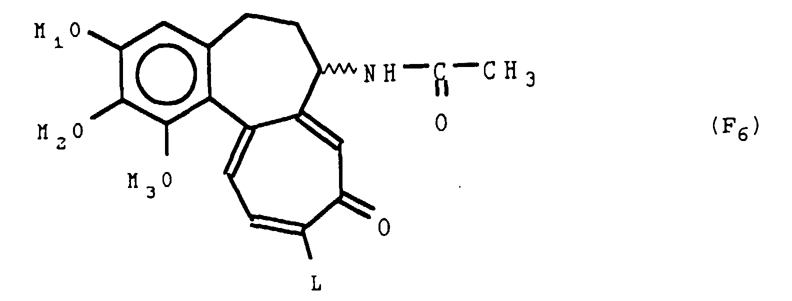 dans laquelle le trait ondulé indique que le substituant se trouve dans la configuration (R), (S) ou (R,S) et
- soit M₁ et M₂ représentent un atome d'hydrogène, M₃ représente un radical Ra tel que défini à la revendication 27 et L représente un radical OK, K étant tel que défini à la revendication 13,
- soit M₂ et M₃ représentent un atome d'hydrogène, M₁ représente un radical Ra tel que défini ci-dessus et L représente un radical OK tel que défini ci-dessus,
- soit M₁ représente un radical Ra tel que défini ci-dessus, M₂ et M₃ représentent un radical méthyle et L représente un radical thiométhyle.

## Patentansprüche

1. Verbindungen der Formel (I): 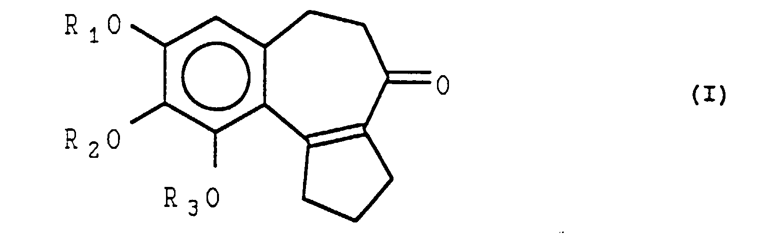 worin:
entweder (a) R₁ und R₂ beide eine Alkylgruppe bedeuten und R₃ ein Wasserstoffatom oder die Gruppe A-SO₂- wiedergibt,
oder (b) R₂ und R₃ beide ein Wasserstoffatom oder beide eine Alkylgruppe bedeuten und R₁ für eine Gruppe A-SO₂- steht,
oder (c) R₁, R₂ und R₃ alle drei ein Wasserstoffatom oder alle drei eine Alkylgruppe bedeuten,
oder (d) R₁ für die Gruppe -A-SO₂- oder ein Wasserstoffatom steht und R₂ und R₃ gemeinsam mit den Sauerstoffatomen, an die sie gebunden sind, eine Gruppe der Formel -O-X-O- bilden, worin X für eine Gruppe der Formel -B(OR₄)- steht, wobei R₄ ein Wasserstoffatom, eine Alkylgruppe, eine Gruppe -C(O)- oder eine Gruppe der Formel -CR₅R₆- bedeutet, wobei R₅ und R₆ ein Wasserstoffatom, eine Alkyl- oder Phenylgruppe, gegebenenfalls substituiert durch 1 bis 3 Gruppen, ausgewählt unter den Alkyl-, Hydroxy- und Alkoxyresten, wiedergeben oder R₅ und R₆ gemeinsam mit dem Kohlenstoffatom, an das sie gebunden sind, eine carbocyclische Gruppe mit 5 oder 6 Gliedern darstellen,
oder (e) R₅ die Gruppe A-SO₂- wiedergibt und R₁ und R₂ gemeinsam mit den Sauerstoffatomen, an die sie gebunden sind, die Gruppe -O-X-O- bilden,
oder (f) R₁ ein Wasserstoffatom ist und R₂ und R₃ beide eine Alkylgruppe darstellen oder gemeinsam mit den Sauerstoffatomen, an die sie gebunden sind, eine Gruppe der Formel -O-X-O-, wie vorstehend definiert, bilden,
"A" eine Alkylgruppe, eine unsubstituierte Phenylgruppe oder eine Phenylgruppe, substituiert durch 1 bis 3 Alkylgruppen, oder eine unsubstituierte Naphthylgruppe oder eine Naphthylgruppe, substituiert durch 1 bis 3 Alkylgruppen, bedeutet,
wobei die vorstehend erwähnten Alkyl- oder Alkoxygruppen verzweigt oder unverzeigt sind und 1 bis 6 Kohlenstoffatome umfassen.

2. Verbindungen der Formel (I) gemäß Anspruch 1, dadurch gekennzeichnet, daß R₁, R₂ und R₃ alle drei ein Wasserstoffatom oder alle drei eine Alkylgruppe, wie in Anspruch 1 definiert, bedeuten.

3. Verbindung gemäß Anspruch 2, dadurch gekennzeichnet, daß R₁, R₂ und R₃ alle drei eine Methylgruppe bedeuten oder alle drei ein Wasserstoffatom wiedergeben.

4. Verbindungen der Formel (I) gemäß Anspruch 1, dadurch gekennzeichnet, daß R₁ eine Gruppe A-SO₂-, wie in Anspruch 1 definiert, bedeutet und R₂ und R₃ beide eine Alkylgruppe, wie in Anspruch 1 definiert, oder beide ein Wasserstoffatom darstellen.

5. Verbindung gemäß Anspruch 4, dadurch gekennzeichnet, daß R₁ eine Tosylgruppe bedeutet und R₂ und R₃ beide eine Methylgruppe oder beide ein Wasserstoffatom wiedergeben.

6. Verbindungen der Formel (I) gemäß Anspruch 1, dadurch gekennzeichnet, daß R₃ eine Gruppe A-SO₂-, wie in Anspruch 1 definiert, bedeutet und R₁ und R₂ beide einen Alkylrest, wie in Anspruch 1 definiert, wiedergeben.

7. Verbindung gemäß Anspruch 6, dadurch gekennzeichnet, daß R₃ eine Tosylgruppe ist und R₁ und R₂ beide eine Methylgruppe bedeuten.

8. Verbindungen der Formel (I) gemäß Anspruch 1, dadurch gekennzeichnet, daß R₁ und R₂ beide eine Alkylgruppe bedeuten und R₃ für ein Wasserstoffatom steht.

9. Verbindung gemäß Anspruch 8, dadurch gekennzeichnet, daß R₁ und R₂ beide eine Methylgruppe bedeuten.

10. Verbindung gemäß Anspruch 1, dadurch gekennzeichnet, daß R₁ ein Wasserstoffatom ist und R₂ und R₃ beide eine Methylgruppe wiedergeben.

11. Verfahren zur Herstellung der Verbindungen der Formel (I), wie in Anspruch 1 definiert, dadurch gekennzeichnet, daß
(i) man eine Verbindung der Formel (a): 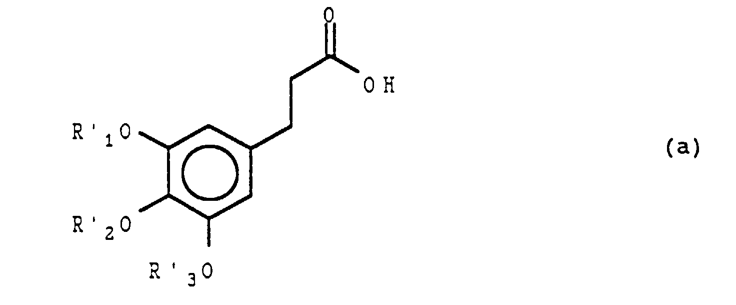 worin
R₁', R₂' und R₃' jeweils die Bedeutung der Gruppen R₁, R₂ und R₃, wie in Anspruch 1 definiert, mit Ausnahme der Bedeutung Wasserstoff und für R₃' der Bedeutung A-SO₂- besitzen,
der Einwirkung eines Halogenierungsmittels unterzieht, um zu dem entsprechenden Acylhalogenid zu gelangen,
(ii) welches man der Einwirkung eines Reagens der Formel(b): 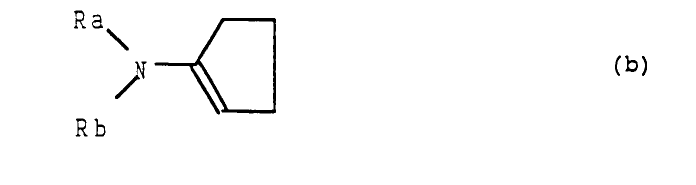 unterzieht, worin Ra und Rb, identisch oder verschieden, eine Alkylgruppe bedeuten oder Ra und Rb gemeinsam mit dem Stickstoffatom, an das sie gebunden sind, einen Heterocyclus mit 5 oder 6 Gliedern, der gegebenenfalls ein weiteres Heteroatom, ausgewählt unter O und N, umfaßt, bilden,
um zu einer Verbindung der Formel (c) 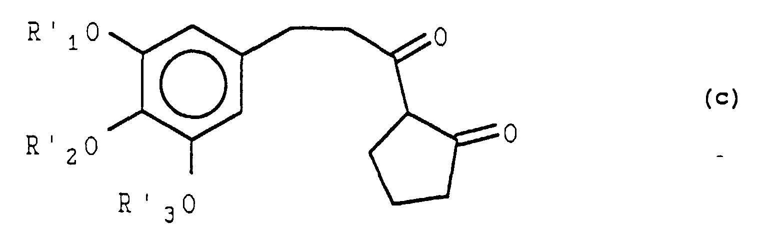 zu gelangen,
(iii) welche man der Einwirkung eines Halogenierungsmittels unterzieht, um zu einer Verbindung der Formel (d): 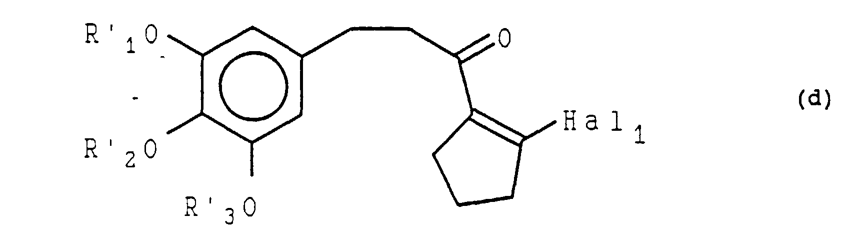 zu gelangen, worin Hal₁ für ein Halogenatom steht;
(iv) welche man der Einwirkung einer Lewissäure unterzieht, um zu einer Verbindung der Formel (e): 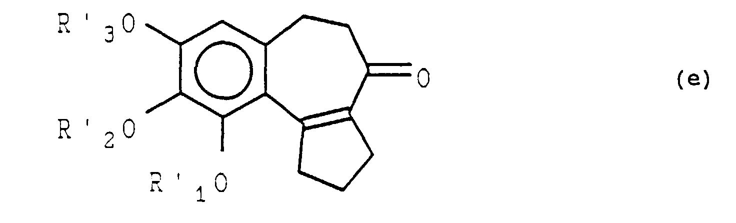 zu gelangen, die den Verbindungen der Formel (I)(a), wie in Anspruch 1 definiert, worin R₃ eine Gruppe A-SO₂- bedeutet, den Verbindungen der Formel (I)(c),wie in Anspruch 1 definiert, worin R₁, R₂ und R₃ alle drei für eine Alkylgruppe stehen, und den Verbindungen der Formel (I)(e), wie in Anspruch 1 definiert entspricht; und die Verbindung der Formel (e) gegebenenfalls
(v) - entweder, wenn R'₁ eine Gruppe A-SO₂- bedeutet und R'₂ und R'₃ nicht gemeinsam die Gruppe -X- bilden, man der Einwirkung eines Hydrolysemittels unterzieht, um eine Verbindung der Formel (f): 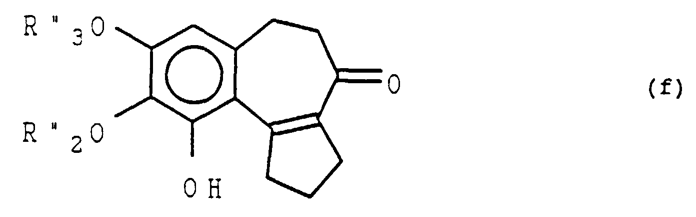 zu erhalten, worin R"₂ und R"₃ beide einen Alkylrest bedeuten; entsprechend den Verbindungen der Formel (I)(a), wie in Anspruch 1 definiert, worin R₃ für ein Wasserstoffatom steht;
welche man gegebenenfalls
(vi) der Einwirkung eines Alkylierungsmittels unterzieht, um zu einer Verbindung der Formel (g): 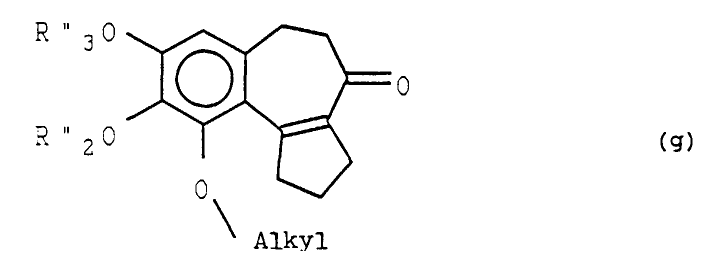 entsprechend den Verbindungen der Formel (I)(c), wie in Anspruch 1 definiert, worin R₁, R₂ und R₃ einen Alkylrest bedeuten, zu gelangen, die gegebenenfalls
(vii) man einer Hydrolyse der Gesamtheit der Alkoxygruppen unterzieht, um die Verbindung der Formel (h): 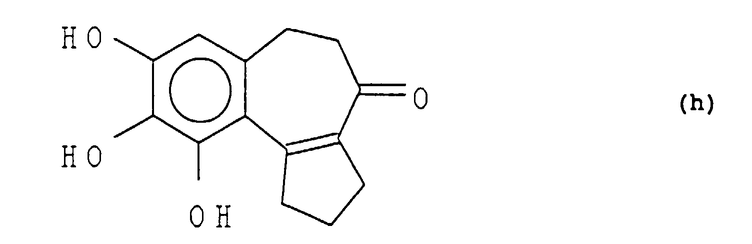 entsprechend der Verbindung der Formel (I)(c), wie in Anspruch 1 definiert, worin R₁, R₂ und R₃ ein Wasserstoffatom bedeuten, zu erhalten,
(viii) oder man eine Verbindung der Formel (e), worin R'₁, R'₂ und R'₃ alle drei einen Alkylrest wiedergeben, einer Hydrolyse der Gesamtheit der Alkoxygruppen unterzieht, um zu der Verbindung der Formel (h), wie vorstehend definiert, zu gelangen,
(ix) die man gegebenenfalls mit einem Reagens zum Schutz der Diole, ausgewählt unter den Verbindungen der Formeln:
B(OR₄)₃, C(O)R₅R₆, C(O)Y₂ und CR₅R₆(Y)₂,
behandelt, worin R₄, R₅ und R₆ wie in Anspruch 1 definiert sind und Y ein Halogenatom oder eine Gruppe der Formel Ar-O- bedeutet, worin Ar eine Phenylgruppe, gegebenenfalls substituiert durch 1 bis 3 Substituenten, ausgewählt unter den Alkyl-, Alkoxy-, Hydroxy-, Amino-, Alkylamino-, Dialkylamino- und Nitrogruppen, wiedergibt;
oder mit einem Reagens der Formel CH₂(Y)₂, worin Y wie vorstehend definiert ist, in Gegenwart eines Reagens der Formel P(Hal)₅, worin Hal für ein Halogenatom steht, woran sich eine Hydrolyse anschließt, um zu einer Verbindung der Formel (i): 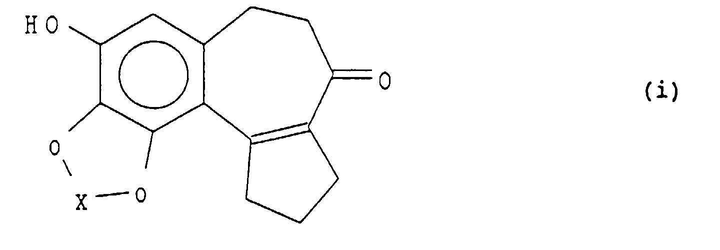 entsprechend den Verbindungen der Formel (I)(d), wie in Anspruch 1 definiert, worin R₁ ein Wasserstoffatom bedeutet, zu gelangen,
die man mit einem Mittel der Formel A-SO₂Y, worin A wie in Anspruch 1 definiert ist und Y wie vorstehend definiert ist, behandelt, um zu einer Verbindung der Formel (j): 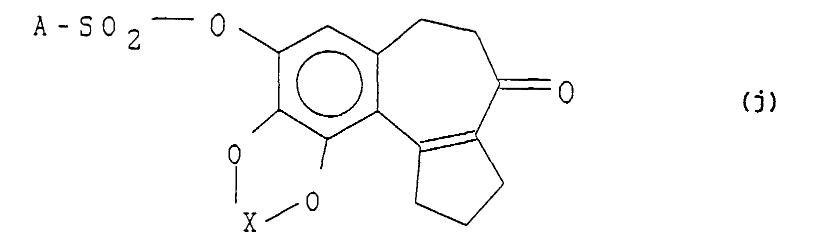 entsprechend den Verbindungen der Formel (I)(d), wie in Anspruch 1 definiert, worin R₁ die Gruppe A-SO₂- wiedergibt, zu gelangen, die gegebenenfalls
(x) man der Einwirkung eines Reagens für die Abspaltung der Schutzgruppe des Diols unterzieht, um eine Verbindung der Formel (k): 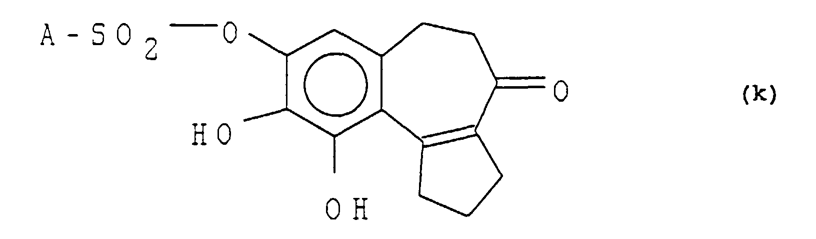 entsprechend den Verbindungen der Formel (I)(b), wie in Anspruch 1 definiert, worin R₂ und R₃ für ein Wasserstoffatom stehen, zu erhalten, die gegebenenfalls
(xi) man der Einwirkung eines Alkylierungsmittels unterzieht, um eine Verbindung der Formel (l): 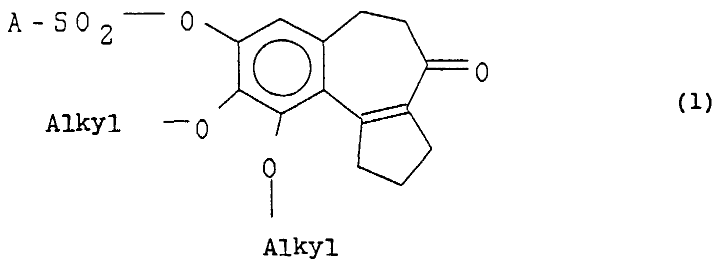 entsprechend den Verbindungen der Formel (I)(b), wie in Anspruch 1 definiert, worin Alkyl die gleiche Bedeutung wie in Anspruch 1 besitzt, zu erhalten;
(xii) oder gegebenenfalls eine Verbindung der Formel (j) oder (l) der Einwirkung eines Mittels für die Hydrolyse des Restes A-SO₂-O- unterzieht, um zu einer Verbindung der Formel (i) 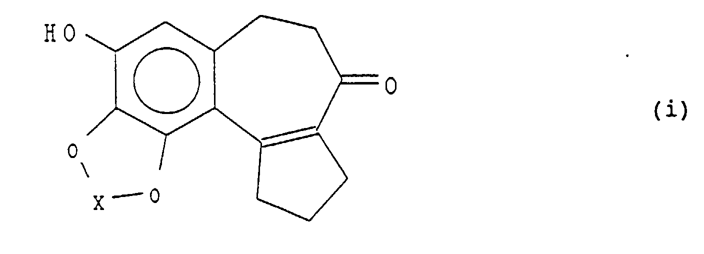 oder (1') 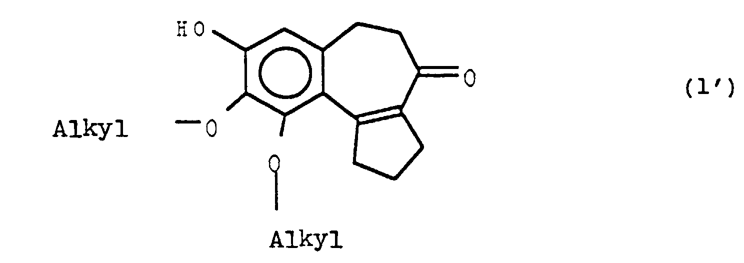 entsprechend den Verbindungen der Formel (I)(f), wie in Anspruch 1 definiert, zu gelangen.

12. Verfahren gemäß Anspruch 11, dadurch gekennzeichnet, daß
- das in Stufe (i) verwendete Halogenierungsmittel das Thionylchlorid ist;
- das in Stufe (ii) verwendete Reagens der Formel (b) 1-(N-Morpholinyl)-cyclopenten ist;
- das in Stufe (iii) verwendete Halogenierungsmittel das Oxalylchlorid - (COCl)₂ - ist;
- die in Stufe (iv) verwendete Lewissäure Ferrichlorid oder Zinntetrachlorid ist;
- das in Stufe (ix) verwendete Reagens für den Schutz der Diole das Trimethyl- oder Triethylborat ist;
- das Reagens ASO₂Y, welches man in Stufe (ix) einsetzt, derart ist, daß A für einen Alkyl- oder Phenylrest, substituiert durch ein bis drei Alkylgruppen, steht.

13. Verwendung einer Verbindung der Formel (I), wie in Anspruch 1 definiert, bei der Synthese der Verbindungen der Formel (II): 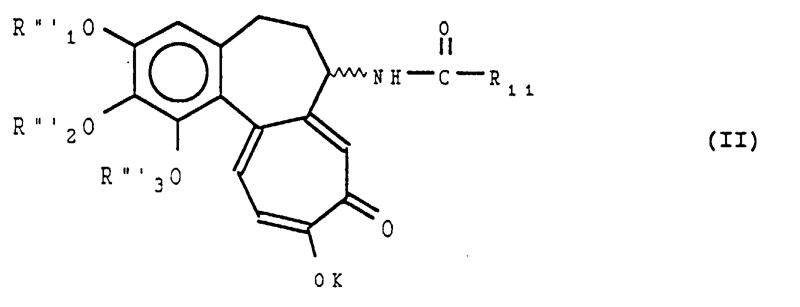 worin R"'₁, R"'₂ und R"'₃ die gleichen Bedeutungen wie die Gruppen R₁, R₂ und R₃, wie in Anspruch 1 definiert, besitzen mit Ausnahme der Bedeutung Wasserstoff und für R₂-R₃ der Bedeutung X, verschieden von -CR₅R₆-,und für R₁-R₂ der Bedeutung X, verschieden von -CR₅R₆, R₁₁ einen Alkylrest bedeutet, die gewellte Linie anzeigt, daß die Gruppe NH-C(O)R₁₁ sich in α- oder β-Stellung befindet oder daß die Verbindung in Form eines Gemisches der α- + β-Isomeren vorliegt, und K einen Alkylrest, einen an dem Phenyl gegebenenfalls durch 1 bis 3 Alkylreste substituierten Benzylrest oder einen Rest -SO₂-A' bedeutet, worin A' unabhängig unter den vorstehend erwähnten Bedeutungen von A ausgewählt ist, dadurch gekennzeichnet, daß:
(i) entweder man eine Verbindung der Formel (I), wie vorstehend definiert, worin die Gruppen R₁, R₂ oder R₃ kein Wasserstoffatom bedeuten, der Einwirkung eines Mittels der Formel (m): 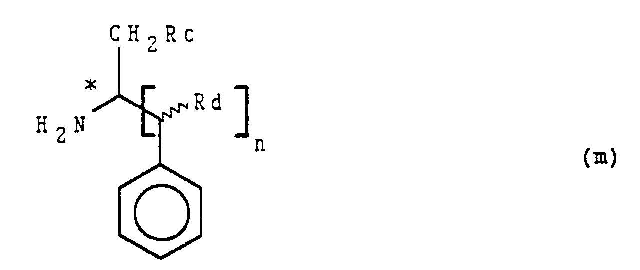 in racemischer oder optisch aktiver Form, worin Rc und Rd, identisch oder verschieden, ein Wasserstoffatom oder einen Hydroxyrest bedeuten und n die Zahl 0 oder 1 wiedergibt, unterzieht;
hiernach das so erhaltene Imino-Zwischenprodukt mit einem Reduktionsmittel behandelt, um zu einer Verbindung der Formel (n): 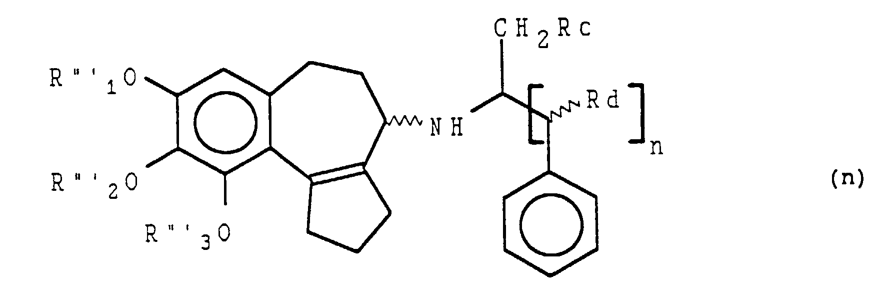 zu gelangen, worin Rc und Rd die vorstehend angegebene Bedeutung haben und die gewellte, NH mit dem Ring verbindende Linie jenachdem, ob man ein racemisches oder optisch aktives Mittel der Formel (m) eingesetzt hat, ein Gemisch der Isomeren oder das eine oder das andere Isomere anzeigt,
die Verbindung (n) entweder der Hydrogenolyse der Alkylaminogruppe unterzieht, um zu einer Verbindung der Formel (o): 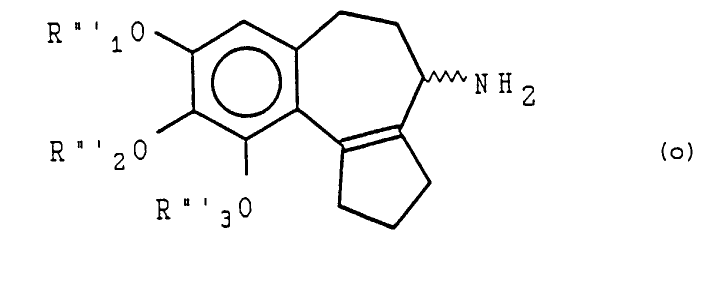 zu gelangen, worin die gewellte Linie wie vorstehend definiert ist, deren Aminogruppe man mit einer zweiwertigen Schutzgruppe schützt, um eine Verbindung der Formel (p): 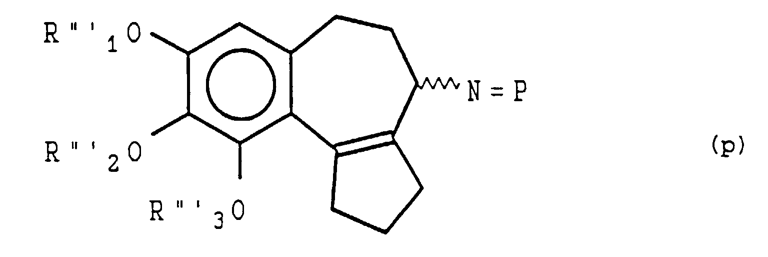 zu erhalten, worin die gewellte Linie wie vorstehend definiert ist und P eine zweiwertige Schutzgruppe darstellt,
oder man die Verbindung der Formel (n), wie vorstehend definiert, der Einwirkung eines Hydrolysemittels für die Gruppe R"'₁O unterzieht, um zu einer Verbindung der Formel (n'): 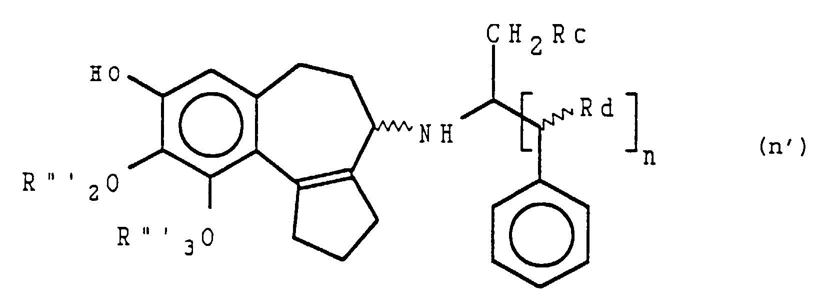 zu gelangen, worin R"'₂, R"'₃, Rc, Rd, n und die gewellten Linien die vorstehend angegebene Bedeutung besitzen, die man einer Hydrogenolyse der Alkylaminogruppe unterzieht, um zu einer Verbindung der Formel (o'): 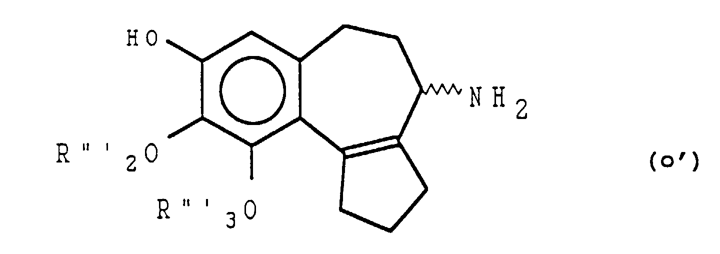 zu gelangen, worin die gewellte Linie wie vorstehend definiert ist, deren Aminogruppe man mit einer zweiwertigen Schutzgruppe schützt, um zu einer Verbindung der Formel (p'): 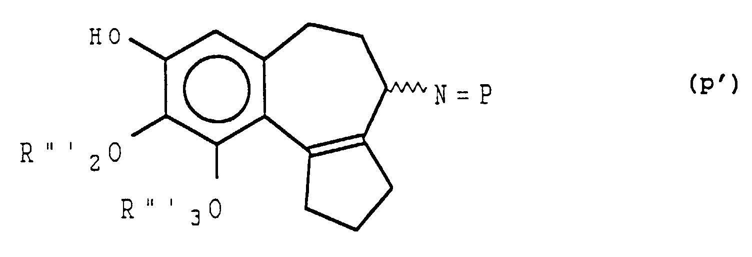 zu gelangen, worin P und die gewellte Linie die vorstehend angegebene Bedeutung besitzen, welche man der Einwirkung eines Mittels zum Schutz der Hydroxygruppe unterzieht, um zu einer Verbindung der Formel (p), wie vorstehend definiert zu gelangen;
(ii) oder man eine Verbindung der Formel (I)(f), wie vorstehend definiert, der Einwirkung eines Mittels der Formel (m), wie vorstehend definiert, unterzieht, das erhaltene Imino-Zwischenprodukt mit einem Reduktionsmittel behandelt, um zu einer Verbindung der Fornel (n'), wie vorstehend definiert, zu gelangen, wonach man die Synthese, wie vorstehend angegeben, fortführt;
(iii) oder man eine Verbindung der Formel (I), wie vorstehend definiert, der Einwirkung eines Mittels der Formel R₈O-NH₂ oder eines Salzes dieser Verbindung, worin R₈ ein Wasserstoffatom oder einen Alkylrest bedeutet, unterzieht, um zu einer Verbindung der Formel (q): 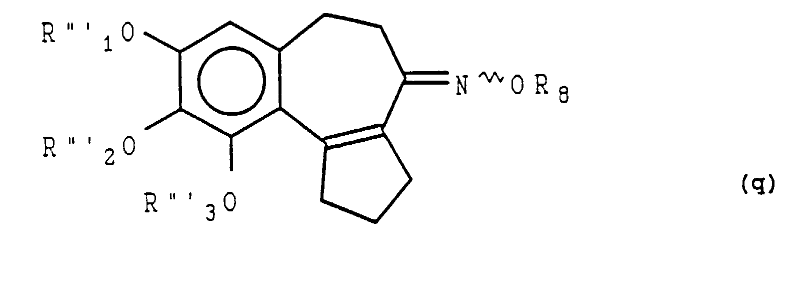 zu gelangen, worin die gewellte Linie anzeigt, daß die Verbindung aus einem Gemisch der syn- und anti-Isomeren besteht,
(iv) oder man eine Verbindung der Formel (I), wie vorstehend definiert, der Einwirkung entweder eines chiralen Reduktionsmittels oder eines nicht-chiralen Reduktionsmittels unterzieht, um zu dem entsprechenden Alkohol der Formel(r): 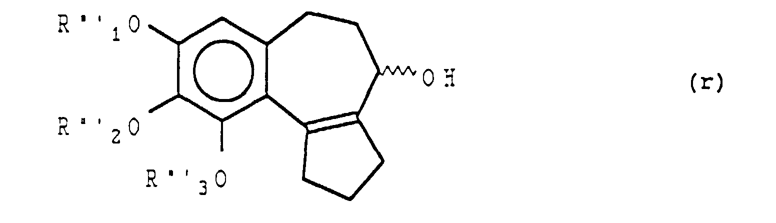 zu gelangen, worin die gewellte Linie zwischen dem Stickstoffatom und dem Ring mit 7 Gliedern anzeigt, daß das Produkt entsprechend dem verwendeten Reduktionsmittel entweder aus einem α- oder β-Isomeren oder aus einem Gemisch der α- und β-Isomeren besteht, welches Gemisch man gegebenenfalls auftrennt, hiernach entweder den Alkohol der Einwirkung eines Mittels der Formel HN=P unterzieht, worin P die vorstehend angegebene Bedeutung besitzt, um zu einer Verbindung der Formel (p): 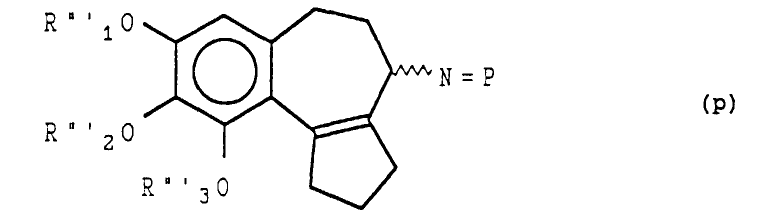 zu gelangen, worin die gewellte Linie zwischen dem Stickstoffatom und dem Ring mit 7 Gliedern anzeigt, daß das Produkt aus einem α- oder β-Isomeren oder aus einem racemischen Gemisch der Isomeren besteht,
oder den Alkohol der Einwirkung eines Reagens der Formel ASO₂Hal unterzieht, worin A wie vorstehend definiert ist und Hal für ein Halogenatom steht, um zu der Verbindung der Formel (r₁): 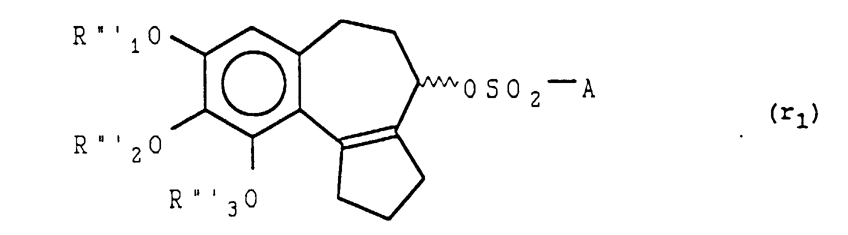 zu gelangen, worin die gewellte Linie und A die vorstehenden Bedeutungen besitzen, welche man mit Ammoniak umsetzt, um zu der vorstehenden Verbindung der Formel (o) zu gelangen, deren Aminogruppe man mit einer zweiwertigen Schutzgruppe schützt, um zu der vorstehenden Verbindung der Formel (p) zu gelangen, wonach
(v) man eine Verbindung der Formel (p) oder (q) der Einwirkung eines Bromierungsmittels in Anwesenheit einer Verbindung der Formel R₇OH unterzieht, worin R₇ ein Wasserstoffatom oder einen Alkylrest bedeutet, um zu einer Verbindung der Formel (s): 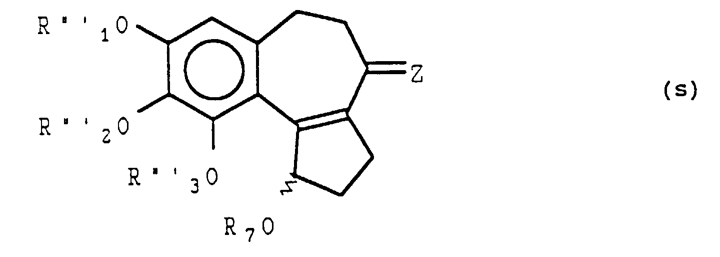 zu gelangen, worin die gewellte Linie anzeigt, daß die Bindung sich in α- oder β-Position befinden kann oder daß dieses Produkt auf einem Isomerengemisch bestehen kann, und worin die Gruppe =Z eine Gruppe der Formel = N 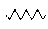 OR₈ oder 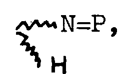 wie für die vorstehenden Formeln p und q definiert, wiedergibt,
(iv) welche man einer Dehydratation oder einer Desalkoxylierung in wasserfreiem, saurem Milieu unterzieht, um zu einer Verbindung der Formel (t): 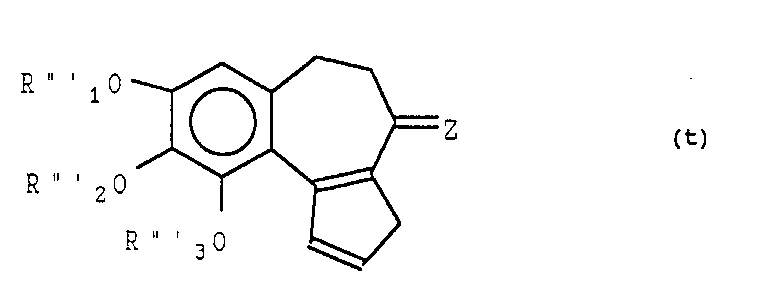 zu gelangen,
(vii) welche man in Anwesenheit einer Base oder eines reduzierenden Metalls der Einwirkung eines Mittels der Formel 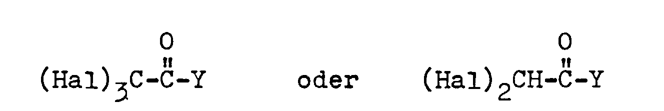 unterzieht, worin Hal ein Halogenatom bedeutet und Y wie vorstehend definiert ist, um zu einer Verbindung der Formel (u): 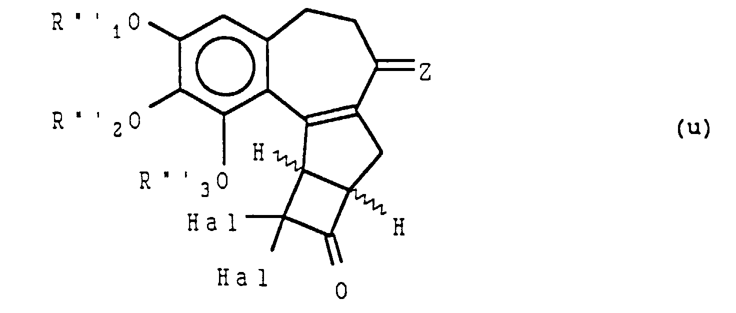 zu gelangen, worin die gewellten Linien anzeigen, daß die Wasserstoffatome sich beide in α- oder beide in β-Position befinden;
(viii) welche man der Einwirkung einer Carbonsäure in Gegenwart einer Base in wäßrigem Milieu unterzieht, um zu einer Verbindung der Formel (v) zu gelangen, die im Gleichgewicht mit ihrem Tautomeren (v') vorliegt: 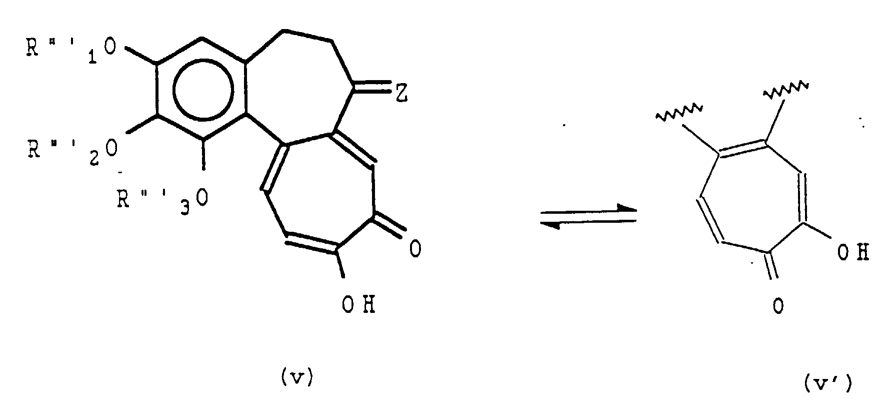 welche Verbindung entweder
(ix) wenn =Z eine Gruppe der Formel 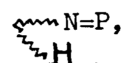 wie vorstehend definiert bedeutet, man entweder einer Reaktion zur Abspaltung der Schutzgruppe von der Aminogruppe unterzieht, um entweder das freie Amin der Formel (w) zu erhalten, welches sich im Gleichgewicht mit seinem Tautomeren (w') befindet: 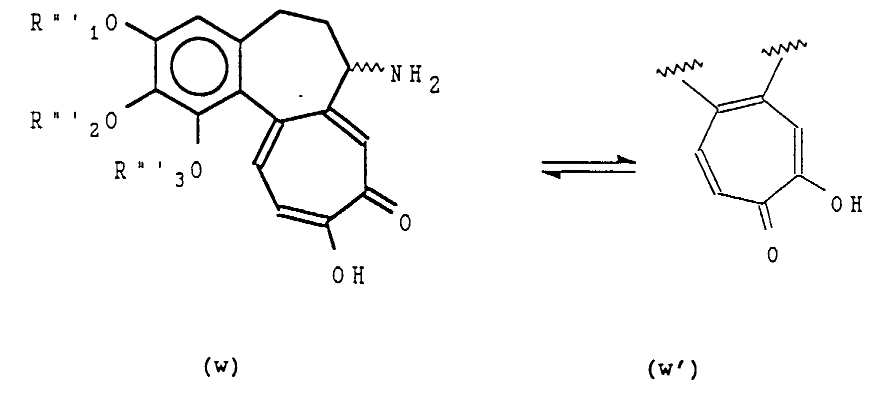 worin die gewellte Linie anzeigt, daß sich die Gruppe NH₂ in α- oder β-Position befindet oder daß das Produkt aus einem Gemisch der Isomeren besteht, welches man der Einwirkung eines Reagens der Formel R₁₁-C(O)Y oder der Formel (R₁₁CO)₂O unterzieht, worin die Gruppe R₁₁ eine Alkylgruppe bedeutet und Y wie in Anspruch 11 definiert ist, um zu einer Verbi ndung der Formel (x): 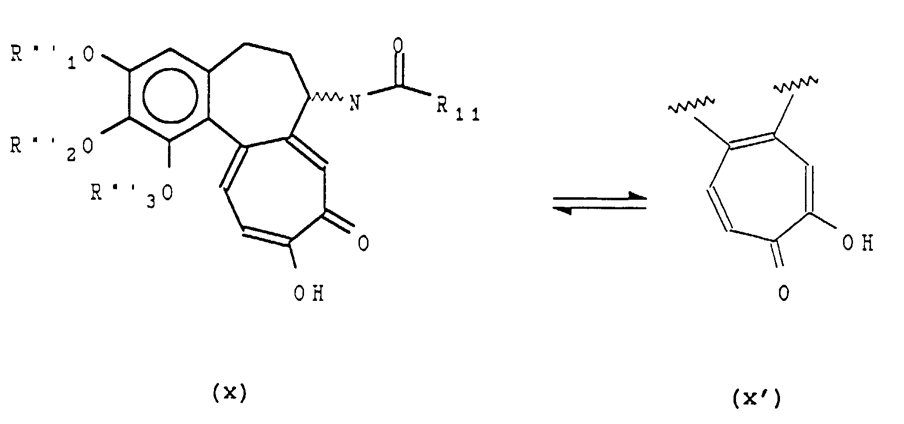 zu gelangen, worin die gewellte Linie die vorstehende Bedeutung besitzt, oder einer Reaktion zur Abspaltung der Aminogruppe und einem Hydrolysemittel für die Gruppe R"'₁, wenn diese Gruppe die Bedeutung A-SO₂-, wie vorstehend definiert, aufweist, unterzieht, um zu einer Verbindung der Formel (w₁): 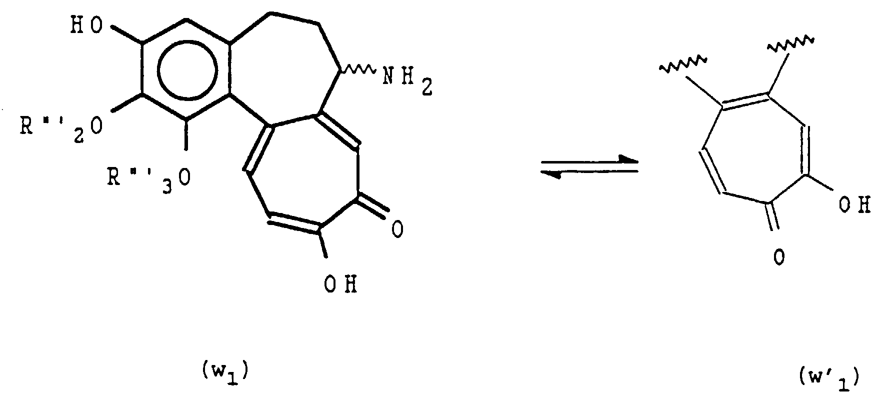 zu gelangen, welche man der Einwirkung einer Verbindung der Formel R₁₁C(O)Y oder (R₁₁CO)₂O, wie vorstehend definiert, unterzieht, um zu einer Verbindung der Formel (x₁): 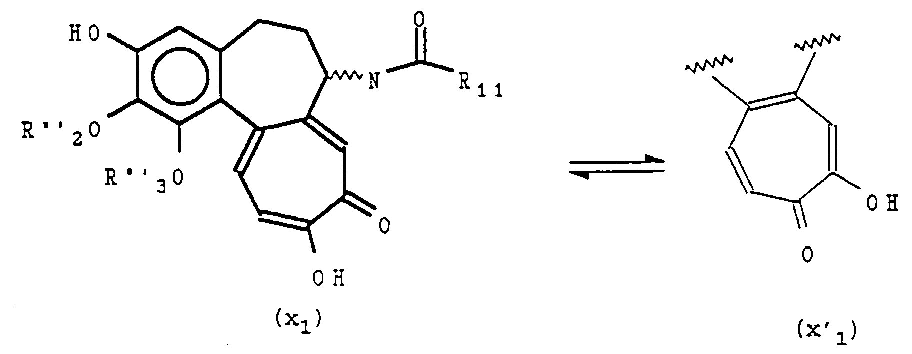 zu gelangen, deren OH-Funktion man durch Einwirken eines Reagens der Formel ASO₂Y, wie vorstehend definiert, schützt, um zu einer entsprechenden Verbindung der Formel (x)/(x'), wie vorstehend definiert, zu gelangen, oder
(x) wenn =Z eine Gruppe der Formel =N-OR₈ bedeutet, man der Einwirkung eines Schutzmittels der Formel B(Hal)₃ oder B(OR₄)₃, worin R₄ und Hal wie vorstehend definiert sind, danach der Einwirkung eines Reduktionsmittels unterzieht, um zu einer Verbindung der Formel (y): 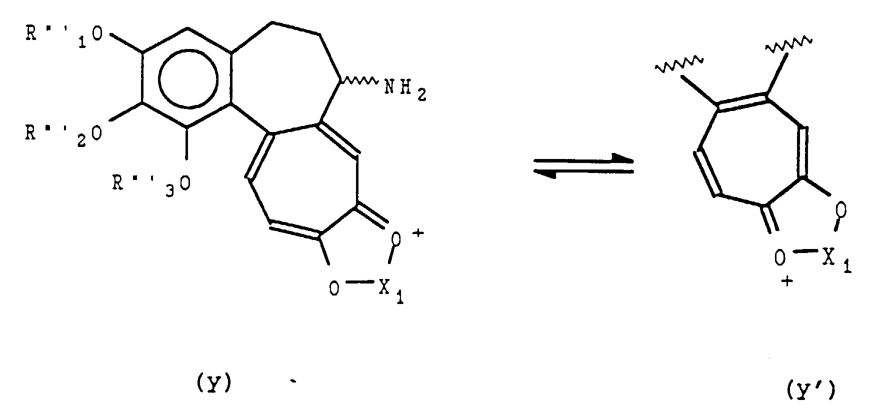 zu gelangen, worin die gewellte Linie anzeigt, daß das Produkt in Form eines Isomerengemisches vorliegt, und X₁ eine Gruppe der Formel -B(OR₄)₂- oder -B(Hal)₂ bedeutet, welche man der Einwirkung einer Verbindung der Formel R₁₁C(O)Y oder der Formel (R₁₁CO)₂O, wie vorstehend definiert, danach der Einwirkung eines Reagens zur Abspaltung der Schutzgruppe von der α-Hydroxyketongruppe unterzieht, um zu einer Verbindung der Formel (x")/(x"₁) entsprechend dem Produkt der Formel (x)/(x'), wie vorstehend definiert, worin die gewellte Linie anzeigt, daß das Produkt in Form eines Isomerengemisches vorliegt, zu gelangen;
(xi) welche Verbindung der Formel (x) oder (x') man der Einwirkung eines Mittels für die Einführung der Gruppe K, wie vorstehend definiert, unterzieht, um eine Verbindung der Formel (II): 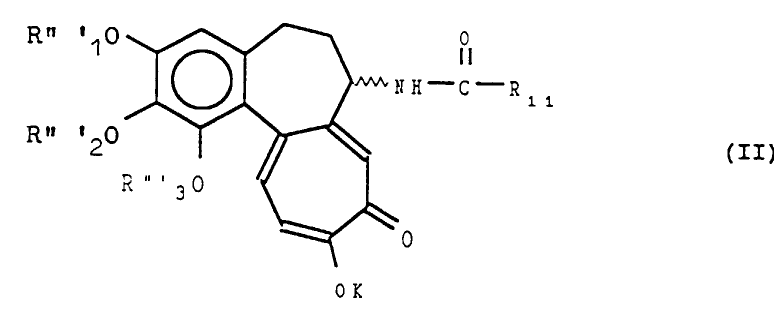 zu erhalten, die im Gemisch mit seinem Regioisomeren der Formel: 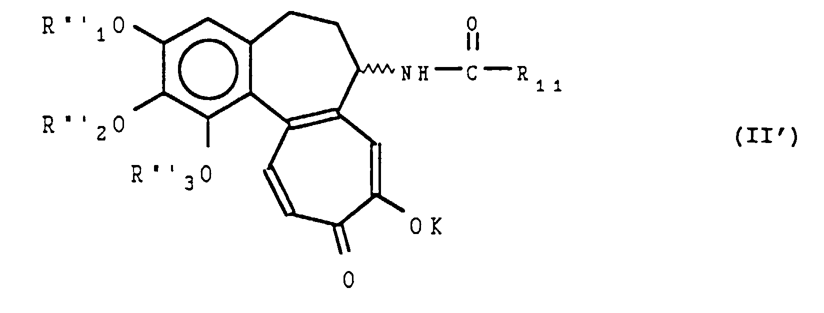 vorliegt, wobei in den Formeln (II) und (II') die gewellte Linie anzeigt, daß sich die Gruppe in α- oder β-Position befindet oder daß das Produkt als Isomerengemisch vorliegt, und die Ausdrücke R"'₁, R"'₂, R"'₃, R₁₁, K und Alkyl die vorstehend angegebenen Bedeutungen besitzen, welches Gemisch man in seine Bestandteile auftrennt.

14. Verwendung gemäß Anspruch 13, dadurch gekennzeichnet, daß das Amin der Formel (m), das in Stufe (i) oder (ii) eingesetzt wird, das (S)-α-Methylbenzylamin, das Norephedrin oder das Norpseudoephedrin ist.

15. Verwendung gemäß Anspruch 13 oder 14, dadurch gekennzeichnet, daß die Schutzgruppe des Aminorestes eine Phthalimidogruppe oder eine Gruppe vom Maleimido-Typ ist.

16. Verwendung gemäß Anspruch 13, dadurch gekennzeichnet, daß das Mittel der Formel R₈O-NH₂ Methylhydroxylamin ist.

17. Verwendung gemäß einem der Ansprüche 13 bis 16, dadurch gekennzeichnet, daß das Reagens R₇OH Methanol ist.

18. Verwendung gemäß einem der Ansprüche 13 bis 17, dadurch gekennzeichnet, daß das Halogenderivat, welches man mit der Verbindung (t) umsetzt, das Dichloracetylchlorid ist.

19. Verwendung gemäß einem der Ansprüche 13 bis 18, dadurch gekennzeichnet, daß R₁₁ ein Methylrest ist.

20. Verwendung gemäß einem der Ansprüche 13 bis 19, dadurch gekennzeichnet, daß das Reagens ASO₂Y, welches man mit der Verbindung (x₁)/(x'₁) umsetzt, das Mesyl- oder Tosylchlorid ist.

21. Verwendung gemäß einem der Ansprüche 13 bis 20, dadurch gekennzeichnet, daß das Mittel für die Einführung der Gruppe K das Mesyl-, Tosyl- oder 2,4,6-Triisopropylphenylsulfonylchlorid ist.

22. Verwendung gemäß einem der Ansprüche 13 bis 21, dadurch gekennzeichnet, daß man
- das Mittel (m) mit der Verbindung (I) in Anwesenheit von Titantetrachlorid und einer tertiären Aminbase oder in Gegenwart eines sauren Katalysators umsetzt;
- der Schutz der Aminogruppe durch Einwirkenlassen von Phthalsäureanhydrid auf die Verbindung (o) oder (o') bewirkt wird;
- das Mittel der Formel HN=P Phthalsäureanhydrid ist und die Reaktion mit der Verbindung (r) in Anwesenheit von Triphenylphosphin und eines Alkylazobicarboxylats erfolgt;
- die Umlagerung zum Tropolon durch Einwirkenlassen von Essigsäure auf die Verbindung (u) in Anwesenheit einer Aminbase durchgeführt wird;
- die Schutzgruppenabspaltung vom Amin mit Hydrazin erfolgt; - die selektive Hydrolyse der Verbindung (x)/(x') durch Einwirkenlassen eines Alkalithioalkoholats erfolgt;
- das die Schutzgruppe für die Hydroxy- und Ketonfunktionen bildende Reagens Bortrifluorid ist;
- das Reduktionsmittel, welches man mit dem aus der Verbindung (v)/(v') hervorgegangenen Oxim umsetzt, das Zink in Gegenwart einer Sulfon- oder Carbonsäure ist.

23. Verwendung gemäß Anspruch 13, dadurch gekennzeichnet, daß man eine Verbindung der Formel (v)/(v'), wie in Anspruch 13 definiert, worin Z eine Gruppe 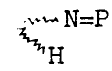 wiedergibt, der Einwirkung eines Mittels für die Einführung der Gruppe K, wie in Anspruch 13 definiert, unterzieht, um zu einer Verbindung der Formel (III): 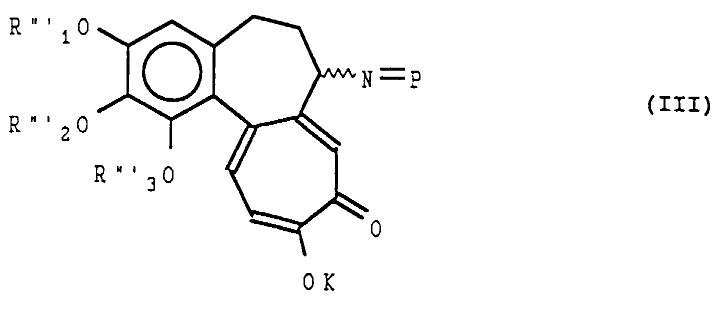 zu gelangen, die im Gemisch mit ihren Regioisomeren der Formel: 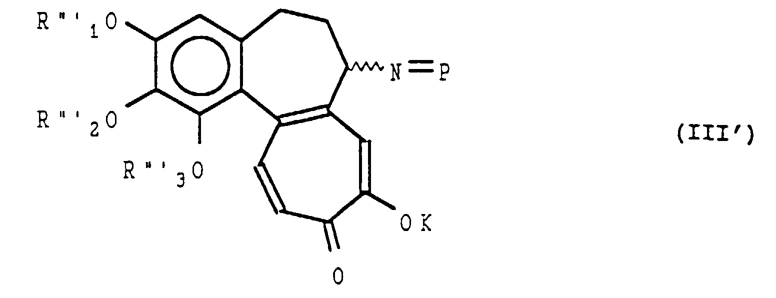 vorliegt, wobei in den Formeln (III) und (III') die gewellte Linie anzeigt, daß sich die Gruppe in α- oder β-Position befindet oder daß das Produkt als ein Isomerengemisch vorliegt, und die Ausdrücke R"'₁, R"'₂, R"'₃, R₁₁ und K und Alkyl die in Anspruch 13 angegebenen Bedeutungen besitzen, welches Gemisch man gegebenenfalls in seine Bestandteile auftrennt, hiernach das Gemisch oder die getrennten Bestandteile einer Reaktion zur Abspaltung der Schutzgruppe von der Aminofunktion unterzieht, um entweder die Verbindung der Formel (IV): 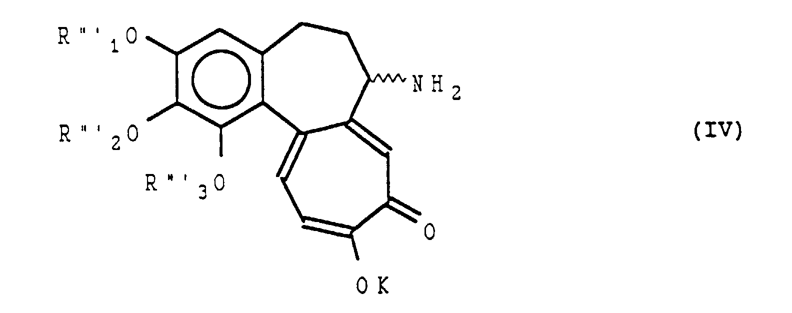 in Form des Gemisches mit ihrem Regioisomeren der Formel 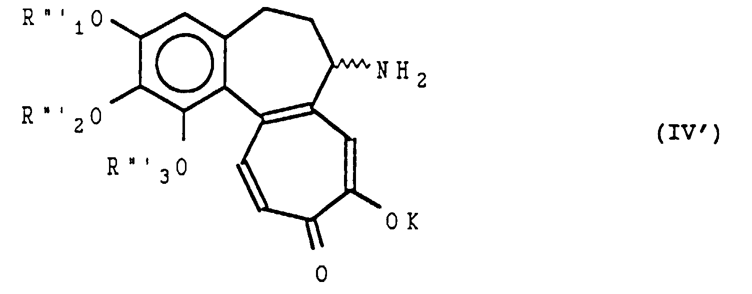 welches Gemisch man gegebenenfalls auftrennt,
oder das eine oder das andere ihrer Regioisomeren zu erhalten, hiernach das Gemisch oder die getrennten Bestandteile der Einwirkung einer Verbindung der Formel R₁₁C(O)Y oder (R₁₁CO)₂O, wie in Anspruch 13 definiert, unterzieht, um entweder die Verbindung der Formel (II), wie vorstehend definiert, im Gemisch mit ihrem Regioisomeren der Formel (II'), welches Gemisch man gegebenenfalls in seine Bestandteile auftrennt, oder das eine oder das andere dieser Regioisomeren zu erhalten.

24. Verwendung gemäß Anspruch 23, dadurch gekennzeichnet, daß das Mittel für die Einführung der Gruppe K das Reagens für die Schutzgruppenabspaltung von der Aminofunktion und R₁₁ wie in den Ansprüchen 19, 21 und 22 definiert sind.

25. Verwendung gemäß Anspruch 13, dadurch gekennzeichnet, daß man eine Verbindung der Formel (v)/(v'), wie vorstehend definiert, worin (z) eine Gruppe =N-OR₈ bedeutet, der Einwirkung eines Mittels für die Einführung der Gruppe K unterzieht, um zu einer Verbindung der Formel (V): 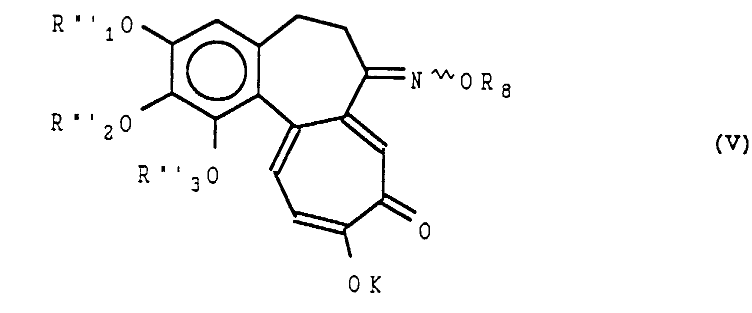 zu gelangen, welche im Gemisch mit ihrem Regioisomeren der Formel 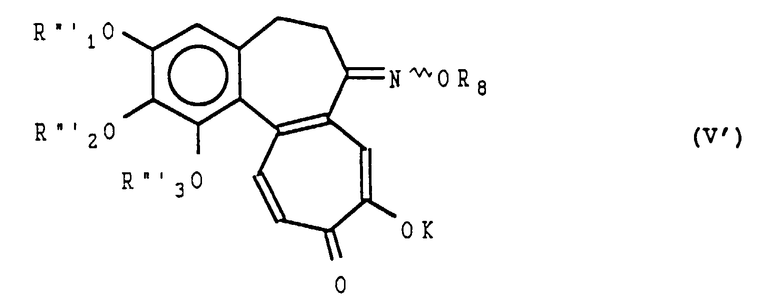 vorliegt, wobei in den Formeln (V) und (V') die gewellte Linie, R₈ und die Ausdrücke R"'₁, R"'₂, R"'₃, K und Alkyl die in Anspruch 13 angegebenen Bedeutungen besitzen, welches Gemisch man gegebenenfalls in seine Bestandteile trennt, hiernach das Gemisch oder die getrennten Bestandteile der Einwirkung eines Reduktionsmittels unterzieht, um entweder die Verbindung der Formel (IV₁): 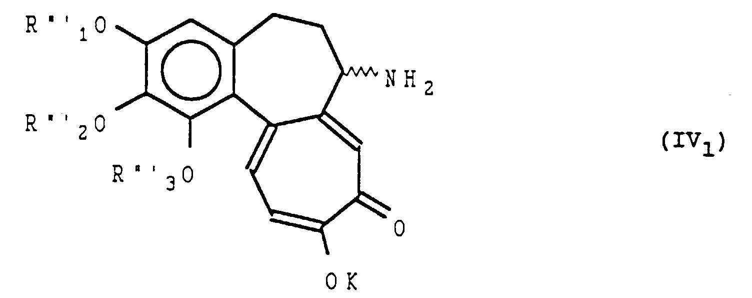 in Form des Gemisches mit ihrem Regioisomeren der Formel 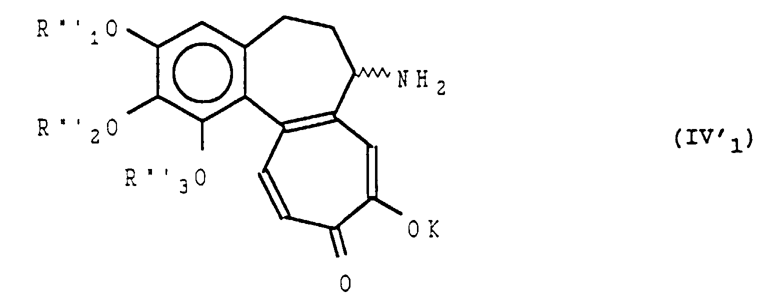 worin die gewellte Linie ein Gemisch der Isomeren symbolisiert, das Gemisch der Regioisomeren, welches man gegebenenfalls auftrennt, oder das eine oder andere dieser Regioisomeren zu erhalten, wonach man die Synthese, wie in Anspruch 13 für die Verbindungen der Formel (IV)/(IV') beschrieben, fortsetzt.

26. Verwendung gemäß Anspruch 25, dadurch gekennzeichnet, daß das Mittel für die Einführung der Gruppe K und das Reduktionsmittel des Oxims wie in den Ansprüchen 21 und 22 definiert sind.

27. Verwendung gemäß Anspruch 13, dadurch gekennzeichnet, daß man außerdem ein Produkt der Formel (II₁): 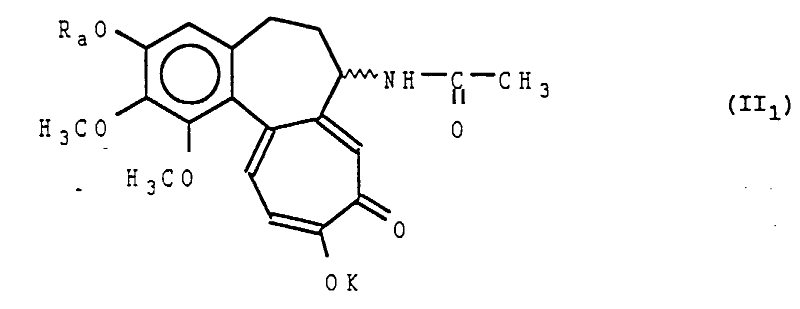 worin K wie in Anspruch 13 definiert ist, Rₐ einen Rest ASO₂-, wie in Anspruch 13 definiert, wiedergibt und die gewellte Linie das eine oder andere Isomere oder ein Gemisch der Isomeren symbolisiert, der Einwirkung eines Reagens der Formel CH₃S⁻Na⁺ unterzieht, um zu einer Verbindung der Formel (VI): 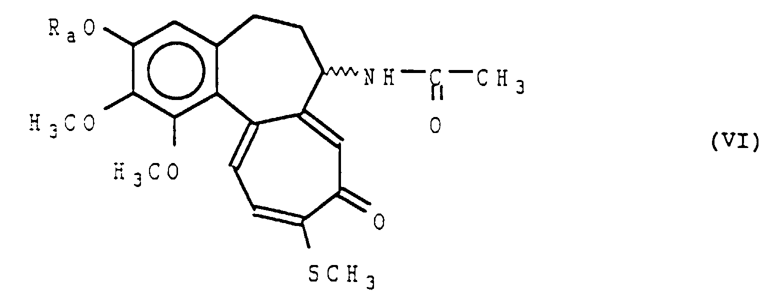 zu gelangen, worin Rₐ und die gewellte Linie die vorstehende Bedeutung besitzen, welche man der Einwirkung eines Reagens für die Schutzgruppenabspaltung von der Hydroxygruppe unterzieht, um zu der Verbindung der Formel (VII): 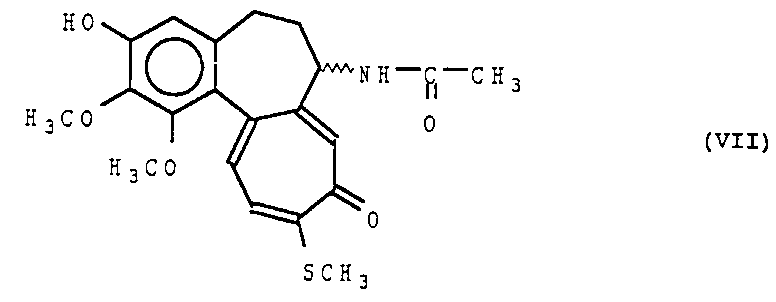 in optisch aktiver oder racemischer Form zu gelangen.

28. Verwendung gemäß Anspruch 27, dadurch gekennzeichnet, daß man die Substitution der Gruppe OK und die Schutzgruppenabspaltung von der Hydroxygruppe mit dem gleichen Reagens CH₃SNa vornimmt.

29. Verwendung gemäß Anspruch 13, dadurch gekennzeichnet, daß man außerdem eine Verbindung der Formel (II₂): 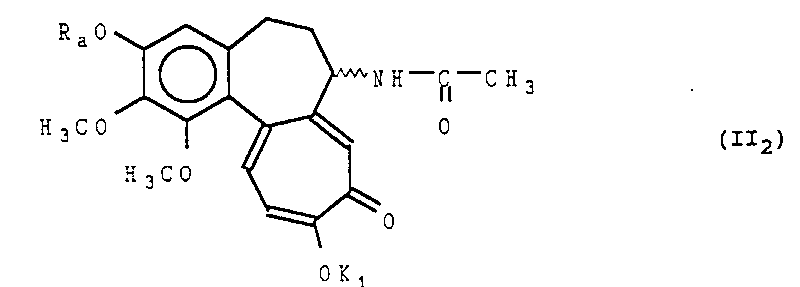 worin Rₐ wie in Anspruch 13 definiert ist, K₁ eine Methylgruppe bedeutet und die gewellte Linie das eine oder andere Isomere oder ein Gemisch der Isomeren symbolisiert, der Einwirkung eines Mittels für die Schutzgruppenabspaltung von der Hydroxygruppe unterzieht, um zu einer Verbindung der Formel (VIII): 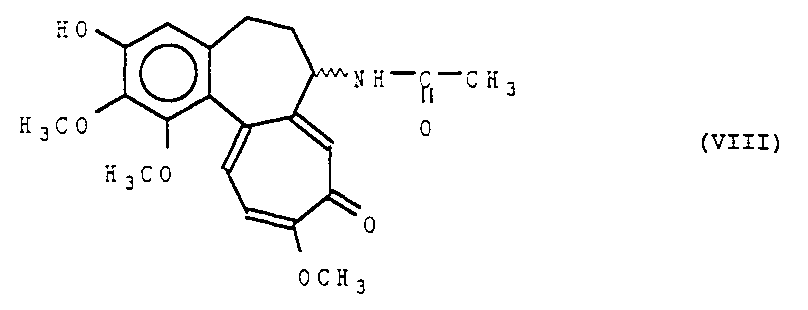 in optisch aktiver oder racemischer Form zu gelangen.

30. Verwendung gemäß Anspruch 13, dadurch gekennzeichnet, daß man außerdem eine Verbindung der Formel (II₃): 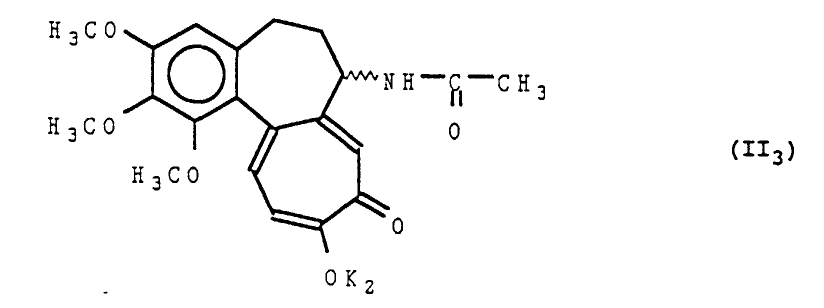 worin K₂ einen gegebenenfalls an dem Phenyl durch 1 bis 3 Alkylreste substituierten Benzylrest oder einen Rest -SO₂A', wie vorstehend definiert, bedeutet und die gewellte Linie das eine oder das andere Isomere oder ein Gemisch der Isomeren symbolisiert, der Einwirkung eines Reagens der Formel CH₃S⁻Na⁺ unterzieht, um zu der Verbindung der Formel (IX): 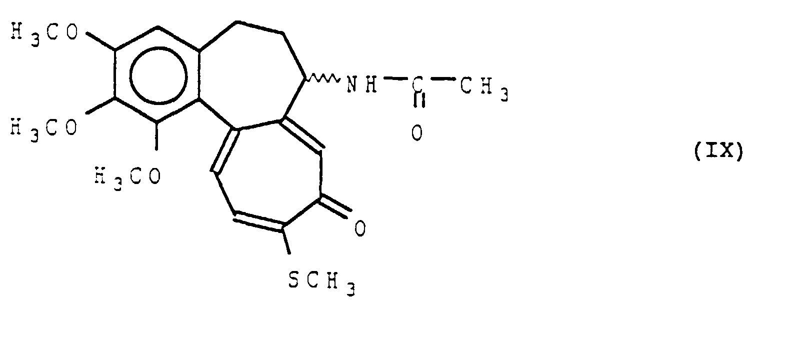 in optisch aktiver oder racemischer Form zu gelangen.

31. Verwendung gemäß Anspruch 13, dadurch gekennzeichnet, daß man außerdem eine Verbindung der Formel (II₄): 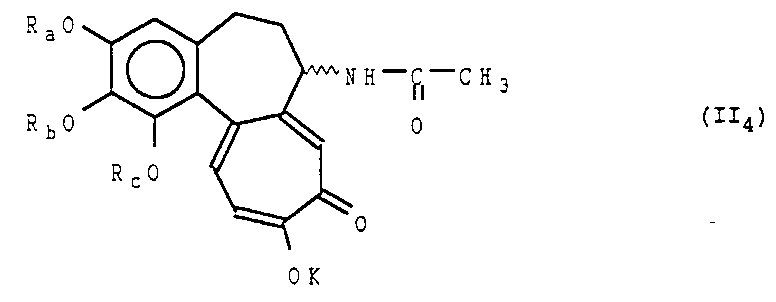 worin Rₐ und K wie in Anspruch 13 definiert sind, R_{b} und R_{c} gemeinsam eine Gruppe CR₅R₆, wie in Anspruch 1 definiert, bilden und die gewellte Linie das eine oder andere Isomere oder ein Gemisch der Isomeren symbolisiert, der Einwirkung eines Reagens für die Schutzgruppenentfernung vom Diol unterzieht, um zu einer Verbindung der Formel (X): 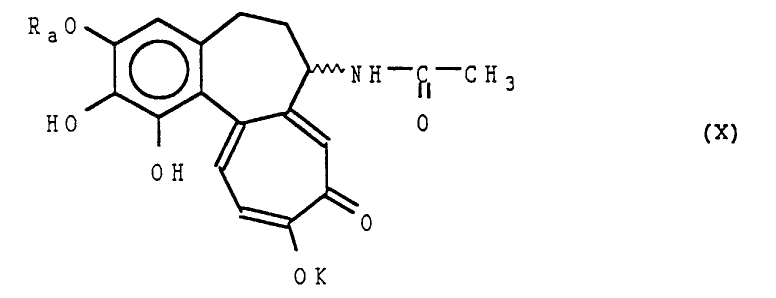 zu gelangen, worin Rₐ, K und die gewellte Linie die vorstehende Bedeutung besitzen, welche man mit einem Methylierungsreagens behandelt, um zu einer Verbindung der Formel (II₁), wie in Anspruch 27 definiert, zu gelangen, wonach man die Synthese wie in Anspruch 27, ausgehend von der Verbindung der Formel (II₁), fortführt.

32. Verwendung gemäß Anspruch 13, dadurch gekennzeichnet, daß man außerdem eine Verbindung der Formel (II₅): 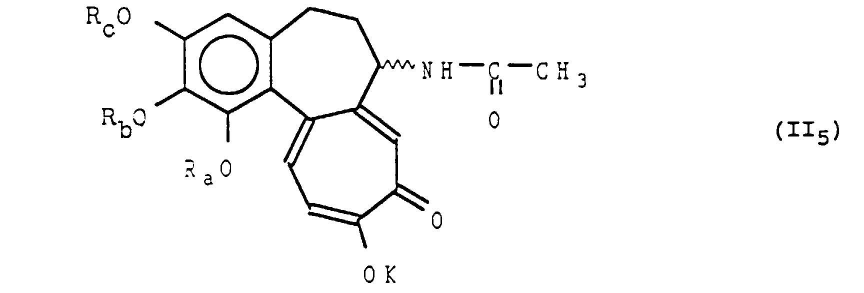 worin Rₐ, R_{b}, R_{c}, K und die gewellte Linie wie für Formel (II₄) in Anspruch 31 definiert sind, der Einwirkung eines Mittels für die Schutzgruppenabspaltung vom Diol unterzieht, um zu einer Verbindung der Formel (XI): 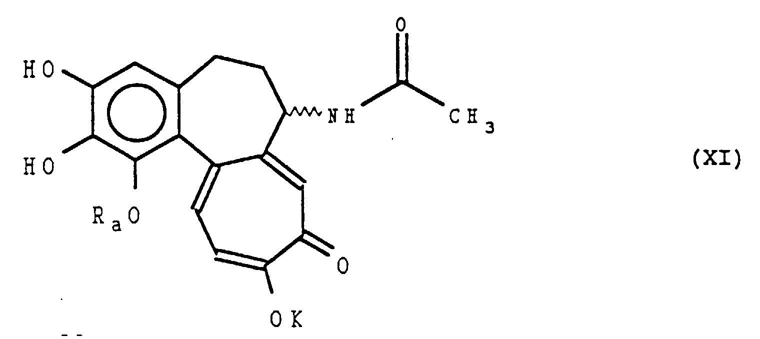 zu gelangen, worin Rₐ, K und die gewellte Linie die vorstehende Bedeutung besitzen, welche man der Einwirkung eines Hydrolysemittels unterzieht, um die Verbindung der Formel (XII): 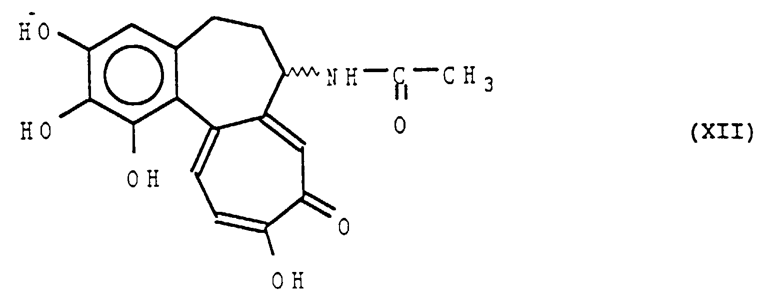 zu erhalten, welche man einem Methylierungsmittel unterzieht, um das optisch aktive oder racemische Colchicin zu erhalten.

33. Als neue industrielle Verbindungen die Verbindungen der Formel (F₁): 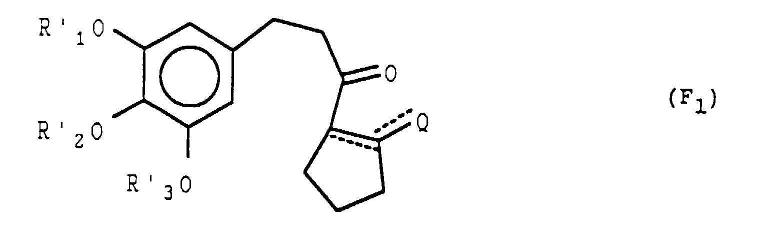 worin entweder Q eine Oxogruppe bedeutet oder die gestrichelte Linie in dem Ring eine zweite Bindung darstellt und Q ein Halogenatom bedeutet, R'₁, R'₂ und R'₃ die in Anspruch 11 angegebene Bedeutung besitzen.

34. Als neue industrielle Verbindungen die Verbindungen der Formel (F₂): 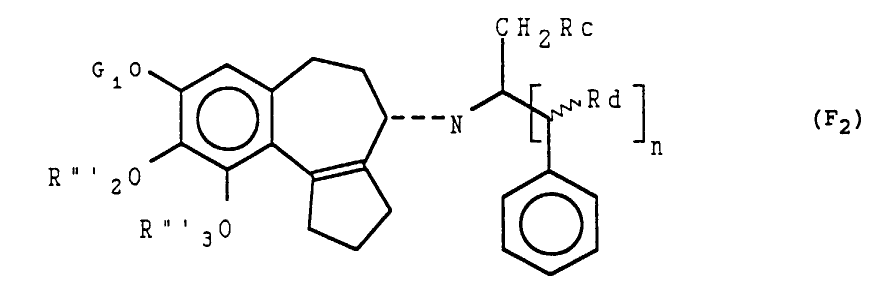 worin G₁ ein Wasserstoffatom oder den Rest R"'₁, wie vorstehend definiert, bedeutet und Rc und Rd sowie R"'₂ und R"'₃ die in Anspruch 13 angegebene Bedeutung besitzen, die gestrichelte Linie entweder eine Doppelbindung symbolisiert oder eine Einfachbindung und, in diesem Fall, der Stickstoff ein Wasserstoffatom trägt und die Verbindung in Form eines (R,S)-Isomerengemisches oder des Isomeren (R) oder (S) vorliegt.

35. Als neue industrielle Produkte die Verbindungen der Formel (F₃): 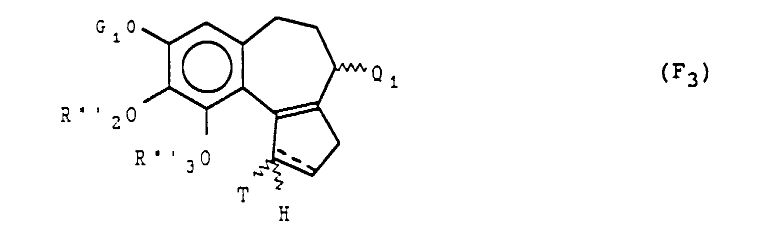 worin R"'₂ und R"'₃ die in Anspruch 13 angegebene Bedeutung besitzen und entweder G₁ den Rest R"'₁, wie vorstehend definiert, bedeutet und:
- entweder Q₁ eine Gruppe NH₂ der Konfiguration (R), (S) oder (R,S) wiedergibt und T ein Wasserstoffatom bedeutet;
- oder Q₁ eine Gruppe N=P der Konfiguration (R),(S) oder (R,S) bedeutet, wobei P wie in Anspruch 13 definiert ist und entweder T ein Wasserstoffatom oder einen Hydroxy- oder Alkoxyrest mit 1 bis 6 Kohlenstoffatomen der Konfiguration (R), (S) oder (R,S) bedeutet oder die gestrichelte Linie eine zweite Bindung wiedergibt;
- oder Q₁ eine Gruppe = N 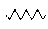 OR₈ darstellt, worin R₈ die in Anspruch 13 angegebene Bedeutung besitzt und die gewellte Linie anzeigt, daß die Bindung entweder syn oder anti sein kann oder daß das Produkt als Gemisch der syn- und anti-Isomeren vorliegen kann, und entweder T ein Wasserstoffatom oder eine Hydroxy- oder Alkoxygruppe mit 1 bis 6 Kohlenstoffatomen der Konfiguration (R), (S) oder (R,S) bedeutet, oder die gestrichelte Linie eine zweite Bindung anzeigt;
- oder Q₁ eine Gruppe OH oder OSO₂A, wobei A wie vorstehend definiert ist, der Konfiguration (R), (S) oder (R,S) bedeutet und T ein Wasserstoffatom wiedergibt,
oder G₁ ein Wasserstoffatom bedeutet und Q₁ entweder einen Rest NH₂ der Konfiguration (R), (S) oder (R,S) oder eine Gruppe N=P der Konfiguration (R), (S) oder (R,S) bedeutet, wobei P wie in Anspruch 13 definiert ist und T ein Wasserstoffatom bedeutet, wobei die gestrichelte Linie keine Doppelbindung wiedergibt.

36. Als neue industrielle Produkte die Verbindungen der Formel (u): 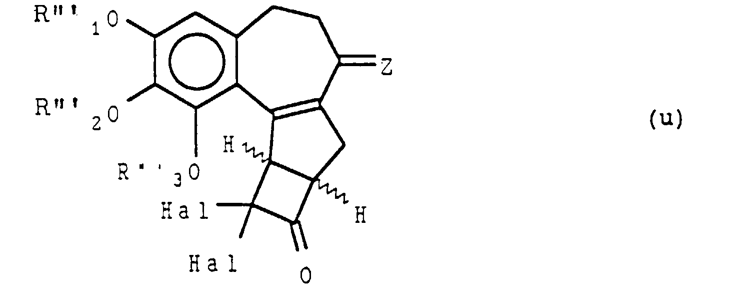 worin R"'₁, R"'₂, R"'₃, Hal und Z wie in Anspruch 13 definiert sind.

37. Als neue industrielle Produkte die Verbindungen der Formel (F₄): 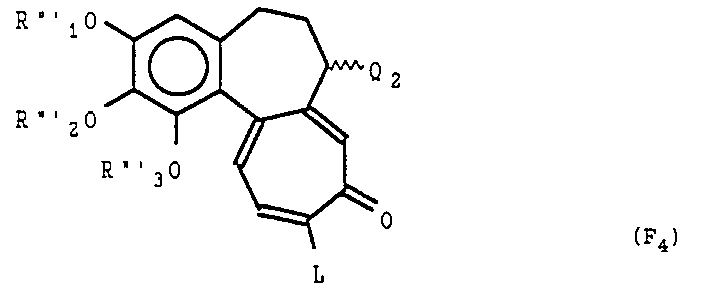 sowie deren Tautomere oder Regioisomere der Formel: 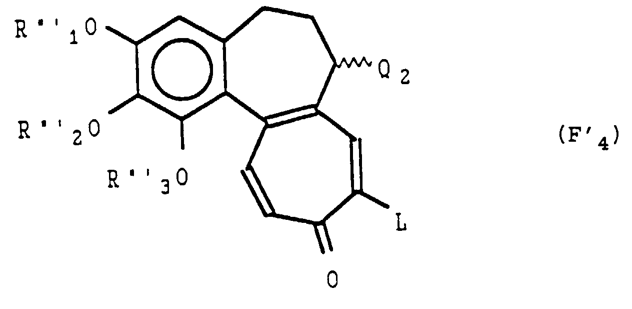 worin R"'₁, R"'₂ und R"'₃ die in Anspruch 13 angegebene Bedeutung besitzen und 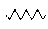 Q₂ bedeutet:
- entweder eine Gruppe = N 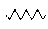 OR₈, wie vorstehend definiert, und L eine Hydroxygruppe bedeutet,
- oder eine Gruppe 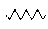 N = P, worin P die in Anspruch 13 angegebene Bedeutung besitzt, die gewellte Linie anzeigt, daß sich die Gruppe in der Konfiguration (R), (S) oder (R,S) befindet, und L eine Hydroxygruppe oder -OK, wie in Anspruch 13 definiert, wiedergibt, mit Ausnahme der Verbindungen, worin R"'₁ , R"'₂ und R"'₃ einen Alkylrest bedeuten, L eine Hydroxy- oder Alkoxygruppe darstellt und P eine Gruppe =C-Phenyl oder CH₃-C-OC₂H₅ wiedergibt;
- oder eine Gruppe 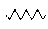 NH₂ der Konfiguration (R), (S) oder (R,S) und L eine Hydroxygruppe bedeutet,mit Ausnahme der Verbindungen, worin R"'₁, R"'₂ und R'"₃ eine Alkylgruppe bedeuten;
- oder eine Gruppe 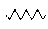 NH₂ der Konfiguration (R,S) und L mit dem Sauerstoff des Ketons in 9-Stellung eine Gruppe O-X₁-O⁺, wie in Anspruch 13 definiert, bildet;
- oder eine Gruppe 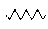 NH₂ der Konfiguration (R), (S) oder (R,S) und L einen Rest OK, wie in Anspruch 13 definiert, bildet, mit Ausnahme der Verbindungen, worin R"'₁, R"'₂ und R'"₃ einen Alkylrest bedeuten und K einen Alkylrest wiedergibt;
- oder eine Gruppe = N 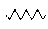 OR₈, wie in Anspruch 13 definiert, und L einen Rest OK, wie in Anspruch 13 definiert, bedeutet.

38. Als neue industrielle Produkte die Verbindungen der Formel (F₅): 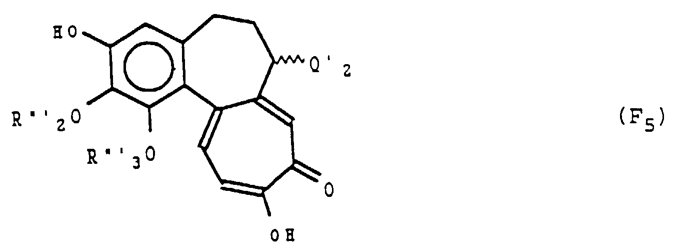 sowie deren Tautomere der Formel: 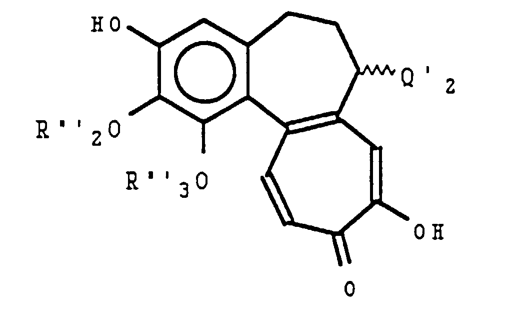 worin R'"₂ und R"'₃ die in Anspruch 13 angegebene Bedeutung besitzen, mit Ausnahme der Bedeutung Alkyl, und 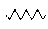 Q'₂ bedeutet:
- entweder eine Gruppe 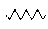 NH - CO - R₁₁, worin R₁₁ die in Anspruch 13 angegebene Bedeutung besitzt,
- oder eine Gruppe 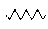 N H₂ der Konfiguration (R), (S) oder (R,S).

39. Als neue industrielle Produkte die Verbindungen der Formel (F₆): 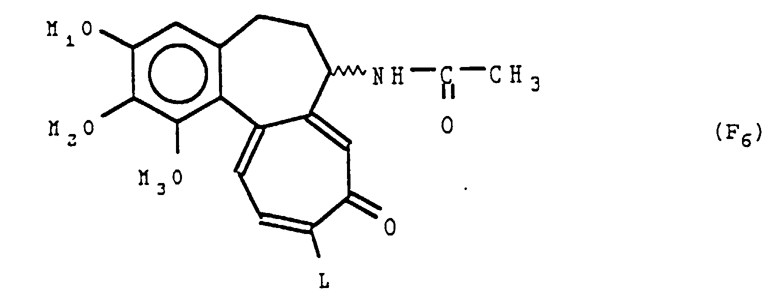 worin die gewellte Linie anzeigt, daß sich der Substituent in der (R)-, (S)- oder (R,S)-Konfiguration befindet und
- entweder M₁ und M₂ ein Wasserstoffatom bedeuten, M₃ für einen Rest Ra, wie in Anspruch 27 definiert, steht und L einen Rest OK wiedergibt, wobei K wie in Anspruch 13 definiert ist,
- oder M₂ und M₃ ein Wasserstoffatom bedeuten, M₁ für einen Rest Ra, wie vorstehend definiert, steht und L einen Rest OK, wie vorstehend definiert, wiedergibt,
- oder M₁ einen Rest Ra, wie vorstehend definiert, bedeutet, M₂ und M₃ eine Methylgruppe wiedergeben und L einen Thiomethylrest darstellt.

## Claims

1. The compounds of formula (I): 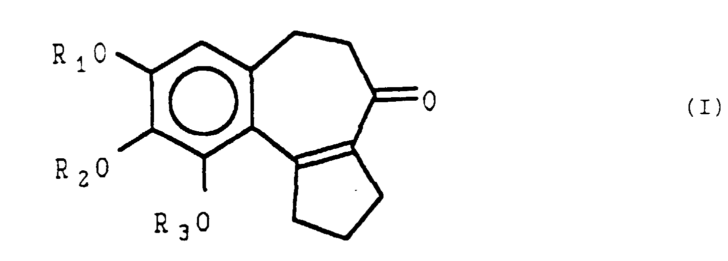 in which:
either (a) R₁ and R₂ both represent an alkyl group and R₃ represents a hydrogen atom or the A-SO₂- group,
or (b) R₂ and R₃ both represent a hydrogen atom or both represent an alkyl group and R₁ represents an A-SO₂- group, or (c) R₁, R₂ and R₃ all represent a hydrogen atom or all represent an alkyl group,
or (d) R₁ represents the A-SO₂- group or a hydrogen atom and R₂ and R_{3,} form together with the oxygen atoms to which they are linked, a group of formula -O-X-O- in which X represents a group of formula -B(OR₄)-, where R₄ represents a hydrogen atom, an alkyl group, a -C(O)- group, or a group of formula -CR₅R₆-, where R₅ and R₆ represent a hydrogen atom, an alkyl or phenyl group optionally substituted by 1 to 3 groups chosen from the alkyl, hydroxy and alkyloxy radicals or R₅ and R₆, together with the carbon atom to which they are linked, represent a carbocyclic group with 5 or 6 members,
or (e) R₃ represents the A-SO₂- group and R₁ and R_{2,} form together with the oxygen atoms to which they are linked, the -O-X-O- group,
or (f) R₁ represents a hydrogen atom and R₂ and R₃ both represent an alkyl group or, form together with the oxygen atoms to which they are linked, a group of formula -O-X-O- as defined above,
"A" represents an alkyl group, a phenyl group non substituted or substituted by 1 to 3 alkyl groups or a naphthyl group non substituted or substituted by 1 to 5 alkyl groups,
the alkyl or alkyloxy groups mentioned above, being branched or non branched, and containing 1 to 6 carbon atoms.

2. The compounds of formula (I) as defined in claim 1, characterised in that R₁, R₂ and R₃ all represent a hydrogen atom or an alkyl group as defined in claim 1.

3. A compound according to claim 2, characterized in that R₁, R₂ and R₃ all represent a methyl radical, or all represent a hydrogen atom.

4. The compounds of formula (I) as defined in claim 1, characterised in that R₁ represents an A-SO₂- group, as defined in claim 1, and R₂ and R₃ both represent an alkyl group as defined in claim 1, or both represent a hydrogen atom.

5. A compound according to claim 4, characterized in that R₁ represents a tosyl group and R₂ and R₃ both represent a methyl radical or both represent a hydrogen atom.

6. The compounds of formula (I) as defined in claim 1, characterised in that R₃ represents an A-SO₂- group as defined in claim 1 and R₁ and R₂ both represent an alkyl radical as defined in claim 1.

7. A compound according to claim 6, characterized in that R₃ represents a tosyl group and R₁ and R₂ both represent a methyl radical.

8. The compounds of formula (I) as defined in claim 1, characterised in that R₁ and R₂ both represent an alkyl group and R₃ represents a hydrogen atom.

9. A compound according to claim 8, characterized in that R₁ and R₂ both represent a methyl radical.

10. A compound according to claim 1, characterized in that R₁ represents a hydrogen atom and R₂ and R₃ both represent a methyl radical.

11. Process for the preparation of the compounds of formula (I) as defined in claim 1, characterized in that (i) a compound of formula (a): 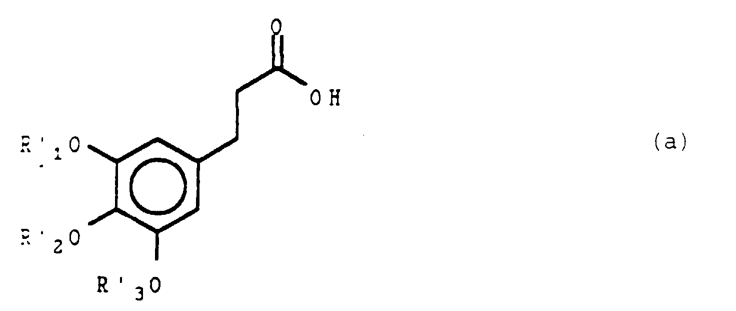 in which
R₁', R₂' and R₃' have the same meaning as the R₁, R₂ and R₃ groups respectively as defined in claim 1, with the exception of the hydrogen value and for R₃' the A-SO₂- value,
is subjected to the action of a halogenation agent, in order to obtain the corresponding acyl halide,
(ii) which is subjected to the action of a reagent of formula (b) : 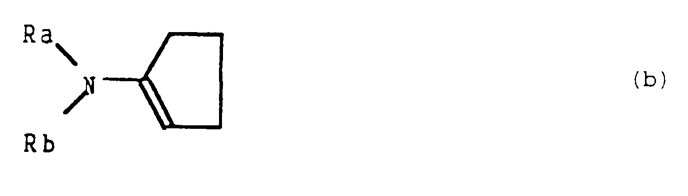 in which Ra and Rb, identical or different, represent an alkyl group, or Ra and Rb, together with the nitrogen atom to which they are linked, represent a heterocycle with 5 or 6 members optionally comprising another heteroatom chosen from O and N;
in order to obtain a compound of formula (c) 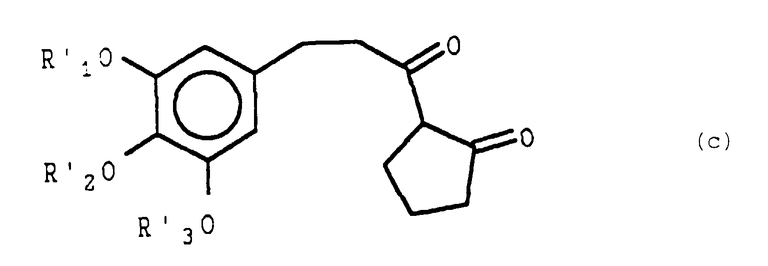
(iii) which is subjected to the action of a halogenation agent in order to obtain a compound of formula (d): 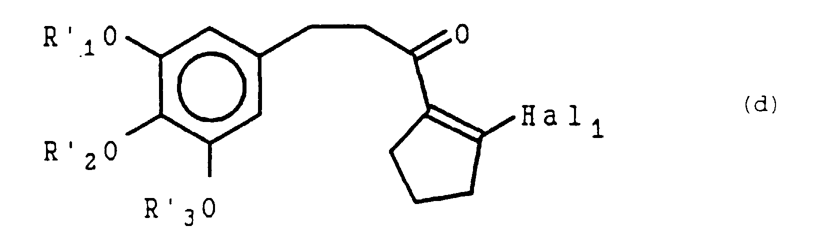 in which Hal₁ represents a halogen atom;
(iv) which is subjected to the action of a Lewis acid, in order to obtain a compound of formula (e) : 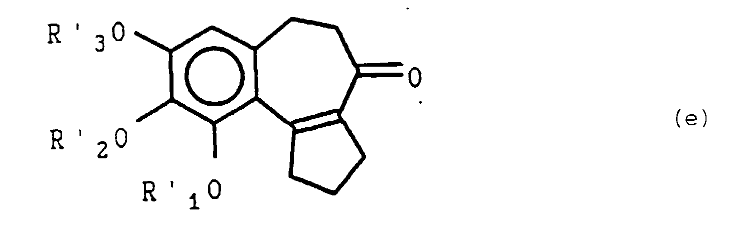 corresponding to the compounds of formula (I)(a) as defined in claim 1, in which R₃ represents an A-SO₂- group, to the compounds of formula (I)(c) as defined in claim 1, in which R₁, R₂ and R₃ all represent an alkyl radical, and to the compounds of formula (I)(e), as defined in claim 1 and compound of formula (e), which, if appropriate,
(v) -either if R'₁ represents an A-SO₂- group and R'₂ and R'₃ together do not represent the -X- group, is subjected to the action of a hydrolysis agent in order to obtain a compound of formula (f): 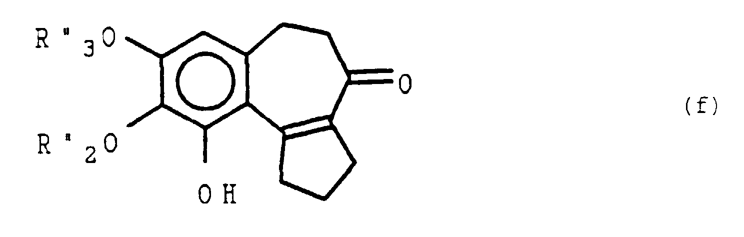 in which R"₂ and R"₃ both represent an alkyl radical; corresponding to the compounds of formula (I)(a) as defined in claim 1 in which R₃ represents a hydrogen atom;
which, if appropriate,
(vi) it is subjected to the action of an alkylation agent, in order to obtain a compound of formula (g) : 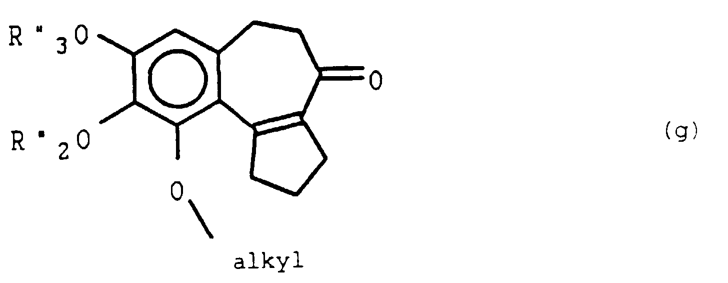 corresponding to the compounds of formula (I) (c) as defined in claim 1, in which R₁, R₂ and R₃ represent an alkyl radical, which if appropriate,
(vii) is subjected to hydrolysis of all of alkyloxy groups in order to obtain the compound of formula (h): 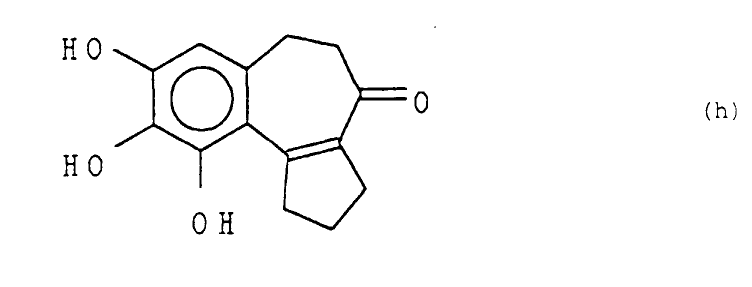 corresponding to the compound of formula (I)(c) as defined in claim 1, in which R₁, R₂ and R₃ represent a hydrogen atom,
(viii) or a compound of formula (e) in which R'₁, R'₂ and R'₃ all represent an alkyl radical, is subjected to hydrolysis of all of the alkyloxy groups, in order to obtain the compound of formula (h) as defined above,
(ix) which, if appropriate, is treated with a diol protection reagent chosen from the compounds of formulae:
B(OR₄)₃, C(O)R₅R₆, C(O)Y₂ and CR₅R₆(Y)₂,
in which R₄, R₅ and R₆ are as defined in claim 1 and Y represents a halogen atom or a group of formula Ar-O-, in which Ar represents a phenyl group optionally substituted by 1 to 3 substituants chosen from the alkyl, alkyloxy, hydroxy, amino, alkylamino, dialkylamino and nitro radicals;
or with a reagent of formula CH₂(Y)₂, Y being defined as above, in the presence of a reagent of formula P(Hal)₅, in which Hal represents a halogen atom, followed by hydrolysis, in order to obtain a compound of formula (i): 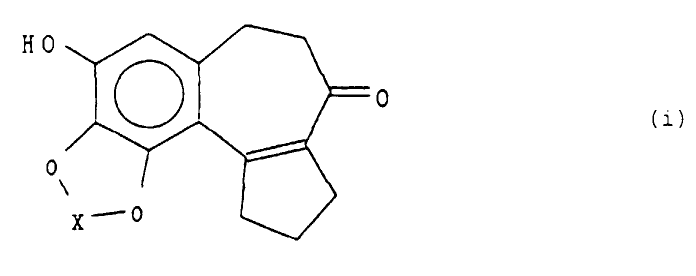 corresponding to the compounds of formula (I) (d) as defined in claim 1, in which R₁ represents a hydrogen atom,
which is treated with an agent of formula A-SO₂Y, in which A is defined as in claim 1 and Y is defined as above, in order to obtain a compound of formula (j) : 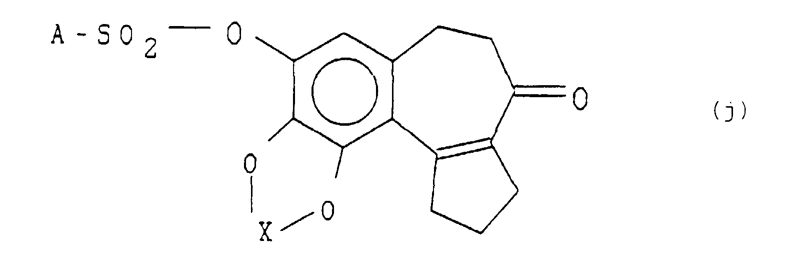 corresponding to the compounds of formula (I)(d) as defined in claim 1, in which R₁ represents the A-SO₂- group, which, if appropriate,
(x) is subjected to the action of a deprotection reagent of the diol, in order to obtain a compound formula (k) : 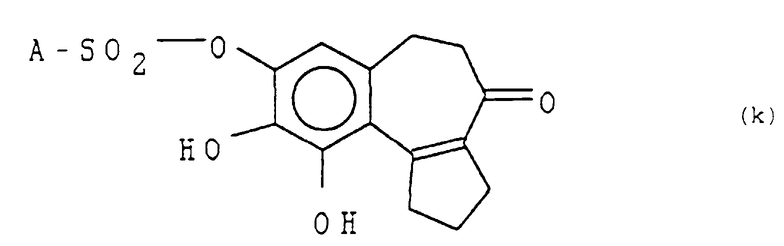 corresponding to the compounds of formula (I) (b) as defined in claim 1, in which R₂ and R₃ represent a hydrogen atom, which, if appropriate,
(xi) is subjected to the action of a alkylation agent in order to obtain a compound formula (1): 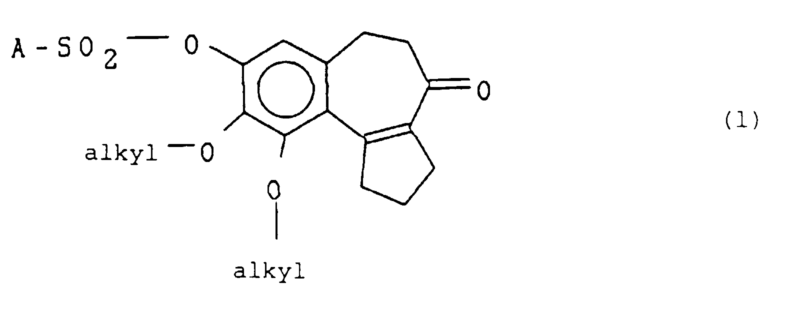 corresponding to the compounds of formula (I)(b) as defined in claim 1, in which alkyl has the same meaning as in claim 1,
(xii) or if appropriate a compound of formula (j) or (l) is subjected to the action of a hydrolysis agent of the A-SO₂- O- radical in order to respectively obtain a compound of formula (i) 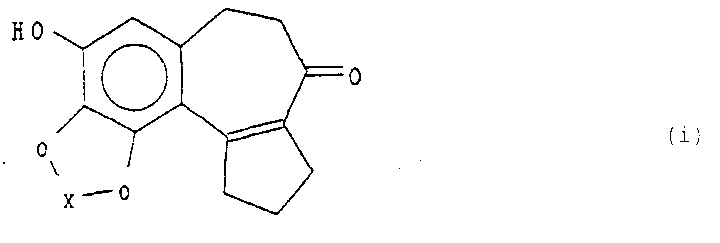 or 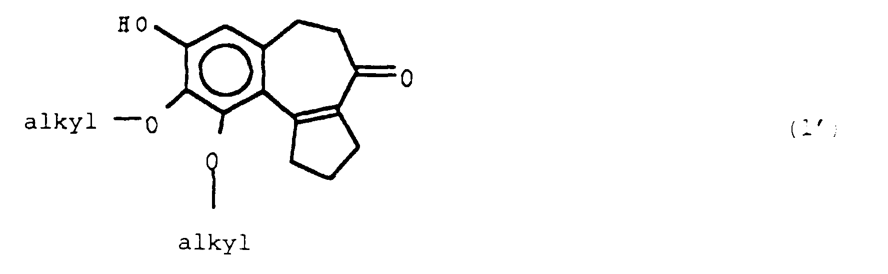 corresponding to the compounds of formula (I) (f) as defined in claim 1.

12. Process according to claim 11, characterized in that
- the halogenation agent used in stage (i) is thionyl chloride;
- the reagent of formula (b) used in stage (ii) is 1-(N-morpholinyl)-cyclopentene;
- the halogenation agent used in stage (iii) is oxalyl chloride-(COCl)₂;
- the Lewis acid used in stage (iv) is ferric chloride or tin tetrachloride;
- the diol protection reagent used in stage (ix) is trimethyl or triethyl borate;
- the ASO₂Y reagent which is reacted in stage (ix) and such that A represents an alkyl or phenyl radical substituted by one to three alkyl radicals.

13. Use of a compound of formula (I) as defined in claim 1, for the synthesis of the compounds of formula (II): 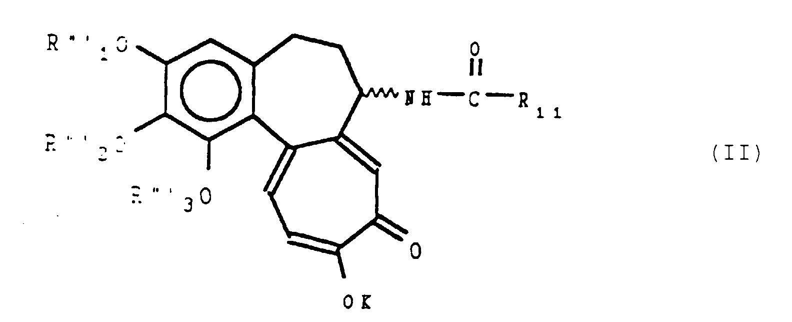 in which R"'₁, R"'₂ and R"'₃ have the same meaning as the R₁, R₂ and R₃ groups as defined in claim 1, with the exception of the hydrogen value, and for R₂-R₃, the value of X other than -CR₅R₆- and, for R₁-R₂, the value of X other than -CR₅R₆-, R₁₁ represents an alkyl radical, the wavy line indicating that the NH-C(O) R₁₁ group is in position α or β, or that the compound is in the form of a mixture of α + β isomers, and K represents an alkyl radical, a benzyl radical optionally substituted on the phenyl by 1 to 3 alkyl radicals, or an -SO₂-A' radical, in which A' is chosen independently from the values of A mentioned above, characterized in that:
(i) either a compound of formula (I), as defined above, in which the R₁, R₂ or R₃ groups do not represent a hydrogen atom, is subjected to the action of an agent of formula (m): 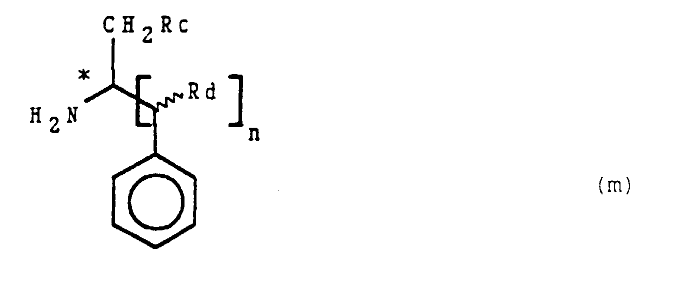 in the racemic or optically active form, in which Rc and Rd, identical or different, represent a hydrogen atom or a hydroxy radical and n represents the number 0 or 1; then the imino intermediate thus obtained is treated with a reducing agent in order to obtain a compound of formula (n): 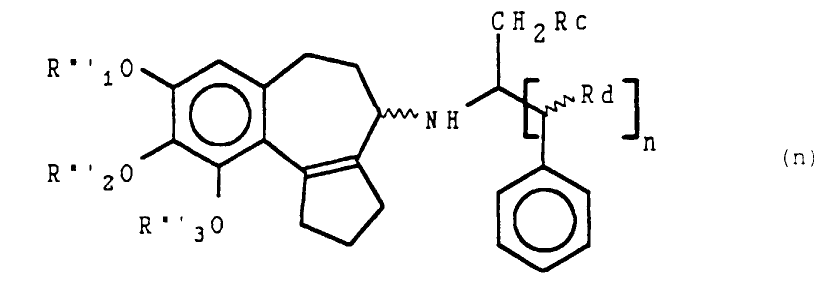 in which Rc and Rd have the meaning indicated above, and the wavy line linking NH to the ring symbolizes, according to whether an optically active or racemic agent of formula (m) is used, a mixture of isomers or a mixture of one or another isomer respectively,
compound (n) which either is subjected to hydrogenolysis of the alkylamino group, in order to obtain a compound of formula (o): 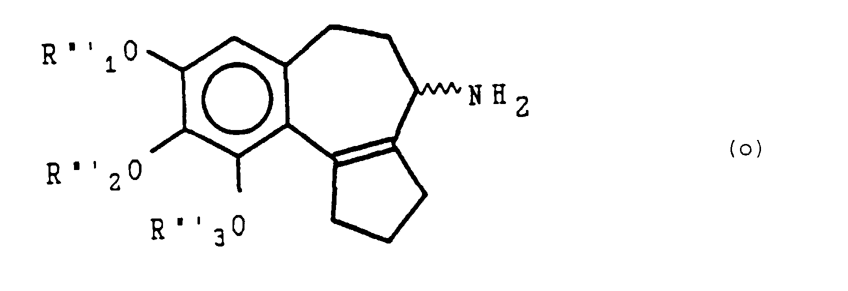 in which the wavy line is defined as above,
the amino group of which is protected with a divalent protective group in order to obtain a compound of formula (p): 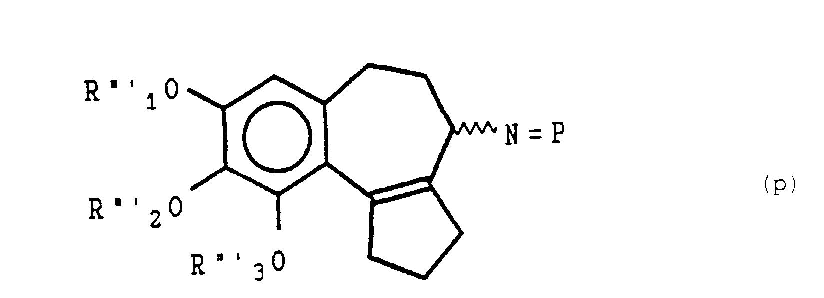 in which the wavy line is defined as above and P represents a divalent protective group,
or the compound of formula (n) as defined above, which is subjected to the action of an hydrolysis agent of the R"'₁O group in order to obtain a compound of formula (n') : 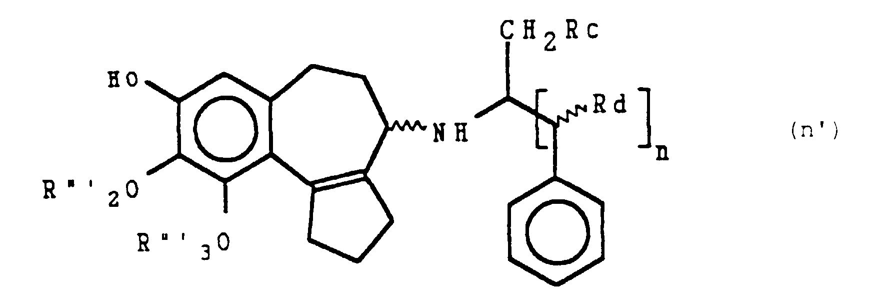 in which R"'₂, R"'₃, Rc, Rd, n and the wavy lines have the meaning indicated previously, which is subjected to hydrogenolysis of the alkylamino group, in order to obtain a compound of formula (o'): 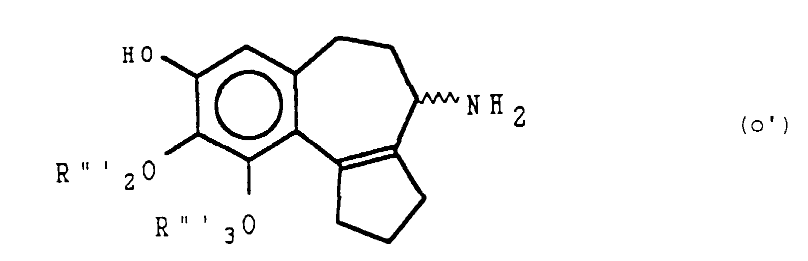 in which the wavy line is defined as above, the amino group of which is protected with a divalent protective group in order to obtain a compound of formula (p'): 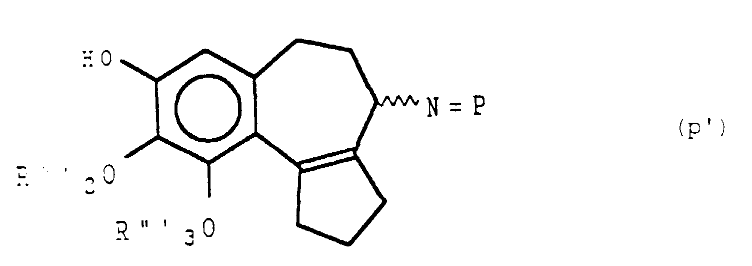 in which P and the wavy line have the meaning indicated above, which is subjected to the action of a protective agent of the hydroxy radical in order to obtain a compound of formula (p) as defined previously;
(ii) or a compound of formula (I)(f) as defined above, is subjected to the action of an agent of formula (m) defined above, the imino intermediate obtained is treated with a reducing agent in order to obtain a compound of formula (n') as defined above, then the synthesis is continued as indicated above;
(iii) or a compound of formula (I) as defined above, is subjected to the action of an agent of formula R₈O-NH₂, or a salt of this compound, in which R₈ represents a hydrogen atom or an alkyl radical, in order to obtain a compound of formula (q) : 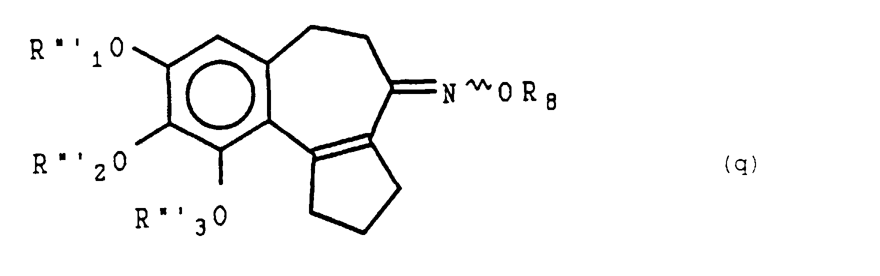 in which the wavy line indicates that the compound consists of a mixture of syn and anti isomers,
(iv) or a compound of formula (I) as defined above, is subjected to the action of a chiral reducing agent, or a non-chiral reducing agent, in order to obtain the corresponding alcohol, of formula (r): 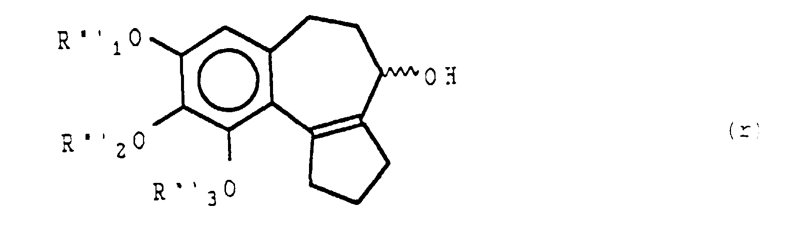 in which the wavy line between the nitrogen atom and the ring with 7 members indicates that this product consists, according to the reducing agent used, either of an α or β isomer, or a mixture of α + β isomers, which mixure, if appropriate, is resolved, then either the alcohol is subjected to the action of an agent of formula HN=P, in which P has the meaning given previously, in order to obtain a compound of formula (p): 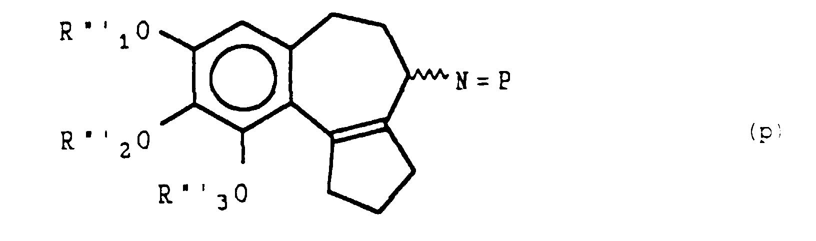 in which the wavy line between the nitrogen atom and the ring with 7 members indicates that this product consists of an α or β isomer, or a racemic mixture of isomers,
or the alcohol is subjected to the action of a reagent of formula ASO₂Hal, in which A is defined as above and Hal represents a halogen atom in order to obtain the compound of formula (r₁): 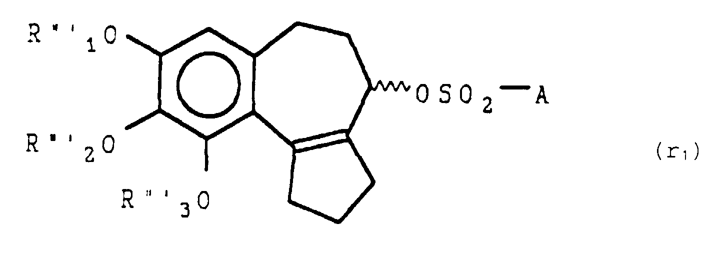 in which the wavy line and A have the meanings above, which is reacted with ammonia, in order to obtain the compound of formula (o) above, the amino group of which is protected with a divalent protective group, in order to obtain the compound of formula (p) above, then,
(v) a compound of formula (p) or (q) is subjected to the action of a bromination agent in the presence of a compound of formula R₇OH, in which R₇ represents a hydrogen atom or an alkyl radical, in order to obtain a compound of formula (s): 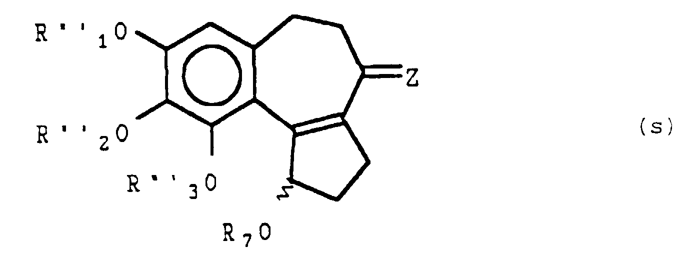 in which the wavy line indicates that the bond can be found in alpha or beta position or that this product can consist of a mixture of isomers and in which the =Z group represents a group of formula = N 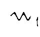 OR₈ or 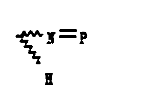 as definied in formulae p and q, above,
(vi) which is subjected to dehydration or dealcoxylation in an anhydrous acid medium in order to obtain a compound of formula (t) : 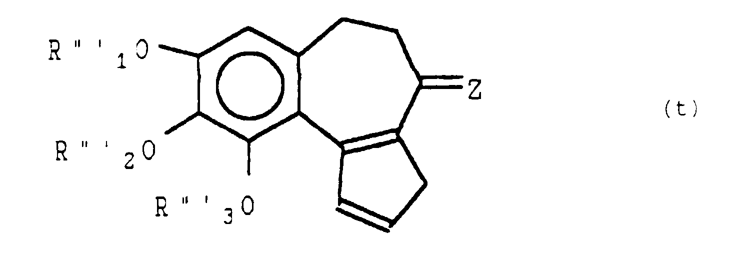
(vii) which, is subjected in the presence of a base or a metal reducing agent, to the action of a agent of formula 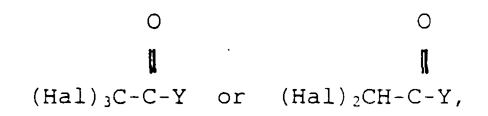 in which Hal represents a halogen atom and Y is as defined above, in order to obtain a compound of formula (u) : 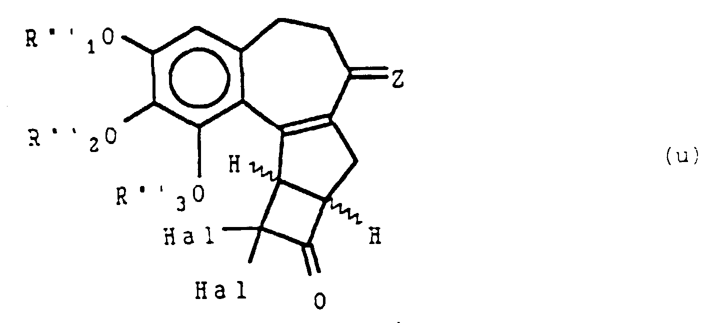 in which the wavy lines indicate that the hydrogen atoms are both found in alpha position or are both found in beta position;
(viii) which is subjected to the action of a carboxylic acid in the presence of a base and in an aqueous medium, in order to obtain a compound of formula (v), which exists in equilibrium with its tautomer (v'): 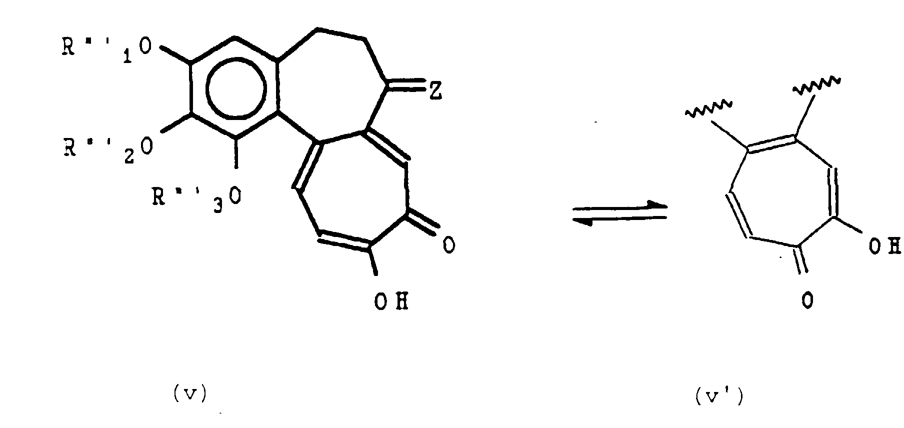 which compound, either,
(ix) if =Z represents a group of formula 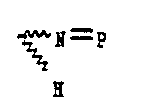 as defined above, either is subjected to a deprotection reaction of the amino group in order to obtain either the free amine of formula (w), which exists in equilibrium with its tautomer (w'): 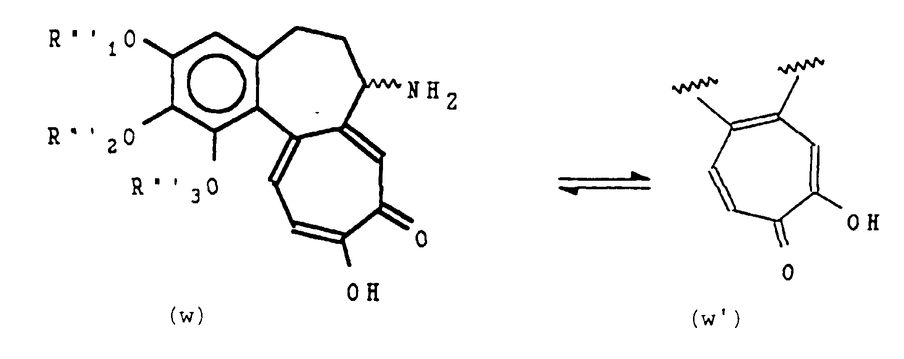 in which the wavy line indicates that the NH₂ group is found in alpha or beta position, or that the product consists of a mixture of isomers,
which is subjected to the action of a reagent of formula R₁₁-C(O)Y or formula (R₁₁CO)₂O, in which the R₁₁ group represents an alkyl group and Y is defined as in claim 11, in order to obtain a compound of formula (x): 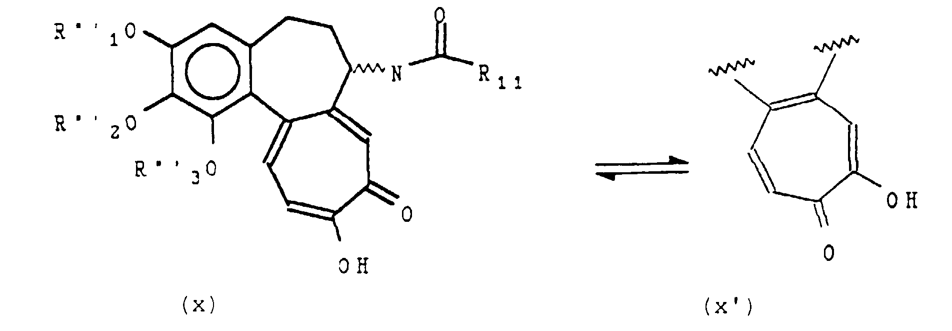 in which the wavy line has the previous meaning,
or is subjected to a deprotection reaction of the amino group and to a hydrolysis agent of the R"'₁ group in the case where this group represents the A-SO₂- value as defined above, in order to obtain a compound formula (w₁): 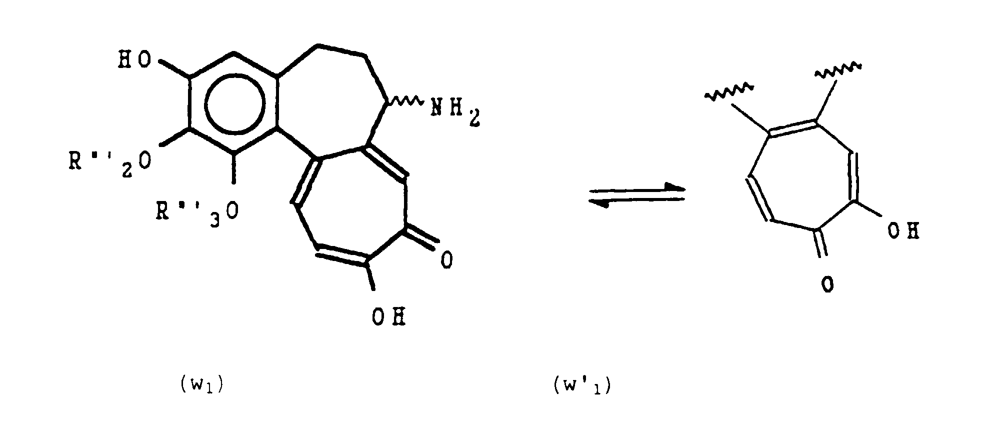 which is subjected to the action of a compound of formula R₁₁C(O)Y or (R₁₁CO)O as defined above, in order to obtain a compound of formula (x₁): 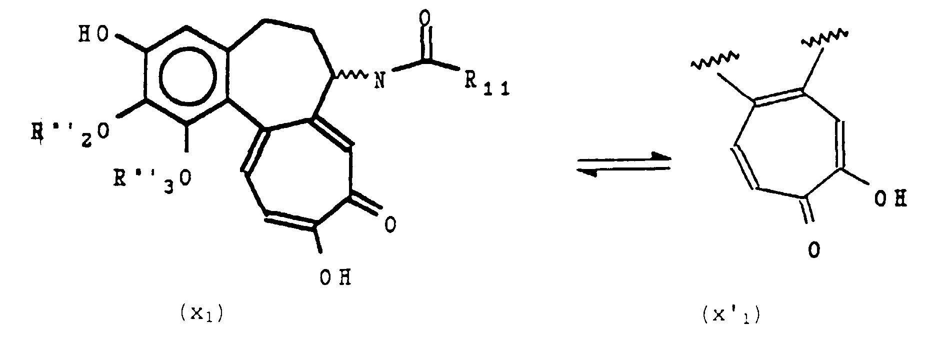 the OH function of which is protected by the action of a reagent of formula ASO₂Y as defined above, in order to obtain a corresponding compound of formula (x)/(x') as defined above, or,
(x) if =Z represents a group of formula =N~OR₈, is subjected to the action of a protective agent of formula B(Hal)₃ or B(OR₄)₃ in which R₄ and Hal are as defined previously, then to the action of a reducing agent in order to obtain a compound of formula (y): 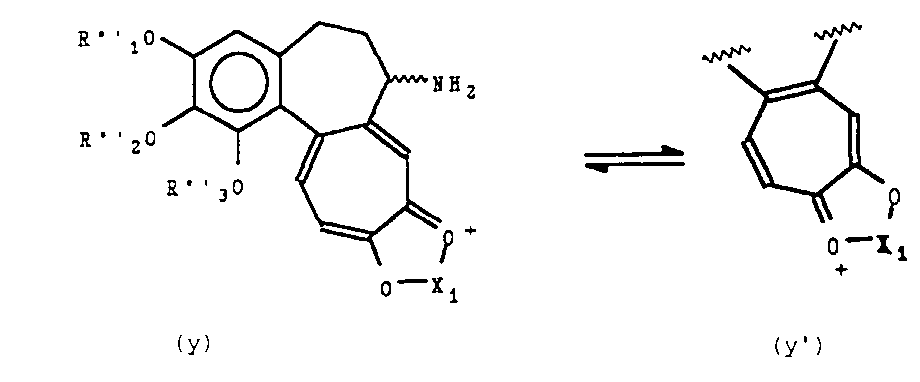 in which the wavy line indicates that this product consists of a mixture of isomers, and X₁ represents a group of formula -B(OR₄)₂- or -B(Hal)₂ which is subjected to the action of a compound of formula R₁₁C(O)Y or of formula (R₁₁CO)₂O, as defined above, then to the action of a deprotection reagent of the alpha-hydroxy ketone group, in order to obtain a compound of formula (x")/(x"₁) corresponding to the product of formula (x)/(x') as defined above, in which the wavy line indicates that the product consists of a mixture of isomers;
(xi) which compound of formula (x) or (x') is subjected to the action of an agent capable of introducing the K group as defined above, in order to obtain a compound of formula (II): 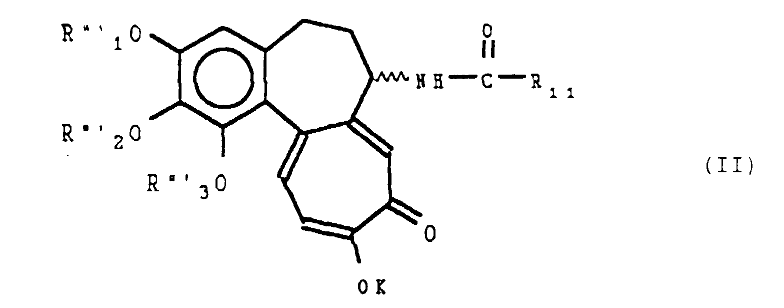 which exists in a mixture with its regioisomer of formula: 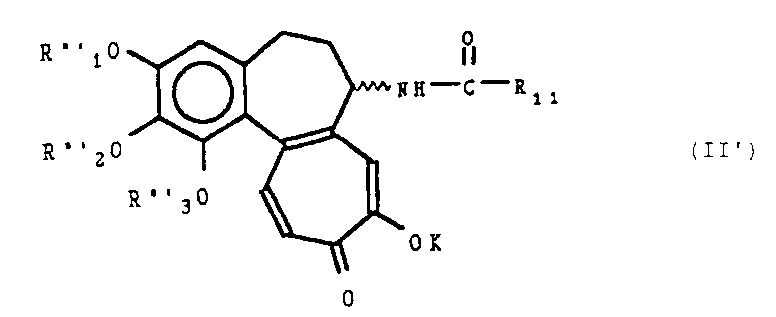 in which formulae (II) and (II') the wavy line indicates that the group is found in alpha or beta position, or that this product consists of a mixture of isomers and the terms R"'₁, R"'₂, R"'₃, R₁₁, K and alkyl having the meanings given previously, which mixture is separated into its constituents.

14. Use according to claim 13, characterized in that the amine of formula (m) used in stage (i) or (ii) is (S)-α-methylbenzylamine, norephedrine or norpseudoephedrine.

15. Use according to claim 13 or 14, characterized in that the protective group of the amino radical is a phthalimidc or maleimido group.

16. Use according to claim 13, characterized in that the agent of formula R₈O-NH₂ is methylhydroxylamine.

17. Use according to any one of claims 13 to 16, characterized in that the R₇OH reagent is methanol.

18. Use according to any one of claims 13 to 17, characterized in that the halogenated derivatives which is reacted with compound (t) is dichloroacetyl chloride.

19. Use according to any one of claims 13 to 18, characterized in that R₁₁ represents a methyl radical.

20. Use according to any one of claims 13 to 19, characterized in that the ASO₂Y reagent which is reacted with the (x₁)/(x'₁) compound is mesyl or tosyl chloride.

21. Use according to any one of claims 13 to 20, characterized in that the agent capable of introducing the K group is mesyl, tosyl or 2,4,6-triisopropyl sulphonyl chloride.

22. Use according to any one of claims 13 to 21, characterized in that:
- the agent (m) is reacted with compound (I) in the presence of titanium tetrachloride and a tertiary aminated base or in the presence of an acid catalyst;
- the protection of the amino group is carried out by the action of phthalic anhydride on compound (o) or (o');
- the agent of formula HN=P is the phthalic anhydride and the reaction with compound (r) is carried out in the presence of triphenylphosphine and an alkyl azobicarboxylate;
- the rearrangement into tropolone is carried out by the action of acetic acid on compound (u), in the presence of an animated base;
- the deprotection of the amine is carried out with hydrazine;
- the selective hydrolysis of compound (x)/(x') is carried out by the action of an alkaline thioalcoholate;
- the reagent forming the protective group of the hydroxy and ketone functions is boron trifluoride;
- the reducing agent which is reacted on the oxime derived from compound (v)/(v') is zinc, in the presence of a sulphonic or carboxylic acid.

23. Use according to claim 13, characterized in that a compound of formula (v)/(v') as defined in claim 13 in which Z represents a group 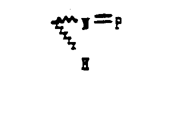 is subjected to the action of an agent capable of introducing the K group as defined in claim 13, in order to obtain a compound of formula (III) : 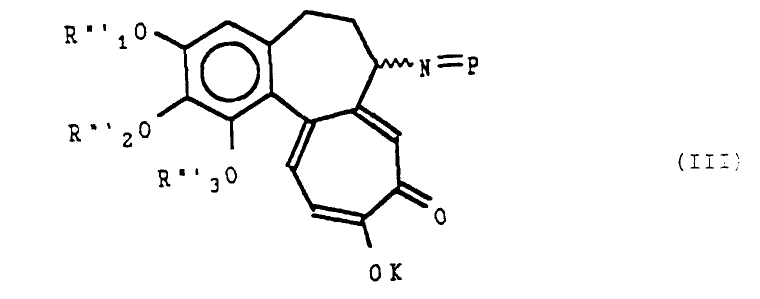 which exists in a mixture with its regioisomer of formula: 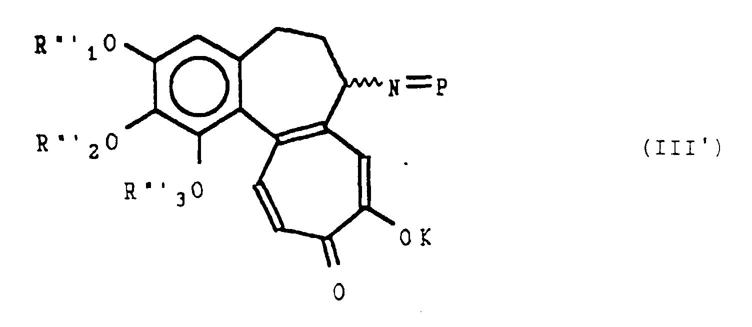 in which formulae (III) and (III') the wavy line indicates that the group is found in alpha or beta position, or that this product consists of a mixture of isomers and the terms R"'₁, R"'₂, R"'₃, R₁₁ and K and alkyl having the meanings given in claim 13, which mixture, if appropriate, is separated into its constituents, then the mixture or the separate constituants, are subjected to a deprotection reaction of the amino function, in order to obtain either the compound of formula (IV): 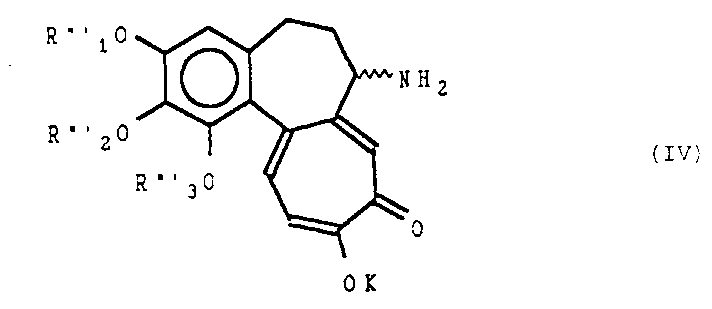 in the form of a mixture with its regioisomer of formula 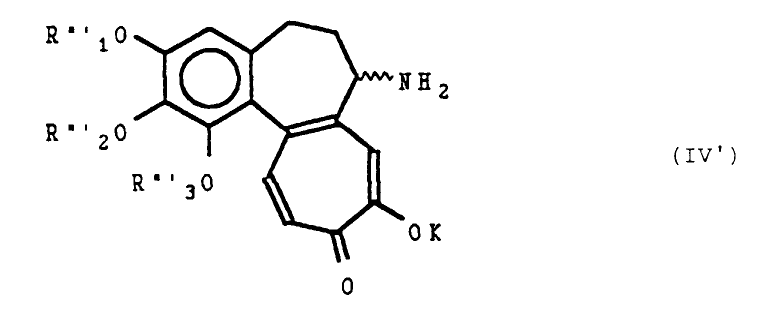 which mixture, if appropriate, either one or other of these regioisomers is separated, then the mixture or the separate constituants are subjected to the action of a compound of formula R₁₁C(O)Y or (R₁₁CO)₂O, as defined in claim 13, in order to obtain either the compound of formula (II) as defined above, in a mixture with its regioisomer of formula (II'), which mixture, if appropriate,is separated into its constituants, or one or other of these regioisomers.

24. Use according to claim 23, characterized in that the agent capable of introduing the K group, the deprotection reagent of the amino function and R₁₁ are as defined in claims 19, 21 and 22.

25. Use according to claim 13, characterized in that a compound of formula (v)/(v') as defined previously, in which (z) represents an =N-OR₈ group, is subjected to the action of an agent capable of introducing the K group, in order to obtain a compound of formula (V): 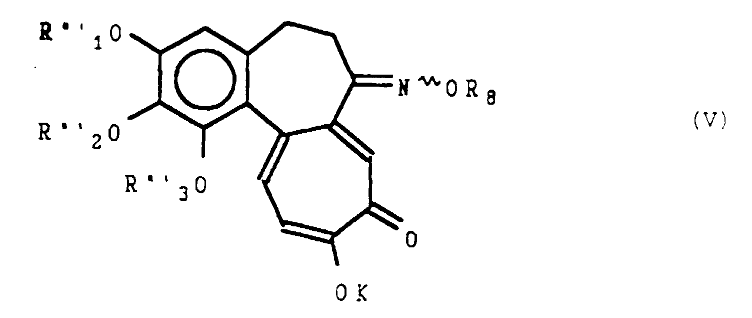 which exists in a mixture with its regioisomer of formula 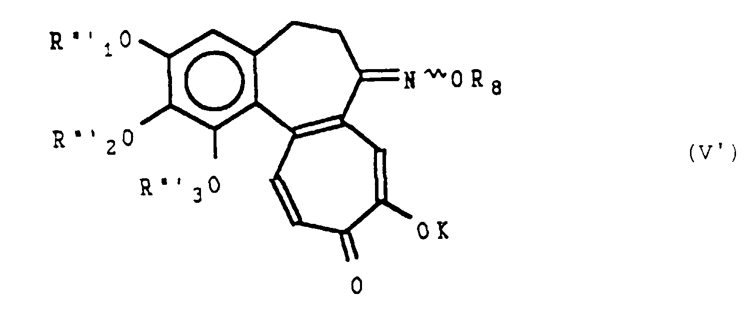 in which formulae (V) and (V') the wavy line, R₈ and the terms R"'₁, R"'₂, R"'₃, K and alkyl have the meanings given in claim 13, which mixture, if appropriate, is separated into its constituents, then the mixture or the separate constituants, are subjected to the action of a reducing agent, in order to obtain either the compound of formula (IV₁): 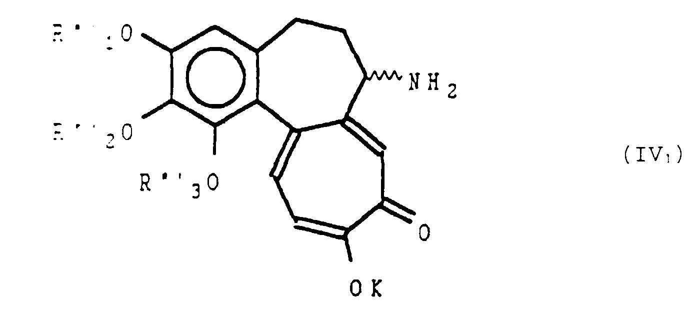 in the form of a mixture with its regioisomer of formula 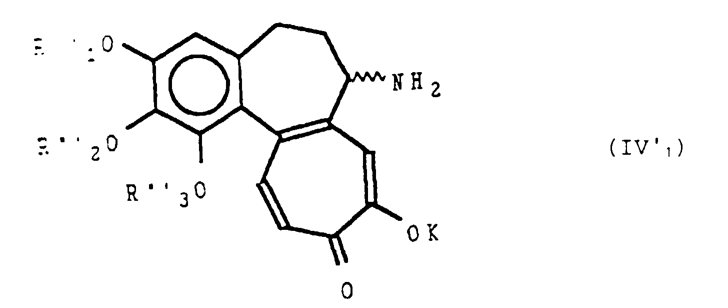 the wavy line symbolizing a mixture of isomers, which mixture of regioisomers, if appropriate, is separated, or one or the other of these regioisomers, then the synthesis is continued as described in claim 13 for the compounds of formula (IV)/(IV').

26. Use according to claim 25, characterized in that the agent capable of introducing the K group and the reducing agent of the oxime are as defined in claims 21 and 22.

27. Use according to claim 13, characterized in that in addition a product of formula (II₁): 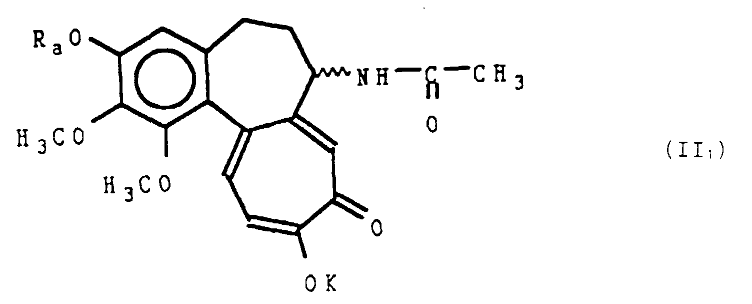 in which K is defined as in claim 13, Rₐ represents an ASO₂-radical as defined in claim 13 and the wavy line symbolizes one or other isomer or a mixture of isomers,
is subjected to the action of a reagent of formula CH₃S⁻Na⁺ in order to obtain a compound of formula (VI): 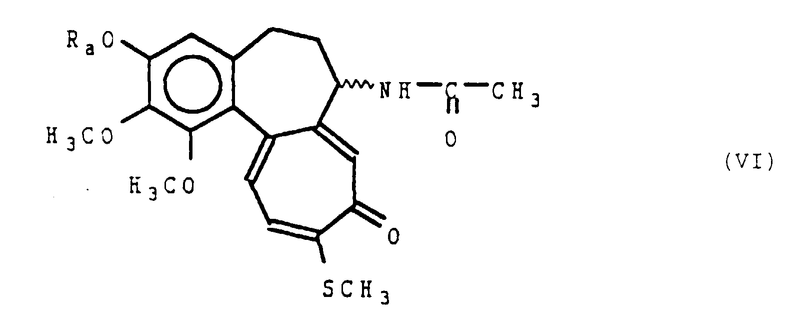 in which Rₐ and the wavy line have the previous meaning which is subjected to a deprotection reagent of the hydroxy radical in order to obtain the optically active or racemic compound of formula (VII):

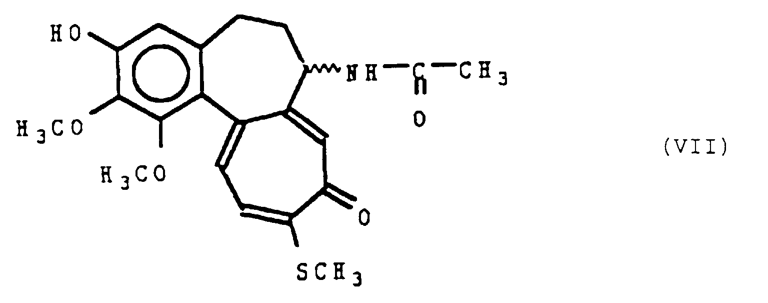

28. Use according to claim 27, characterized in that the OK group is substituted and the hydroxy radical is deprotected with the same CH₃SNa reagent.

29. Use according to claim 13, characterized in that in addition a compound of formula (II₂): 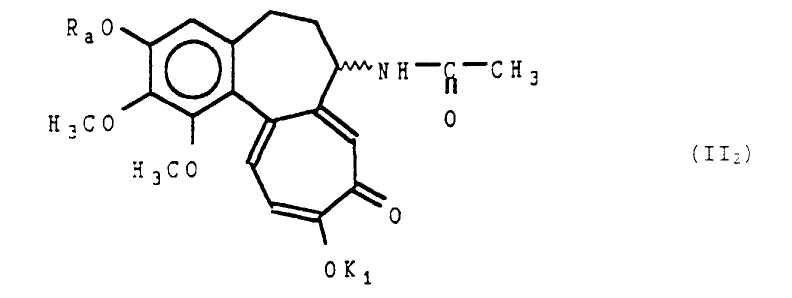 in which Rₐ is defined as in claim 13, K₁ represents a methyl radical and the wavy line symbolizes one or other isomer or a mixture of isomers, is subjected to the action of a deprotection agent of the hydroxy radical, in order to obtain the optically active or racemic compound of formula (VIII):

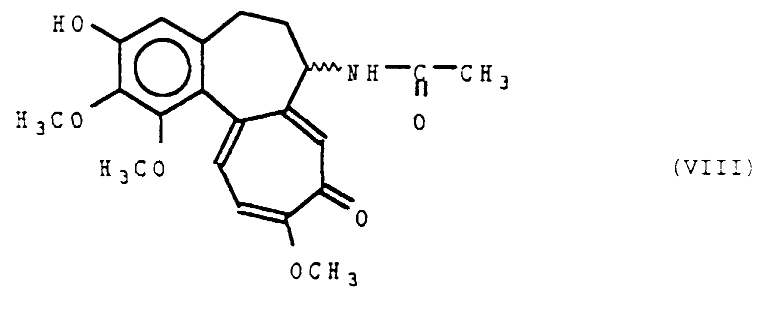

30. Use according to claim 13, characterized in that a compound of formula (II₃): 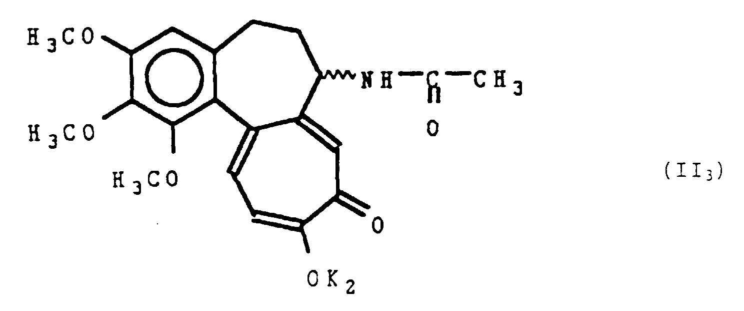 in which K₂ represents a benzyl radical optionally substituted on the phenyl by 1 to 3 alkyl radicals, or an -SO₂A' radical as defined previously and the wavy line symbolizes one or other isomer, or a mixture of isomers, is subjected to the action of a reagent of formula CH₃S⁻Na⁺, in order to obtain the optically active or racemic compound of formula (IX):

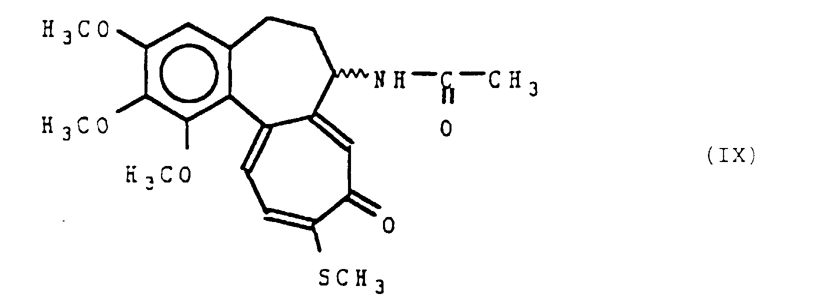

31. Use according to claim 13, characterized in that a compound of formula (II₄): 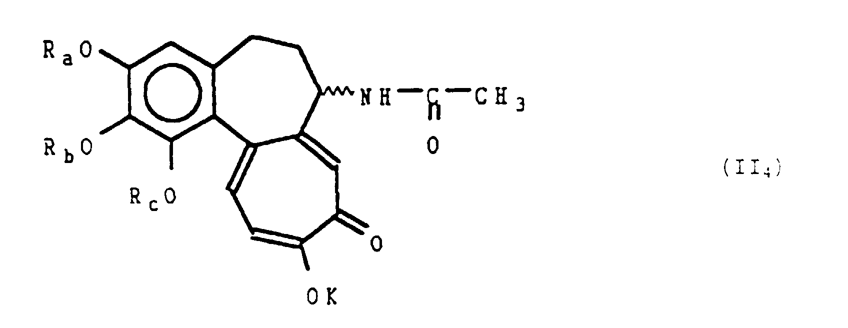 in which Rₐ and K are as defined in claim 13, R_{b} and R_{c} together form a CR₅R₆ group as defined in claim 1 and the wavy line symbolizes one or other isomer or a mixture of isomers, is subjected to the action of a diol deprotection reagent, in order to obtain a compound of formula (X): 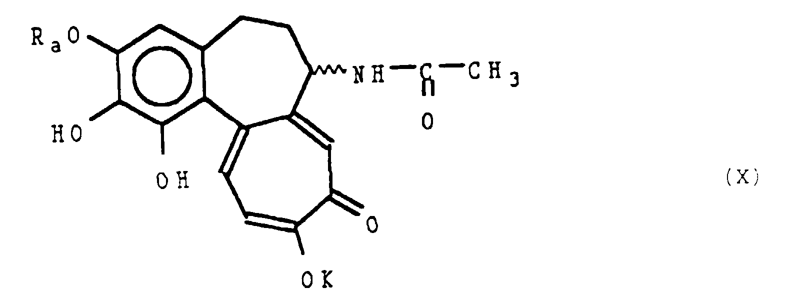 in which Rₐ, K and the wavy line have the previous meaning which is treated with a methylation agent in order to obtain a compound of formula (II₁) as defined in claim 27, then the synthesis is continued as described in claim 27 starting with said compound of formula (II₁).

32. Use according to claim 13, characterized in that a compound of formula (II₅): 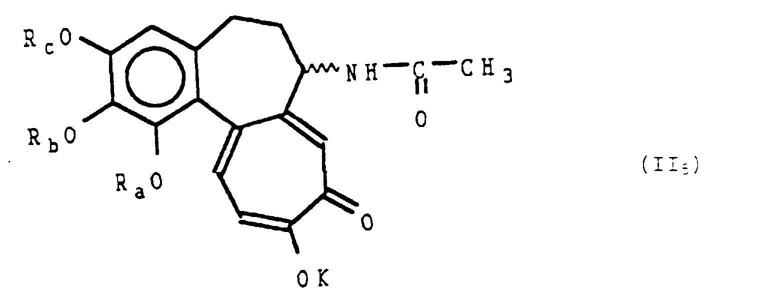 in which Rₐ, R_{b}, R_{c}, K and the wavy line are defined as in formula (II₄) in claim 31, is subjected to the action of a diol deprotection agent, in order to obtain a compound of formula (XI): 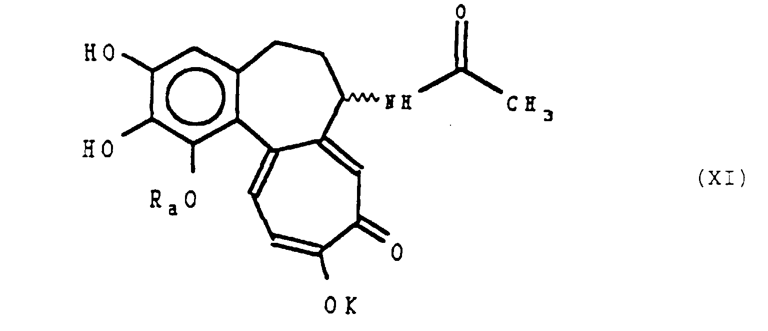 in which Rₐ, K and the wavy line have the previous meaning, which is subjected to the action of a hydrolysis agent in order to obtain the compound of formula (XII): 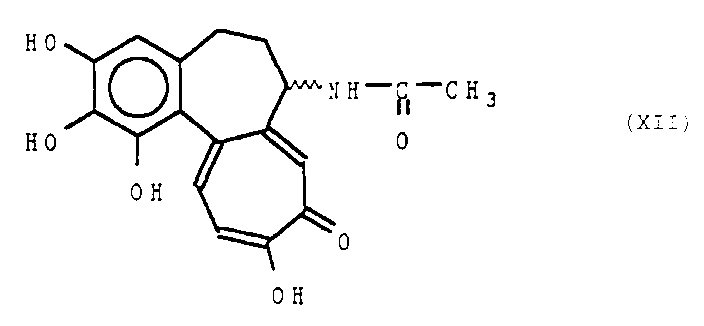 which is subjected to a methylation agent, in order to obtain the optically active or racemic colchicine.

33. As new industrial compounds, the compounds of formula (F₁): 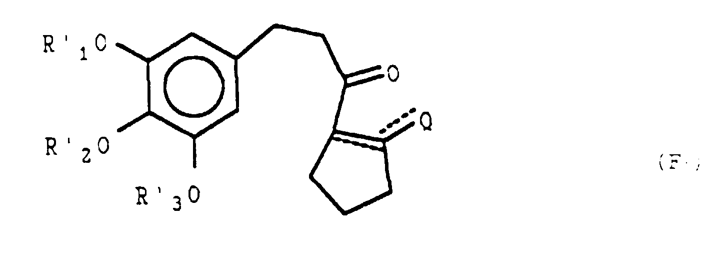 in which Q represents either an oxo radical, or the dotted line in the ring represents a second bond and Q represents a halogen atom, R'₁, R'₂ and R'₃ have the meaning indicated in claim 11.

34. As new industrial compounds, the compounds of formula (F₂): 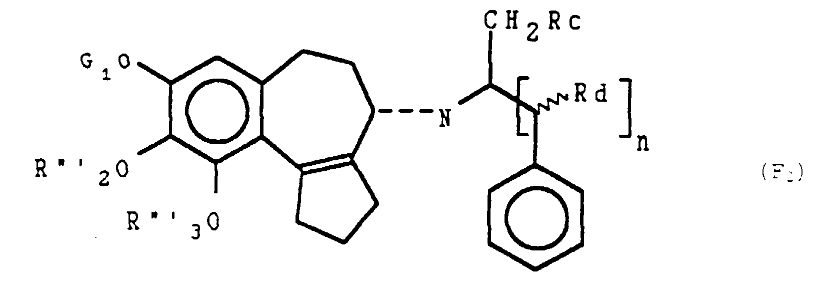 in which G₁ represents a hydrogen atom or the R"'₁ radical as defined above and Rc and Rd as well as R"'₂ and R"'₃ have the meaning indicated in claim 13, the dotted line symbolizes either a double bond, or a single bond and, in this case, the nitrogen carries a hydrogen atom and the compound is presented in the form of a mixture of (R,S) isomers or (R) or (S) isomer.

35. As new industrial compounds, the compounds of formula (F₃): 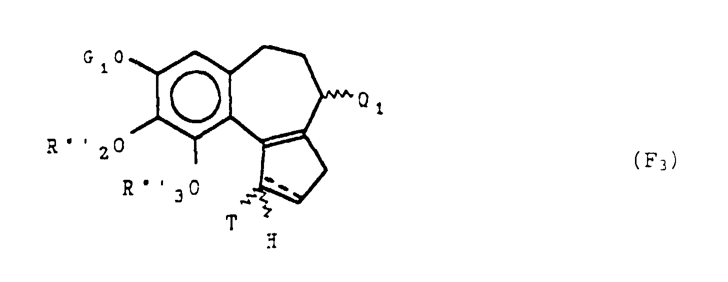 in which R"'₂ and R"'₃ have the meaning indicated in claim 13, and either G₁ represents the R"'₁ radical as defined previously and:
- either Q₁ represents an NH₂ radical of (R), (S) or (R,S) configuration and T represents a hydrogen atom;
- or Q₁ represents an N=P group of (R), (S) or (R,S) configuration, P being defined as in claim 13, and either T represents a hydrogen atom or a hydroxy or alkoxy radical containing 1 to 6 carbon atoms, of (R), (S) or (R,S) configuration, or the dotted line represents a second bond;
- or Q₁ represents an = N 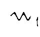 N OR₈group in which R₈ has the meaning indicated in claim 13, and the wavy line indicates that the bond can be syn or anti or that the product can consist of a mixture of syn and anti isomers and or T represents a hydrogen atom or a hydroxy or alkoxy radical containing 1 to 6 carbon atoms, of (R), (S) or (R,S) configuration, or the dotted line represents a second bond;
- or Q₁ represents an OH or OSO₂A radical, A being defined as above, of (R), (S) or (R,S) configuration and T represents a hydrogen atom,or G₁ represents a hydrogen atom and Q₁ represents either a NH₂ radical of (R), (S) or (R,S) configuration or an N=P group of (R), (S) or (R,S) configuration, P being defined as in claim 13 and T represents a hydrogen atom, the dotted line does not represent a double bond.

36. As new industrial compounds, the compounds of formula (u): 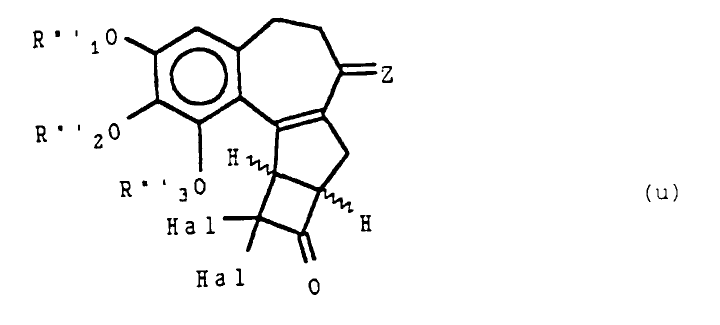 in which R"'₁, R"'₂, R"'₃, Hal and Z are as defined in claim 13.

37. As new industrial compounds, the compounds of formula (F₄): 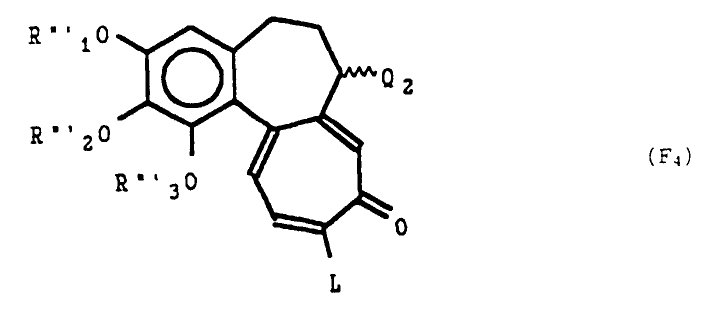 as well as their tautomers or regioisomers of formula: 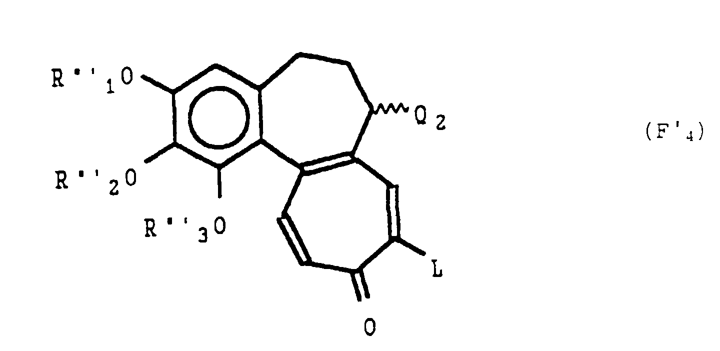 in which R"'₁, R"'₂ and R"'₃ have the meaning indicated in claim 13 and 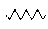 Q₂ represents:
- either an = N 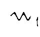 OR₈ group as defined above and L represents a hydroxy radical,
- or an 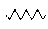 N = P group in which P has the meaning indicated in claim 13, the wavy line indicates that this group is in (R), (S) or (R,S) configuration and L represents a hydroxy or -OK radical as defined in claim 13, with the exception of the compounds in which R"'₁, R"'₂ and R"'₃ represent an alkyl radical, L represents a hydroxy or alkoxy radical and P represents a =C-phenyl or CH₃-C-OC₂H₅ group;
- or an 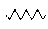 NH₂ group of (R), (S) or (R,S) configuration and L represents a hydroxy radical, with the exception of the compounds in which R"'₁, R"'₂ and R"'₃ represent an alkyl radical;
- or an 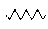 NH₂ group (R,S) configuration and L, form with the oxygen of the ketone in position 9, an 0-X₁-O⁺ group as defined in claim 13;
- or an 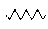 NH₂ group of (R), (S) or (R,S) configuration and L represents an OK radical as defined in claim 13, with the exception of the compounds in which R"'₁, R"'₂ and R"'₃ represent an alkyl radical and K represents an alkyl radical;
- or an = N 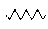 OR₈ group as defined in claim 13 and L represents an OK radical as defined in claim 13.

38. As new industrial compounds, the compounds of formula (F₅): 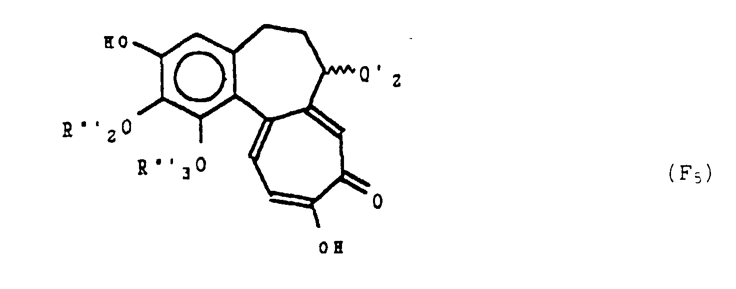 as well as their tautomers of formula: 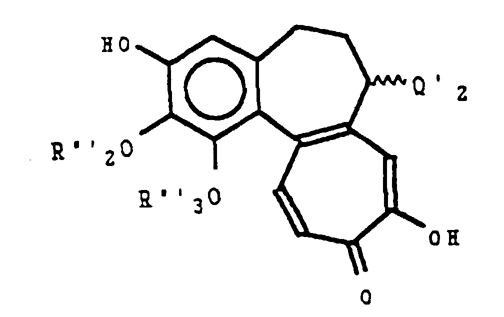 in which R"'₂ and R"'₃ have the meaning indicated in claim 13, with the exception of the alkyl value and 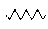 Q'₂ represents:
- either an 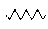 NH-CO-R₁₁ group in which R₁₁ has the meaning indicated in claim 13,
- or an 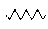 NH₂ group of (R), (S) or (R,S) configuration.

39. As new industrial compounds, the compounds of formula (F₆): 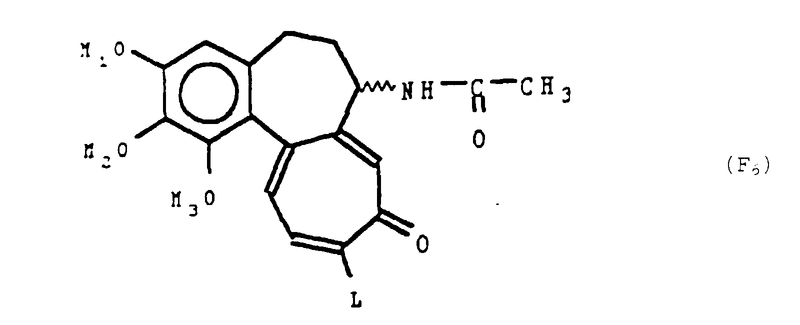 in which the wavy line indicates that the substituant is in the (R), (S) or (R,S) configuration and
- either M₁ and M₂ represent a hydroxy radical, M₃ represents an Ra radical as defined in claim 27 and L represents an OK radical, K being as defined in claim 13,
- or M₂ and M₃ represent a hydrogen atom, M₁ represents an Ra radical as defined above and L represents an OK radical as defined above,
- or M₁ represents an Ra radical as defined above, M₂ and M₃ represent a methyl radical and L represents a thiomethyl radical.
